# EUROPEAN PATENT SPECIFICATION

(11) **EP 4 514 807 B1**
(45) Date of publication and mention of the grant of the patent: **17.12.2025**
(21) Application number: 23725912.2
(22) Date of filing: 27.04.2023
(51) Int. Cl.: C07D 513/04, C07D 519/00, A61P 25/00, A61K 31/429, A61K 31/4439, A61K 31/454, A61K 31/4709, A61K 31/5377

(54) **COMPOSITIONS USEFUL FOR MODULATING SPLICING**
ZUSAMMENSETZUNGEN ZUR MODULATION DES SPLEISSENS
COMPOSITIONS UTILES POUR MODULER L'ÉPISSAGE

(30) Priority: 27.04.2022 US 202263335570 P
(43) Date of publication of application: 05.03.2025
(73) Proprietor: Skyhawk Therapeutics, Inc., Waltham, Massachusetts 02451 (US)
(72) Inventor: LUZZIO, Michael, Waltham, Massachusetts 02451 (US); LUCAS, Brian, Waltham, Massachusetts 02451 (US); DANDAPANI, Sivaraman, Waltham, MA 02451 (US); RATNI, Hasane, 68440 Habsheim (FR)
(74) Representative: Mewburn Ellis LLP
(86) International application number: PCT/US2023/020267
(87) International publication number: WO 2023/212239

(56) References cited:
- WO-A1-2016/147146
- WO-A1-2017/156429
- WO-A1-2020/245233
- WO-A1-2021/021816

## Description

### CROSS REFERENCE

This application claims the benefit of priority to U.S. Provisional Application No. 63/335,570, filed April 27, 2022.

### BACKGROUND

Spinocerebellar Ataxia 3 (SCA3 or Machado-Joseph Disease) is a rare, inherited, neurodegenerative, autosomal dominant disease. It is characterized by progressive degeneration of the brainstem, cerebellum and spinal cord, however, neurons in other areas of the brain are also affected. Presenting features include gait problems, speech difficulties, clumsiness, and often visual blurring and diplopia; saccadic eye movements become slow and ophthalmoparesis develops, resulting initially in up-gaze restriction. Ambulation becomes increasingly difficult, leading to the need for assistive devices 10 to 15 years following onset. Late in the disease course, individuals are wheelchair bound and have severe dysarthria, dysphagia, facial and temporal atrophy. The disease progresses relentlessly until death occurs at any time from 6 to approximately 30 years after onset through pulmonary complications.

SCA3 is caused by CAG tri-nucleotide repeats in exon 10 of the Ataxin 3 (ATXN3) gene. ATXN3 encodes for a deubiquitinase with wide-ranging functions, but it does not appear to be an essential gene. Disease causing variants of the ATXN3 gene have approximately 40 to over 200 CAG tri-nucleotide repeats in exon 10. Expanded CAG repeats in the ATXN3 gene are translated into expanded polyglutamine repeats (polyQ) in the ataxin-3 protein and this toxic Ataxin 3 protein is associated with aggregates. The polyglutamine expanded ataxin-3 protein in these aggregates is ubiquitinated and the aggregates contain other proteins, including heat shock proteins and transcription factors. Aggregates are frequently observed in the brain tissue of SCA3 patients. There are currently no treatments for SCA3.

### SUMMARY

The invention is set out in the appended set of claims. In addition, any reference to methods of treatment in the subsequent paragraphs of this description is to be interpreted as references to the compounds, pharmaceutical compositions and medicaments of the present invention for use in a method for treatment of the human or animal body by therapy. In one aspect, described herein is a compound of Formula (I), or a pharmaceutically acceptable salt thereof. 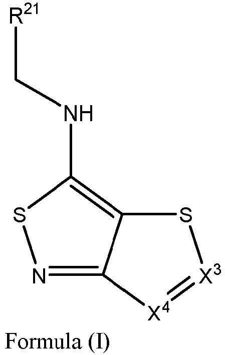 wherein X³, X⁴, and R²¹ are as defined herein.

Also provided herein are pharmaceutical compositions comprising a compound disclosed herein, or a pharmaceutically acceptable salt thereof, and a pharmaceutically acceptable excipient or carrier.

In some aspects, described herein, is a method of modulating splicing of a Ataxin3 (ATXN3) pre-mRNA, comprising contacting a small molecule splicing modulator compound disclosed herein (SMSM) to the ATXN3 pre-mRNA with a splice site sequence or cells comprising the ATXN3 pre-mRNA, wherein the SMSM binds to the ATXN3 pre-mRNA and modulates splicing of the ATXN3 pre-mRNA in a cell of a subject to produce a spliced product of the ATXN3 pre-mRNA.

In some aspects, described herein, is a method of treating, preventing, delaying of progress, or ameliorating symptoms of a disease or a condition associated with Ataxin 3 (ATXN3) expression level or activity level in a subject in need thereof, comprising administering a therapeutically effective amount of a small molecule splicing modulator compound disclosed herein (SMSM), wherein the SMSM binds to a pre-mRNA encoded by ATXN3 and modulates splicing of the ATXN3 pre-mRNA in a cell of the subject to produce a spliced product of the ATXN3 pre-mRNA, wherein the amount of full length ATXN3 is reduced.

### DETAILED DESCRIPTION

Unless otherwise defined, all technical and scientific terms used herein have the same meaning as commonly understood by one of ordinary skill in the art to which this disclosure belongs. Although methods and materials similar or equivalent to those described herein can be used in the practice or testing of the present disclosure, suitable methods, and materials are described below.

### Definitions

The term "small molecule splicing modulator" or "SMSM" denotes a small molecule compound that binds to a cell component (*e.g.*, DNA, RNA, pre-mRNA, protein, RNP, snRNA, carbohydrates, lipids, co-factors, nutrients, and/or metabolites) and modulates splicing. For example, a SMSM can bind to a polynucleotide, *e.g.,* an RNA (*e.g.,* a pre-mRNA) with an aberrant splice site, resulting in steric modulation of the polynucleotide. For example, a SMSM can bind to a protein, e.g., a spliceosome protein or a ribonuclear protein, resulting in steric modulation of the protein. For example, a SMSM can bind to a spliceosome component, e.g., a spliceosome protein or snRNA resulting in steric modulation of the spliceosome protein or snRNA. For example, a SMSM is a compound of Formula (I). The term "small molecule splicing modulator" or "SMSM" specifically excludes compounds consisting of oligonucleotides.

"Steric alteration," "steric modification," or "steric modulation" herein refers to changes in the spatial orientation of chemical moieties with respect to each other. A person of ordinary skill in the art would recognize steric mechanisms include, but are not limited to, steric hindrance, steric shielding, steric attraction, chain crossing, steric repulsions, steric inhibition of resonance, and steric inhibition of protonation.

Any open valency appearing on a carbon, oxygen, sulfur or nitrogen atom in the structures herein indicates the presence of a hydrogen, unless indicated otherwise.

The definitions described herein apply irrespective of whether the terms in question appear alone or in combination. It is contemplated that the definitions described herein can be appended to form chemically relevant combinations, such as *e.g.*, "heterocycloalkylaryl," "haloalkylheteroaryl," "arylalkylheterocycloalkyl," or "alkoxyalkyl." The last member of the combination is the radical which is binding to the rest of the molecule. The other members of the combination are attached to the binding radical in reversed order in respect of the literal sequence, *e.g.*, the combination arylalkylheterocycloalkyl refers to a heterocycloalkyl-radical which is substituted by an alkyl which is substituted by an aryl.

When indicating the number of substituents, the term "one or more" refers to the range from one substituent to the highest possible number of substitutions, *i.e.*, replacement of one hydrogen up to replacement of all hydrogens by substituents.

The term "optional" or "optionally" denotes that a subsequently described event or circumstance can but need not occur, and that the description includes instances where the event or circumstance occurs and instances in which it does not.

The term "substituent" denotes an atom or a group of atoms replacing a hydrogen atom on the parent molecule.

The term "substituted" denotes that a specified group bears one or more substituents. Where any group can carry multiple substituents and a variety of possible substituents is provided, the substituents are independently selected and need not to be the same. The term "unsubstituted" means that the specified group bears no substituents. The term "optionally substituted" means that the specified group is unsubstituted or substituted by one or more substituents, independently chosen from the group of possible substituents. When indicating the number of substituents, the term "one or more" means from one substituent to the highest possible number of substitutions, *i.e.*, replacement of one hydrogen up to replacement of all hydrogens by substituents.

The terms "compound(s) of this disclosure," "compound(s) of the present disclosure," "small molecule steric modulator," "small molecule splicing modulator," "steric modulator," "splicing modulator," "compounds that modify splicing," and "compounds modifying splicing" are interchangeably used herein and refer to compounds as disclosed herein and stereoisomers, tautomers, solvates, and salts (e.g., pharmaceutically acceptable salts) thereof.

The following abbreviations are used throughout the specification: acetic acid (AcOH); ethyl acetate (EtOAc); butyl alcohol (n-BuOH); 1,2-dichloroethane (DCE); dichloromethane (CH₂Cl₂, DCM); diisopropylethylamine (Diipea); dimethylformamide (DMF); hydrogen chloride (HCl); methanol (MeOH); methoxymethyl bromide (MOMBr); N-methyl-2-pyrrolidone (NMP); methyl Iodide (MeI); n-propanol (n-PrOH); p-methoxybenzyl (PMB); triethylamine (Et₃N); [1,1'-Bis(diphenylphosphino)ferrocene] dichloropalladium(II); (Pd(dppf)Cl₂); sodium ethane thiolate (EtSNa); sodium acetate (NaOAc); sodium hydride (NaH); sodium hydroxide (NaOH); tetrahydropyran (THP); tetrahydrofuran (THF).

As used herein, C₁-Cₓ includes C₁-C₂, C₁-C₃... C₁-Cₓ. By way of example only, a group designated as "C₁-C₄" indicates that there are one to four carbon atoms in the moiety, *i.e.* groups containing 1 carbon atom, 2 carbon atoms, 3 carbon atoms or 4 carbon atoms. Thus, by way of example only, "C₁-C₄ alkyl" indicates that there are one to four carbon atoms in the alkyl group, *i.e.,* the alkyl group is selected from among methyl, ethyl, propyl, *iso*-propyl, *n*-butyl, *iso*-butyl, *sec-*butyl, and *t*-butyl.

The term "oxo" refers to the =O substituent.

"Carboxyl" refers to -COOH.

"Cyano" refers to -CN.

The term "thioxo" refers to the =S substituent.

The term "halo," "halogen," and "halide" are used interchangeably herein and denote fluoro, chloro, bromo, or iodo.

The term "alkyl" refers to a straight or branched hydrocarbon chain radical, having from one to twenty carbon atoms, and which is attached to the rest of the molecule by a single bond. An alkyl comprising up to 10 carbon atoms is referred to as a C₁-C₁₀ alkyl, likewise, for example, an alkyl comprising up to 6 carbon atoms is a C₁-C₆ alkyl. Alkyls (and other moieties defined herein) comprising other numbers of carbon atoms are represented similarly. Alkyl groups include, but are not limited to, C₁-C₁₀ alkyl, C₁-C₉ alkyl, C₁-C₈ alkyl, C₁-C₇ alkyl, C₁-C₆ alkyl, C₁-C₅ alkyl, C₁-C₄ alkyl, C₁-C₃ alkyl, C₁-C₂ alkyl, C₂-C₈ alkyl, C₃-C₈ alkyl and C₄-C₈ alkyl. Representative alkyl groups include, but are not limited to, methyl, ethyl, *n*-propyl, 1-methylethyl (*i*-propyl), *n*-butyl, *i-*butyl, *s*-butyl, *n*-pentyl, 1,1-dimethylethyl (*t*-butyl), 3-methylhexyl, 2-methylhexyl, 1-ethyl-propyl, and the like. In some embodiments, the alkyl is methyl or ethyl. In some embodiments, the alkyl is-CH(CH₃)₂ or -C(CH₃)₃. Unless stated otherwise specifically in the specification, an alkyl group may be optionally substituted as described below. "Alkylene" or "alkylene chain" refers to a straight or branched divalent hydrocarbon chain linking the rest of the molecule to a radical group. In some embodiments, the alkylene is -CH₂-, -CH₂CH₂-, or -CH₂CH₂CH₂-. In some embodiments, the alkylene is -CH₂-. In some embodiments, the alkylene is -CH₂CH₂-. In some embodiments, the alkylene is -CH₂CH₂CH₂-.

The term "alkoxy" refers to a radical of the formula -OR where R is an alkyl radical as defined. Unless stated otherwise specifically in the specification, an alkoxy group may be optionally substituted as described below. Representative alkoxy groups include, but are not limited to, methoxy, ethoxy, propoxy, butoxy, pentoxy. In some embodiments, the alkoxy is methoxy. In some embodiments, the alkoxy is ethoxy.

The term "alkylamino" refers to a radical of the formula -NHR or -NRR where each R is, independently, an alkyl radical as defined above. Unless stated otherwise specifically in the specification, an alkylamino group may be optionally substituted as described below.

The term "alkenyl" refers to a type of alkyl group in which at least one carbon-carbon double bond is present. In one embodiment, an alkenyl group has the formula -C(R)=CR₂, wherein R refers to the remaining portions of the alkenyl group, which may be the same or different. In some embodiments, R is H or an alkyl. In some embodiments, an alkenyl is selected from ethenyl (*i.e.,* vinyl), propenyl (*i.e.,* allyl), butenyl, pentenyl, pentadienyl, and the like. Non-limiting examples of an alkenyl group include -CH=CH₂, -C(CH₃)=CH₂, -CH=CHCH₃, -C(CH₃)=CHCH₃, and-CH₂CH=CH₂.

The term "alkynyl" refers to a type of alkyl group in which at least one carbon-carbon triple bond is present. In one embodiment, an alkynyl group has the formula -C≡C-R, wherein R refers to the remaining portions of the alkynyl group. In some embodiments, R is H or an alkyl. In some embodiments, an alkynyl is selected from ethynyl, propynyl, butynyl, pentynyl, hexynyl, and the like. Non-limiting examples of an alkynyl group include -C≡CH, -C≡CCH₃ -C≡CCH₂CH₃, -CH₂C≡CH.

The term "aromatic" refers to a planar ring having a delocalized π-electron system containing 4n+2 π electrons, where n is an integer. Aromatics can be optionally substituted. The term "aromatic" includes both aryl groups (*e.g.,* phenyl, naphthalenyl) and heteroaryl groups (*e.g.,* pyridinyl, furanyl, quinolinyl).

The term "aryl" refers to a radical derived from a hydrocarbon ring system comprising at least one aromatic ring wherein each of the atoms forming the ring is a carbon atom. Aryl groups can be optionally substituted. Examples of aryl groups include, but are not limited to phenyl, and naphthyl. In some embodiments, the aryl is phenyl. Depending on the structure, an aryl group can be a monoradical or a diradical (*i.e.,* an arylene group). Unless stated otherwise specifically in the specification, the term "aryl" or the prefix "ar-"(such as in "aralkyl") is meant to include aryl radicals that are optionally substituted. In some embodiments, an aryl group is partially reduced to form a cycloalkyl group defined herein. In some embodiments, an aryl group is fully reduced to form a cycloalkyl group defined herein.

The term "haloalkyl" denotes an alkyl group wherein at least one of the hydrogen atoms of the alkyl group has been replaced by same or different halogen atoms, particularly fluoro atoms. Examples of haloalkyl include monofluoro-, difluoro-or trifluoro-methyl, -ethyl or -propyl, for example, 3,3,3-trifluoropropyl, 2-fluoroethyl, 2,2,2-trifluoroethyl, fluoromethyl, or trifluoromethyl. The term "perhaloalkyl" denotes an alkyl group where all hydrogen atoms of the alkyl group have been replaced by the same or different halogen atoms.

"Hydroxyalkyl" refers to an alkyl radical, as defined above, that is substituted by one or more hydroxyls. In some embodiments, the alkyl is substituted with one hydroxyl. In some embodiments, the alkyl is substituted with one, two, or three hydroxyls. Hydroxyalkyl include, for example, hydroxymethyl, hydroxyethyl, hydroxypropyl, hydroxybutyl, or hydroxypentyl. In some embodiments, the hydroxyalkyl is hydroxymethyl.

"Aminoalkyl" refers to an alkyl radical, as defined above, that is substituted by one or more amines. In some embodiments, the alkyl is substituted with one amine. In some embodiments, the alkyl is substituted with one, two, or three amines. Aminoalkyl include, for example, aminomethyl, aminoethyl, aminopropyl, aminobutyl, or aminopentyl. In some embodiments, the aminoalkyl is aminomethyl.

"Cyanoalkyl" refers to an alkyl radical, as defined above, that is substituted by one or more cyano groups. In some embodiments, the alkyl is substituted with one cyano group. In some embodiments, the alkyl is substituted with one, two, or three cyano groups. Aminoalkyl include, for example, cyanomethyl, cyanoethyl, cyanopropyl, cyanobutyl, or cyanopentyl.

The term "haloalkoxy" denotes an alkoxy group wherein at least one of the hydrogen atoms of the alkoxy group has been replaced by same or different halogen atoms, particularly fluoro atoms. Examples of haloalkoxyl include monofluoro-, difluoro-or trifluoro-methoxy, -ethoxy or -propoxy, for example, 3,3,3-trifluoropropoxy, 2-fluoroethoxy, 2,2,2-trifluoroethoxy, fluoromethoxy, or trifluoromethoxy. The term "perhaloalkoxy" denotes an alkoxy group where all hydrogen atoms of the alkoxy group have been replaced by the same or different halogen atoms.

The term "bicyclic ring system" denotes two rings which are fused to each other via a common single or double bond (annelated bicyclic ring system), via a sequence of three or more common atoms (bridged bicyclic ring system) or via a common single atom (spiro bicyclic ring system). Bicyclic ring systems can be saturated, partially unsaturated, unsaturated, or aromatic. Bicyclic ring systems can comprise heteroatoms selected from N, O, and S.

The terms "carbocyclic" or "carbocycle" refer to a ring or ring system where the atoms forming the backbone of the ring are all carbon atoms. The term thus distinguishes carbocyclic from "heterocyclic" rings or "heterocycles" in which the ring backbone contains at least one atom which is different from carbon. In some embodiments, at least one of the two rings of a bicyclic carbocycle is aromatic. In some embodiments, both rings of a bicyclic carbocycle are aromatic. Carbocycle includes cycloalkyl and aryl.

The term "cycloalkyl" refers to a monocyclic or polycyclic non-aromatic radical, wherein each of the atoms forming the ring (*i.e.,* skeletal atoms) is a carbon atom. In some embodiments, cycloalkyls are saturated or partially unsaturated. In some embodiments, cycloalkyls are spirocyclic or bridged compounds. In some embodiments, cycloalkyls are fused with an aromatic ring (in which case the cycloalkyl is bonded through a non-aromatic ring carbon atom). Cycloalkyl groups include groups having from 3 to 10 ring atoms. Representative cycloalkyls include, but are not limited to, cycloalkyls having from three to ten carbon atoms, from three to eight carbon atoms, from three to six carbon atoms, or from three to five carbon atoms. Monocyclic cycloalkyl radicals include, for example, cyclopropyl, cyclobutyl, cyclopentyl, cyclohexyl, cycloheptyl, and cyclooctyl. In some embodiments, the monocyclic cycloalkyl is cyclopropyl, cyclobutyl, cyclopentyl or cyclohexyl. In some embodiments, the monocyclic cycloalkyl is cyclopentenyl or cyclohexenyl. In some embodiments, the monocyclic cycloalkyl is cyclopentenyl. Polycyclic radicals include, for example, adamantyl, 1,2-dihydronaphthalenyl, 1,4-dihydronaphthalenyl, tetrainyl, decalinyl, 3,4-dihydronaphthalenyl-1(2H)-one, spiro[2.2]pentyl, norbornyl and bicyclo[1.1.1]pentyl. Unless otherwise stated specifically in the specification, a cycloalkyl group may be optionally substituted.

The term "bridged" refers to any ring structure with two or more rings that contains a bridge connecting two bridgehead atoms. The bridgehead atoms are defined as atoms that are the part of the skeletal framework of the molecule and which are bonded to three or more other skeletal atoms. In some embodiments, the bridgehead atoms are C, N, or P. In some embodiments, the bridge is a single atom or a chain of atoms that connects two bridgehead atoms. In some embodiments, the bridge is a valence bond that connects two bridgehead atoms. In some embodiments, the bridged ring system is cycloalkyl. In some embodiments, the bridged ring system is heterocycloalkyl.

The term "fused" refers to any ring structure described herein which is fused to an existing ring structure. When the fused ring is a heterocyclyl ring or a heteroaryl ring, any carbon atom on the existing ring structure which becomes part of the fused heterocyclyl ring or the fused heteroaryl ring may be replaced with one or more N, S, and O atoms. The non-limiting examples of fused heterocyclyl or heteroaryl ring structures include 6-5 fused heterocycle, 6-6 fused heterocycle, 5-6 fused heterocycle, 5-5 fused heterocycle, 7-5 fused heterocycle, and 5-7 fused heterocycle.

The term "haloalkyl" refers to an alkyl radical, as defined above, that is substituted by one or more halo radicals, as defined above, e.g., trifluoromethyl, difluoromethyl, fluoromethyl, trichloromethyl, 2,2,2-trifluoroethyl, 1,2-difluoroethyl, 3-bromo-2-fluoropropyl, 1,2-dibromoethyl, and the like. Unless stated otherwise specifically in the specification, a haloalkyl group may be optionally substituted.

The term "haloalkoxy" refers to an alkoxy radical, as defined above, that is substituted by one or more halo radicals, as defined above, e.g., trifluoromethoxy, difluoromethoxy, fluoromethoxy, trichloromethoxy, 2,2,2-trifluoroethoxy, 1,2-difluoroethoxy, 3-bromo-2-fluoropropoxy, 1,2-dibromoethoxy, and the like. Unless stated otherwise specifically in the specification, a haloalkoxy group may be optionally substituted.

The term "fluoroalkyl" refers to an alkyl in which one or more hydrogen atoms are replaced by a fluorine atom. In one aspect, a fluoroalkyl is a C₁-C₆ fluoroalkyl. In some embodiments, a fluoroalkyl is selected from trifluoromethyl, difluoromethyl, fluoromethyl, 2,2,2-trifluoroethyl, 1-fluoromethyl-2-fluoroethyl, and the like.

The term "heteroalkyl" refers to an alkyl group in which one or more skeletal atoms of the alkyl are selected from an atom other than carbon, *e.g.,* oxygen, nitrogen (*e.g.,* -NH-, -N(alkyl)-, or-N(aryl)-), sulfur (e.g., -S-, -S(=O)-, or -S(=O)₂-), or combinations thereof. In some embodiments, a heteroalkyl is attached to the rest of the molecule at a carbon atom of the heteroalkyl. In some embodiments, a heteroalkyl is attached to the rest of the molecule at a heteroatom of the heteroalkyl. In some embodiments, a heteroalkyl is a C₁-C₆ heteroalkyl. Representative heteroalkyl groups include, but are not limited to -OCH₂OMe, -OCH₂CH₂OH, -OCH₂CH₂OMe, or-OCH₂CH₂OCH₂CH₂NH₂.

"Heteroalkylene" or "heteroalkylene chain" refers to a straight or branched divalent heteroalkyl chain linking the rest of the molecule to a radical group. Unless stated otherwise specifically in the specification, the heteroalkyl or heteroalkylene group may be optionally substituted as described below. Representative heteroalkylene groups include, but are not limited to-OCH₂CH₂O-, -OCH₂CH₂OCH₂CH₂O-, or -OCH₂CH₂OCH₂CH₂OCH₂CH₂O-.

The term "heterocycloalkyl" refers to a cycloalkyl group that includes at least one heteroatom selected from nitrogen, oxygen, and sulfur. Unless stated otherwise specifically in the specification, the heterocycloalkyl radical may be a monocyclic, or bicyclic ring system, which may include fused (when fused with an aryl or a heteroaryl ring, the heterocycloalkyl is bonded through a non-aromatic ring atom) or bridged ring systems. The nitrogen, carbon, or sulfur atoms in the heterocyclyl radical may be optionally oxidized. The nitrogen atom may be optionally quaternized. The heterocycloalkyl radical is partially or fully saturated. Examples of heterocycloalkyl radicals include, but are not limited to, dioxolanyl, thienyl[1,3]dithianyl, tetrahydroquinolyl, tetrahydroisoquinolyl, decahydroquinolyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, 1,1-dioxo-thiomorpholinyl. The term heterocycloalkyl also includes all ring forms of carbohydrates, including but not limited to monosaccharides, disaccharides, and oligosaccharides. Unless otherwise noted, heterocycloalkyls have from 2 to 12 carbons in the ring. In some embodiments, heterocycloalkyls have from 2 to 10 carbons in the ring. In some embodiments, heterocycloalkyls have from 2 to 10 carbons in the ring and 1 or 2 N atoms. In some embodiments, heterocycloalkyls have from 2 to 10 carbons in the ring and 3 or 4 N atoms. In some embodiments, heterocycloalkyls have from 2 to 12 carbons, 0-2 N atoms, 0-2 O atoms, 0-2 P atoms, and 0-1 S atoms in the ring. In some embodiments, heterocycloalkyls have from 2 to 12 carbons, 1-3 N atoms, 0-1 O atoms, and 0-1 S atoms in the ring. It is understood that when referring to the number of carbon atoms in a heterocycloalkyl, the number of carbon atoms in the heterocycloalkyl is not the same as the total number of atoms (including the heteroatoms) that make up the heterocycloalkyl (*i.e.* skeletal atoms of the heterocycloalkyl ring). Unless stated otherwise specifically in the specification, a heterocycloalkyl group may be optionally substituted.

The term "heterocycle" or "heterocyclic" refers to heteroaromatic rings (also known as heteroaryls) and heterocycloalkyl rings (also known as heteroalicyclic groups) that includes at least one heteroatom selected from nitrogen, oxygen and sulfur, wherein each heterocyclic group has from 3 to 12 atoms in its ring system, and with the proviso that any ring does not contain two adjacent O or S atoms. In some embodiments, heterocycles are monocyclic, bicyclic, polycyclic, spirocyclic or bridged compounds. Non-aromatic heterocyclic groups (also known as heterocycloalkyls) include rings having 3 to 12 atoms in its ring system and aromatic heterocyclic groups include rings having 5 to 12 atoms in its ring system. The heterocyclic groups include benzo-fused ring systems. Examples of non-aromatic heterocyclic groups are pyrrolidinyl, tetrahydrofuranyl, dihydrofuranyl, tetrahydrothienyl, oxazolidinonyl, tetrahydropyranyl, dihydropyranyl, tetrahydrothiopyranyl, piperidinyl, morpholinyl, thiomorpholinyl, thioxanyl, piperazinyl, aziridinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, pyrrolin-2-yl, pyrrolin-3-yl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, ₃ h-indolyl, indolin-2-onyl, isoindolin-1-onyl, isoindoline-1,3-dionyl, 3,4-dihydroisoquinolin-1(2H)-onyl, 3,4-dihydroquinolin-2(1H)-onyl, isoindoline-1,3-dithionyl, benzo[d]oxazol-2(3H)-onyl, 1H-benzo[d]imidazol-2(3H)-onyl, benzo[d]thiazol-2(3H)-onyl, and quinolizinyl. Examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. The foregoing groups are either C-attached (or C-linked) or *N-*attached where such is possible. For instance, a group derived from pyrrole includes both pyrrol-1-yl (*N*-attached) or pyrrol-3-yl (C-attached). Further, a group derived from imidazole includes imidazol-1-yl or imidazol-3-yl (both *N*-attached) or imidazol-2-yl, imidazol-4-yl or imidazol-5-yl (all C-attached). The heterocyclic groups include benzo-fused ring systems. Non-aromatic heterocycles are optionally substituted with one or two oxo (=O) moieties, such as pyrrolidin-2-one. In some embodiments, at least one of the two rings of a bicyclic heterocycle is aromatic. In some embodiments, both rings of a bicyclic heterocycle are aromatic.

The term "heteroaryl" refers to an aryl group that includes one or more ring heteroatoms selected from nitrogen, oxygen, and sulfur. The heteroaryl is monocyclic or bicyclic. Illustrative examples of monocyclic heteroaryls include pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, pyridazinyl, triazinyl, oxadiazolyl, thiadiazolyl, furazanyl, indolizine, indole, benzofuran, benzothiophene, indazole, benzimidazole, purine, quinolizine, quinoline, isoquinoline, cinnoline, phthalazine, quinazoline, quinoxaline, 1,8-naphthyridine, and pteridine. Illustrative examples of monocyclic heteroaryls include pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, pyridazinyl, triazinyl, oxadiazolyl, thiadiazolyl, and furazanyl. Illustrative examples of bicyclic heteroaryls include indolizine, indole, benzofuran, benzothiophene, indazole, benzimidazole, purine, quinolizine, quinoline, isoquinoline, cinnoline, phthalazine, quinazoline, quinoxaline, 1,8-naphthyridine, and pteridine. In some embodiments, heteroaryl is pyridinyl, pyrazinyl, pyrimidinyl, thiazolyl, thienyl, thiadiazolyl or furyl. In some embodiments, a heteroaryl contains 0-6 N atoms in the ring. In some embodiments, a heteroaryl contains 1-4 N atoms in the ring. In some embodiments, a heteroaryl contains 4-6 N atoms in the ring. In some embodiments, a heteroaryl contains 0-4 N atoms, 0-1 O atoms, 0-1 P atoms, and 0-1 S atoms in the ring. In some embodiments, a heteroaryl contains 1-4 N atoms, 0-1 O atoms, and 0-1 S atoms in the ring. In some embodiments, heteroaryl is a C₁-C₉ heteroaryl. In some embodiments, monocyclic heteroaryl is a C₁-C₅ heteroaryl. In some embodiments, monocyclic heteroaryl is a 5-membered or 6-membered heteroaryl. In some embodiments, a bicyclic heteroaryl is a C₆-C₉ heteroaryl. In some embodiments, a heteroaryl group is partially reduced to form a heterocycloalkyl group defined herein. In some embodiments, a heteroaryl group is fully reduced to form a heterocycloalkyl group defined herein.

The term "moiety" refers to a specific segment or functional group of a molecule. Chemical moieties are often recognized chemical entities embedded in or appended to a molecule.

The term "optionally substituted" or "substituted" means that the referenced group is optionally substituted with one or more additional group(s) individually and independently selected from D, halogen, -CN, -NH₂, -NH(alkyl), -N(alkyl)₂, -OH, -CO₂H, -CO₂alkyl, -C(=O)NH₂,-C(=O)NH(alkyl), -C(=O)N(alkyl)₂, -S(=O)₂NH₂, -S(=O)₂NH(alkyl), -S(=O)₂N(alkyl)₂, alkyl, cycloalkyl, fluoroalkyl, heteroalkyl, alkoxy, fluoroalkoxy, heterocycloalkyl, aryl, heteroaryl, aryloxy, alkylthio, arylthio, alkylsulfoxide, arylsulfoxide, alkylsulfone, and arylsulfone. In some other embodiments, optional substituents are independently selected from D, halogen, -CN, -NH₂,-NH(CH₃), -N(CH₃)₂, -OH, -CO₂H, -CO₂(C₁-C₄ alkyl), -C(=O)NH₂, -C(=O)NH(C₁-C₄ alkyl),-C(=O)N(C₁-C₄ alkyl)₂, -S(=O)₂NH₂, -S(=O)₂NH(C₁-C₄ alkyl), -S(=O)₂N(C₁-C₄ alkyl)₂, C₁-C₄ alkyl, C₃-C₆ cycloalkyl, C₁-C₄ fluoroalkyl, C₁-C₄ heteroalkyl, C₁-C₄ alkoxy, C₁-C₄ fluoroalkoxy, -SC₁-C₄ alkyl, -S(=O)C₁-C₄ alkyl, and -S(=O)₂(C₁-C₄ alkyl). In some embodiments, optional substituents are independently selected from D, halogen, -CN, -NH₂, -OH, -NH(CH₃), -N(CH₃)₂,-NH(cyclopropyl), -CH₃, -CH₂CH₃, -CF₃, -OCH₃, and -OCF₃. In some embodiments, substituted groups are substituted with one or two of the preceding groups. In some embodiments, an optional substituent on an aliphatic carbon atom (acyclic or cyclic) includes oxo (=O).

The term "tautomer" refers to a proton shift from one atom of a molecule to another atom of the same molecule. The compounds presented herein may exist as tautomers. Tautomers are compounds that are interconvertible by migration of a hydrogen atom, accompanied by a switch of a single bond and adjacent double bond. In bonding arrangements where tautomerization is possible, a chemical equilibrium of the tautomers will exist. All tautomeric forms of the compounds disclosed herein are contemplated. The exact ratio of the tautomers depends on several factors, including temperature, solvent, and pH. Some examples of tautomeric interconversions include:

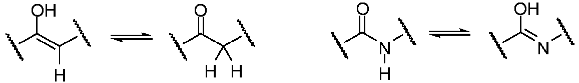

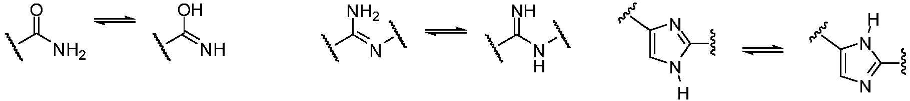

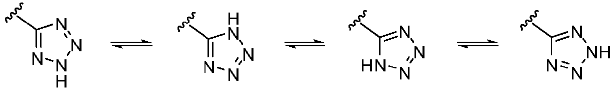

The terms "administer," "administering," "administration," and the like, as used herein, refer to the methods that may be used to enable delivery of compounds or compositions to the desired site of biological action. These methods include but are not limited to oral routes (p.o.), intraduodenal routes (i.d.), parenteral injection (including intravenous (i.v.), subcutaneous (s.c.), intraperitoneal (i.p.), intramuscular (i.m.), intravascular or infusion (inf.)), topical (top.) and rectal (p.r.) administration. Those of skill in the art are familiar with administration techniques that can be employed with the compounds and methods described herein. In some embodiments, the compounds and compositions described herein are administered orally.

The terms "co-administration" or the like, as used herein, are meant to encompass administration of the selected therapeutic agents to a single patient and are intended to include treatment regimens in which the agents are administered by the same or different route of administration or at the same or different time.

The term "subject" or "patient" encompasses mammals. Examples of mammals include, but are not limited to, any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. In one aspect, the mammal is a human. The term "animal" as used herein comprises human beings and non-human animals. In one embodiment, a "non-human animal" is a mammal, for example a rodent such as rat or a mouse. In one embodiment, a non-human animal is a mouse.

The term "pharmaceutically acceptable" denotes an attribute of a material which is useful in preparing a pharmaceutical composition that is generally safe, non toxic, and neither biologically nor otherwise undesirable and is acceptable for veterinary as well as human pharmaceutical use. "Pharmaceutically acceptable" can refer a material, such as a carrier or diluent, which does not abrogate the biological activity or properties of the compound, and is relatively nontoxic, *i.e.*, the material may be administered to an individual without causing undesirable biological effects or interacting in a deleterious manner with any of the components of the composition in which it is contained.

The terms "pharmaceutically acceptable excipient", "pharmaceutically acceptable carrier" and "therapeutically inert excipient" can be used interchangeably and denote any pharmaceutically acceptable ingredient in a pharmaceutical composition having no therapeutic activity and being nontoxic to the subject administered, such as disintegrators, binders, fillers, solvents, buffers, tonicity agents, stabilizers, antioxidants, surfactants, carriers, diluents, excipients, preservatives or lubricants used in formulating pharmaceutical products.

The term "pharmaceutically acceptable salts" denotes salts which are not biologically or otherwise undesirable. Pharmaceutically acceptable salts include both acid and base addition salts. A "pharmaceutically acceptable salt" can refer to a formulation of a compound that does not cause significant irritation to an organism to which it is administered and/or does not abrogate the biological activity and properties of the compound. In some embodiments, pharmaceutically acceptable salts are obtained by reacting a SMSM compound of the present disclosure with acids. Pharmaceutically acceptable salts are also obtained by reacting a compound of the present disclosure with a base to form a salt.

As used herein, a "small molecular weight compound" can be used interchangeably with "small molecule" or "small organic molecule." Small molecules refer to compounds other than peptides or oligonucleotides; and typically have molecular weights of less than about 2000 Daltons, *e.g.,* less than about 900 Daltons.

A ribonucleoprotein (RNP) refers to a nucleoprotein that contains RNA. An RNP can be a complex of a ribonucleic acid and an RNA-binding protein. Such a combination can also be referred to as a protein-RNA complex. These complexes can function in a number of biological functions that include, but are not limited to, DNA replication, gene expression, metabolism of RNA, and pre-mRNA splicing. Examples of RNPs include the ribosome, the enzyme telomerase, vault ribonucleoproteins, RNase P, heterogeneous nuclear RNPs (hnRNPs) and small nuclear RNPs (snRNPs).

Nascent RNA transcripts from protein-coding genes and mRNA processing intermediates, collectively referred to as pre-mRNA, are generally bound by proteins in the nuclei of eukaryotic cells. From the time nascent transcripts first emerge from RNA polymerase (e.g., RNA polymerase II) until mature mRNAs are transported into the cytoplasm, the RNA molecules are associated with an abundant set of splicing complex components (e.g., nuclear proteins and snRNAs). These proteins can be components of hnRNPs, which can contain heterogeneous nuclear RNA (hnRNA) (e.g., pre-mRNA and nuclear RNA complexes) of various sizes.

Splicing complex components function in splicing and/or splicing regulation. Splicing complex components can include, but are not limited to, ribonuclear proteins (RNPs), splicing proteins, small nuclear RNAs (snRNAs), small nuclear ribonucleoproteins (snRNPs), and heterogeneous nuclear ribonucleoproteins (hnRNPs). Splicing complex components include, but are not limited to, those that may be required for splicing, such as constitutive splicing, alternative splicing, regulated splicing, and splicing of specific messages or groups of messages. A group of related proteins, the serine/arginine-rich (SR) proteins, can function in constitutive pre-mRNA splicing and may also regulate alternative splice-site selection in a concentration-dependent manner. SR proteins typically have a modular structure that consists of one or two RNA-recognition motifs (RRMs) and a C-terminal rich in arginine and serine residues (RS domain). Their activity in alternative splicing may be antagonized by members of the hnRNP A/B family of proteins. Splicing complex components can also include proteins that are associated with one or more snRNAs. snRNAs in human include, but are not limited to, U1 snRNA, U2 snRNA, U4 snRNA, U5 snRNA, U6 snRNA, U11 snRNA, U12 snRNA, U4atac snRNA, U5 snRNA, and U6atac snRNA. SR proteins in human include, but are not limited to, SC35, SRp55, SRp40, SRm300, SFRS10, TASR-1, TASR-2, SF2/ASF, 9G8, SRp75, SRp30c, SRp20, and P54/SFRS11. Other splicing complex components in human that can be involved in splice site selection include, but are not limited to, U2 snRNA auxiliary factors (e.g. U2AF65, U2AF35), Urp/U2AF1-RS2, SF1/BBP, CBP80, CBP 20, SF1 and PTB/hnRNP1. hnRNP proteins in humans include, but are not limited to, A1, A2/B1, L, M, K, U, F, H, G, R, I and C1/C2. Human genes encoding hnRNPs include *HNRNPA0, HNRNPA1, HNRNPA1L1, HNRNPA1L2, HNRNPA3, HNRNPA2B1, HNRNPAB, HNRNPB1, HNRNPC, HNRNPCL1, HNRNPD, HNRPDL, HNRNPF, HNRNPH1, HNRNPH2, HNRNPH3, HNRNPK, HNRNPL, HNRPLL, HNRNPM, HNRNPR, HNRNP U, HNRNP UL1, HNRNPUL2, HNRNPUL3,* and *FMR1.*

In some embodiments, the splicing complex component comprises a nucleic acid, a protein, a carbohydrate, a lipid, a co-factor, a nutrient, a metabolite, or an auxiliary splicing factor. In some embodiments, the splicing complex component comprises an auxiliary splicing factor such as a ribonucleoprotein (RNP) which can be a heterogeneous nuclear ribonucleoprotein (hnRNP) or a small nuclear ribonucleoprotein (snRNP). In some embodiments, the auxiliary splicing factor includes, but are not limited to, 9G8, A1 hnRNP, A2 hnRNP, ASD-1, ASD-2b, ASF, B1 hnRNP, C1 hnRNP, C2 hnRNP, CBP20, CBP80, CELF, F hnRNP, FBP11, Fox-1, Fox-2, G hnRNP, H hnRNP, hnRNP C, hnRNP G, hnRNP K, hnRNP M, hnRNP U, Hu, HUR, K hnRNP, KH-type splicing regulatory protein (KSRP), L hnRNP, M hnRNP, mBBP, muscle-blind like (MBNL), NF45, NFAR, Nova-1, Nova-2, P54/SFRS11, polypyrimidine tract binding protein (PTBP) 1, PTBP2, PRP19 complex proteins, R hnRNP, RNPC1, SAM68, SC35, SF, SF1/BBP, SF2, SF3 a, SF3B, SFRS10, Sm proteins, SR proteins, SRm300, SRp20, SRp30c, SRP35C, SRP36, SRP38, SRp40, SRp55, SRp75, SRSF, STAR, GSG, SUP-12, TASR-1, TASR-2, TIA, TIAR, TRA2, TRA2a/b, U hnRNP, U1 snRNP, U11 snRNP, U12 snRNP, U1-70K, U1-A, U1-C, U2 snRNP, U2AF1-RS2, U2AF35, U2AF65, U4 snRNP, U5 snRNP, U6 snRNP, Urp, and YB1.

Splicing complex components may be stably or transiently associated with a snRNP or with a transcript (*e.g.*, pre-mRNA). In some embodiments, the pre-mRNA binds to a splicing complex or a component thereof.

The term "intron" refers to both the DNA sequence within a gene and the corresponding sequence in the unprocessed RNA transcript. As part of the RNA processing pathway, introns can be removed by RNA splicing either shortly after or concurrent with transcription. Introns are found in the genes of most organisms and many viruses. They can be located in a wide range of genes, including those that generate proteins, ribosomal RNA (rRNA), and transfer RNA (tRNA).

An "exon" can be any part of a gene that encodes a part of the final mature RNA produced by that gene after introns have been removed by RNA splicing. The term "exon" refers to both the DNA sequence within a gene and to the corresponding sequence in RNA transcripts.

A "spliceosome" can be assembled from snRNAs and protein complexes. The spliceosome can remove introns from a transcribed pre-mRNA.

The term "cryptic exon" can refer to an intronic sequence that may be flanked by apparent consensus splice sites (*e.g.*, cryptic splice site) but are generally not spliced into the mature mRNA or the product of splicing. The term "poison exon" can refer to a cryptic exon that contains a premature termination codon in the reading frame of the exon when included in an RNA transcript. "Poison exon" can also refer to a cryptic exon inclusion of which in an RNA transcript causes a reading frame shift in downstream exons resulting in a premature stop codon, which was not in frame prior to the frame shift caused by inclusion of the cryptic exon. In some embodiments, the poison exon is a variant of an existing exon. In some embodiments, the poison exon is an extended form of an existing exon. In some embodiments, the poison exon is a truncated form of an existing exon. The terms "poison exon" and "toxic exon" are used interchangeably in the present disclosure. The terms "stop codon" and "termination codon" are used interchangeably in the present disclosure.

A splicing event that promotes inclusion of a poison exon can further promote inclusion of an intron immediately following the poison exon in an RNA transcript. Inclusion of the poison exon and the intron immediately following the poison exon can result in "nuclear retention" of the RNA transcript, *e.g.*, mRNA, wherein the RNA transcript is retained in the nucleus and not transported or exported to the cytoplasm and thus, not translated into a protein.

### Small Molecule Splicing Modulators (SMSMs)

It has now been found that compounds of this disclosure, and pharmaceutically acceptable compositions thereof, are effective as agents for use in treating, preventing, or ameliorating a disease or a condition associated with a target RNA. The present disclosure provides the unexpected discovery that certain small chemical molecules can modify splicing events in pre-mRNA molecules, herein referred to as small molecule splicing modulators (SMSMs). These SMSMs can modulate specific splicing events in specific pre-mRNA molecules. These SMSMs can operate by a variety of mechanisms to modify splicing events. For example, the SMSMs of this disclosure can: 1) interfere with the formation and/or function and/or other properties of splicing complexes, spliceosomes, and/or their components such as hnRNPs, snRNPs, SR-proteins and other splicing factors or elements, resulting in the prevention or induction of a splicing event in a pre-mRNA molecule. As another example, the SMSMs of this disclosure can: 2) prevent and/or modify post-transcriptional regulation (*e.g.*, splicing) of gene products, such as hnRNPs, snRNPs, SR-proteins and other splicing factors, which can subsequently be involved in the formation and/or function of a spliceosome or splicing complex component; 3) prevent and/or modify phosphorylation, glycosylation and/or other modifications of gene products including, but not limited to, hnRNPs, snRNPs, SR-proteins and other splicing factors, which can subsequently be involved in the formation and/or function of a spliceosome or splicing complex component; or 4) bind to and/or otherwise affect specific pre-mRNA so that a specific splicing event is prevented or induced, *e.g.*, via a mechanism that does not involve base-pairing with RNA in a sequence-specific manner. The small molecules of this disclosure are different from and are not related to antisense or antigene oligonucleotides.

Described herein are compounds modifying splicing of gene products for use in the treatment, prevention, and/or delay of progression of diseases or conditions. Described herein are compounds modifying splicing of gene products wherein the compounds induce a transcriptionally inactive variant or transcript of a gene product. Described herein are compounds modifying splicing of gene products wherein the compounds induce a transcriptionally active variant or transcript of a gene product. Described herein are compounds modifying splicing of gene products wherein the compounds repress a transcriptionally active variant or transcript of a gene product. Described herein are compounds modifying splicing of gene products wherein the compounds repress a transcriptionally inactive variant or transcript of a gene product.

Described herein are compounds modifying splicing of gene products wherein the compounds induce a specific variant or isoform of a mature mRNA. Described herein are compounds modifying splicing of gene products wherein the compounds cause increased expression of a protein encoded by a variant or an isoform of an mRNA derived from a pre-mRNA that the compounds bind. Described herein are compounds modifying splicing of gene products wherein the compounds cause increased expression of a variant or an isoform of an mRNA derived from a pre-mRNA that the compounds bind, leading to increased expression of the protein encoded by the variant or the isoform of the mRNA. Described herein are compounds modifying splicing of gene products wherein the compounds cause increased expression of a variant or an isoform of an mRNA containing a specific exon by inducing exon inclusion in a pre-mRNA that compounds bind, leading to increased expression of the protein encoded by the variant or the isoform of the mRNA. Described herein are compounds modifying splicing of gene products wherein the compounds cause exon inclusion in a pre-mRNA that compounds bind, leading to increased expression of the protein encoded by the specific variant or the isoform of the mRNA.

Described herein are compounds modifying splicing of gene products wherein the compounds repress a specific variant or isoform of a mature mRNA. Described herein are compounds modifying splicing of gene products wherein the compounds cause decreased expression of a protein encoded by a variant or an isoform of an mRNA derived from a pre-mRNA that the compounds bind. Described herein are compounds modifying splicing of gene products wherein the compounds cause decreased expression of a variant or an isoform of an mRNA derived from a pre-mRNA that the compounds bind, leading to decreased expression of the protein encoded by the variant or the isoform of the mRNA. Described herein are compounds modifying splicing of gene products wherein the compounds cause decreased expression of a variant or an isoform of an mRNA containing a specific exon by inducing exon inclusion in a pre-mRNA that compounds bind, leading to decreased expression of the protein encoded by the variant or the isoform of the mRNA. Described herein are compounds modifying splicing of gene products wherein the compounds cause exon inclusion in a pre-mRNA that compounds bind, leading to decreased expression of the protein encoded by the specific variant or the isoform of the mRNA.

Described herein are compounds modifying splicing of gene products wherein the compounds induce a post-transcriptionally inactive variant or transcript of a gene product. Described herein are compounds modifying splicing of gene products wherein the compounds repress a post-transcriptionally active variant or transcript of a gene product. Described herein are compounds modifying splicing of gene products wherein the compounds induce a post-transcriptionally destabilized variant or transcript of a gene product. Described herein are compounds modifying splicing of gene products wherein the compounds cause less expression of a protein encoded by an mRNA derived from a pre-mRNA that the compounds bind. Described herein are compounds modifying splicing of gene products wherein the compounds cause less expression of an mRNA derived from a pre-mRNA that the compounds bind, leading to decreased expression of the protein encoded by the mRNA. Described herein are compounds modifying splicing of gene products wherein the compounds cause increased expression of an mRNA containing a poison exon derived from a pre-mRNA that compounds bind, leading to decreased expression of the protein encoded by the mRNA. Described herein are compounds modifying splicing of gene products wherein the compounds cause nonsense-mediated decay (NMD) of an mRNA derived from a pre-mRNA that compounds bind, leading to decreased expression of the protein encoded by the mRNA.

In one aspect, a SMSM described herein is a compound of Formula (IB), or a pharmaceutically acceptable salt thereof: 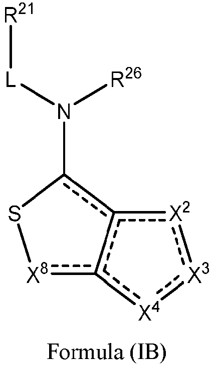 wherein: 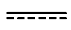
represents a single or a double bond;
X² is S;
X³ is selected from the group consisting of N and CR²³;
X⁴ is selected from the group consisting of N and CR²⁴;
X⁸ is N;
L is -CH₂-;
R²⁶ is hydrogen;
R²¹ is selected from the group consisting of phenyl, 5-6 membered heteroaryl, and 5-6 membered heterocycloalkyl, each of which is unsubstituted or substituted with 1, 2, 3 or 4, independently selected R^{1A} groups; each R^{1A} is independently selected from halo, CN, NO₂, C₁₋₆ alkyl, C₂₋₆ alkenyl, C₂₋₆ alkynyl, C₁₋₆ haloalkyl, C₁₋₆ alkoxy, -C(=O)OH, -C(=O)C₁₋₆ alkyl, -C(=O)C₁₋₆ haloalkyl, and -C(=O)C₁₋₆ alkoxy;
R²³ is selected from the group consisting of hydrogen, azido, halogen, -CN, -NO₂, C₁₋₆ haloalkyl, C₁₋₆ alkyl, C₂₋₆ alkenyl, C₂₋₆ alkynyl, C₁₋₆ heteroalkyl, -(C₁₋₆ alkylene)-C₃₋₁₀ cycloalkyl, -(C₁₋₆ alkylene)-4-10 membered heterocycloalkyl, -(C₁₋₆ heteroalkylene)-C₃₋₁₀ cycloalkyl, -(C₁₋₆ heteroalkylene)-4-10 membered heterocycloalkyl, -(C₁₋₆ alkylene)-C₆₋₁₀ aryl, -(C₁₋₆ alkylene)-5-10 membered heteroaryl, -(C₁₋₆ heteroalkylene)-C₆₋₁₀ aryl, -(C₁₋₆ heteroalkylene)-5-10 membered heteroaryl, C₃₋₁₀ cycloalkyl, C₆₋₁₀ aryl, 5-10 membered heteroaryl, 4- 10 membered heterocycloalkyl, -OR^{a3},-SR^{a3}, -C(=O)R^{b3}, -C(=O)OR^{b3}, -NR^{c3}R^{d3}, -C(=O)NR^{c3}R^{d3}, -OC(=O)NR^{c3}R^{d3}, -NR^{c3}C(=O)R^{b3},-NR^{c3}C(=O)OR^{b3}, -NR^{c3}C(=O)NR^{c3}R^{d3}, -NR^{c3}S(-O)₂R^{b3}, -NR^{c3}S(=O)₂NR^{c3}R^{d3}, -S(O)NR^{c3}R^{d3}, and -S(O)₂NR^{c3}R^{d3}, wherein the C₁₋₆ alkyl, C₁₋₆ haloalkyl, C₁₋₆ alkylene, C₁₋₆ heteroalkylene, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
each R²⁰ is independently selected from the group consisting of -OH, -SH, -CN, -NO₂, halogen, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino;
R²⁴ is selected from the group consisting of hydrogen, azido, halogen, -CN, -NO₂, C₁₋₆ haloalkyl, C₁₋₆ alkyl, C₂₋₆ alkenyl, C₁₋₆ heteroalkyl, C₃₋₁₀ cycloalkyl, C₆₋₁₀ aryl, -OR^{a4}, -SR^{a4}, -S(=O)₂R^{a4},-C(=O)R^{b4}, -C(=O)OR^{b4}, -NR^{c4}R^{d4}, -C(=O)NR^{c4}R^{d4}, -OC(=O)NR^{c4}R^{d4}, -NR^{c4}C(=O)R^{b4},-NR^{c4}C(=O)OR^{b4}, -NR^{c4}C(=O)NR^{c4}R^{d4}, -NR^{c4}S(-O)₂R^{b4}, -NR^{c4}S(=O)₂NR^{c4}R^{d4}, -S(O)NR^{c4}R^{d4}, and -S(O)₂NR^{c4}R^{d4}, wherein the C₁₋₆ alkyl, C₁₋₆ haloalkyl, C₂₋₆ alkenyl, C₁₋₆ heteroalkyl, C₃₋₁₀ cycloalkyl, and C₆₋₁₀ aryl are unsubstituted or substituted independently with 1, 2, 3, or 4 R^{20d} groups;
each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino; and
each R^{a3}, R^{b3}, R^{c3}, R^{d3}, R^{a4}, R^{b4}, R^{c4}, and R^{d4} is independently selected from the group consisting of hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy,-(C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl, wherein the C₁₋₆ alkyl, C₂₋₆ alkenyl, C₂₋₆ alkynyl, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
or R^{c3} and R^{d3} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups;
or R^{c4} and R^{d4} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups.

In one aspect, a SMSM described herein is a compound of Formula (IB), or a pharmaceutically acceptable salt thereof: 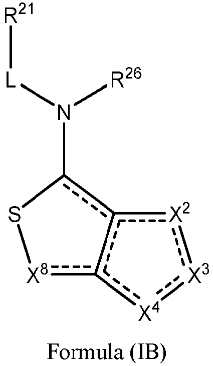 wherein:
-̅-̅-̅-̅-̅-̅ represents a single or a double bond;
X² is S;
X³ is selected from the group consisting of N and CR²³;
X⁴ is selected from the group consisting of N and CR²⁴;
X⁸ is N;
L is -CH₂-;
R²⁶ is hydrogen;
R²¹ is selected from the group consisting of C₆₋₁₀ aryl, 5-10 membered heteroaryl, and 5-10 membered heterocycloalkyl, wherein the C₆₋₁₀ aryl, 5-10 membered heteroaryl, and 5-10 membered heterocycloalkyl are unsubstituted or substituted independently with 1, 2, 3, or 4 R^{1A} groups; wherein each R^{1A} is independently selected from the group consisting of halogen, -CN, amino,-NO₂, C₁₋₆ alkyl, C₂₋₆ alkenyl, C₂₋₆ alkynyl, C₁₋₆ haloalkyl, C₁₋₆ heteroalkyl, C₁₋₆ alkoxy,-C(=O)OH, -C(=O)C₁₋₆ alkyl, -C(=O)C₁₋₆ haloalkyl, and -C(=O)C₁₋₆ alkoxy;
R²³ is selected from the group consisting of hydrogen, azido, halogen, -CN, -NO₂, C₁₋₆ haloalkyl, C₁₋₆ alkyl, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, 4- 10 membered heterocycloalkyl, -OR^{a3}, -SR^{a3}, -C(=O)R^{b3}, -C(=O)OR^{b3}, -NR^{c3}R^{d3},-C(=O)NR^{c3}R^{d3}, -OC(=O)NR^{c3}R^{d3}, -NR^{c3}C(=O)R^{b3}, -NR^{c3}C(=O)OR^{b3}, -NR^{c3}C(=O)NR^{c3}R^{d3},-NR^{c3}S(=O)₂R^{b3}, -NR^{c3}S(=O)₂NR^{c3}R^{d3}, -S(O)NR^{c3}R^{d3}, and -S(O)₂NR^{c3}R^{d3}, wherein the C₁₋₆ alkyl, C₁₋₆ haloalkyl, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
each R²⁰ is independently selected from the group consisting of -OH, -SH, -CN, -NO₂, halogen, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 5-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino;
R²⁴ is selected from the group consisting of hydrogen, azido, halogen, -CN, -NO₂, C₁₋₆ haloalkyl, C₁₋₆ alkyl, C₂₋₆ alkenyl, C₁₋₆ heteroalkyl, C₃₋₁₀ cycloalkyl, C₆₋₁₀ aryl, -OR^{a4}, -C(=O)R^{b4}, -C(=O)OR^{b4},-NR^{c4}R^{d4}, -C(=O)NR^{c4}R^{d4}, - OC(=O)NR^{c4}R^{d4}, -NR^{c4}C(=O)R^{b4}, -NR^{c4}C(=O)OR^{b4},-NR^{c4}C(=O)NR^{c4}R^{d4}, -NR^{c4}S(=O)₂R^{b4}, -NR^{c4}S(=O)₂NR^{c4}R^{d4}, -S(O)NR^{c4}R^{d4}, and -S(O)₂NR^{c4}R^{d4}, wherein the C₁₋₆ alkyl, C₁₋₆ haloalkyl, C₂₋₆ alkenyl, C₁₋₆ heteroalkyl,C₃₋₁₀ cycloalkyl, and C₆₋₁₀ aryl are unsubstituted or substituted independently with 1, 2, 3, or 4 R^{20d} groups;
each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino; and
each R^{a3}, R^{b3}, R^{c3}, R^{d3}, R^{a4}, R^{b4}, R^{c4}, and R^{d4} is independently selected from the group consisting of hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy,-(C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl, wherein the C₁₋₆ alkyl, C₂₋₆ alkenyl, C₂₋₆ alkynyl, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
or R^{c3} and R^{d3} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups;
or R^{c4} and R^{d4} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups.

In one aspect, a SMSM described herein is a compound of Formula (I), or a pharmaceutically acceptable salt thereof: 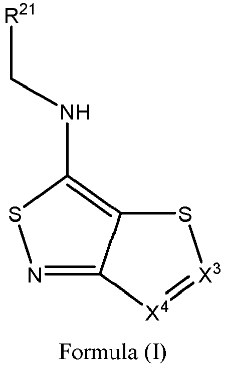 wherein:
X³ is selected from the group consisting of N and CR²³;
X⁴ is selected from the group consisting of N and CR²⁴;

R²¹ is selected from the group consisting of phenyl, 5-6 membered heteroaryl, and 5-6 membered heterocycloalkyl, each of which is unsubstituted or substituted with 1, 2, 3 or 4, independently selected R^{1A} groups; each R^{1A} is independently selected from halo, CN, NO₂, C₁₋₆ alkyl, C₂₋₆ alkenyl, C₂₋₆ alkynyl, C₁₋₆ haloalkyl, C₁₋₆ alkoxy, -C(=O)OH, -C(=O)C₁₋₆ alkyl, -C(=O)C₁₋₆ haloalkyl, and -C(=O)C₁₋₆ alkoxy;
R²³ is selected from the group consisting of hydrogen, azido, halogen, -CN, -NO₂, C₁₋₆ haloalkyl, C₁₋₆ alkyl, C₂₋₆ alkenyl, C₂₋₆ alkynyl, C₁₋₆ heteroalkyl, -(C₁₋₆ alkylene)-C₃₋₁₀ cycloalkyl, -(C₁₋₆ alkylene)-4-10 membered heterocycloalkyl, -(C₁₋₆ heteroalkylene)-C₃₋₁₀ cycloalkyl, -(C₁₋₆ heteroalkylene)-4-10 membered heterocycloalkyl, -(C₁₋₆ alkylene)-C₆₋₁₀ aryl, -(C₁₋₆ alkylene)-5-10 membered heteroaryl, -(C₁₋₆ heteroalkylene)-C₆₋₁₀ aryl, -(C₁₋₆ heteroalkylene)-5-10 membered heteroaryl, C₃₋₁₀ cycloalkyl, C₆₋₁₀ aryl, 5-10 membered heteroaryl, 4- 10 membered heterocycloalkyl, -OR^{a3},-SR^{a3}, -C(=O)R^{b3}, -C(=O)OR^{b3}, -NR^{c3}R^{d3}, -C(=O)NR^{c3}R^{d3}, -OC(=O)NR^{c3}R^{d3}, -NR^{c3}C(=O)R^{b3},-NR^{c3}C(=O)OR^{b3}, -NR^{c3}C(=O)NR^{c3}R^{d3}, -NR^{c3}S(-O)₂R^{b3}, -NR^{c3}S(=O)₂NR^{c3}R^{d3}, -S(O)NR^{c3}R^{d3}, and -S(O)₂NR^{c3}R^{d3}, wherein the C₁₋₆ alkyl, C₁₋₆ haloalkyl, C₁₋₆ alkylene, C₁₋₆ heteroalkylene, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
each R²⁰ is independently selected from the group consisting of -OH, -SH, -CN, -NO₂, halogen, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino;
R²⁴ is selected from the group consisting of hydrogen, azido, halogen, -CN, -NO₂, C₁₋₆ haloalkyl, C₁₋₆ alkyl, C₂₋₆ alkenyl, C₁₋₆ heteroalkyl, C₃₋₁₀ cycloalkyl, C₆₋₁₀ aryl, -OR^{a4}, -SR^{a4}, -S(=O)₂R^{a4},-C(=O)R^{b4}, -C(=O)OR^{b4}, -NR^{c4}R^{d4}, -C(=O)NR^{c4}R^{d4}, - OC(=O)NR^{c4}R^{d4}, -NR^{c4}C(=O)R^{b4},-NR^{c4}C(=O)OR^{b4}, -NR^{c4}C(=O)NR^{c4}R^{d4}, -NR^{c4}S(=O)₂R^{b4}, -NR^{c4}S(=O)₂NR^{c4}R^{d4}, -S(O)NR^{c4}R^{d4}, and -S(O)₂NR^{c4}R^{d4}, wherein the C₁₋₆ alkyl, C₁₋₆ haloalkyl, C₂₋₆ alkenyl, C₁₋₆ heteroalkyl,C₃₋₁₀ cycloalkyl, and C₆₋₁₀ aryl are unsubstituted or substituted independently with 1, 2, 3, or 4 R^{20d} groups;
each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄alkyl)aminocarbonylamino; and
each R^{a3}, R^{b3}, R^{c3}, R^{d3}, R^{a4}, R^{b4}, R^{c4}, and R^{d4} is independently selected from the group consisting of hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy,-(C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl, wherein the C₁₋₆ alkyl, C₂₋₆ alkenyl, C₂₋₆ alkynyl, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
or R^{c3} and R^{d3} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups;
or R^{c4} and R^{d4} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups.

In some embodiments of a compound of Formula (I) or (IB), or a pharmaceutically acceptable salt thereof, R²³ is selected from the group consisting of hydrogen, azido, halogen, -CN,-NO₂, C₁₋₆ haloalkyl, C₁₋₆ alkyl, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, 4- 10 membered heterocycloalkyl, -OR^{a3}, -SR^{a3}, -C(=O)R^{b3}, -C(=O)OR^{b3}, -NR^{c3}R^{d3}, -C(=O)NR^{c3}R^{d3}, -OC(=O)NR^{c3}R^{d3}, -NR^{c3}C(=O)R^{b3}, -NR^{c3}C(=O)OR^{b3},-NR^{c3}C(=O)NR^{c3}R^{d3}, -NR^{c3}S(-O)₂R^{b3}, -NR^{c3}S(=O)₂NR^{c3}R^{d3}, -S(O)NR^{c3}R^{d3}, and -S(O)₂NR^{c3}R^{d3}, wherein the C₁₋₆ alkyl, C₁₋₆ haloalkyl, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups.

In some embodiments of a compound of Formula (I) or (IB), or a pharmaceutically acceptable salt thereof, R²³ is unsubstituted or substituted independently with 1, 2, 3, or 4 R²⁰ groups, and wherein each R²⁰ is independently selected from the group consisting of -OH, -SH, -CN, -NO₂, halogen, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino.

In some embodiments of a compound of Formula (I) or (IB), or a pharmaceutically acceptable salt thereof,
X³ is selected from the group consisting of N and CR²³;
X⁴ is selected from the group consisting of N and CR²⁴;

R²¹ is selected from the group consisting of 5 membered heteroaryl, and 5 membered heterocycloalkyl, each of which is unsubstituted or substituted with 1, 2, or 3, independently selected R^{1A} groups; each R^{1A} is independently selected from halo, CN, NO₂, C₁₋₆ alkyl, C₂₋₆ alkenyl, C₂₋₆ alkynyl, C₁₋₆ haloalkyl, C₁₋₆ alkoxy, -C(=O)OH, -C(=O)C₁₋₆ alkyl, -C(=O)C₁₋₆ haloalkyl, and -C(=O)C₁₋₆ alkoxy;
R²³ is selected from the group consisting of hydrogen, azido, halogen, -CN, -NO₂, C₁₋₆ haloalkyl, C₁₋₆ alkyl, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, 4- 10 membered heterocycloalkyl, -OR^{a3}, -SR^{a3}, -C(=O)R^{b3}, -C(=O)OR^{b3}, -NR^{c3}R^{d3},-C(=O)NR^{c3}R^{d3}, -OC(=O)NR^{c3}R^{d3}, -NR^{c3}C(=O)R^{b3}, -NR^{c3}C(=O)OR^{b3}, -NR^{c3}C(=O)NR^{c3}R^{d3},-NR^{c3}S(=O)₂R^{b3}, -NR^{c3}S(=O)₂NR^{c3}R^{d3}, -S(O)NR^{c3}R^{d3}, and -S(O)₂NR^{c3}R^{d3}, wherein the C₁₋₆ alkyl, C₁₋₆ haloalkyl, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
each R²⁰ is independently selected from the group consisting of -OH, -SH, -CN, -NO₂, halogen, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino;
R²⁴ is selected from the group consisting of hydrogen, azido, halogen, -CN, -NO₂, C₁₋₆ haloalkyl, C₁₋₆ alkyl, C₂₋₆ alkenyl, C₁₋₆ heteroalkyl, C₃₋₁₀ cycloalkyl, C₆₋₁₀ aryl, -OR^{a4}, -C(=O)R^{b4}, -C(=O)OR^{b4},-NR^{c4}R^{d4}, -C(=O)NR^{c4}R^{d4}, - OC(=O)NR^{c4}R^{d4}, -NR^{c4}C(=O)R^{b4}, -NR^{c4}C(=O)OR^{b4},-NR^{c4}C(=O)NR^{c4}R^{d4}, -NR^{c4}S(=O)₂R^{b4}, -NR^{c4}S(=O)₂NR^{c4}R^{d4}, -S(O)NR^{c4}R^{d4}, and -S(O)₂NR^{c4}R^{d4}, wherein the C₁₋₆ alkyl, C₁₋₆ haloalkyl, C₂₋₆ alkenyl, C₁₋₆ heteroalkyl,C₃₋₁₀ cycloalkyl, and C₆₋₁₀ aryl are unsubstituted or substituted independently with 1, 2, 3, or 4 R^{20d} groups;
each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino; and
each R^{a3}, R^{b3}, R^{c3}, R^{d3}, R^{a4}, R^{b4}, R^{c4}, and R^{d4} is independently selected from the group consisting of hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy,-(C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl, wherein the C₁₋₆ alkyl, C₂₋₆ alkenyl, C₂₋₆ alkynyl, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
or R^{c3} and R^{d3} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups;
or R^{c4} and R^{d4} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups.

In some embodiments of a compound of Formula (I) or (IB), or a pharmaceutically acceptable salt thereof,
X³ is selected from the group consisting of N and CR²³;
X⁴ is selected from the group consisting of N and CR²⁴;

R²¹ is selected from the group consisting of 5 membered heteroaryl, and 5 membered heterocycloalkyl, each of which is unsubstituted or substituted with 1, 2, or 3, independently selected R^{1A} groups; each R^{1A} is independently selected from halo, CN, NO₂, C₁₋₆ alkyl, C₂₋₆ alkenyl, C₂₋₆ alkynyl, C₁₋₆ haloalkyl, C₁₋₆ alkoxy, -C(=O)OH, -C(=O)C₁₋₆ alkyl, -C(=O)C₁₋₆ haloalkyl, and -C(=O)C₁₋₆ alkoxy;
R²³ is selected from the group consisting of hydrogen, azido, halogen, -CN, -NO₂, C₁₋₆ haloalkyl, C₁₋₆ alkyl, C₂₋₆ alkenyl, C₂₋₆ alkynyl, C₁₋₆ heteroalkyl, -(C₁₋₆ alkylene)-C₃₋₁₀ cycloalkyl, -(C₁₋₆ alkylene)-4-10 membered heterocycloalkyl, -(C₁₋₆ heteroalkylene)-C₃₋₁₀ cycloalkyl, -(C₁₋₆ heteroalkylene)-4-10 membered heterocycloalkyl, -(C₁₋₆ alkylene)-C₆₋₁₀ aryl, -(C₁₋₆ alkylene)-5-10 membered heteroaryl, -(C₁₋₆ heteroalkylene)-C₆₋₁₀ aryl, -(C₁₋₆ heteroalkylene)-5-10 membered heteroaryl, C₃₋₁₀ cycloalkyl, C₆₋₁₀ aryl, 5-10 membered heteroaryl, 4- 10 membered heterocycloalkyl, -OR^{a3}, - SR^{a3}, -C(=O)R^{b3}, -C(=O)OR^{b3}, -NR^{c3}R^{d3}, -C(=O)NR^{c3}R^{d3}, -OC(=O)NR^{c3}R^{d3}, -NR^{c3}C(=O)R^{b3}, - NR^{c3}C(=O)OR^{b3}, -NR^{c3}C(=O)NR^{c3}R^{d3}, -NR^{c3}S(=O)₂R^{b3}, -NR^{c3}S(=O)₂NR^{c3}R^{d3}, -S(O)NR^{c3}R^{d3}, and -S(O)₂NR^{c3}R^{d3}, wherein the C₁₋₆ alkyl, C₁₋₆ haloalkyl, C₁₋₆ alkylene, C₁₋₆ heteroalkylene, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
each R²⁰ is independently selected from the group consisting of -OH, -SH, -CN, -NO₂, halogen, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino;
R²⁴ is selected from the group consisting of hydrogen, azido, halogen, -CN, -NO₂, C₁₋₆ haloalkyl, C₁₋₆ alkyl, C₂₋₆ alkenyl, C₁₋₆ heteroalkyl, C₃₋₁₀ cycloalkyl, C₆₋₁₀ aryl, -OR^{a4}, -SR^{a4}, -S(=O)₂R^{a4}, - C(=O)R^{b4}, -C(=O)OR^{b4}, -NR^{c4}R^{d4}, -C(=O)NR^{c4}R^{d4}, - OC(=O)NR^{c4}R^{d4}, -NR^{c4}C(=O)R^{b4}, - NR^{c4}C(=O)OR^{b4}, -NR^{c4}C(=O)NR^{c4}R^{d4}, -NR^{c4}S(=O)₂R^{b4}, -NR^{c4}S(=O)₂NR^{c4}R^{d4}, -S(O)NR^{c4}R^{d4}, and -S(O)₂NR^{c4}R^{d4}, wherein the C₁₋₆ alkyl, C₁₋₆ haloalkyl, C₂₋₆ alkenyl, C₁₋₆ heteroalkyl,C₃₋₁₀ cycloalkyl, and C₆₋₁₀ aryl are unsubstituted or substituted independently with 1, 2, 3, or 4 R^{20d} groups;
each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino; and
each R^{a3}, R^{b3}, R^{c3}, R^{d3}, R^{a4}, R^{b4}, R^{c4}, and R^{d4} is independently selected from the group consisting of hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl, wherein the C₁₋₆ alkyl, C₂₋₆ alkenyl, C₂₋₆ alkynyl, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
or R^{c3} and R^{d3} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups;
or R^{c4} and R^{d4} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups.

In some embodiments of a compound of Formula (I) or (IB), or a pharmaceutically acceptable salt thereof,
X³ is selected from the group consisting of N and CR²³;
X⁴ is selected from the group consisting of N and CR²⁴;

R²¹ is selected from the group consisting of phenyl, 6 membered heteroaryl, and 6 membered heterocycloalkyl, each of which is unsubstituted or substituted by 1, 2, 3, or 4 independently selected R^{1A} groups; each R^{1A} is independently selected from halo, CN, NO₂, C₁₋₆ alkyl, C₂₋₆ alkenyl, C₂₋₆ alkynyl, C₁₋₆ haloalkyl, C₁₋₆ alkoxy, -C(=O)OH, -C(=O)C₁₋₆ alkyl, -C(=O)C₁₋₆ haloalkyl, and -C(=O)C₁₋₆ alkoxy;
R²³ is selected from the group consisting of hydrogen, oxo, azido, halogen, -CN, -NO₂, C₁₋₆ haloalkyl, C₁₋₆ alkyl, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, 4- 10 membered heterocycloalkyl, -OR^{a3}, -SR^{a3}, -C(=O)R^{b3}, -C(=O)OR^{b3}, -NR^{c3}R^{d3}, - C(=O)NR^{c3}R^{d3}, -OC(=O)NR^{c3}R^{d3}, -NR^{c3}C(=O)R^{b3}, -NR^{c3}C(=O)OR^{b3}, -NR^{c3}C(=O)NR^{c3}R^{d3}, - NR^{c3}S(=O)₂R^{b3}, -NR^{c3}S(=O)₂NR^{c3}R^{d3}, -S(O)NR^{c3}R^{d3}, and -S(O)₂NR^{c3}R^{d3}, wherein the C₁₋₆ alkyl, C₁₋₆ haloalkyl, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
each R²⁰ is independently selected from the group consisting of -OH, -SH, -CN, -NO₂, halogen, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino;
R²⁴ is selected from the group consisting of hydrogen, oxo, azido, halogen, -CN, -NO₂, C₁₋₆ haloalkyl, C₁₋₆ alkyl, C₂₋₆ alkenyl, C₁₋₆ heteroalkyl, C₃₋₁₀ cycloalkyl, C₆₋₁₀ aryl, -OR^{a4}, -C(=O)R^{b4}, - C(=O)OR^{b4}, -NR^{c4}R^{d4}, -C(=O)NR^{c4}R^{d4}, - OC(=O)NR^{c4}R^{d4}, -NR^{c4}C(=O)R^{b4}, -NR^{c4}C(=O)OR^{b4}, - NR^{c4}C(=O)NR^{c4}R^{d4}, -NR^{c4}S(=O)₂R^{b4}, -NR^{c4}S(=O)₂NR^{c4}R^{d4}, -S(O)NR^{c4}R^{d4}, and -S(O)₂NR^{c4}R^{d4}, wherein the C₁₋₆ alkyl, C₁₋₆ haloalkyl, C₂₋₆ alkenyl, C₁₋₆ heteroalkyl,C₃₋₁₀ cycloalkyl, C₆₋₁₀ aryl, are unsubstituted or substituted independently with 1, 2, 3, or 4 R^{20d} groups;
each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino; and;
each R^{a3}, R^{b3}, R^{c3}, R^{d3}, R^{a4}, R^{b4}, R^{c4}, and R^{d4} is independently selected from the group consisting of hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl, wherein the C₁₋₆ alkyl, C₂₋₆ alkenyl, C₂₋₆ alkynyl, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
or R^{c3} and R^{d3} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups;
or R^{c4} and R^{d4} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups.

In some embodiments of a compound of Formula (I) or (IB), or a pharmaceutically acceptable salt thereof,
X³ is selected from the group consisting of N and CR²³;
X⁴ is selected from the group consisting of N and CR²⁴;

R²¹ is selected from the group consisting of phenyl, 6 membered heteroaryl, and 6 membered heterocycloalkyl, each of which is unsubstituted or substituted by 1, 2, 3, or 4 independently selected R^{1A} groups; each R^{1A} is independently selected from halo, CN, NO₂, C₁₋₆ alkyl, C₂₋₆ alkenyl, C₂₋₆ alkynyl, C₁₋₆ haloalkyl, C₁₋₆ alkoxy, -C(=O)OH, -C(=O)C₁₋₆ alkyl, -C(=O)C₁₋₆ haloalkyl, and -C(=O)C₁₋₆ alkoxy;
R²³ is selected from the group consisting of hydrogen, oxo, azido, halogen, -CN, -NO₂, C₁₋₆ haloalkyl, C₁₋₆ alkyl, C₂₋₆ alkenyl, C₂₋₆ alkynyl, C₁₋₆ heteroalkyl, -(C₁₋₆ alkylene)-C₃₋₁₀ cycloalkyl, -(C₁₋₆ alkylene)-4-10 membered heterocycloalkyl, -(C₁₋₆ heteroalkylene)-C₃₋₁₀ cycloalkyl, -(C₁₋₆ heteroalkylene)-4-10 membered heterocycloalkyl, -(C₁₋₆ alkylene)-C₆₋₁₀ aryl, -(C₁₋₆ alkylene)-5-10 membered heteroaryl, -(C₁₋₆ heteroalkylene)-C₆₋₁₀ aryl, -(C₁₋₆ heteroalkylene)-5-10 membered heteroaryl, C₃₋₁₀ cycloalkyl, C₆₋₁₀ aryl, 5-10 membered heteroaryl, 4- 10 membered heterocycloalkyl, -OR^{a3}, -SR^{a3}, -C(=O)R^{b3}, -C(=O)OR^{b3}, -NR^{c3}R^{d3}, -C(-O)NR^{c3}R^{d3}, - OC(=O)NR^{c3}R^{d3} -NR^{c3}C(=O)R^{b3}, -NR^{c3}C(=O)OR^{b3}, -NR^{c3}C(=O)NR^{c3}R^{d3}, -NR^{c3}S(=O)₂R^{b3}, - NR^{c3}S(=O)₂NR^{c3}R^{d3}, -S(O)NR^{c3}R^{d3}, and -S(O)₂NR^{c3}R^{d3}, wherein the C₁₋₆ alkyl, C₁₋₆ haloalkyl, C₁₋₆ alkylene, C₁₋₆ heteroalkylene, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
each R²⁰ is independently selected from the group consisting of -OH, -SH, -CN, -NO₂, halogen, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino;
R²⁴ is selected from the group consisting of hydrogen, oxo, azido, halogen, -CN, -NO₂, C₁₋₆ haloalkyl, C₁₋₆ alkyl, C₂₋₆ alkenyl, C₁₋₆ heteroalkyl, C₃₋₁₀ cycloalkyl, C₆₋₁₀ aryl, -OR^{a4}, -SR^{a4}, -S(=O)₂R^{a4}, - C(=O)R^{b4}, -C(=O)OR^{b4}, -NR^{c4}R^{d4}, -C(=O)NR^{c4}R^{d4}, - OC(=O)NR^{c4}R^{d4}, -NR^{c4}C(=O)R^{b4}, - NR^{c4}C(=O)OR^{b4}, -NR^{c4}C(=O)NR^{c4}R^{d4}, -NR^{c4}S(=O)₂R^{b4}, -NR^{c4}S(=O)₂NR^{c4}R^{d4}, -S(O)NR^{c4}R^{d4}, and -S(O)₂NR^{c4}R^{d4}, wherein the C₁₋₆ alkyl, C₁₋₆ haloalkyl, C₂₋₆ alkenyl, C₁₋₆ heteroalkyl,C₃₋₁₀ cycloalkyl, C₆₋₁₀ aryl, are unsubstituted or substituted independently with 1, 2, 3, or 4 R^{20d} groups;
each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino; and;
each R^{a3}, R^{b3}, R^{c3}, R^{d3}, R^{a4}, R^{b4}, R^{c4}, and R^{d4} is independently selected from the group consisting of hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl, wherein the C₁₋₆ alkyl, C₂₋₆ alkenyl, C₂₋₆ alkynyl, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
or R^{c3} and R^{d3} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups;
or R^{c4} and R^{d4} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups.

In some embodiments of Formula (I), (IB), (Ia), (Ib) or (Ic), X³ is CR²³. In some embodiments, X³ is N.

In some embodiments of Formula (I), (IB), (Ia), (Ib) or (Ic), X⁴ is CR²⁴. In some embodiments, R²⁴ is hydrogen. In some embodiments, R²⁴ is halogen. In some embodiments, R²⁴ is - Br. In some embodiments, R²⁴ is -F. In some embodiments, R²⁴ is -Cl. In some embodiments, R²⁴ is - CN. In some embodiments, R²⁴ is C₁₋₄ alkyl. In some embodiments, R²⁴ is methyl. In some embodiments, R²⁴ is ethyl. In some embodiments, R²⁴ is cycloalkyl. In some embodiments, R²⁴ is cyclopropyl.

In some embodiments, disclosed herein is a compound of the Formula (Ia):

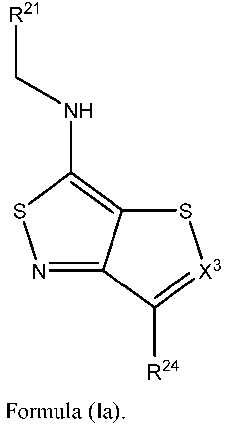

In some embodiments, disclosed herein is a compound of the Formula (Ib):

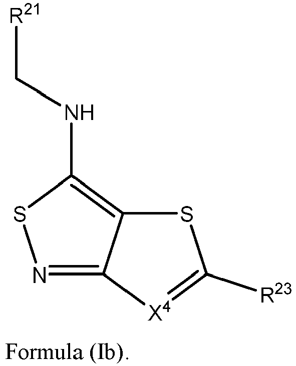

In some embodiments, disclosed herein is a compound of the Formula (Ic):

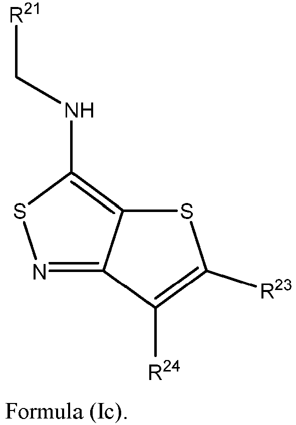

In some embodiments of Formula (I), (IB), (Ia), (Ib), (Ic), (IIa), (IIb), (IIc) or (IId), R²¹ is unsubstituted or substituted 5 membered heteroaryl. In some embodiments, R²¹ is unsubstituted or substituted 5 membered heterocycloalkyl. In some embodiments, R²¹ is unsubstituted. In some embodiments, R²¹ is substituted with 1, 2, or 3, independently selected R^{1A} groups; wherein each R^{1A} is independently selected from halo, CN, NO₂, alkyl, alkenyl, C₂₋₆ alkynyl, alkoxy, -C(=O)OH, an ether group, or an ester group, each of which is unsubstituted or substituted. In some embodiments, R²¹ is substituted with 1, 2, or 3 substituents independently selected R^{1A} groups; wherein each R^{1A} is independently selected from halo, C₁₋₆alkyl, C₁₋₆haloalkyl, and C₁₋₆alkoxy. In some embodiments, R²¹ is substituted with 1, 2, or 3 substituents independently selected R^{1A} groups; wherein each R^{1A} is independently selected from halo, C₁₋₃alkyl, C₁₋₃haloalkyl, and C₁₋₃alkoxy. In some embodiments, R²¹ is 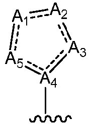 wherein -̅ -̅ -̅ -̅ -̅ -̅ represents a single or a double bond; each of A₁, A₂, A₃, and A₅ is independently selected from the group consisting of O, S, N, NH, NR^{1A}, CH, CR^{1A}, CH₂, and CHR^{1A}; and A₄ is selected from the group consisting of N, C, CH and CR^{1A}. In some embodiments, R²¹ is 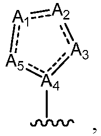 wherein -̅ -̅ -̅ -̅ -̅ -̅ represents a single or a double bond; each of A₁, A₂, A₃, and A₅ is independently selected from the group consisting of O, S, N, NH, NR^{1A}, C, CH, CR^{1A}, CH₂, and CHR^{1A}; and A4 is selected from the group consisting of N, C, CH and CR^{1A} .

In some embodiments of Formula (I), (IB), (Ia), (Ib), (Ic), (IIa), (IIb), (IIc) or (IId), R²¹ is selected from the group consisting of

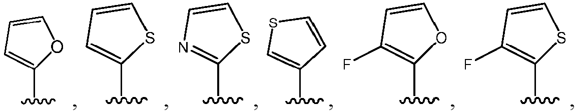

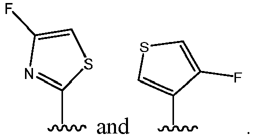

In some embodiments of Formula (I), (IB), (Ia), (Ib), (Ic), (IIa), (IIb), (IIc) or (IId), R²¹ is 5 membered heteroaryl. In some embodiments, R²¹ is furanyl, or thiazolyl each of which is substituted or unsubstituted.

In some embodiments of Formula (I), (IB), (Ia), (Ib), (Ic), (IIa), (IIb), (IIc) or (IId), R²¹ is unsubstituted furanyl. In some embodiments, R²¹ is substituted furanyl. In some embodiments, R²¹ is unsubstituted thiazolyl. In some embodiments, R²¹ is substituted thiazolyl. In some embodiments, R²¹ is 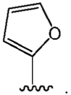 In some embodiments, R²¹ is 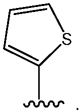 In some embodiments, R²¹ is 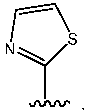 In some embodiments, R²¹ is 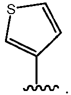 In some embodiments, R²¹ is 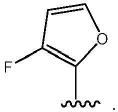 In some embodiments, R²¹ is 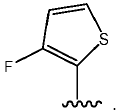 In some embodiments, R²¹ is 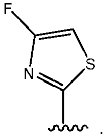 In some embodiments, R²¹ is 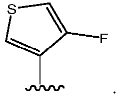 In some embodiments, R²¹ is 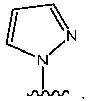 In some embodiments, R²¹ is 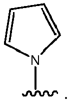 In some embodiments, R²¹ is 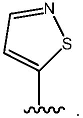 In some embodiments, R²¹ is 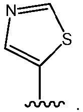 In some embodiments, R²¹ is 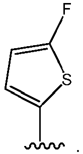 In some embodiments, R²¹ is 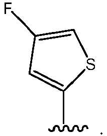 In some embodiments, R²¹ is 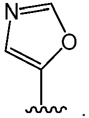 In some embodiments, R²¹ is 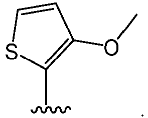 In some embodiments, R²¹ is 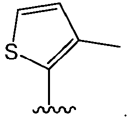 In some embodiments, R²¹ is 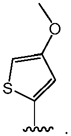 In some embodiments, R²¹ is 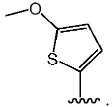 In some embodiments, R²¹ is

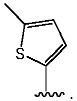

In some embodiments of Formula (I), (IB), (Ia), (Ib), (Ic), (IIa), (IIb), (IIc) or (IId), R²¹ is unsubstituted or substituted phenyl. In some embodiments, R²¹ is unsubstituted or substituted 6 membered heteroaryl. In some embodiments, R²¹ is unsubstituted or substituted 6 membered heterocycloalkyl. In some embodiments, R²¹ is unsubstituted. In some embodiments, R²¹ is substituted with 1, 2, 3, or 4 independently selected R^{1A} groups; wherein each R^{1A} is independently selected from halo, CN, NO₂, alkyl, alkenyl, C₂₋₆ alkynyl, alkoxy, -C(=O)OH, an ether group, or an ester group, each of which is unsubstituted or substituted. In some embodiments, R²¹ is substituted with 1, 2, 3, or 4 substituents independently selected R^{1A} groups; wherein each R^{1A} is independently selected from halo, C₁₋₆alkyl, C₁₋₆haloalkyl, and C₁₋₆alkoxy. In some embodiments, R²¹ is substituted with 1, 2, 3, or 4 substituents independently selected R^{1A} groups; wherein each R^{1A} is independently selected from halo, C₁₋₃alkyl, C₁₋₃haloalkyl, and C₁₋₃alkoxy. In some embodiments, R²¹ is 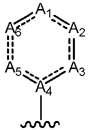 whereinrepresents a single or a double bond; each of A₁, A₂, A₃, As and A₆ is independently selected from the group consisting of O, S, N, NH, NR^{1A}, CH, CR^{1A}, CH₂, and CHR^{1A}; and A₄ is selected from the group consisting of N, C, CH and CR^{1A}. In some embodiments, R²¹ is 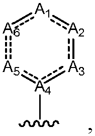 whereinrepresents a single or a double bond; each of A₁, A₂, A₃, A₅ and A₆ is independently selected from the group consisting of O, S, N, NH, NR^{1A}, C, CH, CR^{1A}, CH₂, and CHR^{1A}; and A₄ is selected from the group consisting of N, C, CH, and CR^{1A} . In some embodiments, R²¹ is selected from the group consisting of

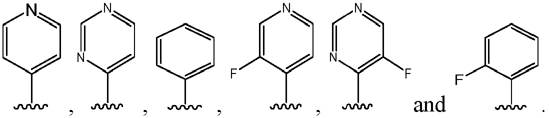

In some embodiments of Formula (I), (IB), (Ia), (Ib), (Ic), (IIa), (IIb), (IIc) or (IId), R²¹ is substituted or unsubstituted phenyl. In some embodiments, R²¹ is 6 membered heteroaryl. In some embodiments, R²¹ is pyridinyl, thiophenyl, pyrimidinyl, each of which is substituted or unsubstituted.

In some embodiments of Formula (I), (IB), (Ia), (Ib), (Ic), (IIa), (IIb), (IIc) or (IId), R²¹ is unsubstituted pyridinyl. In some embodiments, R²¹ is substituted pyridinyl. In some embodiments, R²¹ is unsubstituted thiophenyl. In some embodiments, R²¹ is substituted thiophenyl. In some embodiments, R²¹ is unsubstituted pyrimidinyl. In some embodiments, R²¹ is substituted pyrimidinyl. In some embodiments, R²¹ is 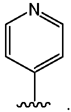 In some embodiments, R²¹ is 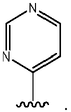 In some embodiments, R²¹ is 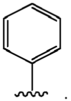 In some embodiments, R²¹ is 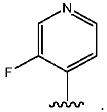 In some embodiments, R²¹ is 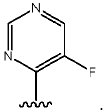 In some embodiments, R²¹ is

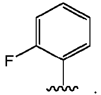

In some embodiments of Formula (I) or (IB), X³ is CH. In some embodiments, X³ is CR²³. In some embodiments, X³ is CR²³, wherein R²³ is C₁₋₆ alkyl or C₁₋₆ heteroalkyl, wherein the C₁₋₆ alkyl and C₁₋₆ heteroalkyl are unsubstituted or substituted independently with 1, 2, 3, or 4 R²⁰ groups. In some embodiments, X³ is CCH₂CHNH₂CH₃. In some embodiments, X³ is CCH₂CHNH₂CH₂OH. In some embodiments, X³ is CCH₂CHNH₂CH₂CH₃. In some embodiments, X³ is CCH₂CHNH₂CH₂CH₂OH. In some embodiments, X³ is CCH₂CHNH₂CH₂CH₂F. In some embodiments, X³ is CCH₂CHNH₂CH₂CHF₂. In some embodiments, X³ is CCH₂CHNH₂CH₂CH(CH₃)₂. In some embodiments, X⁴ is N. In some embodiments, X⁴ is CH. In some embodiments, X⁴ is CR²⁴, wherein R²⁴ is selected from the group consisting of halo, CN, and substituted or unsubstituted C₁₋₆ alkyl. In some embodiments, X⁴ is CCl. In some embodiments, X⁴ is CBr. In some embodiments, X⁴ is CF. In some embodiments, X⁴ is CCN. In some embodiments, X⁴ is CCH₃. In some embodiments, X⁴ is C(cyclopropyl).

In some embodiments of Formula (I), (IB), (Ia), (Ib), or (Ic), R²³ is C₁₋₆ alkyl or C₁₋₆ heteroalkyl, and wherein the C₁₋₆ alkyl and C₁₋₆ heteroalkyl are substituted independently with 1, 2, or 3 R²⁰ groups. In some embodiments, R²³ is C₁₋₆ heteroalkyl, wherein the C₁₋₆ heteroalkyl is substituted with 1, 2, or 3 independently selected R²⁰ groups. In some embodiments, C₁₋₆ alkyl is substituted with 1 R²⁰ group. In some embodiments, C₁₋₆ alkyl is substituted with 2 independently selected R²⁰ groups. In some embodiments, C₁₋₆ alkyl is substituted with 3 independently selected R²⁰ groups. In some embodiments, C₁₋₆ heteroalkyl is substituted with 1 R²⁰ group. In some embodiments, C₁₋₆ heteroalkyl is substituted with 2 independently selected R²⁰ groups. In some embodiments, C₁₋₆ heteroalkyl is substituted with 3 independently selected R²⁰ groups.

In some embodiments of Formula (I), (IB), (Ia), (Ib), or (Ic), R²³ is substituted or unsubstituted C₁₋₆ alkyl or substituted or unsubstituted C₁₋₆ heteroalkyl. In some embodiments, R²³ is substituted or unsubstituted C₁₋₆ heteroalkyl. In some embodiments, the C₁₋₆ heteroalkyl is - CH₂CH(NH₂)CH₂-S(=O)₂-CH₃ or -CH₂CH(NH₂)CH₂-S(=O)-CH₃. In some embodiments, R²³ is - CH₂CH(NH₂)CH₂-S(=O)₂-CH₃. In some embodiments, R²³ is -CH₂CH(NH₂)CH₂-S(=O)-CH₃. In some embodiments, R²³ is CH₂CHNH₂CH₃. In some embodiments, R²³ is CH₂CHNH₂CH₂OH. In some embodiments, R²³ is CH₂CHNH₂CH₂CH₃. In some embodiments, R²³ is CH₂CHNH₂CH₂CH₂OH. In some embodiments, R²³ is CH₂CHNH₂CH₂CH₂F. In some embodiments, R²³ is CH₂CHNH₂CH₂CHF₂. In some embodiments, R²³ is CH₂CHNH₂CH₂CH(CH₃)₂. In some embodiments, R²³ is CH₂CHNH₂CHFCH₃. In some embodiments, R²³ is CH₂CHNH₂CH₂F. In some embodiments, R²³ is CH₂CHNH₂CH₂OCH₃. In some embodiments, R²³ is CH₂CHNH₂CH₂OCD₃. In some embodiments, R²³ is CF₂CH(NH₂)CH₃. In some embodiments, R²³ is CH₂CH(NH₂)CH₂OCH₃. In some embodiments, R²³ is CF₂CH(NH₂)CH₃. In some embodiments, R²³ is CH₂CH(NH₂)CHF₂.

In some embodiments of Formula (I), (IB), (Ia), (Ib), or (Ic), R²³ is H.

In some embodiments of Formula (I), (IB), (Ia), (Ib), or (Ic), R²³ is substituted with 1, 2, or 3 independently selected R²⁰ groups, wherein each R²⁰ group is independently selected from the group consisting of OH, SH, CN, NO₂, halo, oxo, amino, C₁₋₃alkyl, C₁₋₃alkoxy, cycloalkyl, aryl, heteroaryl, heterocycloalkyl, carbamyl, and carbamoyl. In some embodiments, R²³ is substituted with 1, 2, or 3 independently selected R²⁰ groups, wherein each R²⁰ group is independently selected from the group consisting of OH, halo, and C₁₋₃alkoxy.

In some embodiments of Formula (I), (IB), (Ia), (Ib), or (Ic), R²³ is substituted or unsubstituted C₁₋₆ alkyl. In some embodiments, R²³ is C₁₋₆ alkyl, wherein C₁₋₆ alkyl is substituted with 1, 2, or 3 independently selected R²⁰ groups. In some embodiments, R²³ is C₁₋₆ alkyl, wherein C₁₋₆ alkyl is substituted with 1, 2, or 3 independently selected R²⁰ groups, wherein each R²⁰ group is independently selected from the group consisting of OH, SH, CN, NO₂, halo, oxo, amino, C₁₋₃alkyl, C₁₋₃alkoxy, cycloalkyl, aryl, heteroaryl, heterocycloalkyl, carbamyl, and carbamoyl.. In some embodiments, R²³ is C₁₋₆ alkyl, wherein C₁₋₆ alkyl is substituted with 1, 2, or 3 independently selected R²⁰ groups, wherein each R²⁰ group is independently selected from the group consisting of OH, halo, and C₁₋₃alkoxy.

In some embodiments of Formula (I), (IB), (Ia), (Ib), or (Ic), R²³ is substituted or unsubstituted C₁₋₆ alkenyl. In some embodiments, R²³ is C₁₋₆ alkenyl, wherein C₁₋₆ alkenyl is substituted with 1, 2, or 3 independently selected R²⁰ groups.

In some embodiments of Formula (I), (IB), (Ia), (Ib), or (Ic), R²³ is substituted or unsubstituted C₁₋₆ alkynyl. In some embodiments, R²³ is C₁₋₆ alkynyl, wherein C₁₋₆ alkynyl is substituted with 1, 2, or 3 independently selected R²⁰ groups.

In some embodiments of Formula (I), (IB), (Ia), (Ib), (Ic), (IIa), (IIb), (IIc) or (IId), each R²⁰ is independently selected from the group consisting of -OH, -SH, -CN, -NO₂, halogen, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, and wherein each of the alkyl, alkenyl, phenyl, cycloalkyl, alkenyl, heteroaryl, and heterocycloalkyl is optionally substituted with 1, 2, 3, or 4 groups independently selected from OH, -SH, -CN, -NO₂, halogen, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, C₁₋₄ haloalkoxy, C₃₋₆ cycloalkyl, amino, oxo, C₁₋₄ alkylamino, and di(C₁₋₄ alkyl)amino. In some embodiments of Formula (I), (IB), (Ia), (Ib), (Ic), (IIa), (IIb), (IIc) or (IId), R²⁰ is optionally substituted.

In some embodiments of Formula (I), (IB), (Ia), (Ib), (Ic), (IIa), (IIb), (IIc) or (IId), each R²⁰ is independently selected from the group consisting of -OH, -SH, -CN, -NO₂, halogen, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, C₁₋₄ haloalkoxy, C₃₋₆ cycloalkyl, phenyl, 5-6 membered heteroaryl, 4-6 membered heterocycloalkyl, amino, C₁₋₄ alkylamino, and di(C₁₋₄ alkyl)amino, and wherein each of the alkyl, alkenyl, phenyl, cycloalkyl, alkenyl, heteroaryl, and heterocycloalkyl is optionally substituted with 1, 2, 3, or 4 groups independently selected from OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, C₁₋₄ haloalkoxy, C₃₋₆ cycloalkyl, amino, C₁₋₄ alkylamino, and di(C₁₋₄ alkyl)amino.

In some embodiments of Formula (I), (IB), (Ia), (Ib), (Ic), (IIa), (IIb), (IIc) or (IId), each R²⁰ is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein alkyl, alkenyl, alkynyl, heteroalkyl, cycloalkyl, phenyl, heteroaryl and heterocycloalkyl are each optionally substituted with 1, 2, 3 or 4 R³¹ groups. In some embodiments, each R³¹ is individually -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy. In some embodiments, R³¹ is -OH. In some embodiments, R³¹ is -SH. In some embodiments, R³¹ is -CN. In some embodiments, R³¹ is -NO₂. In some embodiments, R³¹ is halogen. In some embodiments, R³¹ is oxo. In some embodiments, R³¹ is C₁₋₄ alkyl. In some embodiments, R³¹ is C₂₋₄ alkenyl. In some embodiments, R³¹ is C₁₋₄ haloalkyl. In some embodiments, R³¹ is C₁₋₄ cyanoalkyl. In some embodiments, R³¹ is C₁₋₄ hydroxyalkyl. In some embodiments, R³¹ is C₁₋₄ alkoxy.

In some embodiments of Formula (I), (IB), (Ia), (Ib), (Ic), (IIa), (IIb), (IIc) or (IId), each R²⁰ is independently selected from the group consisting of -OH, -SH, -CN, -NO₂, halogen, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein alkyl, alkenyl, alkynyl, heteroalkyl, cycloalkyl, phenyl, heteroaryl and heterocycloalkyl are each optionally substituted with 1, 2, 3 or 4 R³¹ groups. In some embodiments, each R³¹ is individually oxo, halogen, methyl, ethyl, -CN, -CF₃, -OH, -OMe, -NH₂, or -NO₂. In some embodiments, R³¹ is oxo. In some embodiments, R³¹ halogen. In some embodiments, R³¹ methyl, ethyl. In some embodiments, R³¹ -CN. In some embodiments, R³¹ -CF₃. In some embodiments, R³¹ -OH. In some embodiments, R³¹ -OMe. In some embodiments, R³¹ -NH₂. In some embodiments, R³¹ -NO₂.

In some embodiments of Formula (I), (IB), (Ia), (Ib), or (Ic), R²³ is selected from the group consisting of hydrogen, oxo, azido, halogen, -CN, -NO₂, C₁₋₆ haloalkyl, C₁₋₆ alkyl, C₂₋₆ alkenyl, C₂₋₆ alkynyl, C₁₋₆heteroalkyl, C₃₋₁₀ cycloalkyl, C₆₋₁₀ aryl, 5-10 membered heteroaryl, 4-10 membered heterocycloalkyl, -OR^{a3}, -SR^{a3}, -C(=O)R^{b3}, -C(=O)OR^{b3}, -NR^{c3}R^{d3}, -C(=O)NR^{C3}R^{d3}, -OC(=O)NR^{c3}R^{d3}, -NR^{c3}C(=O)R^{b3}, -NR^{c3}C(=O)OR^{b3}, -NR^{c3}C(=O)NR^{c3}R¹³, -NR^{c3}S(=O)₂R^{b3}, -NR^{c3}S(=O)₂NR^{c3}R^{a3},-S(O)NR^{C3}R^{d3}, and -S(O)₂NR^{c3}R^{d3}, wherein the C₁₋₆ alkyl, C₁₋₆ haloalkyl, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups. In some embodiments, R²³ is selected from the group consisting of hydrogen, oxo, azido, halogen, -CN, -NO₂, C₁₋₆ haloalkyl, C₁₋₆ alkyl, C₁₋₆heteroalkyl, C₃₋₁₀ cycloalkyl, C₆₋₁₀ aryl, 5-10 membered heteroaryl, 4-10 membered heterocycloalkyl, -OR^{a3}, and -NR^{c3}R^{d3}, wherein the C₁₋₆ alkyl, C₁₋₆ haloalkyl, C₁₋₆ heteroalkyl, C₃₋₁₀ cycloalkyl, C₆₋₁₀ aryl, 5-10 membered heteroaryl, and 4-10 membered heterocycloalkyl are unsubstituted or substituted. In some embodiments, R²³ is hydrogen.

In some embodiments of Formula (I), (IB), (Ia), (Ib), or (Ic), R²³ is -NR^{c3}R^{d3}. In some embodiments, R^{c3} and R^{d3} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups. In some embodiments, R²³ is 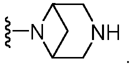 In some embodiments, R²³ is

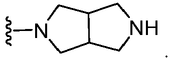

In some embodiments, R²³ is 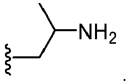 In some embodiments, R²³ is 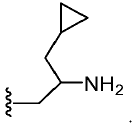 In some embodiments, R²³ is 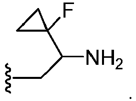 In some embodiments, R²³ is 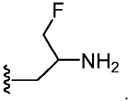 In some embodiments, R²³ is 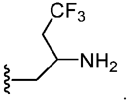 In some embodiments, R²³ is 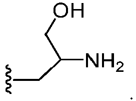 In some embodiments, R²³ is 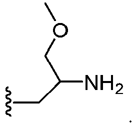 In some embodiments, R²³ is 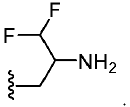 In some embodiments, R²³ is

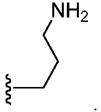

In some embodiments of Formula (I), (IB), (Ia), (Ib), or (Ic), R²³ is -(C₁₋₆ alkylene)-C₃₋₁₀ cycloalkyl, -(C₁₋₆ alkylene)-4-10 membered heterocycloalkyl, -(C₁₋₆ heteroalkylene)-C₃₋₁₀ cycloalkyl, -(C₁₋₆ heteroalkylene)-4-10 membered heterocycloalkyl, -(C₁₋₆ alkylene)-C₆₋₁₀ aryl, -(C₁₋₆ alkylene)-5-10 membered heteroaryl, -(C₁₋₆ heteroalkylene)-C₆₋₁₀ aryl, -(C₁₋₆ heteroalkylene)-5-10 membered heteroaryl. In some embodiments, R²³ is -(C₁₋₆ alkylene)-C₃₋₁₀ cycloalkyl, optionally substituted with one or more R²⁰. In some embodiments, R²³ is -(C₁₋₆ alkylene)-4-10 membered heterocycloalkyl, optionally substituted with one or more R²⁰. In some embodiments, R²³ is -(C₁₋₆ heteroalkylene)-4-10 membered heterocycloalkyl, optionally substituted with one or more R²⁰. In some embodiments, R²³ is -(C₁₋₆ heteroalkylene)-C₃₋₁₀ cycloalkyl, optionally substituted with one or more R²⁰. In some embodiments, R²³ is -(C₁₋₆ alkylene)-C₆₋₁₀ aryl, optionally substituted with one or more R²⁰. In some embodiments, R²³ is -(C₁₋₆ alkylene)-5-10 membered heteroaryl, optionally substituted with one or more R²⁰. In some embodiments, R²³ is -(C₁₋₆ heteroalkylene)-C₆₋₁₀ aryl, optionally substituted with one or more R²⁰. In some embodiments, R²³ is -(C₁₋₆ heteroalkylene)-5-10 membered heteroaryl, optionally substituted with one or more R²⁰. In some embodiments, the C₁₋₆ heteroalkylene is

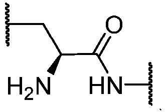

In some embodiments of Formula (I), (IB), (Ia), (Ib), or (Ic), R²³ is substituted or unsubstituted -(C₁₋₆ alkylene)-C₃₋₁₀ cycloalkyl. In some embodiments, R¹³ is -(C₁₋₆ alkylene)-C₃₋₁₀ cycloalkyl, wherein -(C₁₋₆ alkylene)-C₃₋₁₀ cycloalkyl is substituted with 1, 2, or 3 independently selected R²⁰ groups. In some embodiments, the C₁₋₆ alkylene is C₁₋₃ alkylene. In some embodiments, the C₁₋₆ alkylene is CH₂. In some embodiments, the C₃₋₁₀ cycloalkyl is an optionally substituted a 3-6 membered ring. In some embodiments, the C₃₋₁₀ cycloalkyl is an optionally substituted a 3 membered ring. In some embodiments, the C₃₋₁₀ cycloalkyl is an optionally substituted a 4 membered ring. In some embodiments, the C₃₋₁₀ cycloalkyl is an optionally substituted a 5 membered ring. In some embodiments, the C₃₋₁₀ cycloalkyl is an optionally substituted a 6 membered ring. In some embodiments, the -C₃₋₁₀ cycloalkyl is

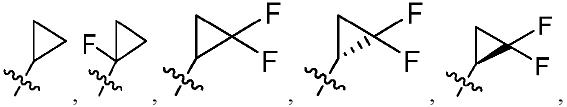

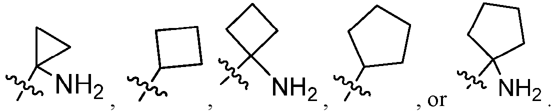

In some embodiments of Formula (I), (IB), (Ia), (Ib), or (Ic), R²³ is substituted or unsubstituted -(C₁₋₆ alkylene)-4-10 membered heterocycloalkyl. In some embodiments, R²³ is -(C₁₋₆ alkylene)-4-10 membered heterocycloalkyl, wherein -(C₁₋₆ alkylene)-4-10 membered heterocycloalkyl is substituted with 1, 2, or 3 independently selected R²⁰ groups. In some embodiments, the C₁₋₆ alkylene is C₁₋₃ alkylene. In some embodiments, the C₁₋₆ alkylene is CH₂. In some embodiments, the 4-10 membered heterocycloalkyl is an optionally substituted a 4-6 membered ring. In some embodiments, the 4-10 membered heterocycloalkyl is an optionally substituted a 4 membered ring. In some embodiments, the 4-10 membered heterocycloalkyl is an optionally substituted a 5 membered ring. In some embodiments, the 4-10 membered heterocycloalkyl is an optionally substituted a 6 membered ring. n some embodiments, the 4-10 membered heterocycloalkyl contains 0-1 oxygen and 0-2 nitrogen atoms. In some embodiments, the -4-10 membered heterocycloalkyl is

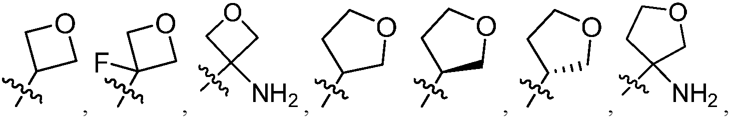

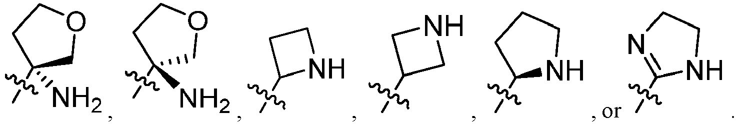

In some embodiments of Formula (I), (IB), (Ia), (Ib), or (Ic), R²³ is substituted or unsubstituted -(C₁₋₆ heteroalkylene)-C₃₋₁₀ cycloalkyl. In some embodiments, R²³ is -(C₁₋₆ heteroalkylene)-C₃₋₁₀ cycloalkyl, wherein -(C₁₋₆ heteroalkylene)-C₃₋₁₀ cycloalkyl is substituted with 1, 2, or 3 independently selected R²⁰ groups. In some embodiments, the heteroalkylene is C₁₋₃ heteroalkylene. In some embodiments, the C₃₋₁₀ cycloalkyl is an optionally substituted a 3-6 membered ring. In some embodiments, the C₃₋₁₀ cycloalkyl is an optionally substituted a 3 membered ring. In some embodiments, the C₃₋₁₀ cycloalkyl is an optionally substituted a 4 membered ring. In some embodiments, the C₃₋₁₀ cycloalkyl is an optionally substituted a 5 membered ring. In some embodiments, the C₃₋₁₀ cycloalkyl is an optionally substituted a 6 membered ring. In some embodiments, the heteroalkylene is C₁₋₃ heteroalkylene. In some embodiments, the -C₃₋₁₀ cycloalkyl is

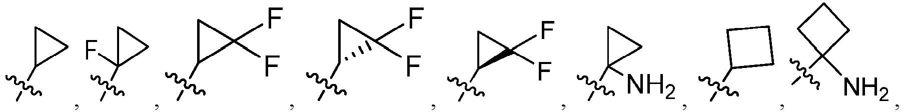

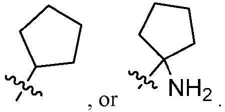

In some embodiments of Formula (I), (IB), (Ia), (Ib), or (Ic), R²³ is substituted or unsubstituted -(C₁₋₆ heteroalkylene)-4-10 membered heterocycloalkyl. In some embodiments, R²³ is-(C₁₋₆ heteroalkylene)-4-10 membered heterocycloalkyl, wherein -(C₁₋₆ heteroalkylene)-4-10 membered heterocycloalkyl is substituted with 1, 2, or 3 independently selected R²⁰ groups. In some embodiments, the heteroalkylene is C₁₋₃ heteroalkylene. In some embodiments, the 4-10 membered heterocycloalkyl is an optionally substituted a 4-6 membered ring. In some embodiments, the 4-10 membered heterocycloalkyl is an optionally substituted a 4 membered ring. In some embodiments, the 4-10 membered heterocycloalkyl is an optionally substituted a 5 membered ring. In some embodiments, the 4-10 membered heterocycloalkyl is an optionally substituted a 6 membered ring. n some embodiments, the 4-10 membered heterocycloalkyl contains 0-1 oxygen and 0-2 nitrogen atoms. In some embodiments, the -4-10 membered heterocycloalkyl is

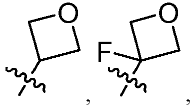

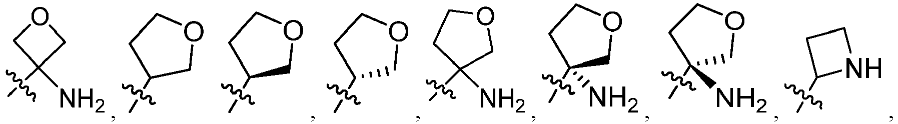

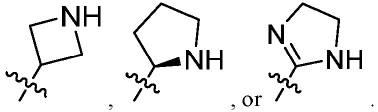

In some embodiments of Formula (I), (IB), (Ia), (Ib), or (Ic), R²³ is any one selected from the group consisting of: 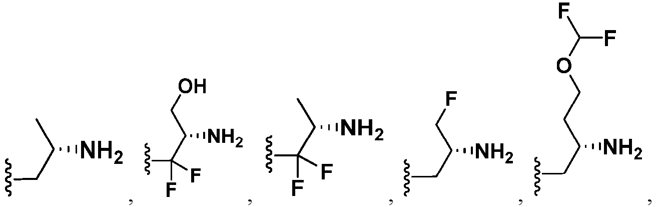 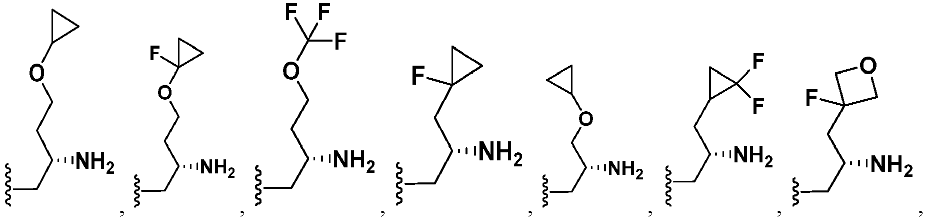 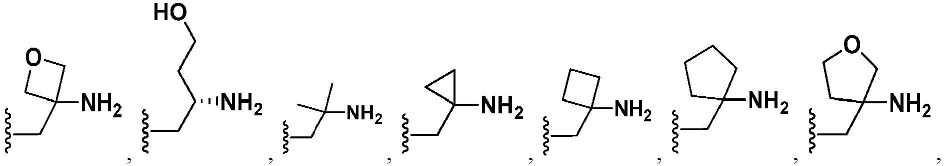 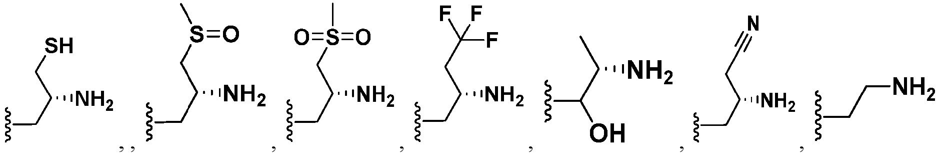 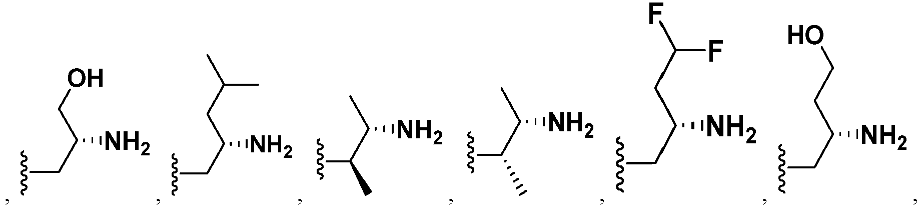 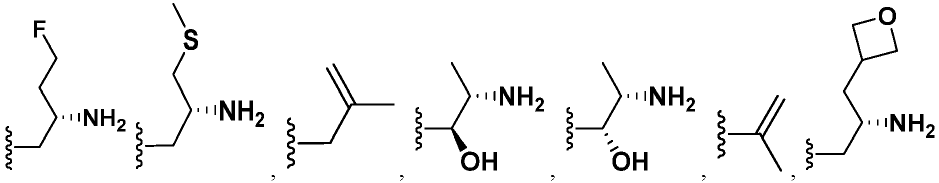 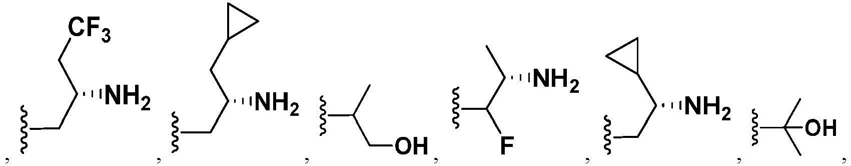 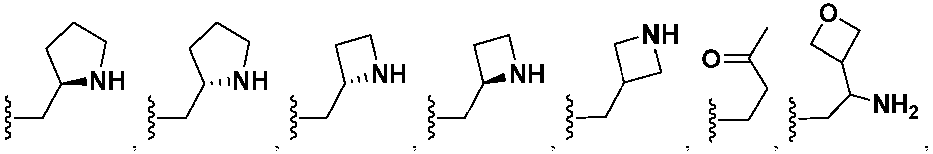 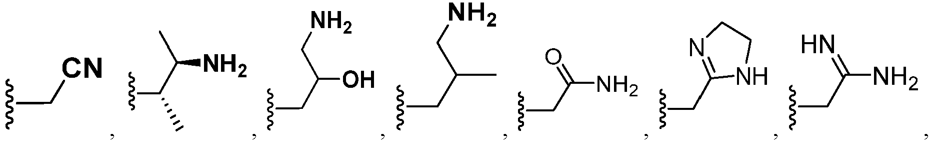 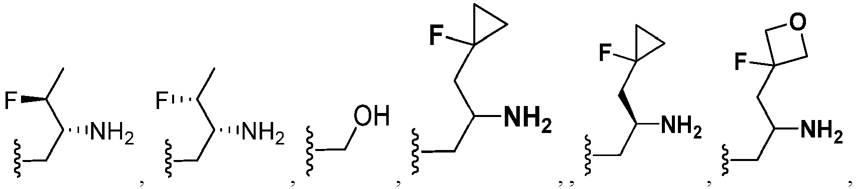 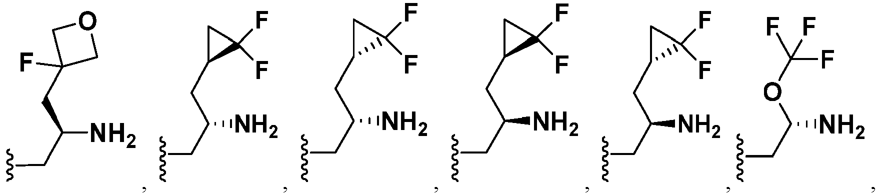 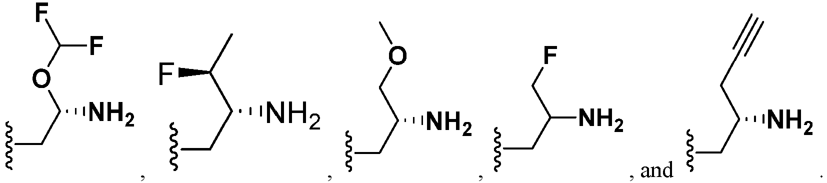 In some embodiments, R²³ is any one selected from the group consisting of: 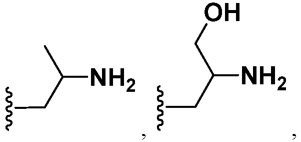 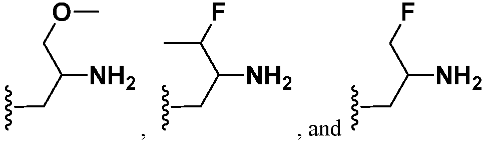 In some embodiments, R²³ is 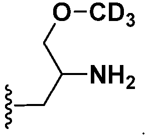 In some embodiments, R²³ is 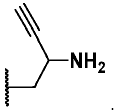 In some embodiments, R²³ is 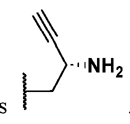 In some embodiments, R²³ is 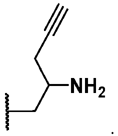 In some embodiments, R²³ is 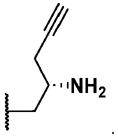 In some embodiments, R²³ is 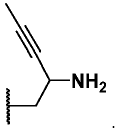 In some embodiments, R²³ is 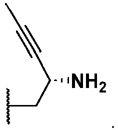 In some embodiments, R²³ is 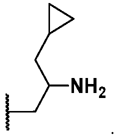 In some embodiments, R²³ is 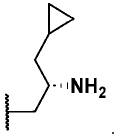 In some embodiments, R²³ is 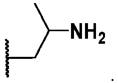 In some embodiments, R²³ is 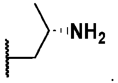 In some embodiments, R²³ is 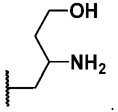 In some embodiments, R²³ is 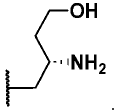 In some embodiments, R²³ is 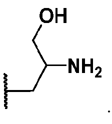 . In some embodiments, R²³ is 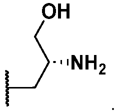 In some embodiments, R²³ is 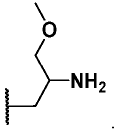 In some embodiments, R²³ is 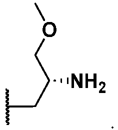 In some embodiments, R²³ is 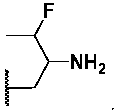 In some embodiments, R²³ is 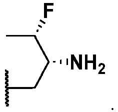 In some embodiments, R²³ is 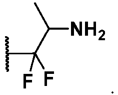 In some embodiments, R²³ is 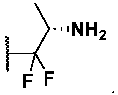 In some embodiments, R²³ is 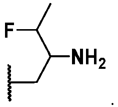 In some embodiments, R²³ is 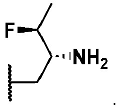 In some embodiments, R²³ is 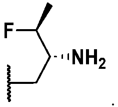 In some embodiments, R²³ is 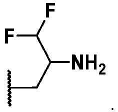 In some embodiments, R²³ is 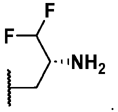 In some embodiments, R²³ is 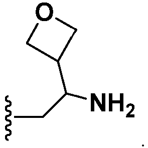 In some embodiments, R²³ is 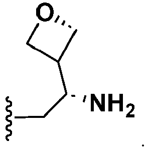 In some embodiments, R²³ is 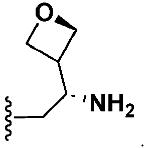 In some embodiments, R²³ is 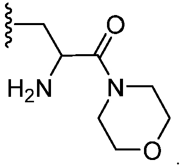 In some embodiments, R²³ is 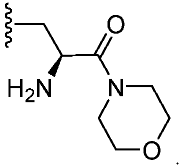 In some embodiments, R²³ is 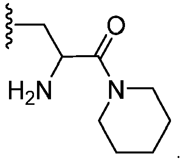 In some embodiments, R²³ is 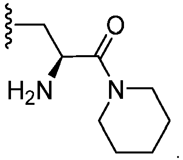 In some embodiments, R²³ is 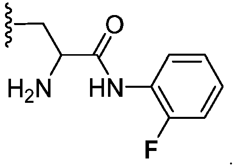 In some embodiments, R²³ is 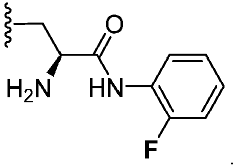 In some embodiments, R²³ is 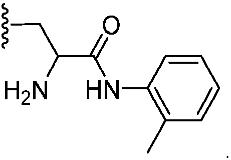 In some embodiments, R²³ is 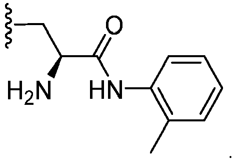 In some embodiments, R²³ is 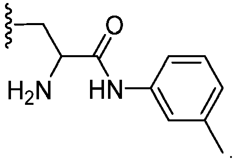 In some embodiments, R²³ is 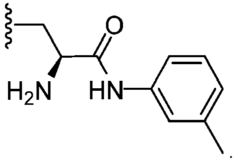 In some embodiments, R²³ is 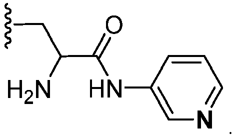 In some embodiments, R²³ is 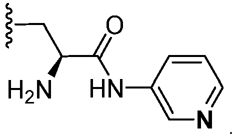 In some embodiments, R²³ is 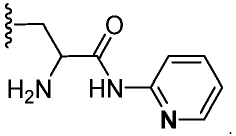 In some embodiments, R²³ is 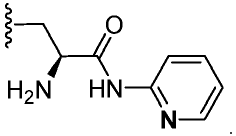 In some embodiments, R²³ is 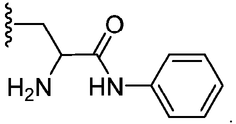 In some embodiments, R²³ is 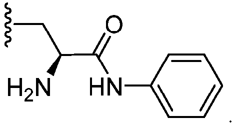 In some embodiments, R²³ is 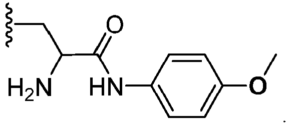 In some embodiments, R²³ is 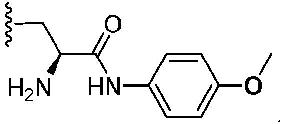 In some embodiments, R²³ is 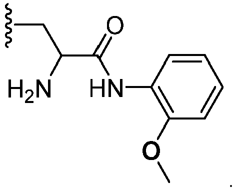 In some embodiments, R²³ is 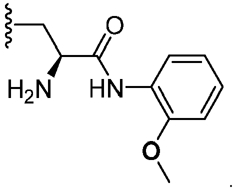 In some embodiments, R²³ is 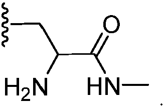 In some embodiments, R²³ is 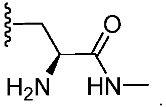 In some embodiments, R²³ is 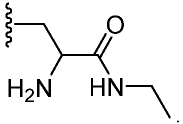 In some embodiments, R²³ is 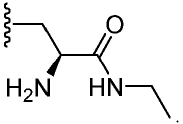 In some embodiments, R²³ is 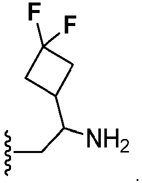 In some embodiments, R²³ is 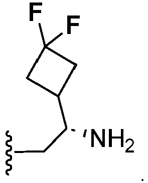 In some embodiments, R²³ is 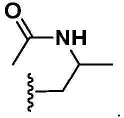 In some embodiments, R²³ is 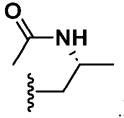 In some embodiments, R²³ is 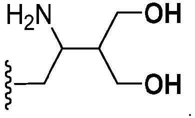 In some embodiments, R²³ is 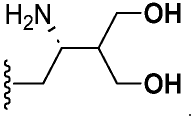 In some embodiments, R²³ is 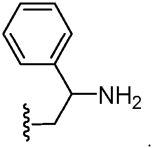 In some embodiments, R²³ is 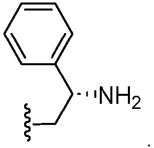 In some embodiments, R²³ is 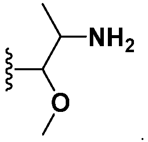 In some embodiments, R²³ is 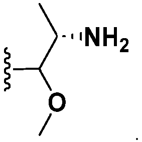 In some embodiments, R²³ is 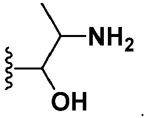 In some embodiments, R²³ is 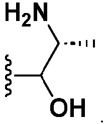 In some embodiments, R²³ is 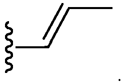 In some embodiments, R²³ is 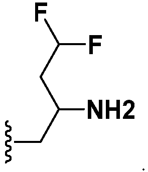 In some embodiments, R²³ is 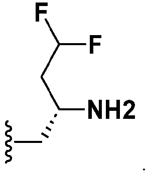 In some embodiments, R²³ is 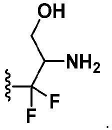 In some embodiments, R²³ is 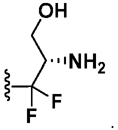 In some embodiments, R²³ is 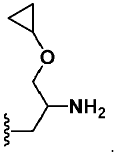 In some embodiments, R²³ is

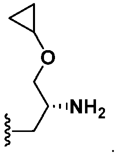

I n some embodiments, R²³ is 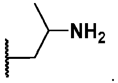 In some embodiments, R²³ is 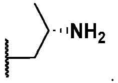 In some embodiments, R²³ is 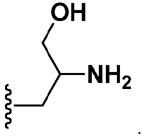 In some embodiments, R²³ is 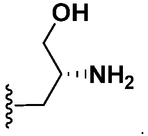 In some embodiments, R²³ is 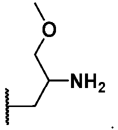 In some embodiments, R²³ is 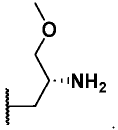 In some embodiments, R²³ is 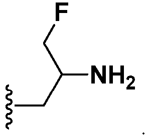 In some embodiments, R²³ is 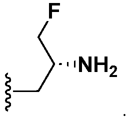 In some embodiments, R²³ is 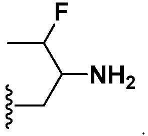 In some embodiments, R²³ is 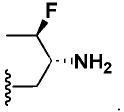 In some embodiments, R²³ is 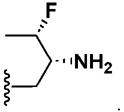 In some embodiments, R²³ is 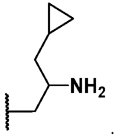 In some embodiments, R²³ is 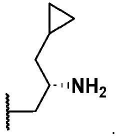 In some embodiments, R²³ is 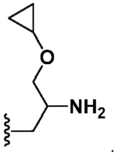 In some embodiments, R²³ is 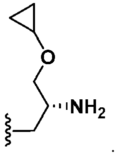 In some embodiments, R²³ is 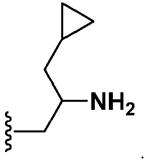 In some embodiments, R²³ is 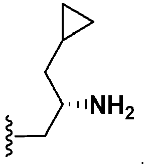 In some embodiments, R²³ is 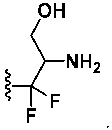 In some embodiments, R²³ is 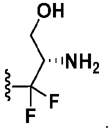 In some embodiments, R²³ is 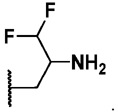 In some embodiments, R²³ is 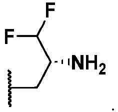 In some embodiments, R²³ is 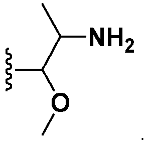 In some embodiments, R²³ is 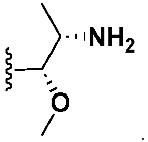 In some embodiments, R²³ is

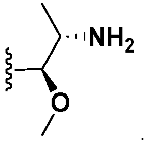

In some embodiments, R²³ is 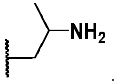 In some embodiments, R²³ is 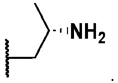 In some embodiments, R²³ is 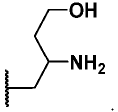 In some embodiments, R²³ is 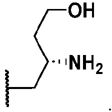 In some embodiments, R²³ is 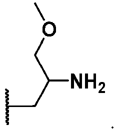 In some embodiments, R²³ is 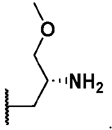 In some embodiments, R²³ is 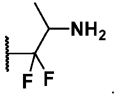 In some embodiments, R²³ is 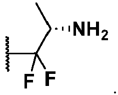 In some embodiments, R²³ is 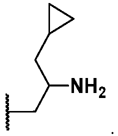 In some embodiments, R²³ is 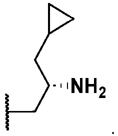 In some embodiments, R²³ is 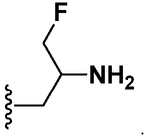 In some embodiments, R²³ is 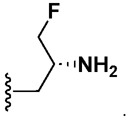 In some embodiments, R²³ is 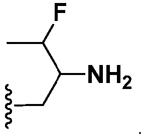 In some embodiments, R²³ is 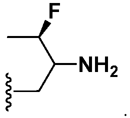 In some embodiments, R²³ is 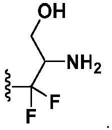 In some embodiments, R²³ is 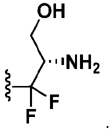 In some embodiments, R²³ is 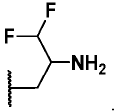 In some embodiments, R²³ is

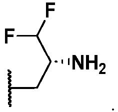

In some embodiments of Formula (I), (IB), (Ia), (Ib), or (Ic), R²³ is a methyl substituted with 1, 2, or 3 independently selected R²⁰ groups. In some embodiments, each R²⁰ is independently halogen, C₁₋₆ alkyl, C₁₋₆ heteroalkyl, C₁₋₆ haloalkyl, C₁₋₆ hydroxyalkyl, C₁₋₆ alkoxyl, amino, CN, -OH, C₁₋₆ aminoalkyl, cycloalkyl, heterocycloalkyl, aryl or heteroaryl. In some embodiments, each R²⁰ is independently halogen, C₁₋₃ alkyl, C₁₋₃ heteroalkyl, C₁₋₃ hydroxyalkyl, C₁₋₃ haloalkyl, C₁₋₃ alkoxyl, amino, CN, -OH, C₁₋₃ aminoalkyl, cycloalkyl, heterocycloalkyl, aryl or heteroaryl. In some embodiments, each R²⁰ is independently, optionally substituted with one or more R³¹. In some embodiments, each R²⁰ is independently halogen, C₁₋₃ alkyl, C₁₋₃ haloalkyl, C₁₋₃ hydroxyalkyl, C₁₋₃ heteroalkyl, C₁₋₃ alkoxyl, amino, CN, -OH, or C₁₋₃ aminoalkyl. In some embodiments, R²⁰ is methyl, ethyl, NH₂, CH₂OH, CH₂CH₂OH, CH₂CH₂F, CH₂CHF₂, or CH₂CH(CH₃)₂. In some embodiments, R²⁰ is NH₂ and methyl. In some embodiments, R²⁰ is NH₂ and CH₂OH. In some embodiments, R²⁰ is NH₂ and CH₂CH(CH₃)₂. In some embodiments, R²⁰ is NH₂ and CH₂CHF₂. In some embodiments, R²⁰ is NH₂ and CH₂CH₂F. In some embodiments, R²⁰ is NH₂ and CH₂CH₂OH. In some embodiments, R²⁰ is NH₂ and ethyl. In some embodiments, R²⁰ is 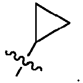 In some embodiments, R²⁰ is CH₃. In some embodiments, R²⁰ is OH. In some embodiments, R²⁰ is -OCH₃. In some embodiments, R²⁰ is NH₂. In some embodiments, R²⁰ is SH. In some embodiments, R²⁰ is CN. In some embodiments, R²⁰ is F. In some embodiments, R²⁰ is carbamyl. In some embodiments, R²⁰ is 4-6 membered heterocycloalkyl. In some embodiments, R²⁰ is 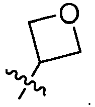 In some embodiments, R²⁰ is oxo.

In some embodiments of Formula (I), (IB), (Ia), (Ib), (Ic), (IIa), (IIb), (IIc) or (IId), R²⁴ is selected from the group consisting of hydrogen, oxo, azido, halogen, -CN, -NO₂, C₁₋₆ haloalkyl, C₁₋₆ alkyl, C₂₋₆ alkenyl, C₁₋₆ heteroalkyl, -NR^{c4}C(=O)R^{b4}, -NR^{c4}C(=O)OR^{b4}, C₃₋₁₀ cycloalkyl, C₆₋₁₀ aryl,-OR^{a4}, -NR^{c4}R^{d4}, and -C(=O)NR^{c4}R^{d4}, wherein the C₁₋₆ alkyl, C₁₋₆ haloalkyl, C₂₋₆ alkenyl, C₁₋₆ heteroalkyl,C₃₋₁₀ cycloalkyl, C₆₋₁₀ aryl are unsubstituted or substituted independently with 1, 2, 3, or 4 R^{20d} groups. In some embodiments, each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino.

In some embodiments of Formula (I), (IB), (Ia), (Ib), (Ic), (IIa), (IIb), (IIc) or (IId), R²⁴ is selected from the group consisting of hydrogen, oxo, azido, halogen, -CN, -NO₂, C₁₋₆ haloalkyl, C₁₋₆ alkyl, C₂₋₆ alkenyl, C₁₋₆ heteroalkyl, -NR^{c4}C(=O)R^{b4}, -NR^{c4}C(=O)OR^{b4}, C₃₋₁₀ cycloalkyl, C₆₋₁₀ aryl,-OR^{a4}, -SR^{a4}, -S(=O)₂R^{a4}, -NR^{c4}R^{d4}, and -C(=O)NR^{c4}R^{d4}, wherein the C₁₋₆ alkyl, C₁₋₆ haloalkyl, C₂₋₆ alkenyl, C₁₋₆ heteroalkyl,C₃₋₁₀ cycloalkyl, C₆₋₁₀ aryl are unsubstituted or substituted independently with 1, 2, 3, or 4 R^{20d} groups, wherein each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino.

In some embodiments of Formula (I), (IB), (Ia), (Ib), (Ic), (IIa), (IIb), (IIc) or (IId), R²⁴ is selected from the group consisting of hydrogen, oxo, azido, halogen, -CN, -NO₂, C₁₋₆ haloalkyl, C₁₋₆ alkyl, C₂₋₆ alkenyl, C₁₋₆ heteroalkyl, -NR^{c4}C(=O)R^{b4}, -NR^{c4}C(=O)OR^{b4}, C₃₋₁₀ cycloalkyl, C₆₋₁₀ aryl,-OR^{a4}, -SR^{a4}, -S(=O)₂R^{a4}, -NR^{c4}R^{d4}, and ^{_}C(=O)NR^{c4}R^{a4}, wherein the C₁₋₆ alkyl, C₁₋₆ haloalkyl, C₂₋₆ alkenyl, C₁₋₆ heteroalkyl,C₃₋₁₀ cycloalkyl, C₆₋₁₀ aryl are unsubstituted or substituted independently with 1, 2, 3, or 4 R^{20d} groups, wherein each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C_{1 4} hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino.

In some embodiments of Formula (I), (IB), (Ia), (Ib), (Ic), (IIa), (IIb), (IIc) or (IId), each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ heteroalkyl, C₁₋₄ cyanoalkyl, C₁₋₄ hydroxyalkyl, C₁₋₄ alkoxy,-(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein each of the alkyl, alkenyl, phenyl, cycloalkyl, and heteroalkyl is optionally substituted with 1, 2, 3, or 4 groups independently selected from OH, -SH, -CN, -NO₂, halogen, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, C₁₋₄ haloalkoxy, C₃₋₆ cycloalkyl, amino, oxo, C₁₋₄ alkylamino, and di(C₁₋₄ alkyl)amino. In some embodiments, R^{20d} is F. In some embodiments, R^{20d} is OH.

In some embodiments of Formula (I), (IB), (Ia), (Ib), (Ic), (IIa), (IIb), (IIc) or (IId), each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ heteroalkyl, C₁₋₄ cyanoalkyl, C₁₋₄ hydroxyalkyl, C₁₋₄ alkoxy,-(C₁₋₄ alkyl)-(C₁₋₄ alkoxy), C₁₋₄ haloalkoxy, C₃₋₆ cycloalkyl, phenyl, amino, C₁₋₄ alkylamino, di(C₁₋₄ alkyl)amino, and C₁₋₄ alkylcarbamyl, wherein each of the alkyl, alkenyl, phenyl, cycloalkyl, and heteroalkyl is optionally substituted with 1, 2, 3, or 4 groups independently selected from OH, -SH,-CN, -NO₂, halogen, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, C₁₋₄ haloalkoxy, C₃₋₆ cycloalkyl, amino, oxo, C₁₋₄ alkylamino, and di(C₁₋₄ alkyl)amino. In some embodiments of Formula (I), (IB), (Ia), (Ib), (Ic), (IIa), (IIb), (IIc) or (IId), each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ heteroalkyl, C₁₋₄ cyanoalkyl, C₁₋₄ hydroxyalkyl, C₁₋₄ alkoxy, -(C₁₋₄ alkyl)-(C₁₋₄ alkoxy), C₁₋₄ haloalkoxy, C₃₋₆ cycloalkyl, phenyl, amino, C₁₋₄ alkylamino, di(C₁₋₄ alkyl)amino, and C₁₋₄ alkylcarbamyl, wherein each of the alkyl, alkenyl, phenyl, cycloalkyl, and heteroalkyl is optionally substituted with 1, 2, 3, or 4 groups independently selected from OH, -SH, - CN, -NO₂, halogen, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, C₁₋₄ haloalkoxy, C₃₋₆ cycloalkyl, amino, and oxo.

In some embodiments of Formula (I), (IB), (Ia), (Ib), (Ic), (IIa), (IIb), (IIc) or (IId), each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, C₁₋₄ haloalkoxy, C₃₋₆ cycloalkyl, phenyl, amino, C₁₋₄ alkylamino, and di(C₁₋₄ alkyl)amino, wherein each of the alkyl, alkenyl, phenyl, cycloalkyl, and alkenyl is optionally substituted with 1, 2, 3, or 4 groups independently selected from OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, C₁₋₄ haloalkoxy, C₃₋₆ cycloalkyl, amino, C₁₋₄ alkylamino, and di(C₁₋₄ alkyl)amino. In some embodiments, each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, C₁₋₄ haloalkoxy, C₃₋₆ cycloalkyl.

In some embodiments of Formula (I), (IB), (Ia), (Ib), (Ic), (IIa), (IIb), (IIc) or (IId), each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ heteroalkyl, C₁₋₄ cyanoalkyl, C₁₋₄ hydroxyalkyl, C₁₋₄ alkoxy,-(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein alkyl, alkenyl, heteroalkyl, cycloalkyl, and phenyl are each optionally substituted with 1, 2, 3 or 4 R³² groups. In some embodiments, each R³² is individually-OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, or C₁₋₄ alkoxy. In some embodiments, R³² is -OH. In some embodiments, R³² is -SH. In some embodiments, R³² is -CN. In some embodiments, R³² is -NO₂. In some embodiments, R³² is halogen. In some embodiments, R³² is oxo. In some embodiments, R³² is C₁₋₄ alkyl. In some embodiments, R³² is C₂₋₄ alkenyl. In some embodiments, R³² is C₁₋₄ haloalkyl. In some embodiments, R³² is C₁₋₄ cyanoalkyl. In some embodiments, R³² is C₁₋₄ hydroxyalkyl. In some embodiments, R³² is C₁₋₄ alkoxy.

In some embodiments of Formula (I), (IB), (Ia), (Ib), (Ic), (IIa), (IIb), (IIc) or (IId), each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein alkyl, alkenyl, heteroalkyl, cycloalkyl, and phenyl are each optionally substituted with 1, 2, 3 or 4 R³² groups. In some embodiments, each R³² is individually oxo, halogen, methyl, ethyl, -CN, -CF₃, -OH, -OMe, -NH₂, or -NO₂. In some embodiments, R³² is oxo. In some embodiments, R³² halogen. In some embodiments, R³² methyl, ethyl. In some embodiments, R³² -CN. In some embodiments, R³² -CF₃. In some embodiments, R³² -OH. In some embodiments, R³²-OMe. In some embodiments, R³² -NH₂. In some embodiments, R³² -NO₂.

In some embodiments of Formula (I), (IB), (Ia), (Ib), (Ic), (IIa), (IIb), (IIc) or (IId), R²⁴ is-OR^{a4}. In some embodiments, R²⁴ is OH. In some embodiments, R²⁴ is -OCH₃. In some embodiments, R²⁴ is C₁₋₆ alkyl. In some embodiments, R²⁴ is C₁₋₆ alkyl, optionally substituted with 1, 2, 3, or 4 R^{20d} In some embodiments, R²⁴ is methyl. In some embodiments, R²⁴ is halo. In some embodiments, R²⁴ is fluoro, bromo, or chloro. In some embodiments, R²⁴ is F. In some embodiments, R²⁴ is Br. In some embodiments, R²⁴ is Cl. In some embodiments, R²⁴ is hydrogen. In some embodiments, R²⁴ is CN. In some embodiments, R²⁴ is C₃₋₁₀ cycloalkyl (e.g., cyclopropyl). In some embodiments, R²⁴ is phenyl. In some embodiments, R²⁴ is -NR^{c4}C(=O)R^{b4}. In some embodiments, R²⁴ is -NHC(=O)CH³. In some embodiments, R²⁴ is unsubstituted or substituted C₁₋₆ haloalkyl. In some embodiments, R²⁴ is CF₃, CHF₂, CH₂F, CH₂CH₂F or CH₂CF₃. In some embodiments, R²⁴ is CF₃. In some embodiments, R²⁴ is CHF₂. In some embodiments, R²⁴ is CH₂F.

In some embodiments of Formula (I), (IB), (Ia), (Ib), (Ic), (IIa), (IIb), (IIc) or (IId), R²⁴ is C₁₋₆ alkyl, which is optionally substituted with 1, 2, 3, or 4 R^{20d}. In some embodiments, R²⁴ is CH₂OH. In some embodiments, R²⁴ is C₂₋₆ alkenyl, which is optionally substituted with 1, 2, 3, or 4 R^{20d}. In some embodiments, R²⁴ is 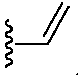 In some embodiments, R²⁴ is C₁₋₆ heteroalkyl. In some embodiments, R²⁴ is C₃₋₁₀ cycloalkyl, which is optionally substituted with 1, 2, 3, or 4 R^{20d}. In some embodiments, R²⁴ is 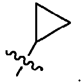 In some embodiments, R²⁴ is C₆₋₁₀ aryl, which is optionally substituted with 1, 2, 3, or 4 R^{20d}. In some embodiments, R²⁴ is -OR^{a4}. In some embodiments, R²⁴ is -SR^{a4}. In some embodiments, R²⁴ is SCH₃. In some embodiments, R²⁴ is -S(=O)₂R^{a4}. In some embodiments, R²⁴ is SO₂CH₃. In some embodiments, R²⁴ is -C(=O)R^{b4}. In some embodiments, R²⁴ is -C(=O)OR^{b4}. In some embodiments, R²⁴ is -NR^{c4}R^{d4}. In some embodiments, R²⁴ is -C(=O)NR^{C4}R^{d4}_{.} In some embodiments, R²⁴ is - OC(=O)NR^{c4}R^{d4}. In some embodiments, R²⁴ is -NR^{c4}C(=O)R^{b4}. In some embodiments, R²⁴ is 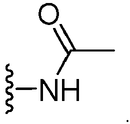 In some embodiments, R²⁴ is -NR^{c4}C(=O)OR^{b4}. In some embodiments, R²⁴ is -NR^{c4}C(=O)NR^{c4}R^{d4}. In some embodiments, R²⁴ is -NR^{c4}S(=O)₂R^{b4}. In some embodiments, R²⁴ is -NR^{c4}S(=O)₂NR^{c4}R^{d4}. In some embodiments, R²⁴ is -S(O)NR^{c4}R^{d4}. In some embodiments, R²⁴ is -S(O)₂NR^{c4}R^{d4}.

In some embodiments, the compound is of the Formula (IIa): 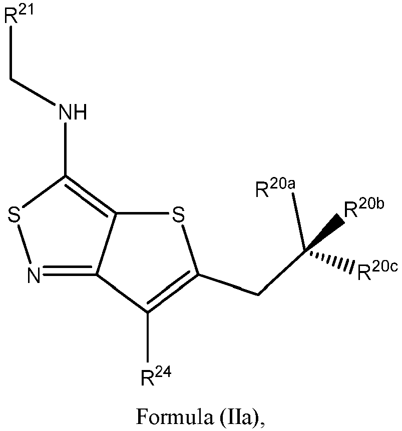 wherein each R^{20a}, R^{20b}, and R^{20c} is independently selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 5-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein each of the alkyl, alkenyl, alkynyl, alkoxy, heteroalkyl, cycloalkyl, aryl, heteroaryl, and heterocycloalkyl are each unsubstituted or substituted independently with 1, 2, or 3 R²⁰ groups,
or a pharmaceutically acceptable salt thereof.

In some embodiments, R^{20a} is methyl. In some embodiments, R^{20a} is ethyl. In some embodiments, R^{20a} is CH₂OH. In some embodiments, R^{20a} is CH₂CH₂OH. In some embodiments, R^{20a} is CH₂CH₂F. In some embodiments, R^{20a} is CH₂CHF₂. In some embodiments, R^{20a} is CH₂CH(CH₃)₂. In some embodiments, R^{20c} is NH₂. In some embodiments, R^{20b} is hydrogen.

In some embodiments, the compound is of the Formula (IIb): 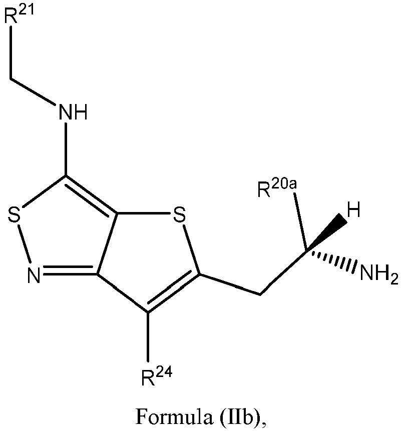 wherein R^{20a} is selected from the group consisting of OH, SH, CN, NO₂, halo, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 5-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein each of the alkyl, alkenyl, alkynyl, alkoxy, heteroalkyl, cycloalkyl, aryl, heteroaryl, and heterocycloalkyl are each unsubstituted or substituted independently with 1, 2, or 3 R²⁰ groups,
or a pharmaceutically acceptable salt thereof.

In some embodiments, R^{20a} is methyl. In some embodiments, R^{20a} is ethyl. In some embodiments, R^{20a} is CH₂OH. In some embodiments, R^{20a} is CH₂CH₂OH. In some embodiments, R^{20a} is CH₂CH₂F. In some embodiments, R^{20a} is CH₂CHF₂. In some embodiments, R^{20a} is CH₂CH(CH₃)₂.

In some embodiments, the compound is of the Formula (IIc):

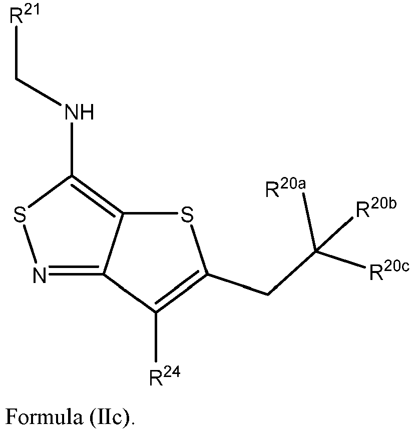

In some embodiments, the compound is of the Formula (IId):

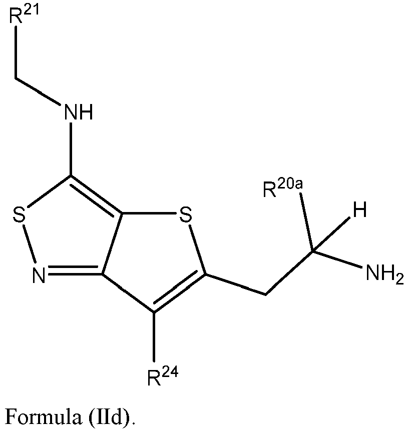

In some embodiments of Formula (IIa), (IIb), (IIc) or (IId), R^{20a} is methyl. In some embodiments, R^{20a} is ethyl. In some embodiments, R^{20a} is CH₂OH. In some embodiments, R^{20a} is CH₂CH₂OH. In some embodiments, R^{20a} is CH₂CH₂F. In some embodiments, R^{20a} is CH₂CHF₂. In some embodiments, R^{20a} is CH₂CH(CH₃)₂. In some embodiments, R^{20c} is NH₂. In some embodiments, R^{20b} is hydrogen. In some embodiments, R^{20a} is 4-6 membered heterocycloalkyl. In some embodiments, R^{20a} is -(C₁₋₃ alkylene)-C₃₋₁₀ cycloalkyl. In some embodiments, R^{20a} is -(C₁₋₃ alkylene)-4-10 membered heterocycloalkyl. In some embodiments, R^{20a} is -(C₁₋₃ heteroalkylene)-C₃₋₁₀ cycloalkyl. In some embodiments, R^{20a} is -(C₁₋₃heteroalkylene)-4-10 membered heterocycloalkyl. In some embodiments, the -C₃₋₁₀ cycloalkyl is 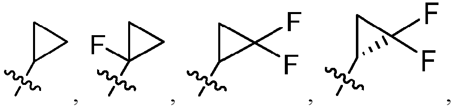 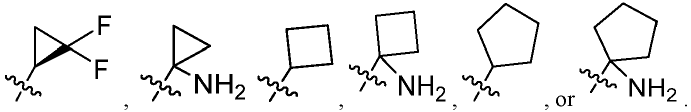 In some embodiments, the -C₃₋₁₀ cycloalkyl is a 3-5 membered ring, which is optionally substituted. In some embodiments, the - 4-10 membered heterocycloalkyl is 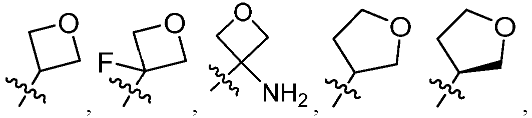 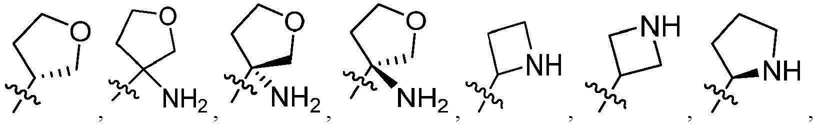 or 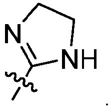 In some embodiments, the -4-10 membered heterocycloalkyl is a 4-5 membered ring, which is optionally substituted. In some embodiments, R^{20a} is C₁₋₄ heteroalkyl. In some embodiments, R^{20a} is C₁₋₄ alkyl. In some embodiments, R^{20a} is optionally substituted C₁₋₄ heteroalkyl. In some embodiments, R^{20a} is optionally substituted C₁₋₄ alkyl. In some embodiments of Formula (IIa), (IIb), (IIc) or (IId), R^{20a} is methyl. In some embodiments, R^{20a} is substituted with one or more R³¹. In some embodiments, R^{20a} is substituted with one or more R²⁰.

In some embodiments of Formula (IIa), (IIb), (IIc) or (IId), each R^{20a}, R^{20b}, and R^{20c} is independently selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-10 membered heteroaryl, 4-10 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino, wherein each of the alkyl, alkenyl, alkynyl, alkoxy, heteroalkyl, cycloalkyl, aryl, heteroaryl, and heterocycloalkyl are each unsubstituted or substituted independently with 1, 2, or 3 R²⁰ groups.

In some embodiments of Formula (IIa), (IIb), (IIc) or (IId), each R^{20a}, R^{20b}, and R^{20c} is independently selected from the group consisting of H, OH, SH, CN, NO₂, halo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-10 membered heteroaryl, 4-10 membered heterocycloalkyl, amino, C₁₋₄ alkylamino, and di(C₁₋₄ alkyl)amino, wherein each of the alkyl, alkenyl, alkynyl, alkoxy, heteroalkyl, cycloalkyl, aryl, heteroaryl, and heterocycloalkyl are each unsubstituted or substituted independently with 1, 2, or 3 R²⁰ groups. In some embodiments, R^{20a} is C₃₋₆ cycloalkyl, phenyl, 5-10 membered heteroaryl, or 4-10 membered heterocycloalkyl, each of which unsubstituted or substituted independently with 1, 2, or 3 R²⁰ groups. In some embodiments, R^{20a} is C₃₋₆ cycloalkyl, phenyl, 5-6 membered heteroaryl, or 4-6 membered heterocycloalkyl, each of which unsubstituted or substituted independently with 1, 2, or 3 R²⁰ groups.

In some embodiments of Formula (I), (IB), (Ia), (Ib), (Ic), (IIa), (IIb), (IIc) or (IId), each R^{a3}, R^{b3}, R^{c3}, R^{d3}, R^{a4}, R^{b4}, R^{c4}, and R^{d4} is independently selected from the group consisting of hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl, wherein the C₁₋₆ alkyl, C₂₋₆ alkenyl, C₂₋₆ alkynyl, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups.

In some embodiments of Formula (I), (IB), (Ia), (Ib), (Ic), (IIa), (IIb), (IIc) or (IId), R^{a3} is hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, or 4-10 membered heterocycloalkyl. In some embodiments, R³³ is hydrogen, C₁₋₆ alkyl, C₂₋₆ alkenyl, C₂₋₆ alkynyl, C₁₋₆ hydroxyalkyl, C₁₋₆ haloalkyl, or C₁₋₆ alkoxy. In some embodiments, R^{a3} is - (C₁₋₆ alkylene)-C₁₋₆ alkoxy, C₃₋₁₀ cycloalkyl, or -(C₁₋₆ alkylene)-C₃₋₁₀ cycloalkyl. In some embodiments, R^{a3} is C₆₋₁₀ aryl, 5-10 membered heteroaryl, or 4-10 membered heterocycloalkyl. In some embodiments, R^{a3} is hydrogen, C₁₋₆ alkyl, or C₁₋₆ haloalkyl. In some embodiments, R^{a3} is hydrogen. In some embodiments, R^{a3} is C₁₋₆ alkyl. In some embodiments, R^{a3} is methyl. In some embodiments, R^{a3} is ethyl. In some embodiments, R^{a3} is propyl. In some embodiments, R^{a3} is C₁₋₆ haloalkyl.

In some embodiments of Formula (I), (IB), (Ia), (Ib), (Ic), (IIa), (IIb), (IIc) or (IId), R^{b3} is hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, or 4-10 membered heterocycloalkyl. In some embodiments, R^{b3} is hydrogen, C₁₋₆ alkyl, C₂₋₆ alkenyl, C₂₋₆ alkynyl, C₁₋₆ hydroxyalkyl, C₁₋₆ haloalkyl, or C₁₋₆ alkoxy. In some embodiments, R^{b3} is - (C₁₋₆ alkylene)-C₁₋₆ alkoxy, C₃₋₁₀ cycloalkyl, or -(C₁₋₆ alkylene)-C₃₋₁₀ cycloalkyl. In some embodiments, R^{b3} is C₆₋₁₀ aryl, 5-10 membered heteroaryl, or 4-10 membered heterocycloalkyl. In some embodiments, R^{b3} is hydrogen, C₁₋₆ alkyl, or C₁₋₆ haloalkyl. In some embodiments, R^{b3} is hydrogen. In some embodiments, R^{b3} is C₁₋₆ alkyl. In some embodiments, R^{b3} is methyl. In some embodiments, R^{b3} is ethyl. In some embodiments, R^{b3} is propyl. In some embodiments, R^{b3} is C₁₋₆ haloalkyl.

In some embodiments of Formula (I), (IB), (Ia), (Ib), (Ic), (IIa), (IIb), (IIc) or (IId), R^{c3} is hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, or 4-10 membered heterocycloalkyl. In some embodiments, R^{c3} is hydrogen, C₁₋₆ alkyl, C₂₋₆ alkenyl, C₂₋₆ alkynyl, C₁₋₆ hydroxyalkyl, C₁₋₆ haloalkyl, or C₁₋₆ alkoxy. In some embodiments, R^{c3} is - (C₁₋₆ alkylene)-C₁₋₆ alkoxy, C₃₋₁₀ cycloalkyl, or -(C₁₋₆ alkylene)-C₃₋₁₀ cycloalkyl. In some embodiments, R^{c3} is C₆₋₁₀ aryl, 5-10 membered heteroaryl, or 4-10 membered heterocycloalkyl. In some embodiments, R^{c3} is hydrogen, C₁₋₆ alkyl, or C₁₋₆ haloalkyl. In some embodiments, R^{c3} is hydrogen. In some embodiments, R^{c3} is C₁₋₆ alkyl. In some embodiments, R^{c3} is methyl. In some embodiments, R^{c3} is ethyl. In some embodiments, R^{c3} is propyl. In some embodiments, R^{c3} is C₁₋₆ haloalkyl.

In some embodiments of Formula (I), (IB), (Ia), (Ib), (Ic), (IIa), (IIb), (IIc) or (IId), R^{d3} is hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, or 4-10 membered heterocycloalkyl. In some embodiments, R^{d3} is hydrogen, C₁₋₆ alkyl, C₂₋₆ alkenyl, C₂₋₆ alkynyl, C₁₋₆ hydroxyalkyl, C₁₋₆ haloalkyl, or C₁₋₆ alkoxy. In some embodiments, R^{d3} is - (C₁₋₆ alkylene)-C₁₋₆ alkoxy, C₃₋₁₀ cycloalkyl, or -(C₁₋₆ alkylene)-C₃₋₁₀ cycloalkyl. In some embodiments, R^{d3} is C₆₋₁₀ aryl, 5-10 membered heteroaryl, or 4-10 membered heterocycloalkyl. In some embodiments, R^{d3} is hydrogen, C₁₋₆ alkyl, or C₁₋₆ haloalkyl. In some embodiments, R^{d3} is hydrogen. In some embodiments, R^{d3} is C₁₋₆ alkyl. In some embodiments, R^{d3} is methyl. In some embodiments, R^{d3} is ethyl. In some embodiments, R^{d3} is propyl. In some embodiments, R^{d3} is C₁₋₆ haloalkyl.

In some embodiments of Formula (I), (IB), (Ia), (Ib), (Ic), (IIa), (IIb), (IIc) or (IId), R^{c3} and R^{d3} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups. In some embodiments, R^{c3} and R^{d3} together with the N atom to which they are connected, come together to form a 5-10 membered heteroaryl or 4-10 membered heterocycloalkyl.

In some embodiments of Formula (I), (IB), (Ia), (Ib), (Ic), (IIa), (IIb), (IIc) or (IId), R^{a4} is hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, or 4-10 membered heterocycloalkyl. In some embodiments, R^{a4} is hydrogen, C₁₋₆ alkyl, C₂₋₆ alkenyl, C₂₋₆ alkynyl, C₁₋₆ hydroxyalkyl, C₁₋₆ haloalkyl, or C₁₋₆ alkoxy. In some embodiments, R^{a4} is - (C₁₋₆ alkylene)-C₁₋₆ alkoxy, C₃₋₁₀ cycloalkyl, or -(C₁₋₆ alkylene)-C₃₋₁₀ cycloalkyl. In some embodiments, R^{a4} is C₆₋₁₀ aryl, 5-10 membered heteroaryl, or 4-10 membered heterocycloalkyl. In some embodiments, R^{a4} is hydrogen, C₁₋₆ alkyl, or C₁₋₆ haloalkyl. In some embodiments, R^{a4} is hydrogen. In some embodiments, R^{a4} is C₁₋₆ alkyl. In some embodiments, R^{a4} is methyl. In some embodiments, R^{a4} is ethyl. In some embodiments, R^{a4} is propyl. In some embodiments, R^{a4} is C₁₋₆ haloalkyl.

In some embodiments of Formula (I), (IB), (Ia), (Ib), (Ic), (IIa), (IIb), (IIc) or (IId), R^{b4} is hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, or 4-10 membered heterocycloalkyl. In some embodiments, R^{b4} is hydrogen, C₁₋₆ alkyl, C₂₋₆ alkenyl, C₂₋₆ alkynyl, C₁₋₆ hydroxyalkyl, C₁₋₆ haloalkyl, or C₁₋₆ alkoxy. In some embodiments, R^{b4} is - (C₁₋₆ alkylene)-C₁₋₆ alkoxy, C₃₋₁₀ cycloalkyl, or -(C₁₋₆ alkylene)-C₃₋₁₀ cycloalkyl. In some embodiments, R^{b4} is C₆₋₁₀ aryl, 5-10 membered heteroaryl, or 4-10 membered heterocycloalkyl. In some embodiments, R^{b4} is hydrogen, C₁₋₆ alkyl, or C₁₋₆ haloalkyl. In some embodiments, R^{b4} is hydrogen. In some embodiments, R^{b4} is C₁₋₆ alkyl. In some embodiments, R^{b4} is methyl. In some embodiments, R^{b4} is ethyl. In some embodiments, R^{b4} is propyl. In some embodiments, R^{b4} is C₁₋₆ haloalkyl.

In some embodiments of Formula (I), (IB), (Ia), (Ib), (Ic), (IIa), (IIb), (IIc) or (IId), R^{c4} is hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, or 4-10 membered heterocycloalkyl. In some embodiments, R^{c4} is hydrogen, C₁₋₆ alkyl, C₂₋₆ alkenyl, C₂₋₆ alkynyl, C₁₋₆ hydroxyalkyl, C₁₋₆ haloalkyl, or C₁₋₆ alkoxy. In some embodiments, R^{c4} is - (C₁₋₆ alkylene)-C₁₋₆ alkoxy, C₃₋₁₀ cycloalkyl, or -(C₁₋₆ alkylene)-C₃₋₁₀ cycloalkyl. In some embodiments, R^{c4} is C₆₋₁₀ aryl, 5-10 membered heteroaryl, or 4-10 membered heterocycloalkyl. In some embodiments, R^{c4} is hydrogen, C₁₋₆ alkyl, or C₁₋₆ haloalkyl. In some embodiments, R^{c4} is hydrogen. In some embodiments, R^{c4} is C₁₋₆ alkyl. In some embodiments, R^{c4} is methyl. In some embodiments, R^{c4} is ethyl. In some embodiments, R^{c4} is propyl. In some embodiments, R^{c4} is C₁₋₆ haloalkyl.

In some embodiments of Formula (I), (IB), (Ia), (Ib), (Ic), (IIa), (IIb), (IIc) or (IId), R^{d4} is hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, or 4-10 membered heterocycloalkyl. In some embodiments, R^{d4} is hydrogen, C₁₋₆ alkyl, C₂₋₆ alkenyl, C₂₋₆ alkynyl, C₁₋₆ hydroxyalkyl, C₁₋₆ haloalkyl, or C₁₋₆ alkoxy. In some embodiments, R^{d4} is - (C₁₋₆ alkylene)-C₁₋₆ alkoxy, C₃₋₁₀ cycloalkyl, or -(C₁₋₆ alkylene)-C₃₋₁₀ cycloalkyl. In some embodiments, R^{d4} is C₆₋₁₀ aryl, 5-10 membered heteroaryl, or 4-10 membered heterocycloalkyl. In some embodiments, R^{d4} is hydrogen, C₁₋₆ alkyl, or C₁₋₆ haloalkyl. In some embodiments, R^{d4} is hydrogen. In some embodiments, R^{d4} is C₁₋₆ alkyl. In some embodiments, R^{d4} is methyl. In some embodiments, R^{d4} is ethyl. In some embodiments, R^{d4} is propyl. In some embodiments, R^{d4} is C₁₋₆ haloalkyl.

In some embodiments of Formula (I), (IB), (Ia), (Ib), (Ic), (IIa), (IIb), (IIc) or (IId), R^{c4} and R^{d4} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups. In some embodiments, R^{c4} and R^{d4} together with the N atom to which they are connected, come together to form a 5-10 membered heteroaryl or 4-10 membered heterocycloalkyl.

In some embodiments, the compound is selected from Table 1.

In some embodiments, a SMSM described herein, possesses one or more stereocenters and each stereocenter exists independently in either the R or S configuration. The compounds presented herein include all diastereomeric, enantiomeric, and epimeric forms as well as the appropriate mixtures thereof. The compounds and methods provided herein include all cis, trans, syn, anti, entgegen (E), and zusammen (Z) isomers as well as the appropriate mixtures thereof. In certain embodiments, compounds described herein are prepared as their individual stereoisomers by reacting a racemic mixture of the compound with an optically active resolving agent to form a pair of diastereoisomeric compounds/salts, separating the diastereomers and recovering the optically pure enantiomers. In some embodiments, resolution of enantiomers is carried out using covalent diastereomeric derivatives of the compounds described herein. In another embodiment, diastereomers are separated by separation/resolution techniques based upon differences in solubility. In other embodiments, separation of stereoisomers is performed by chromatography or by the forming diastereomeric salts and separation by recrystallization, or chromatography, or any combination thereof. Jean Jacques, Andre Collet, Samuel H. Wilen, "Enantiomers, Racemates and Resolutions", John Wiley and Sons, Inc., 1981. In one aspect, stereoisomers are obtained by stereoselective synthesis.

In some embodiments, compounds described herein are prepared as prodrugs. A "prodrug" refers to an agent that is converted into the parent drug *in vivo.* Prodrugs are often useful because, in some situations, they may be easier to administer than the parent drug. They may, for instance, be bioavailable by oral administration whereas the parent is not. The prodrug may also have improved solubility in pharmaceutical compositions over the parent drug. In some embodiments, the design of a prodrug increases the effective water solubility. An example, without limitation, of a prodrug is a compound described herein, which is administered as an ester (the "prodrug") to facilitate transmittal across a cell membrane where water solubility is detrimental to mobility, but which then is metabolically hydrolyzed to the carboxylic acid, the active entity, once inside the cell where water-solubility is beneficial. A further example of a prodrug might be a short peptide (polyaminoacid) bonded to an acid group where the peptide is metabolized to reveal the active moiety. In certain embodiments, upon *in vivo* administration, a prodrug is chemically converted to the biologically, pharmaceutically or therapeutically active form of the compound. In certain embodiments, a prodrug is enzymatically metabolized by one or more steps or processes to the biologically, pharmaceutically or therapeutically active form of the compound.

In one aspect, prodrugs are designed to alter the metabolic stability or the transport characteristics of a drug, to mask side effects or toxicity, to improve the flavor of a drug or to alter other characteristics or properties of a drug. By virtue of knowledge of pharmacokinetic, pharmacodynamic processes and drug metabolism *in vivo,* once a pharmaceutically active compound is known, the design of prodrugs of the compound is possible. (see, for example, Nogrady (1985) Medicinal Chemistry A Biochemical Approach, Oxford University Press, New York, pages 388-392; Silverman (1992), The Organic Chemistry of Drug Design and Drug Action, Academic Press, Inc., San Diego, pages 352-401, Rooseboom et al., Pharmacological Reviews, 56:53-102, 2004; Aesop Cho, "Recent Advances in Oral Prodrug Discovery", Annual Reports in Medicinal Chemistry, Vol. 41, 395-407, 2006; T. Higuchi and V. Stella, Pro-drugs as Novel Delivery Systems, Vol. 14 of the A.C.S. Symposium Series).

In some cases, some of the herein-described compounds may be a prodrug for another derivative or active compound.

In some embodiments, sites on the aromatic ring portion of compounds described herein are susceptible to various metabolic reactions Therefore incorporation of appropriate substituents on the aromatic ring structures will reduce, minimize or eliminate this metabolic pathway. In specific embodiments, the appropriate substituent to decrease or eliminate the susceptibility of the aromatic ring to metabolic reactions is, by way of example only, a halogen, or an alkyl group.

In another embodiment, the compounds described herein are labeled isotopically (e.g. with a radioisotope) or by another other means, including, but not limited to, the use of chromophores or fluorescent moieties, bioluminescent labels, or chemiluminescent labels.

Compounds described herein include isotopically labeled compounds, which are identical to those recited in the various formulae and structures presented herein, but for the fact that one or more atoms are replaced by an atom having an atomic mass or mass number different from the atomic mass or mass number usually found in nature. Examples of isotopes that can be incorporated into the present compounds include isotopes of hydrogen, carbon, nitrogen, oxygen, sulfur, fluorine and chlorine, such as, for example, ²H, ³H, ¹³C, ¹⁴C, ¹⁵N, ¹⁸O, ¹⁷O, ³⁵S, ¹⁸F, ³⁶Cl. In one aspect, isotopically labeled compounds described herein, for example those into which radioactive isotopes such as ³H and ¹⁴C are incorporated, are useful in drug and/or substrate tissue distribution assays. In one aspect, substitution with isotopes such as deuterium affords certain therapeutic advantages resulting from greater metabolic stability, such as, for example, increased *in vivo* half-life or reduced dosage requirements.

In some embodiments of a compound disclosed herein, one or more of R²⁰, R^{20a}, R^{20b}, R^{20c}, R^{20d}, R³¹, R³², R²¹, R²³, R²⁴, R^{a3}, R^{b3}, R^{c3}, R^{d3}, R^{a4}, R^{b4}, R^{c4} and R^{d4} groups comprise deuterium at a percentage higher than the natural abundance of deuterium.

In some embodiments of a compound disclosed herein, one or more ¹H are replaced with one or more deuteriums in one or more of the following groups R²⁰, R^{20a}, R^{20b}, R^{20c}, R^{20d}, R³¹, R³², R²¹, R²³, R²⁴, R^{a3}, R^{b3}, R^{c3}, R^{d3}, R^{a4}, R^{b4}, R^{c4} and R^{d4}.

In some embodiments of a compound disclosed herein, the abundance of deuterium in each of R²⁰, R^{20a}, R^{20b}, R^{20c}, R^{20d}, R³¹, R³², R²¹, R²³, R²⁴, R^{a3}, R^{b3}, R^{c3}, R^{d3}, R^{a4}, R^{b4}, R^{c4} and R^{d4} is independently at least 1%, at least 10%, at least 20%, at least 30%, at least 40%, at least 50%, at least 60%, at least 70%, at least 80%, at least 90%, or 100% by molar.

In additional or further embodiments, the compounds described herein are metabolized upon administration to an organism in need to produce a metabolite that is then used to produce a desired effect, including a desired therapeutic effect.

Compounds described herein may be formed as, and/or used as, pharmaceutically acceptable salts. The type of pharmaceutical acceptable salts, include, but are not limited to: (1) acid addition salts, formed by reacting the free base form of the compound with a pharmaceutically acceptable: inorganic acid, such as, for example, hydrochloric acid, hydrobromic acid, sulfuric acid, phosphoric acid, metaphosphoric acid, and the like; or with an organic acid, such as, for example, acetic acid, propionic acid, hexanoic acid, cyclopentanepropionic acid, glycolic acid, pyruvic acid, lactic acid, malonic acid, succinic acid, malic acid, maleic acid, fumaric acid, trifluoroacetic acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, toluenesulfonic acid, 2-naphthalenesulfonic acid, 4-methylbicyclo-[2.2.2]oct-2-ene-1-carboxylic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, butyric acid, phenylacetic acid, phenylbutyric acid, valproic acid, and the like; (2) salts formed when an acidic proton present in the parent compound is replaced by a metal ion, *e.g.,* an alkali metal ion (*e.g.* lithium, sodium, potassium), an alkaline earth ion (*e.g*. magnesium, or calcium), or an aluminum ion. In some cases, compounds described herein may coordinate with an organic base, such as, but not limited to, ethanolamine, diethanolamine, triethanolamine, tromethamine, N-methylglucamine, dicyclohexylamine, tris(hydroxymethyl)methylamine. In other cases, compounds described herein may form salts with amino acids such as, but not limited to, arginine, lysine, and the like. Acceptable inorganic bases used to form salts with compounds that include an acidic proton, include, but are not limited to, aluminum hydroxide, calcium hydroxide, potassium hydroxide, sodium carbonate, sodium hydroxide, and the like.

It should be understood that a reference to a pharmaceutically acceptable salt includes the solvent addition forms, particularly solvates. Solvates contain either stoichiometric or non-stoichiometric amounts of a solvent and may be formed during the process of crystallization with pharmaceutically acceptable solvents such as water, ethanol, and the like. Hydrates are formed when the solvent is water, or alcoholates are formed when the solvent is alcohol. In some embodiments, solvates of compounds described herein are conveniently prepared or formed during the processes described herein. In addition, the compounds provided herein can exist in unsolvated as well as solvated forms. In general, the solvated forms are considered equivalent to the unsolvated forms for the purposes of the compounds and methods provided herein.

In some embodiments, a SMSM has a molecular weight of at most about 2000 Daltons, 1500 Daltons, 1000 Daltons or 900 Daltons. In some embodiments, a SMSM has a molecular weight of at least 100 Daltons, 200 Daltons, 300 Daltons, 400 Daltons or 500 Daltons. In some embodiments, a SMSM does not comprise a phosphodiester linkage. In some embodiments, a SMSM is a compound with a structure set forth in Table 1 below.

**Table 1: Exemplary SMSM compounds**

| Compound Number | Structure | Iupac Name |
|---|---|---|
| 1 | 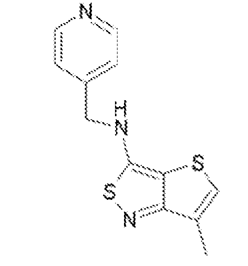 | [(4-pyridyl)methyl](8-methyl-3,6-dithia-2-azabicyclo[3.3.0]oct a-1,4,7-trien-4-yl)amine |
| 2 | 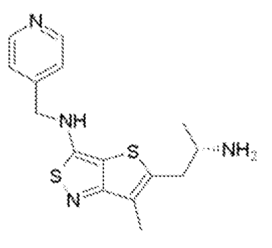 | 7-[(S)-2-aminopropyl]-8-methyl-4-{[(4-pyridyl)methyl]amin o}-3,6-dithia-2-azabicyclo[3.3.0]oct a-1,4,7-triene |
| 3 | 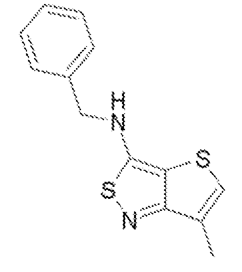 | (benzyl)(8-methyl-3,6-dithia-2-azabicyclo[3.3.0]oct a-1,4,7-trien-4-yl)amine |
| 4 | 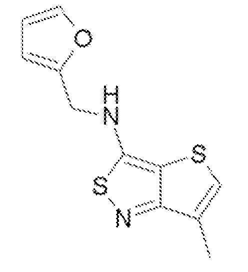 | (furfuryl)(8-methyl-3,6-dithia-2-azabicyclo[3.3.0]oct a-1,4,7-trien-4-yl)amine |
| 5 | 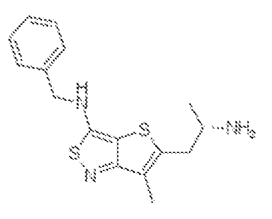 | 7-[(S)-2-aminopropyl]-4-benzylamino-8-methyl-3,6-dithia-2-azabicyclo[3.3.0]oct a-1,4,7-triene |
| 6 | 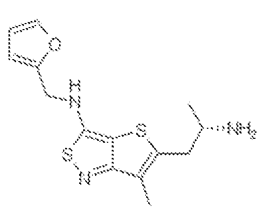 | 7-[(S)-2-aminopropyl]-4-(furfurylamino)-8-methyl-3,6-dithia-2-azabicyclo[3.3.0]oct a-1,4,7-triene |
| 7 | 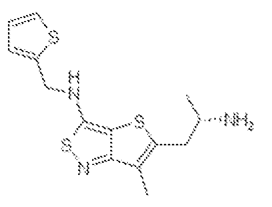 | 7-[(S)-2-aminopropyl]-8-methyl-4-thenylamino-3,6-dithia-2-azabicyclo[3.3.0]oct a-1,4,7-triene |
| 8 | 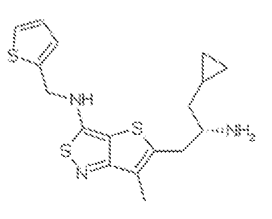 | 7-[(S)-2-amino-3-cyclopropylpropyl]-8-methyl-4-thenylamino-3,6-dithia-2-azabicyclo[3.3.0]oct a-1,4,7-triene |
| 9 | 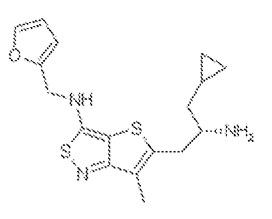 | 7-[(S)-2-amino-3-cyclopropylpropyl]-4-(furfurylamino)-8-methyl-3,6-dithia-2-azabicyclo[3.3.0]oct a-1,4,7-triene |
| 10 | 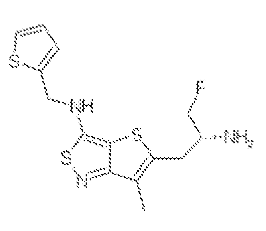 | 7-[(R)-2-amino-3-fluoropropyl]-8-methyl-4-thenylamino-3,6-dithia-2-azabicyclo[3.3.0]oct a-1,4,7-triene |
| 11 | 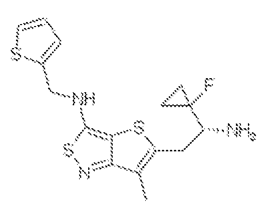 | 7-[(R)-2-amino-2-(1-fluorocyclopropyl)et hyl]-8-methyl-4-thenylamino-3,6-dithia-2-azabicyclo[3.3.0]oct a-1,4,7-triene |
| 12 | 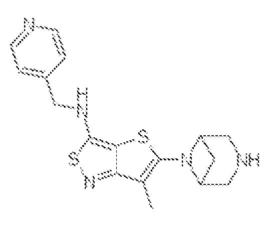 | [(4-pyridyl)methyl]{7-(3,6-diazabicyclo[3.1.1]h ept-6-yl)-8-methyl-3,6-dithia-2-azabicyclo[3.3.0]oct a-1,4,7-trien-4-yl}amine |
| 13 | 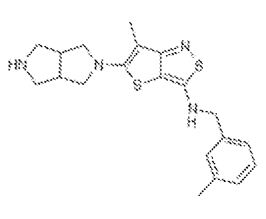 | [(m-tolyl)methyl]{7-(3,7-diazabicyclo[3.3.0]o ct-3-yl)-8-methyl-3,6-dithia-2-azabicyclo[3.3.0]oct a-1,4,7-trien-4-yl}amine |
| 14 | 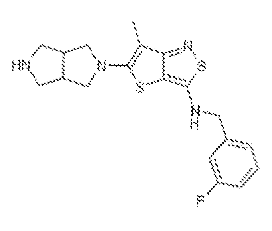 | [(m-fluorophenyl)methyl ]{7-(3,7-diazabicyclo[3.3.0]o ct-3-yl)-8-methyl-3,6-dithia-2-azabicyclo[3.3.0]oct a-1,4,7-trien-4-yl}amine |
| 15 | 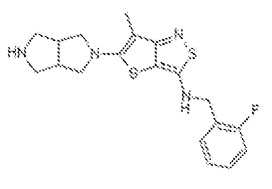 | [(o-fluorophenyl)methyl ]{7-(3,7-diazabicyclo[3.3.0]o ct-3-yl)-8-methyl-3,6-dithia-2-azabicyclo[3.3.0]oct a-1,4,7-trien-4-yl}amine |
| 16 | 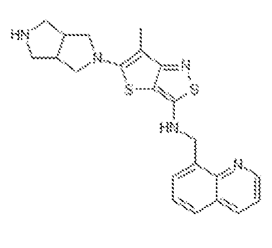 | [(8-quinolyl)methyl]{7-(3,7-diazabicyclo[3.3.0]o ct-3-yl)-8-methyl-3,6-dithia-2-azabicyclo[3.3.0]oct a-1,4,7-trien-4-yl}amine |
| 17 | 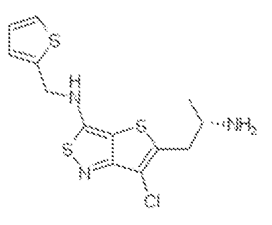 | 7-[(S)-2-aminopropyl]-8-chloro-4-thenylamino-3,6-dithia-2-azabicyclo[3.3.0]oct a-1,4,7-triene |
| 18 | 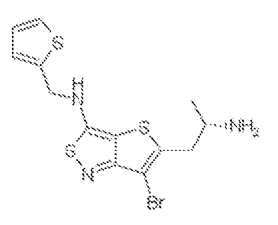 | 7-[(S)-2-aminopropyl]-8-bromo-4-thenylamino-3,6-dithia-2-azabicyclo[3.3.0]oct a-1,4,7-triene |
| 19 | 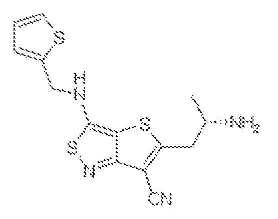 | 7-[(S)-2-aminopropyl]-4-thenylamino-3,6-dithia-2-azabicyclo[3.3.0]oct a-1,4,7-triene-8-carbonitrile |
| 20 | 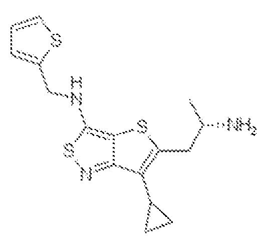 | 7-[(S)-2-aminopropyl]-8-cyclopropyl-4-thenylamino-3,6-dithia-2-azabicyclo[3.3.0]oct a-1,4,7-triene |
| 21 | 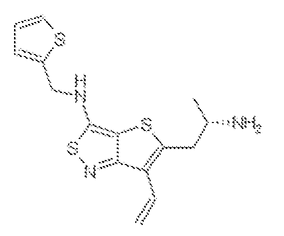 | 7-[(S)-2-aminopropyl]-4-thenylamino-8-vinyl-3,6-dithia-2-azabicyclo[3.3.0]oct a-1,4,7-triene |
| 22 | 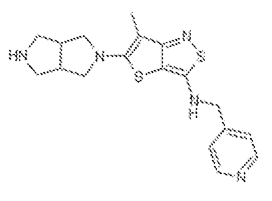 | [(4-pyridyl)methyl] {7-(3,7-diazabicyclo[3.3.0]o ct-3-yl)-8-methyl-3,6-dithia-2-azabicyclo[3.3.0]oct a-1,4,7-trien-4-yl}amine |
| 23 | 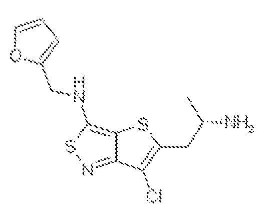 | 7-[(S)-2-aminopropyl]-8-chloro-4-(furfurylamino)-3,6-dithia-2-azabicyclo[3.3.0]oct a-1,4,7-triene |
| 24 | 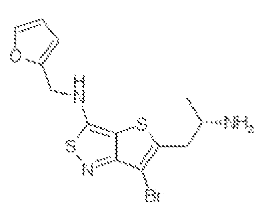 | 7-[(S)-2-aminopropyl]-8-bromo-4-(furfurylamino)-3,6-dithia-2-azabicyclo[3.3.0]oct a-1,4,7-triene |
| 25 | 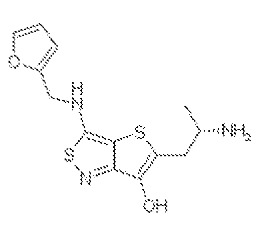 | 7-[(S)-2-aminopropyl]-4-(furfurylamino)-3,6-dithia-2-azabicyclo[3.3.0]oct a-1,4,7-trien-8-ol |
| 26 | 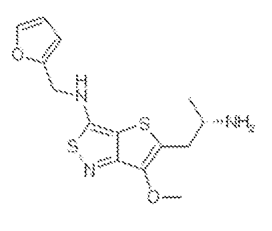 | 7-[(S)-2-aminopropyl]-4-(furfurylamino)-8-methoxy-3,6-dithia-2-azabicyclo[3.3.0]oct a-1,4,7-triene |
| 27 | 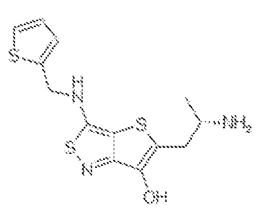 | 7-[(S)-2-aminopropyl]-4-thenylamino-3,6-dithia-2-azabicyclo[3.3.0]oct a-1,4,7-trien-8-ol |
| 28 | 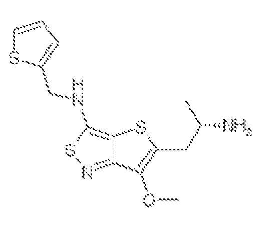 | 7-[(S)-2-aminopropyl]-8-methoxy-4-thenylamino-3,6-dithia-2-azabicyclo[3.3.0]oct a-1,4,7-triene |
| 29 | 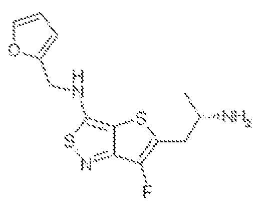 | 7-[(S)-2-aminopropyl]-8-fluoro-4-(furfurylamino)-3,6-dithia-2-azabicyclo[3.3.0]oct a-1,4,7-triene |
| 30 | 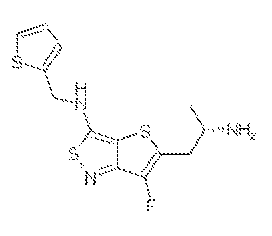 | 7-[(S)-2-aminopropyl]-8-fluoro-4-thenylamino-3,6-dithia-2-azabicyclo[3.3.0]oct a-1,4,7-triene |
| 31 | 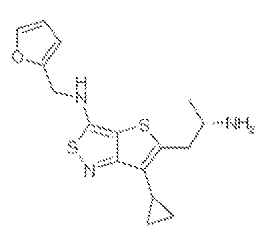 | 7-[(S)-2-aminopropyl]-8-cyclopropyl-4-(furfurylamino)-3,6-dithia-2-azabicyclo[3.3.0]oct a-1,4,7-triene |
| 32 | 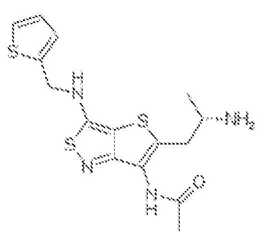 | N-{7-[(S)-2-aminopropyl]-4-thenylamino-3,6-dithia-2-azabicyclo[3.3.0]oct a-1,4,7-trien-8-yl}acetamide |
| 33 | 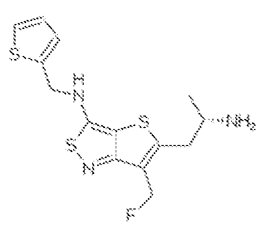 | 7-[(S)-2-aminopropyl]-8-(fluoromethyl)-4-thenylamino-3,6-dithia-2-azabicyclo[3.3.0]oct a-1,4,7-triene |
| 34 | 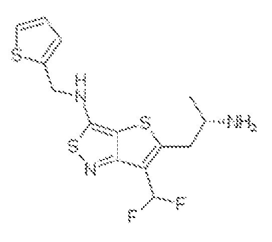 | 7-[(S)-2-aminopropyl]-8-(difluoromethyl)-4-thenylamino-3,6-dithia-2-azabicyclo[3.3.0]oct a-1,4,7-triene |
| 35 | 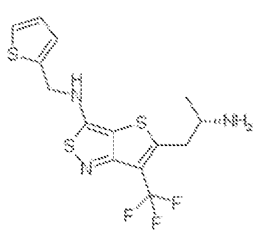 | 7-[(S)-2-aminopropyl]-4-thenylamino-8-(trifluoromethyl)-3,6-dithia-2-azabicyclo[3.3.0]oct a-1,4,7-triene |
| 36 | 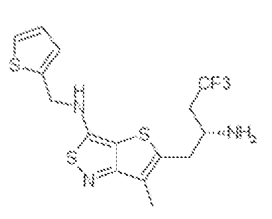 | 7-[(S)-2-amino-4,4,4-trifluorobutyl]-8-methyl-4-thenylamino-3,6-dithia-2-azabicyclo[3.3.0]oct a-1,4,7-triene |
| 37 | 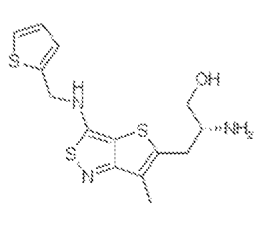 | (R)-2-amino-3-(8-methyl-4-thenylamino-3,6-dithia-2-azabicyclo[3.3.0]oct a-1,4,7-trien-7-yl)-1-propanol |
| 38 | 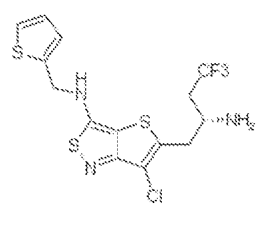 | 7-[(S)-2-amino-4,4,4-trifluorobutyl]-8-chloro-4-thenylamino-3,6-dithia-2-azabicyclo[3.3.0]oct a-1,4,7-triene |
| 39 | 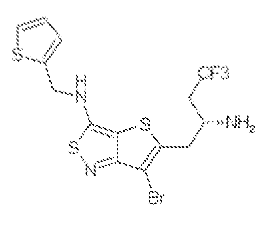 | 7-[(S)-2-amino-4,4,4-trifluorobutyl]-8-bromo-4-thenylamino-3,6-dithia-2-azabicyclo[3.3.0]oct a-1,4,7-triene |
| 40 | 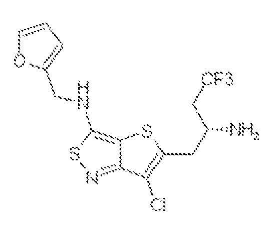 | 7-[(S)-2-amino-4,4,4-trifluorobutyl]-8-chloro-4-(furfurylamino)-3,6-dithia-2-azabicyclo[3.3.0]oct a-1,4,7-triene |
| 41 | 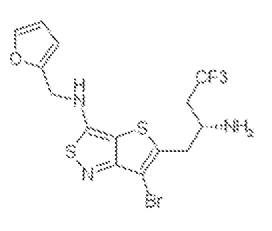 | 7-[(S)-2-amino-4,4,4-trifluorobutyl]-8-bromo-4-(furfurylamino)-3,6-dithia-2-azabicyclo[3.3.0]oct a-1,4,7-triene |
| 42 | 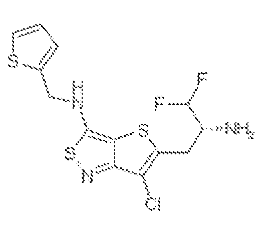 | 7-[(R)-2-amino-3,3-difluoropropyl]-8-chloro-4-thenylamino-3,6-dithia-2-azabicyclo[3.3.0]oct a-1,4,7-triene |
| 43 | 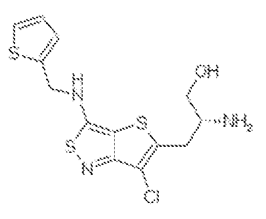 | (R)-2-amino-3-(8-chloro-4-thenylamino-3,6-dithia-2-azabicyclo[3.3.0]oct a-1,4,7-trien-7-yl)-1-propanol |
| 44 | 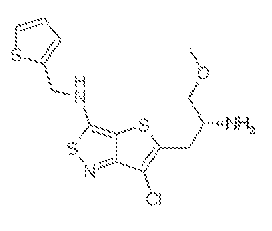 | 7-[(R)-2-amino-3-methoxypropyl]-8-chloro-4-thenylamino-3,6-dithia-2-azabicyclo[3.3.0]oct a-1,4,7-triene |
| 45 | 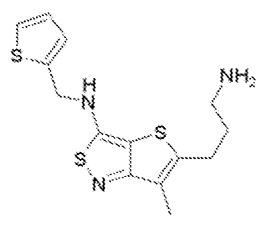 | 7-(3-aminopropyl)-8-methyl-4-thenylamino-3,6-dithia-2-azabicyclo[3.3.0]oct a-1,4,7-triene |
| 46 | 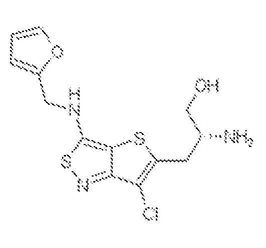 | (R)-2-amino-3-{8-chloro-4-(furfurylamino)-3,6-dithia-2-azabicyclo[3.3.0]oct a-1,4,7-trien-7-yl}-1-propanol |
| 47 | 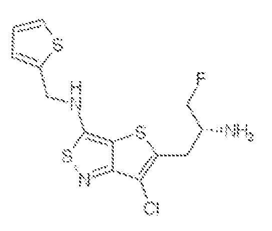 | 7-[(R)-2-amino-3-fluoropropyl]-8-chloro-4-thenylamino-3,6-dithia-2-azabicyclo[3.3.0]oct a-1,4,7-triene |
| 48 | 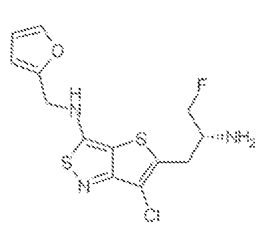 | 7-[(R)-2-amino-3-fluoropropyl]-8-chloro-4-(furfurylamino)-3,6-dithia-2-azabicyclo[3.3.0]oct a-1,4,7-triene |
| 49 | 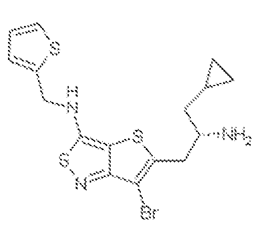 | 7-[(S)-2-amino-3-cyclopropylpropyl]-8-bromo-4-thenylamino-3,6-dithia-2-azabicyclo[3.3.0]oct a-1,4,7-triene |
| 50 | 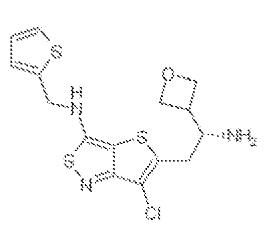 | 7-[(R)-2-amino-2-(3-oxetanyl)ethyl]-8-chloro-4-thenylamino-3,6-dithia-2-azabicyclo[3.3.0]oct a-1,4,7-triene |
| 51 | 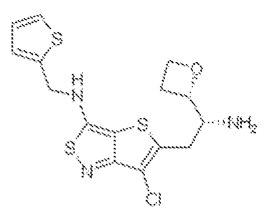 | 7-{(R)-2-[(S)-2-oxetanyl]-2-aminoethyl }-8-chloro-4-thenylamino-3,6-dithia-2-azabicyclo[3.3.0]oct a-1,4,7-triene |
| 52 | 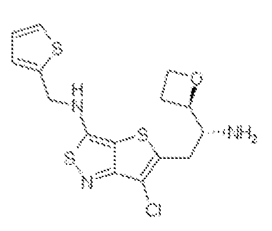 | 7-{(R)-2-[(R)-2-oxetanyl]-2-aminoethyl }-8-chloro-4-thenylamino-3,6-dithia-2-azabicyclo[3.3.0]oct a-1,4,7-triene |
| 53 | 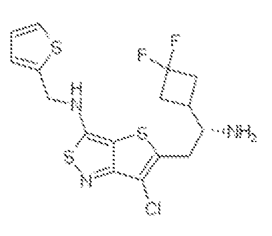 | 7-[(R)-2-amino-2-(3,3-difluorocyclobutyl)e thyl]-8-chloro-4-thenylamino-3,6-dithia-2-azabicyclo[3.3.0]oct a-1,4,7-triene |
| 54 | 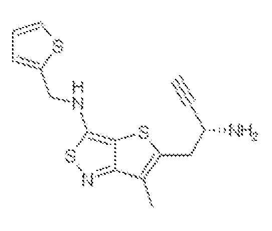 | 7-[(R)-2-amino-3-butynyl]-8-methyl-4-thenylamino-3,6-dithia-2-azabicyclo[3.3.0]oct a-1,4,7-triene |
| 55 | 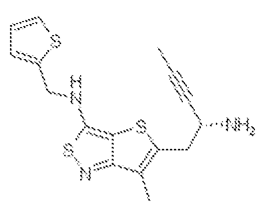 | 7-[(R)-2-amino-3-pentynyl]-8-methyl-4-thenylamino-3,6-dithia-2-azabicyclo[3.3.0]oct a-1,4,7-triene |
| 56 | 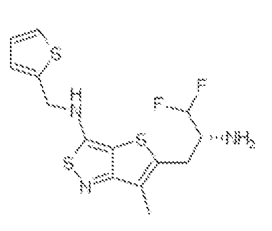 | 7-[(R)-2-amino-3,3-difluoropropyl]-8-methyl-4-thenylamino-3,6-dithia-2-azabicyclo[3.3.0]oct a-1,4,7-triene |
| 57 | 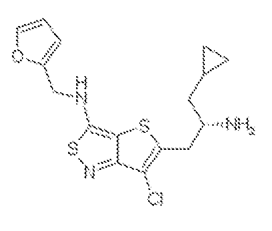 | 7-[(S)-2-amino-3-cyclopropylpropyl]-8-chloro-4-(furfurylamino)-3,6-dithia-2-azabicyclo[3.3.0]oct a-1,4,7-triene |
| 58 | 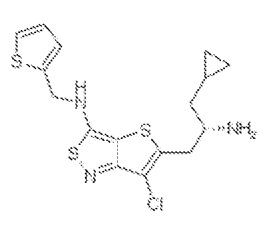 | 7-[(S)-2-amino-3-cyclopropylpropyl]-8-chloro-4-thenylamino-3,6-dithia-2-azabicyclo[3.3.0]oct a-1,4,7-triene |
| 59 | 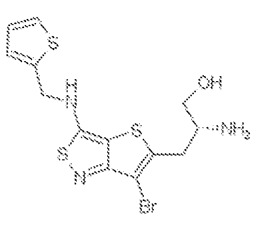 | (R)-2-amino-3-(8-bromo-4-thenylamino-3,6-dithia-2-azabicyclo[3.3.0]oct a-1,4,7-trien-7-yl)-1-propanol |
| 60 | 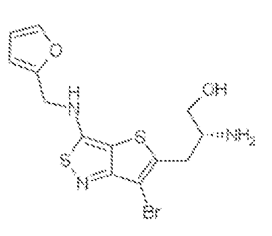 | (R)-2-amino-3-{8-bromo-4-(furfurylamino)-3,6-dithia-2-azabicyclo[3.3.0]oct a-1,4,7-trien-7-yl}-1-propanol |
| 61 | 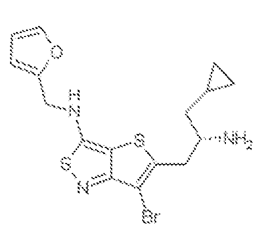 | 7-[(S)-2-amino-3-cyclopropylpropyl]-8-bromo-4-(furfurylamino)-3,6-dithia-2-azabicyclo[3.3.0]oct a-1,4,7-triene |
| 62 | 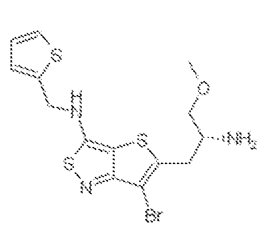 | 7-[(R)-2-amino-3-methoxypropyl]-8-bromo-4-thenylamino-3,6-dithia-2-azabicyclo[3.3.0]oct a-1,4,7-triene |
| 63 | 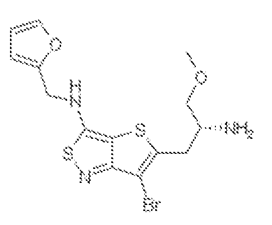 | 7-[(R)-2-amino-3-methoxypropyl]-8-bromo-4-(furfurylamino)-3,6-dithia-2-azabicyclo[3.3.0]oct a-1,4,7-triene |
| 64 | 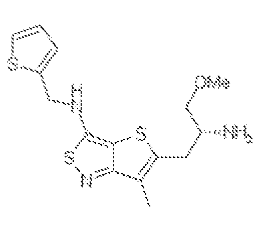 | 7-[(R)-2-amino-3-methoxypropyl]-8-methyl-4-thenylamino-3,6-dithia-2-azabicyclo[3.3.0]oct a-1,4,7-triene |
| 65 | 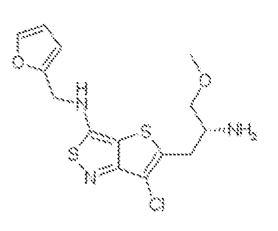 | 7-[(R)-2-amino-3-methoxypropyl]-8-chloro-4-(furfurylamino)-3,6-dithia-2-azabicyclo[3.3.0]oct a-1,4,7-triene |
| 66 | 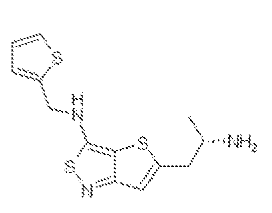 | 7-[(S)-2-aminopropyl]-4-thenylamino-3,6-dithia-2-azabicyclo[3.3.0]oct a-1,4,7-triene |
| 67 | 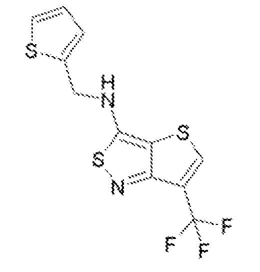 | (thenyl){8-(trifluoromethyl)-3,6-dithia-2-azabicyclo[3.3.0]oct a-1,4,7-trien-4-yl}amine |
| 68 | 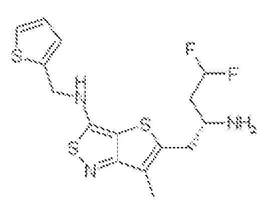 | 7-[(S)-2-amino-4,4-difluorobutyl]-8-methyl-4-thenylamino-3,6-dithia-2-azabicyclo[3.3.0]oct a-1,4,7-triene |
| 69 | 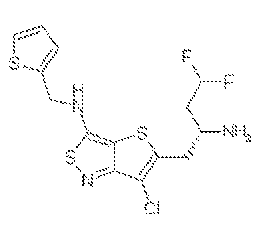 | 7-[(S)-2-amino-4,4-difluorobutyl]-8-chloro-4-thenylamino-3,6-dithia-2-azabicyclo[3.3.0]oct a-1,4,7-triene |
| 70 | 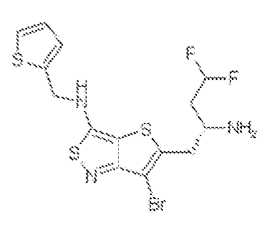 | 7-[(S)-2-amino-4,4-difluorobutyl]-8-bromo-4-thenylamino-3,6-dithia-2-azabicyclo[3.3.0]oct a-1,4,7-triene |
| 71 | 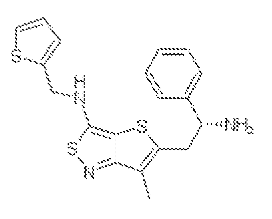 | 7-[(R)-2-amino-2-phenylethyl]-8-methyl-4-thenylamino-3,6-dithia-2-azabicyclo[3.3.0]oct a-1,4,7-triene |
| 72 | 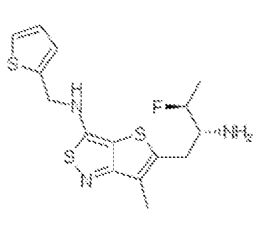 | 7-[(2R,3S)-2-amino-3-fluorobutyl]-8-methyl-4-thenylamino-3,6-dithia-2-azabicyclo[3.3.0]oct a-1,4,7-triene |
| 73 | 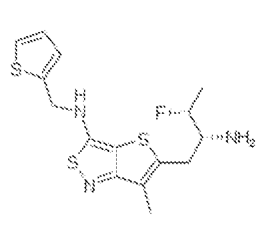 | 7-[(2R,3R)-2-amino-3-fluorobutyl]-8-methyl-4-thenylamino-3,6-dithia-2-azabicyclo[3.3.0]oct a-1,4,7-triene |
| 74 | 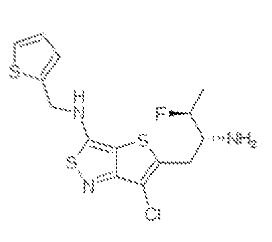 | 7-[(2R,3S)-2-amino-3-fluorobutyl]-8-chloro-4-thenylamino-3,6-dithia-2-azabicyclo[3.3.0]oct a-1,4,7-triene |
| 75 | 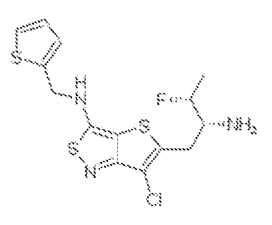 | 7-[(2R,3R)-2-amino-3-fluorobutyl]-8-chloro-4-thenylamino-3,6-dithia-2-azabicyclo[3.3.0]oct a-1,4,7-triene |
| 76 | 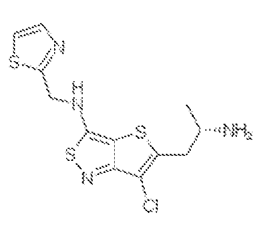 | 7-[(S)-2-aminopropyl]-8-chloro-4-{[(1,3-thiazol-2-yl)methyl]amino}-3,6-dithia-2-azabicyclo[3.3.0]oct a-1,4,7-triene |
| 79 | 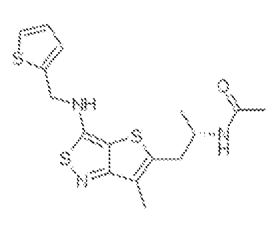 | N-{(S)-1-methyl-2-(8-methyl-4-thenylamino-3,6-dithia-2-azabicyclo[3.3.0]oct a-1,4,7-trien-7-yl)ethyl}acetamide |
| 80 | 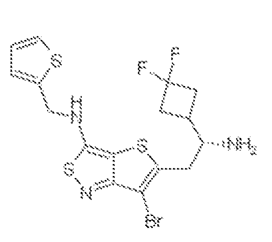 | 7-[(R)-2-amino-2-(3,3-difluorocyclobutyl)e thyl]-8-bromo-4-thenylamino-3,6-dithia-2-azabicyclo[3.3.0]oct a-1,4,7-triene |
| 81 | 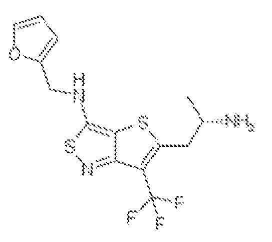 | 7-[(S)-2-aminopropyl]-4-(furfurylamino)-8-(trifluoromethyl)-3,6-dithia-2-azabicyclo[3.3.0]oct a-1,4,7-triene |
| 82 | 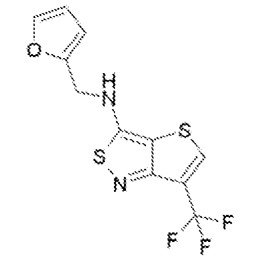 | (furfuryl){8-(trifluoromethyl)-3,6-dithia-2-azabicyclo[3.3.0]oct a-1,4,7-trien-4-yl}amine |
| 83 | 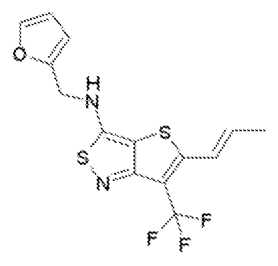 | (furfuryl) {7-[(E)-1-propenyl]-8-(trifluoromethyl)-3,6-dithia-2-azabicyclo[3.3.0]oct a-1,4,7-trien-4-yl}amine |
| 84 | 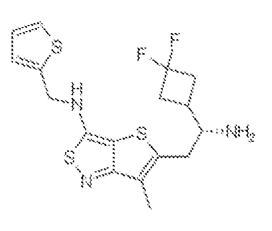 | 7-[(R)-2-amino-2-(3,3-difluorocyclobutyl)e thyl]-8-methyl-4-thenylamino-3,6-dithia-2-azabicyclo[3.3.0]oct a-1,4,7-triene |
| 85 | 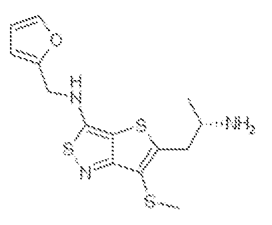 | 7-[(S)-2-aminopropyl]-4-(furfurylamino)-8-(methylthio)-3,6-dithia-2-azabicyclo[3.3.0]oct a-1,4,7-triene |
| 86 | 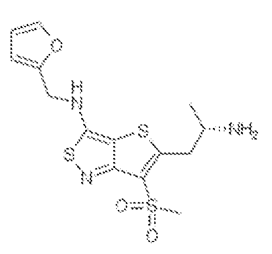 | 7-[(S)-2-aminopropyl]-4-(furfurylamino)-8-mesyl-3,6-dithia-2-azabicyclo[3.3.0]oct a-1,4,7-triene |
| 87 | 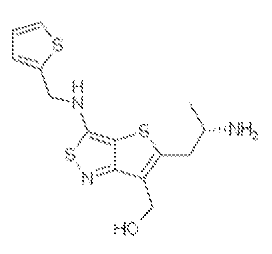 | {7-[(S)-2-aminopropyl]-4-thenylamino-3,6-dithia-2-azabicyclo[3.3.0]oct a-1,4,7-trien-8-yl}methanol |
| 88 | 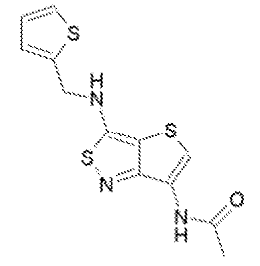 | N-(4-thenylamino-3,6-dithia-2-azabicyclo[3.3.0]oct a-1,4,7-trien-8-yl)acetamide |
| 89 | 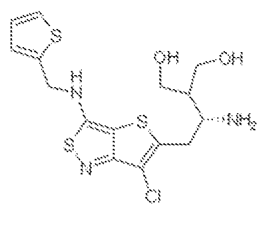 | 2-{(R)-1-amino-2-(8-chloro-4-thenylamino-3,6-dithia-2-azabicyclo[3.3.0]oct a-1,4,7-trien-7-yl)ethyl}-1,3-propanediol |
| 90 | 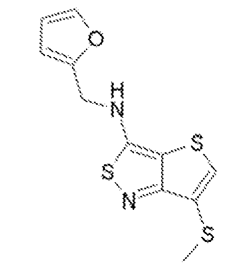 | (furfuryl){8-(methylthio)-3,6-dithia-2-azabicyclo[3.3.0]oct a-1,4,7-trien-4-yl}amine |
| 91 | 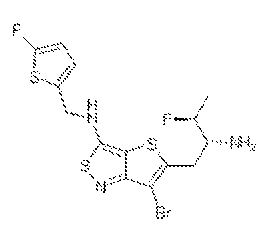 | 7-[(2R,3S)-2-amino-3-fluorobutyl]-8-bromo-4-{[(5-fluoro-2-thienyl)methyl]amin o}-3,6-dithia-2-azabicyclo[3.3.0]oct a-1,4,7-triene |
| 92 | 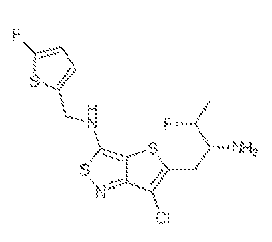 | 7-[(2R,3R)-2-amino-3-fluorobutyl]-8-chloro-4-{[(5-fluoro-2-thienyl)methyl]amin o}-3,6-dithia-2-azabicyclo[3.3.0]oct a-1,4,7-triene |
| 93 | 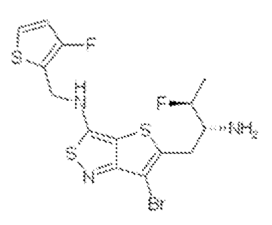 | 7-[(2R,3S)-2-amino-3-fluorobutyl]-8-bromo-4-{[(3-fluoro-2-thienyl)methyl]amin o}-3,6-dithia-2-azabicyclo[3.3.0]oct a-1,4,7-triene |
| 94 | 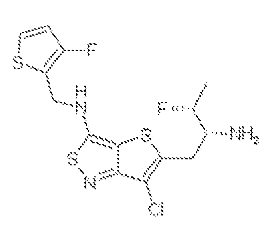 | 7-[(2R,3R)-2-amino-3-fluorobutyl]-8-chloro-4-{[(3-fluoro-2-thienyl)methyl]amin o}-3,6-dithia-2-azabicyclo[3.3.0]oct a-1,4,7-triene |
| 95 | 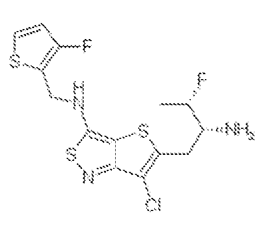 | 7-[(2R,3S)-2-amino-3-fluorobutyl]-8-chloro-4-{[(3-fluoro-2-thienyl)methyl]amin o}-3,6-dithia-2-azabicyclo[3.3.0]oct a-1,4,7-triene |
| 96 | 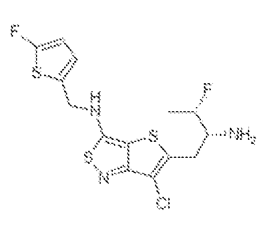 | 7-[(2R,3S)-2-amino-3-fluorobutyl]-8-chloro-4-{[(5-fluoro-2-thienyl)methyl]amin o}-3,6-dithia-2-azabicyclo[3.3.0]oct a-1,4,7-triene |
| 97 | 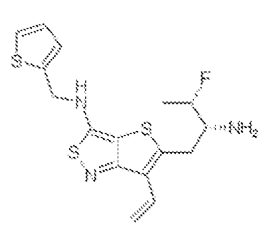 | 7-[(2R,3S)-2-amino-3-fluorobutyl]-4-thenylamino-8-vinyl-3,6-dithia-2-azabicyclo[3.3.0]oct a-1,4,7-triene |
| 98 | 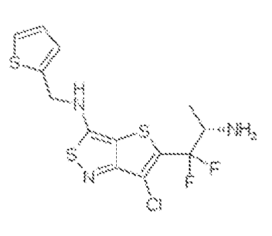 | 7-[(S)-2-amino-1,1-difluoropropyl]-8-chloro-4-thenylamino-3,6-dithia-2-azabicyclo[3.3.0]oct a-1,4,7-triene |
| 99 | 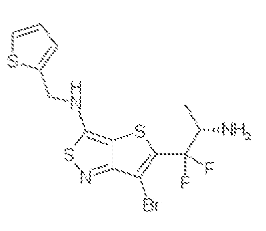 | 7-[(S)-2-amino-1,1-difluoropropyl]-8-bromo-4-thenylamino-3,6-dithia-2-azabicyclo[3.3.0]oct a-1,4,7-triene |
| 100 | 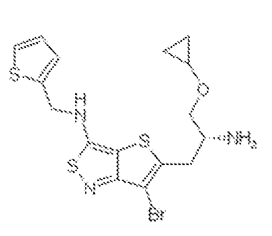 | 7-[(R)-2-amino-3-cyclopropoxypropyl] -8-bromo-4-thenylamino-3,6-dithia-2-azabicyclo[3.3.0]oct a-1,4,7-triene |
| 101 | 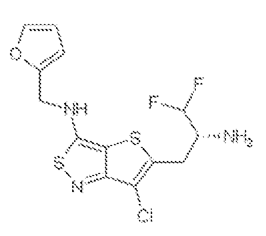 | 7-[(R)-2-amino-3,3-difluoropropyl]-8-chloro-4-(furfurylamino)-3,6-dithia-2-azabicyclo[3.3.0]oct a-1,4,7-triene |
| 102 | 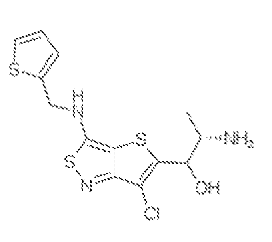 | (2S)-2-amino-1-(8-chloro-4-thenylamino-3,6-dithia-2-azabicyclo[3.3.0]oct a-1,4,7-trien-7-yl)-1-propanol |
| 103 | 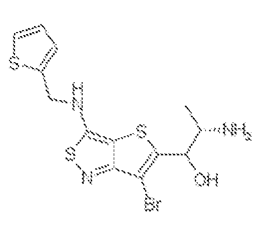 | (2S)-2-amino-1-(8-bromo-4-thenylamino-3,6-dithia-2-azabicyclo[3.3.0]oct a-1,4,7-trien-7-yl)-1-propanol |
| 104 | 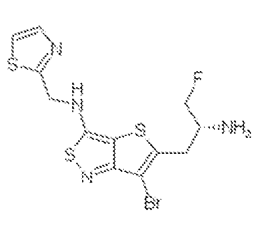 | 7-[(R)-2-amino-3-fluoropropyl]-8-bromo-4-{[(1,3-thiazol-2-yl)methyl]amino }-3,6-dithia-2-azabicyclo[3.3.0]oct a-1,4,7-triene |
| 105 | 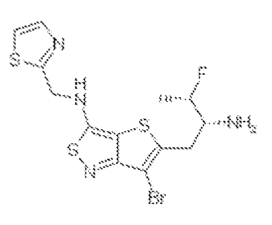 | 7-[(2R,3S)-2-amino-3-fluorobutyl]-8-bromo-4-{[(1,3-thiazol-2-yl)methyl]amino}-3,6-dithia-2-azabicyclo[3.3.0]oct a-1,4,7-triene |
| 106 | 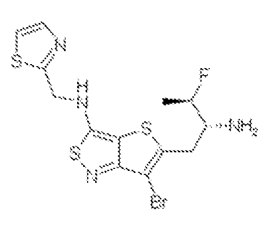 | 7-[(2R,3R)-2-amino-3-fluorobutyl]-8-bromo-4-{[(1,3-thiazol-2-yl)methyl]amino}-3,6-dithia-2-azabicyclo[3.3.0]oct a-1,4,7-triene |
| 107 | 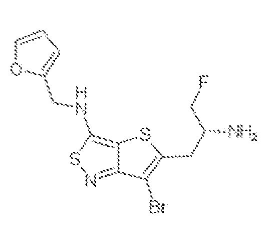 | 7-[(R)-2-amino-3-fluoropropyl]-8-bromo-4-(furfurylamino)-3,6-dithia-2-azabicyclo[3.3.0]oct a-1,4,7-triene |
| 108 | 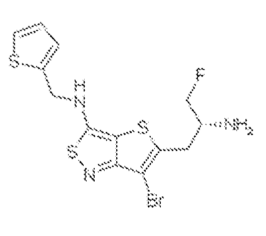 | 7-[(R)-2-amino-3-fluoropropyl]-8-bromo-4-thenylamino-3,6-dithia-2-azabicyclo[3.3.0]oct a-1,4,7-triene |
| 109 | 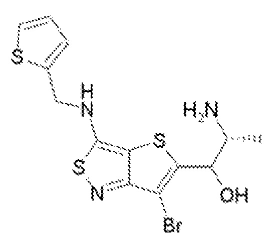 | (2R)-2-amino-1-(8-bromo-4-thenylamino-3,6-dithia-2-azabicyclo[3.3.0]oct a-1,4,7-trien-7-yl)-1-propanol |
| 110 | 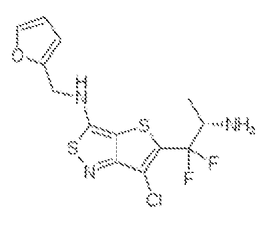 | 7-[(S)-2-amino-1,1-difluoropropyl]-8-chloro-4-(furfurylamino)-3,6-dithia-2-azabicyclo[3.3.0]oct a-1,4,7-triene |
| 111 | 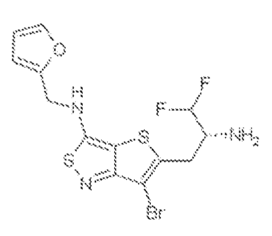 | 7-[(R)-2-amino-3,3-difluoropropyl]-8-bromo-4-(furfurylamino)-3,6-dithia-2-azabicyclo[3.3.0]oct a-1,4,7-triene |
| 112 | 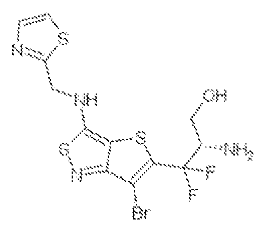 | (S)-2-amino-3-(8-bromo-4-{[(1,3-thiazol-2-yl)methyl]amino }-3,6-dithia-2-azabicyclo[3.3.0]oct a-1,4,7-trien-7-yl)-3,3-difluoro-1-propanol |
| 113 | 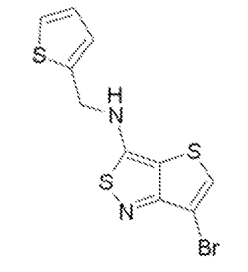 | (thenyl)(8-bromo-3,6-dithia-2-azabicyclo[3.3.0]oct a-1,4,7-trien-4-yl)amine |
| 114 | 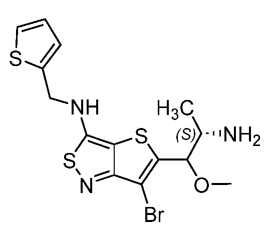 | 7-[(2S)-2-amino-1-methoxypropyl]-8-bromo-4-thenylamino-3,6-dithia-2-azabicyclo[3.3.0]oct a-1,4,7-triene |
| 115 | 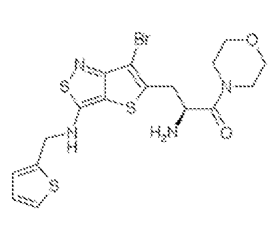 | (S)-2-amino-3-(8-bromo-4-thenylamino-3,6-dithia-2-azabicyclo[3.3.0]oct a-1,4,7-trien-7-yl)-1-morpholino-1-propanone |
| 116 | 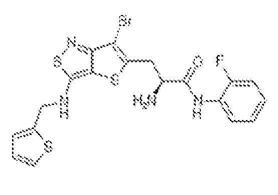 | N-o-fluorophenyl(S)-2-amino-3-(8-bromo-4-thenylamino-3,6-dithia-2-azabicyclo[3.3.0]oct a-1,4,7-trien-7-yl)propionamide |
| 117 | 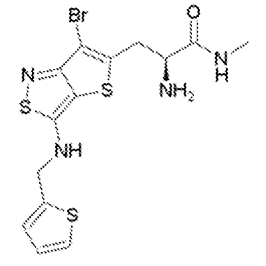 | N-methyl(S)-2-amino-3-(8-bromo-4-thenylamino-3,6-dithia-2-azabicyclo[3.3.0]oct a-1,4,7-trien-7-yl)propionamide |
| 118 | 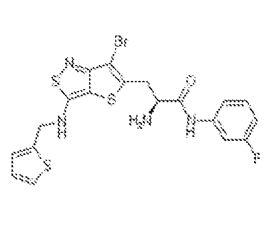 | N-m-fluorophenyl(S)-2-amino-3-(8-bromo-4-thenylamino-3,6-dithia-2-azabicyclo[3.3.0]oct a-1,4,7-trien-7-yl)propionamide |
| 119 | 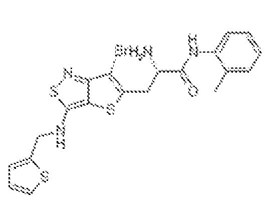 | N-o-tolyl(S)-2-amino-3-(8-bromo-4-thenylamino-3,6-dithia-2-azabicyclo[3.3.0]oct a-1,4,7-trien-7-yl)propionamide |
| 120 | 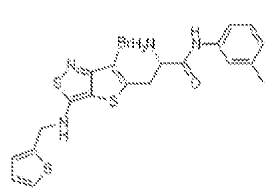 | N-m-tolyl(S)-2-amino-3-(8-bromo-4-thenylamino-3,6-dithia-2-azabicyclo[3.3.0]oct a-1,4,7-trien-7-yl)propionamide |
| 121 | 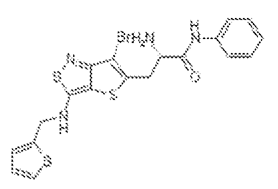 | N-phenyl(S)-2-amino-3-(8-bromo-4-thenylamino-3,6-dithia-2-azabicyclo[3.3.0]oct a-1,4,7-trien-7-yl)propionamide |
| 122 | 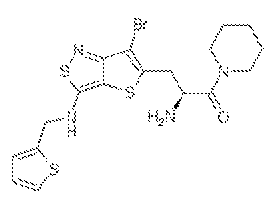 | (S)-2-amino-3-(8-bromo-4-thenylamino-3,6-dithia-2-azabicyclo[3.3.0]oct a-1,4,7-trien-7-yl)-1-piperidino-1-propanone |
| 123 | 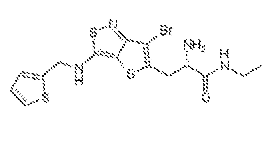 | N-ethyl(S)-2-amino-3-(8-bromo-4-thenylamino-3,6-dithia-2-azabicyclo[3.3.0]oct a-1,4,7-trien-7-yl)propionamide |
| 124 | 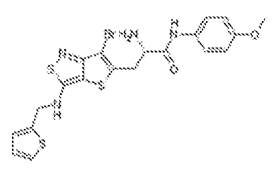 | N-p-methoxyphenyl(S)-2-amino-3-(8-bromo-4-thenylamino-3,6-dithia-2-azabicyclo[3.3.0]oct a-1,4,7-trien-7-yl)propionamide |
| 125 | 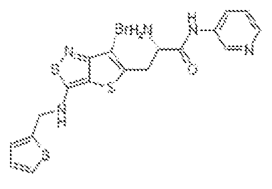 | N-3-pyridyl(S)-2-amino-3-(8-bromo-4-thenylamino-3,6-dithia-2-azabicyclo[3.3.0]oct a-1,4,7-trien-7-yl)propionamide |
| 126 | 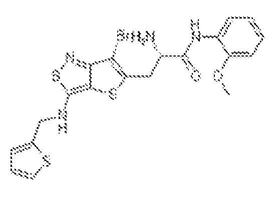 | N-o-methoxyphenyl(S)-2-amino-3-(8-bromo-4-thenylamino-3,6-dithia-2-azabicyclo[3.3.0]oct a-1,4,7-trien-7-yl)propionamide |
| 127 | 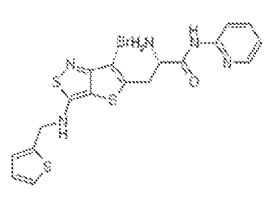 | N-2-pyridyl(S)-2-amino-3-(8-bromo-4-thenylamino-3,6-dithia-2-azabicyclo[3.3.0]oct a-1,4,7-trien-7-yl)propionamide |
| 128 | 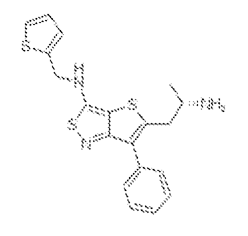 | 7-[(S)-2-aminopropyl]-8-phenyl-4-thenylamino-3,6-dithia-2-azabicyclo[3.3.0]oct a-1,4,7-triene |
| 129 | 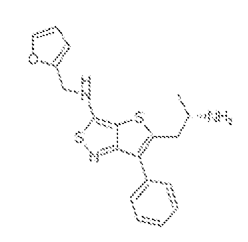 | 7-[(S)-2-aminopropyl]-4-(furfurylamino)-8-phenyl-3,6-dithia-2-azabicyclo[3.3.0]oct a-1,4,7-triene |

### Pharmaceutical Compositions

In some embodiments, the compounds described herein are formulated into pharmaceutical compositions. Pharmaceutical compositions are formulated in a conventional manner using one or more pharmaceutically acceptable inactive ingredients that facilitate processing of the active compounds into preparations that can be used pharmaceutically. Proper formulation is dependent upon the route of administration chosen. A summary of pharmaceutical compositions described herein can be found, for example, in Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins 1999).

A pharmaceutical composition can be a mixture of a SMSM described herein with one or more other chemical components (*i.e.,* pharmaceutically acceptable ingredients), such as carriers, excipients, binders, filling agents, suspending agents, flavoring agents, sweetening agents, disintegrating agents, dispersing agents, surfactants, lubricants, colorants, diluents, solubilizers, moistening agents, plasticizers, stabilizers, penetration enhancers, wetting agents, anti-foaming agents, antioxidants, preservatives, or one or more combination thereof. The pharmaceutical composition facilitates administration of the compound to an organism.

The compositions described herein can be administered to the subject in a variety of ways, including parenterally, intravenously, intradermally, intramuscularly, colonically, rectally, or intraperitoneally. In some embodiments, the small molecule splicing modulator, or a pharmaceutically acceptable salt thereof is administered by intraperitoneal injection, intramuscular injection, subcutaneous injection, or intravenous injection of the subject. In some embodiments, the pharmaceutical compositions can be administered parenterally, intravenously, intramuscularly or orally. The oral agents comprising a small molecule splicing modulator can be in any suitable form for oral administration, such as liquid, tablets, capsules, or the like. The oral formulations can be further coated or treated to prevent or reduce dissolution in stomach. The compositions of the present disclosure can be administered to a subject using any suitable methods known in the art. Suitable formulations for use in the present disclosure and methods of delivery are generally well known in the art. For example, the small molecule splicing modulators described herein can be formulated as pharmaceutical compositions with a pharmaceutically acceptable diluent, carrier, or excipient. The compositions may contain pharmaceutically acceptable auxiliary substances as required to approximate physiological conditions including pH adjusting and buffering agents, tonicity adjusting agents, wetting agents and the like, such as, for example, sodium acetate, sodium lactate, sodium chloride, potassium chloride, calcium chloride, sorbitan monolaurate, triethanolamine oleate, etc.

In some embodiments, the pharmaceutical formulation is in the form of a tablet. In other embodiments, pharmaceutical formulations containing a SMSM described herein are in the form of a capsule. In one aspect, liquid formulation dosage forms for oral administration are in the form of aqueous suspensions or solutions selected from the group including, but not limited to, aqueous oral dispersions, emulsions, solutions, elixirs, gels, and syrups.

For administration by inhalation, a SMSM described herein can be formulated for use as an aerosol, a mist, or a powder. For buccal or sublingual administration, the compositions may take the form of tablets, lozenges, or gels formulated in a conventional manner. In some embodiments, a SMSM described herein can be prepared as transdermal dosage forms. In some embodiments, a SMSM described herein can be formulated into a pharmaceutical composition suitable for intramuscular, subcutaneous, or intravenous injection. In some embodiments, a SMSM described herein can be administered topically and can be formulated into a variety of topically administrable compositions, such as solutions, suspensions, lotions, gels, pastes, medicated sticks, balms, creams, or ointments. In some embodiments, a SMSM described herein can be formulated in rectal compositions such as enemas, rectal gels, rectal foams, rectal aerosols, suppositories, jelly suppositories, or retention enemas.

In some embodiments, disclosed herein is a pharmaceutical composition comprising a compound of the disclosure or a pharmaceutically acceptable salt thereof, and a pharmaceutically acceptable excipient or carrier.

### Splicing Modulation of Target Gene Products

The present disclosure contemplates use of small molecules with favorable drug properties that modulate the activity of splicing of a target RNA. Provided herein are small molecule splicing modulators (SMSMs) that modulate splicing of a polynucleotide. In some embodiments, the SMSMs bind and modulate target RNA. In some embodiments, provided herein is a library of SMSMs that bind and modulate one or more target RNAs. In some embodiments, the target RNA is mRNA. In some embodiments, the target RNA is a noncoding RNA. In some embodiments, the target RNA is a pre-mRNA. In some embodiments, the target RNA is hnRNA. In some embodiments, the small molecules modulate splicing of the target RNA. In some embodiments, a small molecule provided herein modulates splicing at a sequence of the target RNA. In some embodiments, a small molecule provided herein modulates splicing at a cryptic splice site sequence of the target RNA. In some embodiments, a small molecule provided herein modulates splicing at an alternative splice site sequence of the target RNA. In some embodiments, a small molecule provided herein modulates splicing at a native splice site sequence of the target RNA. In some embodiments, a small molecule provided herein binds to a target RNA. In some embodiments, a small molecule provided herein binds to a splicing complex or a component thereof. In some embodiments, a small molecule provided herein binds to a target RNA and a splicing complex or a component thereof. In some embodiments, a small molecule provided herein modulates binding affinity of a splicing complex component to a target RNA such as a pre-mRNA. In some embodiments, a small molecule provided herein modulates binding affinity of a splicing complex component to a target RNA such as a pre-mRNA at a splice site sequence. In some embodiments, a small molecule provided herein modulates binding affinity of a splicing complex component to a target RNA such as a pre-mRNA upstream of a splice site sequence or downstream of a splice site sequence.

Described herein are compounds modifying splicing of gene products, such as Ataxin 3 pre-mRNA for use in the treatment, prevention, and/or delay of progression of diseases or conditions.

In some embodiments, described herein, is a method of treating, preventing, delaying of progress, or ameliorating symptoms of a disease or a condition associated with Ataxin 3 (ATXN3) expression level or activity level in a subject in need thereof, comprising administering a therapeutically effective amount of a small molecule splicing modulator (SMSM), wherein the SMSM binds to a pre-mRNA encoded by ATXN3 and modulates splicing of the ATXN3 pre-mRNA in a cell of the subject to produce a spliced product of the ATXN3 pre-mRNA.

In some embodiments, described herein is a method of treating, preventing, delaying of progress, or ameliorating symptoms of a disease or a condition associated with Ataxin 3 (ATXN3) expression level or activity level in a subject in need thereof, comprising administering a therapeutically effective amount of a compound or salt of Formula (I). In some embodiments, described herein is a method of modulating splicing of a Ataxin3 (ATXN3) pre-mRNA, comprising contacting a compound or salt of Formula (I) to the ATXN3 pre-mRNA with a splice site sequence or cells comprising the ATXN3 pre-mRNA, wherein the compound binds to the ATXN3 pre-mRNA and modulates splicing of the ATXN3 pre-mRNA in a cell of a subject to produce a spliced product of the ATXN3 pre-mRNA. In some embodiments, described herein is use of a compound of Formula (I), or a pharmaceutically acceptable salt thereof, in the manufacture of a medicament for the treatment of a condition or disease associated with Ataxin 3 (ATXN3) expression level or activity level.

In some embodiments, the spliced product of the ATXN3 pre-mRNA undergoes non-sense mediated decay (NMD) and/or nuclear retention. In some embodiments, the nonsense-mediated decay (NMD) and/or nuclear retention of the spliced product of the ATXN3 pre-mRNA is promoted. In some embodiments, the nonsense-mediated decay (NMD) and/or nuclear retention of the spliced product of the ATXN3 pre-mRNA is increased compared to a spliced product of the ATXN3 pre-mRNA produced in the absence of the SMSM.

In some embodiments, described herein is a method of modulating splicing of a Ataxin3 (ATXN3) pre-mRNA, comprising contacting a small molecule splicing modulator (SMSM) to the ATXN3 pre-mRNA with a splice site sequence or cells comprising the ATXN3 pre-mRNA, wherein the SMSM binds to the ATXN3 pre-mRNA and modulates splicing of the ATXN3 pre-mRNA in a cell of a subject to produce a spliced product of the ATXN3 pre-mRNA.

In some embodiments, described herein, is a method of modulating splicing of Ataxin 3 (ATXN3) pre-mRNA, comprising contacting a small molecule splicing modulator (SMSM) to the ATXN3 pre-mRNA with a splice site sequence or cells comprising the ATXN3 pre-mRNA, wherein the SMSM binds to the ATXN3 pre-mRNA and modulates splicing of the ATXN3 pre-mRNA in a cell of a subject to produce a spliced product of the ATXN3 pre-mRNA, wherein the splice site sequence comprises UCCUAU/guaagauucugu.

In some embodiments, described herein, is a method of treating, preventing, delaying of progress, or ameliorating symptoms of a disease or condition associated with Ataxin 3 (ATXN3) expression level or activity level in a subject in need thereof, comprising administering a therapeutically effective amount of a small molecule splicing modulator (SMSM) to the subject, wherein the SMSM binds to a ATXN3 pre-mRNA with a splice site sequence and modulates splicing of the ATXN3 pre-mRNA in a cell of the subject, wherein a spliced product of the ATXN3 pre-mRNA undergoes nonsense-mediated decay (NMD), and wherein the splice site sequence comprises UCCUAU/guaagauucugu.

In some embodiments, the modulating splicing comprises modulating alternative splicing. In some embodiments, the modulating splicing comprises promoting exon skipping. In some embodiments, the modulating splicing comprises promoting exon inclusion. In some embodiments, the modulating splicing comprises modulating nonsense-mediated mRNA decay (NMD). In some embodiments, the modulating NMD comprises promoting NMD. In some embodiments, the modulating splicing comprises modulating nuclear retention of the spliced product of the pre-mRNA. In some embodiments, the modulating intron retention comprises promoting nuclear retention of the spliced product of the pre-mRNA.

In some embodiments, the splice site sequence is a native splice site sequence. In some embodiments, the native splice site is a canonical splice site. In some embodiments, the native splice site is an alternative splice site. In some embodiments, the alternative splice site comprises a 5' splice site sequence. In some embodiments, the alternative splice site sequence comprises UCCUAU/guaagauucugu. In some embodiments, the SMSM induces splicing at the alternative splice site. In some embodiments, the splicing at the alternative splice site results in a frameshift in a downstream exon in the spliced product. In some embodiments, the downstream exon comprises an in-frame stop codon that is not in frame in the absence of splicing at the alternative splice site. In some embodiments, the in-frame stop codon in the downstream exon is at least 50 or at least 60 base pairs upstream of the 3' end of the downstream exon. In some embodiments, the in-frame stop codon in the downstream exon is at least 50 or at least 60 base pairs upstream of a final exon-exon junction.

In some embodiments, the splicing of the pre-mRNA at the alternative splice site promotes NMD of the spliced product of the ATXN3 pre-mRNA. In some embodiments, the spliced product comprises an alternative exon. In some embodiments, the SMSM promotes inclusion of the alternative exon in the spliced product. In some embodiments, the alternative exon comprises a poison exon. In some embodiments, the SMSM promotes inclusion of the poison exon in the spliced product. In some embodiments, the poison exon comprises an in-frame stop codon. In some embodiments, the in-frame stop codon is a premature termination codon. In some embodiments, the in-frame stop codon is at least 50 or 60 base pairs upstream of the 3' end of the poison exon. In some embodiments, the in-frame stop codon is less than 60 base pairs upstream of the 3' end of the poison exon and wherein the exon immediately downstream of the poison exon is not the last exon in the pre-mRNA. In some embodiments, the sum of (a) the number of base pairs in the exon immediately downstream of the poison exon and (b) the number of base pairs between the premature termination codon in the poison exon and the 3' end of the poison exon is at least 50 or at least 60.

In some embodiments, the cells comprise primary cells. In some embodiments, the cells comprise disease cells. In some embodiments, the SMSM modulates proliferation or survival of the cells. In some embodiments, the SMSM modulates the expression level of a protein encoded by the spliced product of the pre-mRNA in the cells.

**Table 2. Exemplary targets for exon skipping**

| Gene | ATXN3 |
|---|---|
| Exon Coordinates | Chr14:92093746-92093831 |
| Splicing Event Region | chr14:92093319-92096092 |
| Strand | - |
| Target site | Exon 4 |
| Exon length | 86 |
| SEQ ID NO: | 1 |
| 5' ss sequence (-6~+12) | UCCUAU/guaagauucugu |
| 5' ss-U1 duplex structure | -1U-C loop |
| Disease | Spinocerebellar Ataxia Type 3 |

### Methods of Treatment

The compositions and methods described herein can be used for treating a human disease or disorder associated with aberrant splicing, such as aberrant pre-mRNA splicing. The compositions and methods described herein can be used for treating a human disease or disorder by modulating mRNA, such as pre-mRNA. In some embodiments, the compositions and methods described herein can be used for treating a human disease or disorder by modulating splicing of a nucleic acid even when that nucleic acid is not aberrantly spliced in the pathogenesis of the disease or disorder being treated.

In some embodiments, an effective amount in the context of the administration of a SMSM or a pharmaceutically acceptable salt thereof, or composition or medicament thereof refers to an amount of a SMSM or a pharmaceutically acceptable salt thereof to a patient which has a therapeutic effect and/or beneficial effect. In certain specific embodiments, an effective amount in the context of the administration of a SMSM or a pharmaceutically acceptable salt thereof, or composition or medicament thereof to a patient results in one, two or more of the following effects: (i) reduces or ameliorates the severity of a disease; (ii) delays onset of a disease; (iii) inhibits the progression of a disease; (iv) reduces hospitalization of a subject; (v) reduces hospitalization length for a subject; (vi) increases the survival of a subject; (vii) improves the quality of life of a subject; (viii) reduces the number of symptoms associated with a disease; (ix) reduces or ameliorates the severity of a symptom associated with a disease; (x) reduces the duration of a symptom associated with a disease associated; (xi) prevents the recurrence of a symptom associated with a disease; (xii) inhibits the development or onset of a symptom of a disease; and/or (xiii) inhibits of the progression of a symptom associated with a disease. In some embodiments, an effective amount of a SMSM or a pharmaceutically acceptable salt thereof is an amount effective to restore the amount of an RNA transcript of a gene to the amount of the RNA transcript detectable in healthy patients or cells from healthy patients. In other embodiments, an effective amount of a SMSM or a pharmaceutically acceptable salt thereof is an amount effective to restore the amount an RNA isoform and/or protein isoform of a gene to the amount of the RNA isoform and/or protein isoform detectable in healthy patients or cells from healthy patients.

In some embodiments, an effective amount of a SMSM or a pharmaceutically acceptable salt thereof is an amount effective to decrease the aberrant amount of an RNA transcript of a gene which associated with a disease. In some embodiments, an effective amount of a SMSM or a pharmaceutically acceptable salt thereof is an amount effective to decrease the amount of the aberrant expression of an isoform of a gene. In some embodiments, an effective amount of a SMSM or a pharmaceutically acceptable salt thereof is an amount effective to result in a substantial change in the amount of an RNA transcript (*e.g*., an mRNA transcript), alternative splice variant, or isoform.

In some embodiments, an effective amount of a SMSM or a pharmaceutically acceptable salt thereof is an amount effective to increase the amount of an RNA transcript (*e.g*., an mRNA transcript) of a gene that is beneficial for the prevention and/or treatment of a disease. In some embodiments, an effective amount of a SMSM or a pharmaceutically acceptable salt thereof is an amount effective to increase the amount of an alternative splice variant of an RNA transcript of a gene that is beneficial for the prevention and/or treatment of a disease. In some embodiments, an effective amount of a SMSM or a pharmaceutically acceptable salt thereof is an amount effective to increase the amount of an isoform of a gene that is beneficial for the prevention and/or treatment of a disease.

In some embodiments, an effective amount of a SMSM or a pharmaceutically acceptable salt thereof is an amount effective to decrease the amount of an RNA transcript (*e.g*., an mRNA transcript) which causes or is related to the symptoms of the condition or disease. In particular embodiments, the SMSM decreases the amount of an RNA transcript that causes or relates to the symptoms of the condition or disease by modulating one or more splicing elements of the RNA transcript. In some embodiments, the SMSM promotes skipping of one or more exons. In some embodiments, the SMSM promotes inclusion of one or more exons. In some embodiments, the SMSM promotes inclusion of one or more exons and/or introns that relate to nonsense-mediated mRNA decay (NMD). In some embodiments, the one or more exons harbor a premature termination codon. In particular embodiments, the premature stop codon is an in-frame codon that does not cause frameshift of the downstream exon(s). In some embodiments, inclusion of the one or more exons causes a reading frameshift in a downstream exon, for example, in the immediately downstream exon, introducing a premature termination codon.

A method of treating a disease or a condition in a subject in need thereof can comprise administering to the subject a therapeutically effective amount of a compound described herein or a pharmaceutically acceptable salt thereof. In some embodiments, the present disclosure relates to a method for the treatment, prevention and/or delay of progression of a disease or a condition associated with a gene listed in Table 2.

Non-limiting examples of effective amounts of a SMSM or a pharmaceutically acceptable salt thereof are described herein. For example, the effective amount may be the amount required to prevent and/or treat a disease associated with the aberrant amount of an mRNA transcript of gene in a human subject. In general, the effective amount will be in a range of from about 0.001 mg/kg/day to about 500 mg/kg/day for a patient having a weight in a range of between about 1 kg to about 200 kg. The typical adult subject is expected to have a median weight in a range of between about 70 and about 100 kg.

In one embodiment, a SMSM described herein can be used in the preparation of medicaments for the treatment of diseases or conditions described herein. In addition, a method for treating any of the diseases or conditions described herein in a subject in need of such treatment, can involve administration of pharmaceutical compositions that include at least one SMSM described herein or a pharmaceutically acceptable salt, thereof, in a therapeutically effective amount to a subject.

In certain embodiments, a SMSM described herein can be administered for prophylactic and/or therapeutic treatments. In certain therapeutic applications, the compositions are administered to a patient already suffering from a disease or a condition, in an amount sufficient to cure or at least partially arrest at least one of the symptoms of the disease or the condition. Amounts effective for this use depend on the severity and course of the disease or the condition, previous therapy, the patient's health status, weight, and response to the drugs, and the judgment of the treating physician. Therapeutically effective amounts are optionally determined by methods including, but not limited to, a dose escalation clinical trial. In prophylactic applications, compositions containing a SMSM described herein can be administered to a patient susceptible to or otherwise at risk of a particular disease, disorder, or condition.

### Methods of Administering

The compositions described herein can be administered to the subject in a variety of ways, including parenterally, intravenously, intradermally, intramuscularly, colonically, rectally or intraperitoneally. In some embodiments, the small molecule splicing modulator (SMSM) or a pharmaceutically acceptable salt thereof is administered by intraperitoneal injection, intramuscular injection, subcutaneous injection, or intravenous injection of the subject. In some embodiments, the pharmaceutical compositions can be administered parenterally, intravenously, intramuscularly or orally. The oral agents comprising a small molecule splicing modulator can be in any suitable form for oral administration, such as liquid, tablets, capsules, or the like. The compositions of the present disclosure can be administered to a subject using any suitable methods known in the art. Suitable formulations for use in the present disclosure and methods of delivery are generally well known in the art. For example, the small molecule splicing modulators described herein can be formulated as pharmaceutical compositions with a pharmaceutically acceptable diluent, carrier, or excipient. Dosing and Schedules

The SMSMs utilized in the methods of the disclosure can be, *e.g.,* administered at dosages that may be varied depending upon the requirements of the subject, the severity of the condition being treated and/or imaged, and/or the SMSM being employed. For example, dosages can be empirically determined considering the type and stage of disease diagnosed in a particular subject and/or the type of imaging modality being used in conjunction with the SMSMs. The dose administered to a subject, in the context of the present disclosure should be sufficient to affect a beneficial diagnostic or therapeutic response in the subject. The size of the dose also can be determined by the existence, nature, and extent of any adverse side-effects that accompany the administration of a SMSM in a particular subject.

Within the scope of the present description, the effective amount of a SMSM or a pharmaceutically acceptable salt thereof for use in the manufacture of a medicament, the preparation of a pharmaceutical kit or in a method for preventing and/or treating a disease in a human subject in need thereof, is intended to include an amount in a range of from about 1 µg to about 50 grams.

The compositions of the present disclosure can be administered as frequently as necessary. Subjects

The subjects that can be treated with the SMSMs and methods described herein can be any subject that produces mRNA that is subject to alternative splicing, *e.g.*, the subject may be a eukaryotic subject, such as a plant or an animal. In some embodiments, the subject is a mammal, *e.g.*, human. In some embodiments, the subject is a human. In some embodiments, the subject is a non-human animal. In some embodiments, the subject is a fetus, an embryo, or a child. In some embodiments, the subject is a non-human primate such as chimpanzee, and other apes and monkey species; farm animals such as cattle, horses, sheep, goats, swine; domestic animals such as rabbits, dogs, and cats; laboratory animals including rodents, such as rats, mice and guinea pigs, and the like. In some embodiments, the subject is prenatal (*e.g.*, a fetus), a child (*e.g.*, a neonate, an infant, a toddler, a preadolescent), an adolescent, a pubescent, or an adult (*e.g.*, an early adult, a middle-aged adult, a senior citizen).

### Methods of Making Compounds

Compounds described herein can be synthesized using standard synthetic techniques or using methods known in the art in combination with methods described herein. Unless otherwise indicated, conventional methods of mass spectroscopy, NMR, HPLC, protein chemistry, biochemistry, recombinant DNA techniques and pharmacology can be employed. Compounds can be prepared using standard organic chemistry techniques such as those described in, for example, March's Advanced Organic Chemistry, 6th Edition, John Wiley and Sons, Inc. Alternative reaction conditions for the synthetic transformations described herein may be employed such as variation of solvent, reaction temperature, reaction time, as well as different chemical reagents and other reaction conditions. The starting materials can be available from commercial sources or can be readily prepared. By way of example only, provided are schemes for preparing the SMSMs described herein.

Suitable reference books and treatise that detail the synthesis of reactants useful in the preparation of compounds described herein, or provide references to articles that describe the preparation, include for example, "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley Interscience, New York, 1992. Additional suitable reference books and treatise that detail the synthesis of reactants useful in the preparation of compounds described herein, or provide references to articles that describe the preparation, include for example, Fuhrhop, J. and Penzlin G. "Organic Synthesis: Concepts, Methods, Starting Materials", Second, Revised and Enlarged Edition (1994) John Wiley & Sons ISBN: 3 527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

In the reactions described, it may be necessary to protect reactive functional groups, for example hydroxy, amino, imino, thio or carboxy groups, where these are desired in the final product, in order to avoid their unwanted participation in reactions. A detailed description of techniques applicable to the creation of protecting groups and their removal are described in Greene and Wuts, Protective Groups in Organic Synthesis, 3rd Ed., John Wiley & Sons, New York, NY, 1999, and Kocienski, Protective Groups, Thieme Verlag, New York, NY, 1994.

SMSMs can be made using known techniques and further chemically modified, in some embodiments, to facilitate intranuclear transfer to, *e.g.*, a splicing complex component, a spliceosome or a pre-mRNA molecule. One of ordinary skill in the art will appreciate the standard medicinal chemistry approaches for chemical modifications for intranuclear transfer (*e.g.*, reducing charge, optimizing size, and/or modifying lipophilicity).

### General Synthesis Scheme 1:

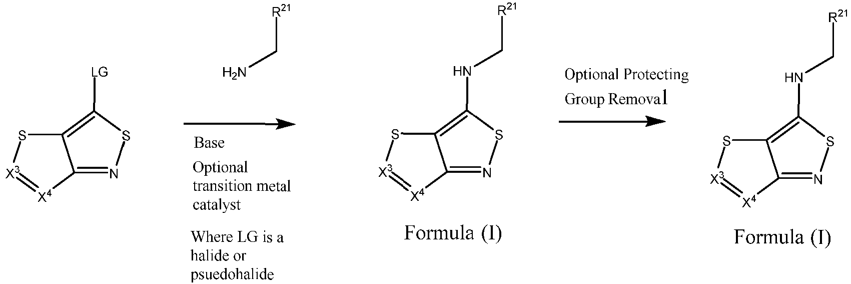

### General Synthesis Scheme 2:

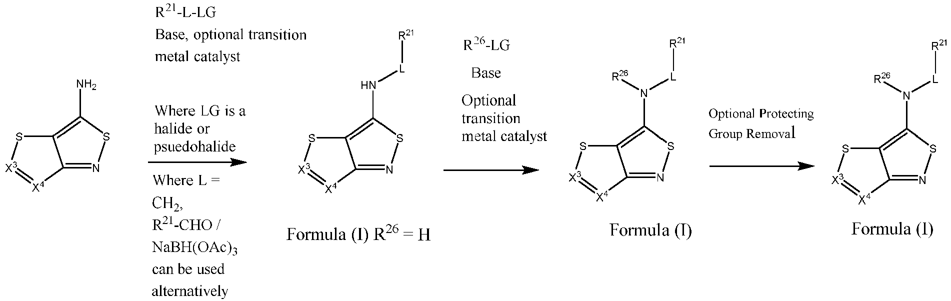

### General Synthesis Scheme A

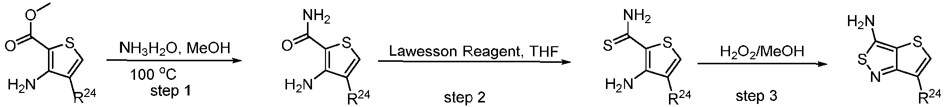

### General Synthesis Scheme B:

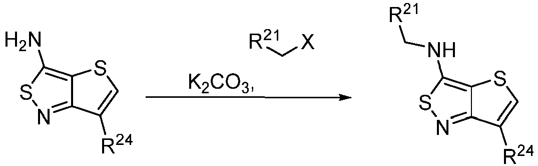

### General Synthesis Scheme C:

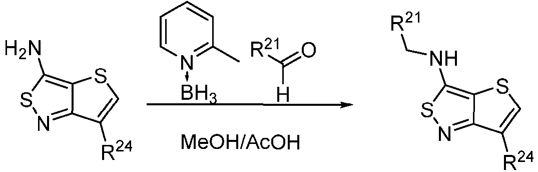

### General Synthesis Scheme D:

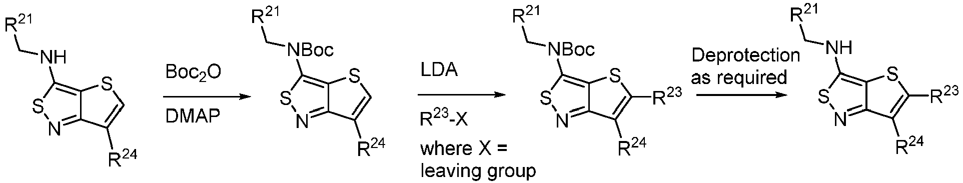

### General Synthesis Scheme E

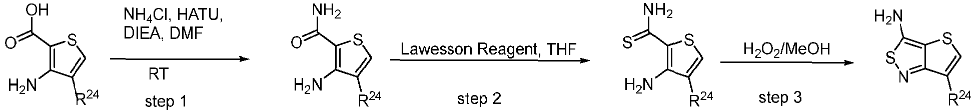

### General Synthesis Scheme F:

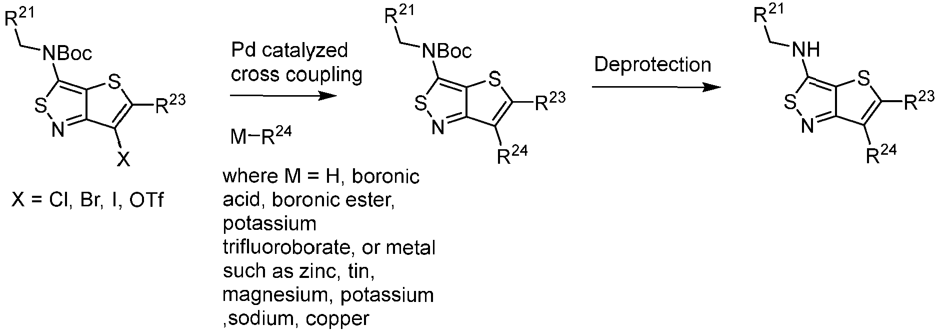

### General Synthesis Scheme G:

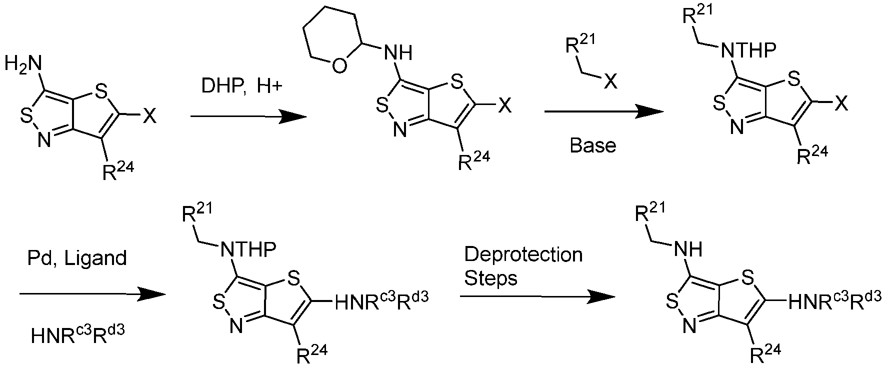

### General Synthesis Scheme H:

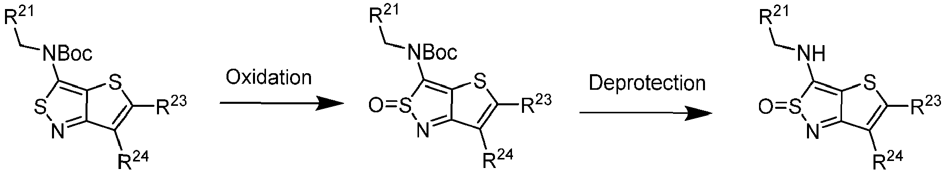

### General Synthesis Scheme I:

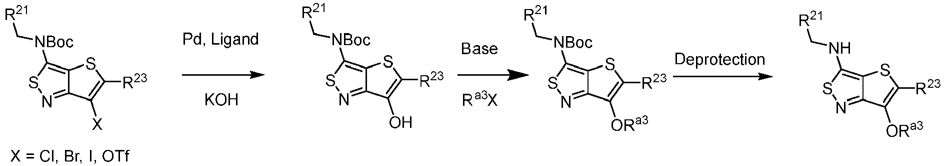

### General Synthesis Scheme J

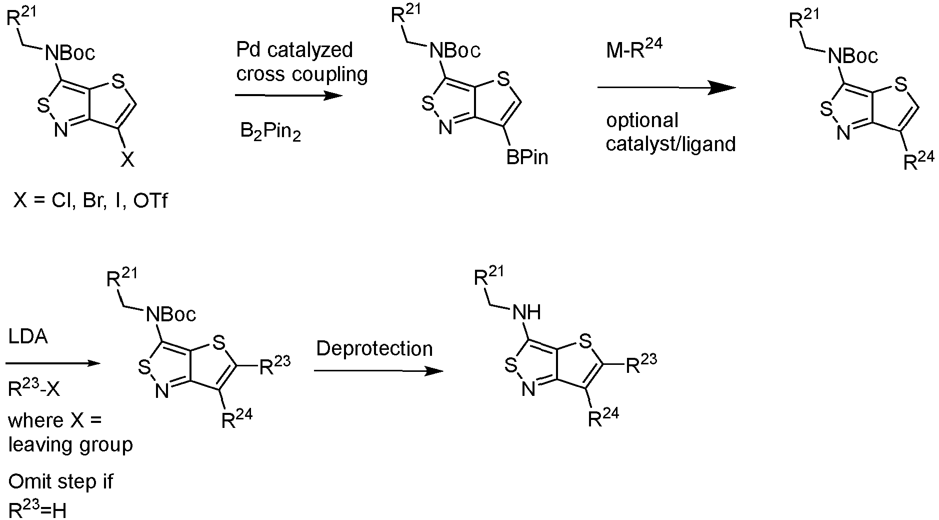

### General Synthesis Scheme K

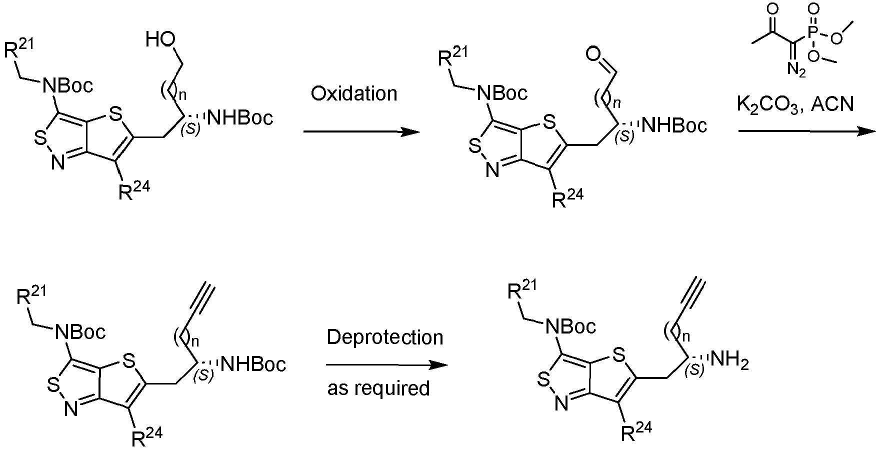

### General Synthesis Scheme L

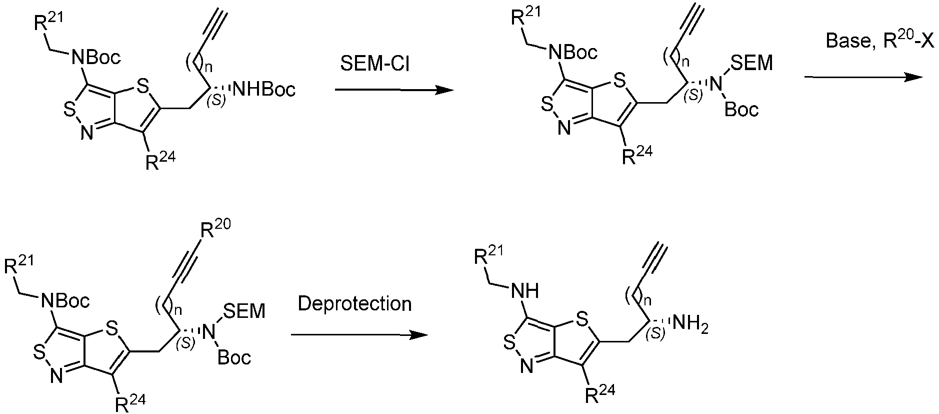

### General Synthesis Scheme M

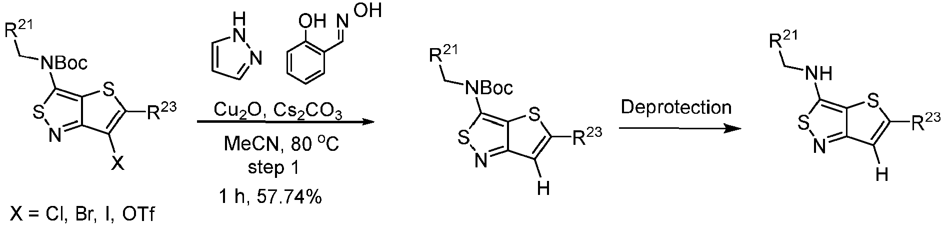

### General Synthesis Scheme N

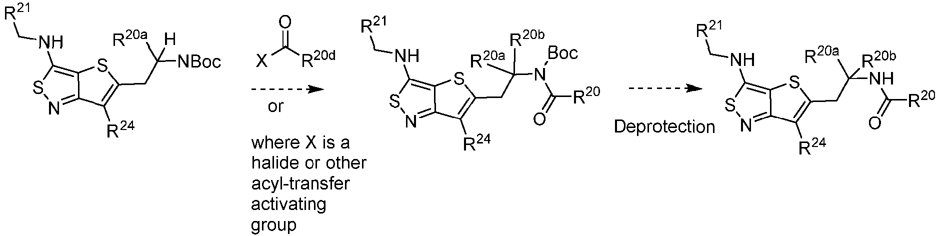

### General Synthesis Scheme O

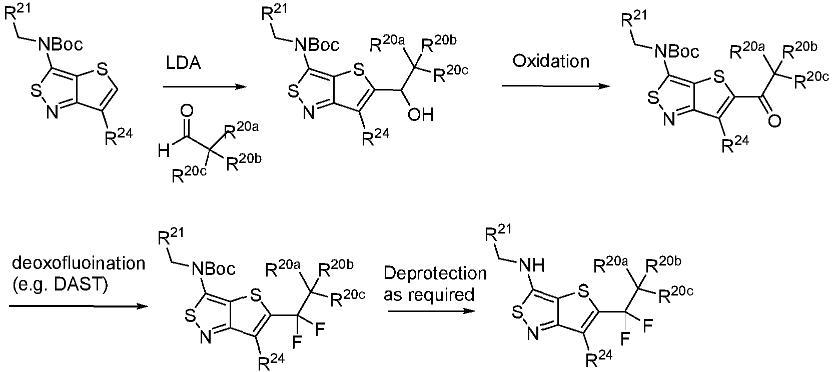

### General Synthesis Scheme P

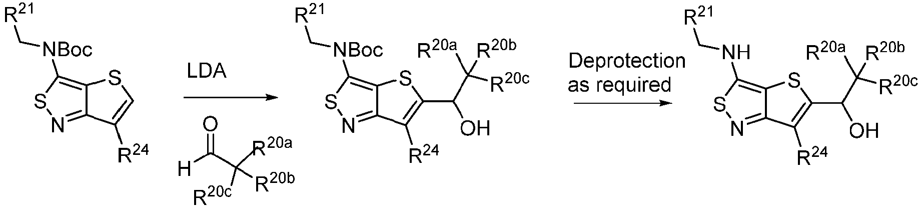

### General Synthesis Scheme Q

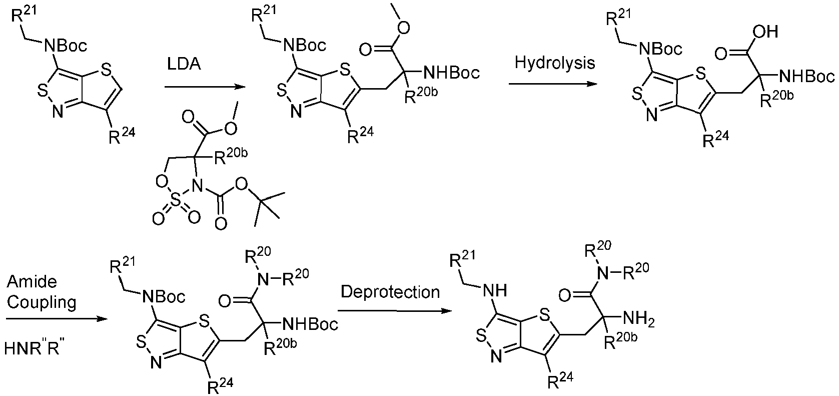

### General Synthesis Scheme R:

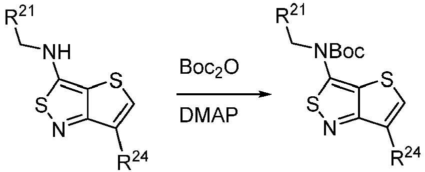

### General Synthesis Scheme S

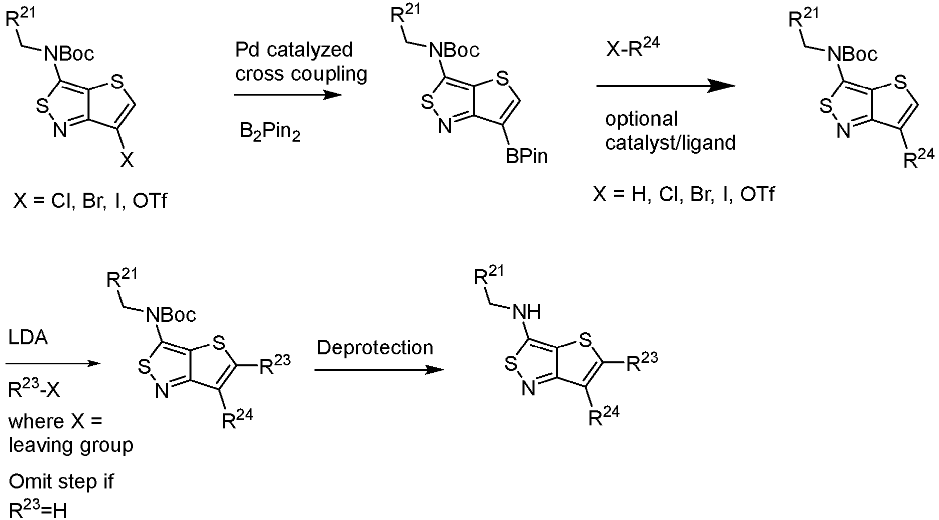

### EXAMPLES

These examples are provided for illustrative purposes only and not to limit the scope of the claims provided herein. The starting materials and reagents used for the synthesis of the compounds described herein may be synthesized or can be obtained from commercial sources, such as, but not limited to, Sigma-Aldrich, Acros Organics, Fluka, and Fisher Scientific.

### Example 1.References and syntheses of common building blocks and starting materials

### General Procedure for the Synthesis of Cyclic Sulfamidates AA:

Cyclic Sulfamidates can be synthesized by the following general method starting from the appropriate amino alcohol.

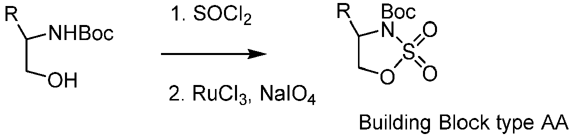

Additional synthetic procedures and/or literature references for known cyclic sulfamidates are provided below.

### Reference for tert-butyl (S)-4-methyl-1,2,3-oxathiazolidine-3-carboxylate 2,2-dioxide (AA-1) Bower, J.F., Szetzo, P.; Gallagher, T. Org. Lett. 2007, 9, 3283-3286.

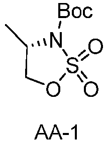

### Synthesis of Tert-butyl (R)-4-((S)-1-fluoroethyl)-1,2,3-oxathiazolidine-3-carboxylate 2,2-dioxide (AA-2)

### Step 1: Synthesis of 3-(tert-butyl) 4-methyl (4S,5R)-5-methyl-1,2,3-oxathiazolidine-3,4-dicarboxylate2,2-dioxide

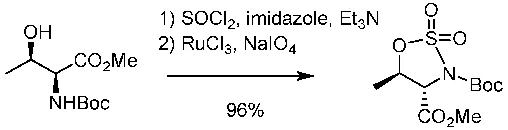

To a solution of imidazole (3.62 g, 4 Eq, 53.2 mmol), triethylamine (3.36 g, 4.63 mL, 2.5 Eq, 33.2 mmol) in DCM (60 mL) at -60°C was added dropwise thionyl chloride (1.74 g, 1.07 mL, 1.10 Eq, 14.6 mmol) followed by methyl (tert-butoxycarbonyl)-L-threoninate (3.10 g, 1 Eq, 13.3 mmol) in DCM (30.00 mL) keeping the reaction mixture below -55°C. The turbid mixture was allowed to warm to rt slowly and stirred 30 minutes. The reaction was quenched with 0.5N HCl (150 mL) and the water layer was extracted with DCM (2 x 75 mL). The combined organic layers were washed with half brine and half water, dried over sodium sulfate, filtered, and concentrated in vacuo.

The crude mixture was redissolved in MeCN (50 mL), cooled to 0°C, and treated with sodium periodate (3.27 g, 1.15 Eq, 15.3 mmol) followed by ruthenium trichloride (276 mg, 88.6 µL, 0.1 Eq, 1.33 mmol) and water (50 mL). The reaction was stirred for 1h at 0°C and diluted with water and TBME and filtered through a pad of celite. The water layer was extracted with TBME two times. The combined organic layers were washed with brine, dried over sodium sulfate, filtered and concentrated in vacuo to afford 3-(tert-butyl) 4-methyl (4S,5R)-5-methyl-1,2,3-oxathiazolidine-3,4-dicarboxylate 2,2-dioxide (3.770 g, 12.77 mmol, 96.1 %).

1H NMR (299 MHz, CDCl₃) δ 5.06 - 4.72 (m, 1H), 4.51 (dd, J = 5.8, 1.4 Hz, 1H), 3.87 (s, 3H), 1.73 (d, J = 6.4 Hz, 3H), 1.57 (s, 9H).

### Step 2: Synthesis of methyl (2R,3S)-2-((tert-butoxycarbonyl)amino)-3-fluorobutanoate

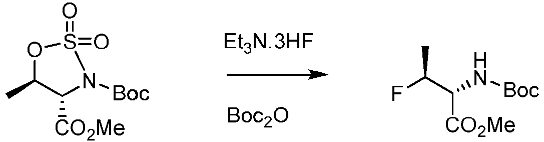

To a solution of 3-(tert-butyl) 4-methyl (4S,5R)-5-methyl-1,2,3-oxathiazolidine-3,4-dicarboxylate 2,2-dioxide (5.770 g, 1 Eq, 19.54 mmol) in THF (100.00 mL) was added triethylamine trihydrofluoride (20.47 g, 20.7 mL, 6.50 Eq, 127.0 mmol) and the reaction mixture was refluxed for 16h. The reaction was neutralized with a saturated solution of sodium bicarbonate until the pH tested basic. Next, boc anhydride (4.264 g, 1 eq, 19.54 mmol) was added in one portion. The reaction mixture was stirred for 30 minutes at room temperature and extracted with EtOAc twice. The combined organic layers were washed with water and brine, dried over sodium sulfate, filtered and concentrated in vacuo. The crude material was purified by column chromatography (Heptanes/EtOAc 90/10) to afford methyl (2R,3S)-2-((tert-butoxycarbonyl)amino)-3-fluorobutanoate (3.150 g, 13.39 mmol, 68.53 %).

¹H NMR (299 MHz, CDCl₃) δ 5.38 (s, 1H), 4.88 (ddd, J = 47.2, 6.4, 3.7 Hz, 1H), 4.47 (dd, J = 22.2, 9.0 Hz, 1H), 3.82 (s, 3H), 1.51 - 1.36 (m, 12H).

### Step 3: Synthesis of Tert-butyl ((2R,3S)-3-fluoro-1-hydroxybutan-2-yl)carbamate

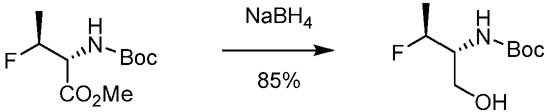

To a solution of methyl (2R,3S)-2-((tert-butoxycarbonyl)amino)-3-fluorobutanoate (3.08 g, 1 Eq, 13.1 mmol) in EtOH (65.00 mL) at 0°C was added NaBH₄ (1.24 g, 2.5 Eq, 32.7 mmol). The mixture was stirred at 0°C for 8h. The mixture was poured into 100 mL of water. The water layer was extracted with DCM (3 x 100 mL). The combined organic layers were washed with brine, dried over sodium sulfate, filtered and concentrated in vacuo. The crude material was purified by column chromatography (500 mL silica, Heptanes/EA, 65/35 to afford tert-butyl ((2R,3S)-3-fluoro-1-hydroxybutan-2-yl)carbamate (2.30 g, 11.1 mmol, 84.8 %).

¹H NMR (299 MHz, CDCl₃) δ 5.12 (s, 1H), 4.80 (dt, J = 48.0, 6.1 Hz, 1H), 4.04 - 3.88 (m, 1H), 3.85 - 3.59 (m, 2H), 1.97 (s, 1H), 1.56 - 1.33 (m, 12H).

### Step 4: Tert-butyl (R)-4-((S)-1-fluoroethyl)-1,2,3-oxathiazolidine-3-carboxylate 2,2-dioxide

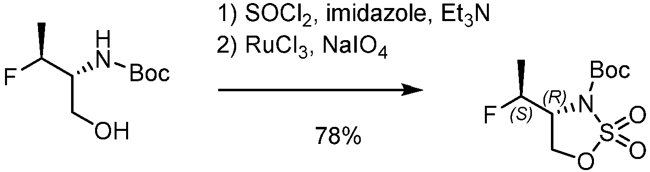

To a solution of imidazole (3.02 g, 4 Eq, 44.4 mmol), triethylamine (2.81 g, 3.87 mL, 2.5 Eq, 27.7 mmol) in DCM (60.00 mL) at -60°C was added dropwise thionyl chloride (1.45 g, 891 µL, 1.1 Eq, 12.2 mmol) followed by tert-butyl ((2R,3S)-3-fluoro-1-hydroxybutan-2-yl)carbamate (2.30 g, 1 Eq, 11.1 mmol) in DCM (30.00 mL) while keeping the temperature of the reaction mixture below - 55°C. The turbid mixture was allowed to warm to rt slowly and stirred for 30 minutes. The reaction was quenched with 0.5N HCl (150 mL) and the phases were separated. The water layer was extracted with DCM (2 x 75 mL). The combined organic layers washed brine (100 mL), dried over sodium sulfate, filtered, and concentrated in vacuo. To a solution of the crude material in MeCN (40.00 mL) at 0°C was added sodium periodate (2.73 g, 1.15 Eq, 12.8 mmol) followed by ruthenium trichloride (230 mg, 74.0 µL, 0.1 Eq, 1.11 mmol) and water (40.00 mL). The reaction was stirred 1h at 0°C and diluted with water (50 mL) and TBME (150 mL) and filtered through a pad of celite. The celite cake was washed with 50 mL of TBME. The water layer was extracted with TBME twice (2 x 75 mL). The combined organic layers were washed with brine (100 mL), dried over sodium sulfate, filtered and concentrated in vacuo. The crude material was purified by column chromatography (500 mL silica, Hept/EtOAc, 80/20 to 75/25) to afford tert-butyl (R)-4-((S)-1-fluoroethyl)-1,2,3-oxathiazolidine-3-carboxylate 2,2-dioxide (2.32 g, 8.62 mmol, 77.6 %) as a white solid. ¹H NMR (299 MHz, CDCl₃) δ 4.95 (d of quintets, J = 47.5, 6.3 Hz, 1H), 4.79 - 4.56 (m, 2H), 4.33 (dtd, J = 13.7, 5.5, 2.5 Hz, 1H), 1.58 (s, 9H), 1.43 (d, J = 24.1, 6.4, 1.1 Hz, 3H).

### Reference for tert-butyl (S)-4-(cyclopropylmethyl)-1,2,3-oxathiazolidine-3-carboxylate 2,2-dioxide (AA-3) WO2022/042657.

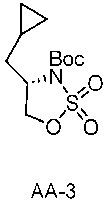

### Reference for tert-butyl (S)-4-(((tert-butyldimethylsilyl)oxy)methyl)-1,2,3-oxathiazolidine-3-carboxylate 2,2-dioxide (AA-4) Hebeisen, P.; Weiss, U.1 Alker, A.1 Ataempfli, A. Tetrahedron Lett. , 2011, 52, 5229-5233.

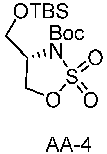

### Synthesis of Tert-butyl (R)-4-(difluoromethyl)-1,2,3-oxathiazolidine-3-carboxylate 2,2-dioxide (AA-5)

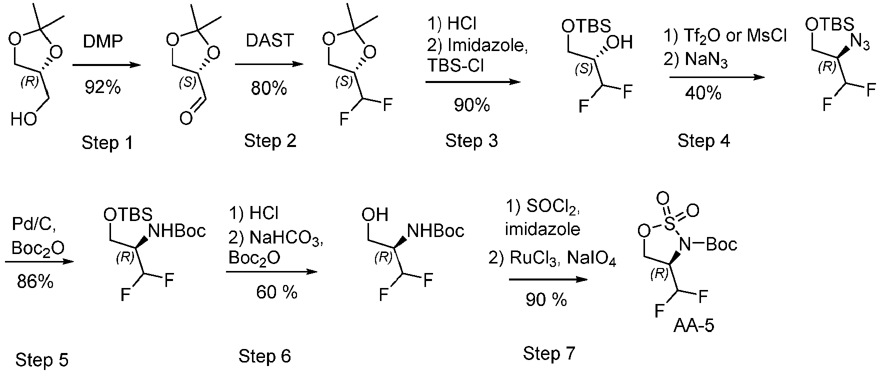

### Step 1. Synthesis of (S)-2,2-dimethyl-1,3-dioxolane-4-carbaldehyde

(*R*)-(2,2-dimethyl-1,3-dioxolan-4-yl)methanol (23.5 mL, 1 Eq, 189 mmol) was dissolved in DCM (800 mL) and sodium bicarbonate (79.5 g, 5 Eq, 946 mmol) was added. To the stirring suspension was then added, Dess-Martin periodinane (DMP) (120 g, 1.5 Eq, 284 mmol). After 4 hours, the mixture was quenched with 500 ml of water and sodium thiosulfate (150 g, 5 Eq, 946 mmol). The mixture was stirred for approximately 15 minutes until a non-cloudy mixture was obtained. The layers were then separated, and the aqueous layer was extracted three times with 300 mL of DCM. The combined organic layers were then dried over Na₂SO₄ and concentrated to afford 24.0 g of a crude pale-yellow oil. The crude oil was filtered and residue washed with diethyl ether. The filtrate was concentrated to afford (*S*)-2,2-dimethyl-1,3-dioxolane-4-carbaldehyde (22.5 g, 173 mmol, 91.4 %) as pale-yellow oil.

### Step 2. Synthesis of (S)-4-(difluoromethyl)-2,2-dimethyl-1,3-dioxolane

(*S*)-2,2-dimethyl-1,3-dioxolane-4-carbaldehyde (10.0 g, 1 Eq, 76.8 mmol) was dissolved in DCM (125 mL) and DAST (14.2 mL, 1.4 Eq, 108 mmol) was added dropwise at 0 °C. The mixture was allowed to warm up to rt and stirred for 3 hours. The mixture was quenched with sat. aq. sodium bicarbonate solution with cooling in an ice-water bath. The layers were then separated, and the aqueous layer was extracted twice with 100 mL of DCM. The combined organic layers were washed with brine, dried over Na₂SO₄, and concentrated to afford (*S*)-4-(difluoromethyl)-2,2-dimethyl-1,3-dioxolane (9.34 g, 61.4 mmol, 79.9 %) as yellow oil.

### Step 3. Synthesis of (S)-3-((tert-butyldimethylsilyl)oxy)-1,1-difluoropropan-2-ol

(*S*)-4-(difluoromethyl)-2,2-dimethyl-1,3-dioxolane (2.6 g, 1 Eq, 17 mmol) was dissolved in MeOH (30 mL) and HCl in diethyl ether (13 mL, 2 molar, 1.5 Eq, 26 mmol) was added at 0 °C. The resulting mixture was stirred overnight at rt. After stirring overnight, TLC (40% EtOAc in heptane) indicated the formation of one new product with an R_{f} of 0.2 and the complete disappearance of the starting material at R_{f} = 0.95. The mixture was concentrated and this resulted in (S)-3,3-difluoropropane-1,2-diol (1.62 g, 14.5 mmol, 85 %) as pale green oil.

(*S*)-3,3-difluoropropane-1,2-diol (1.46 g, 1 Eq, 13.0 mmol) was dissolved in DMF (45 mL) and imidazole (1.95 g, 2.2 Eq, 28.7 mmol) was added. The mixture was cooled down to 0 °C and of tert-butylchlorodimethylsilane (2.00 g, 1.02 Eq, 13.3 mmol) was then added. After two hours the mixture was diluted with 60 mL of TBME and 60 mL of water. The layers were separated, and the water layer was extracted two more times with 40 mL of TBME. The combined organic layers were then washed with a small amount (5 mL) of water (2x). The combined organic layers were then dried over Na₂SO₄ and concentrated to afford (*S*)-3-((tert-butyldimethylsilyl)oxy)-1,1-difluoropropan-2-ol (2.68 g, 11.8 mmol, 90.9 %) as pale-yellow oil.

### Step 4. Synthesis of (R)-(2-azido-3,3-difluoropropoxy)(tert-butyl)dimethylsilane

(*S*)-3-((tert-butyldimethylsilyl)oxy)-1,1-difluoropropan-2-ol (1.65 g, 1 Eq, 7.29 mmol) was dissolved in DCM (50 mL) and cooled down to -20 °C using an acetonitrile/dry ice bath. Next, 2,6-lutidine (1.69 mL, 2 Eq, 14.6 mmol) was added followed by the drop-wise addition of triflic anhydride (1.42 mL, 1.15 Eq, 8.38 mmol) (caution: exothermic). The mixture was stirred at -20 °C during the addition (20 minutes) followed by 60 minutes at -10 °C and two hours at 0 °C. The mixture was diluted with diethyl ether (100 ml) and washed with 15 mL each of water, 1M HCl, sat. aq. bicarbonate and brine. The organic phase was dried over Na₂SO₄ and concentrated to afford (S)-3-((tert-butyldimethylsilyl)oxy)-1,1-difluoropropan-2-yl trifluoromethanesulfonate (2.9 g, 8.1 mmol, 110 %) as orange oil.

**Step 5. Synthesis of tert-butyl (*R*)-(3-((tert-butyldimethylsilyl)oxy)-1,1-difluoropropan-2-yl)carbamate** (*R*)-(2-azido-3,3-difluoropropoxy)(tert-butyl)dimethylsilane (280 mg, 1 Eq, 1.11 mmol) was left to stir at 50 °C in EtOAc (10 mL) with Pd(OH)₂ (46.9 mg, 0.3 Eq, 334 µmol) and Boc₂O (267 mg, 282 µL, 1.1 Eq, 1.23 mmol) in a hydrogen atmosphere overnight. The mixture was then cooled to rt, diluted with 50 mL of EtOAc and filtered over Celite^{®}. The filtrate was dried over Na₂SO₄ and concentrated to afford 400 mg of a colorless oil. The crude was purified on 12 g of silica using 0-20% EtOAc in heptane to afford tert-butyl (R)-(3-((tert-butyldimethylsilyl)oxy)-1,1-difluoropropan-2-yl)carbamate (310 mg, 952 µmol, 85.5 %) as a colorless oil.

### Step 6. Synthesis of Tert-butyl (R)-(1,1-difluoro-3-hydroxypropan-2-yl)carbamate

Tert-butyl (*R*)-(3-((tert-butyldimethylsilyl)oxy)-1,1-difluoropropan-2-yl)carbamate (300 mg, 1 Eq, 922 µmol) was dissolved in THF (5 mL) and water (5 mL).Then, HCl (1.38 mL, 4 molar, 6 Eq, 5.53 mmol) was added and mixture was stirred overnight. Next morning, mixture was neutralized with sodium hydrogen carbonate (619 mg, 8 Eq, 7.37 mmol) and subsequently di-tert-butyl dicarbonate (201 mg, 1 Eq, 922 µmol) was added. After 1.5 hours, the mixture was extracted with 2x 10 mL of EtOAc, washed with 5 mL of 0.5N HCl, 5 mL of sat. aq. sodium bicarbonate solution, dried over Na₂SO₄, filtered, and concentrated to afford 300 mg of a crude colorless oil. Purification by silica gel chromatography afford tert-butyl (*R*)-(1,1-difluoro-3-hydroxypropan-2-yl)carbamate (100 mg, 473 µmol, 51.4 %) as colorless oil.

### Step 7. Synthesis of Tert-butyl (R)-4-(difluoromethyl)-1,2,3-oxathiazolidine-3-carboxylate 2,2-dioxide (AA-5).

To a solution of imidazole (2.32 g, 4 Eq, 34.1 mmol) and triethylamine (2.97 mL, 2.5 Eq, 21.3 mmol) in DCM (60 mL) at -60 °C was added thionyl chloride (1.22 g, 746 µL, 1.2 Eq, 10.2 mmol) dropwise. Subsequently, tert-butyl (R)-(1,1-difluoro-3-hydroxypropan-2-yl)carbamate (1.80 g, 1 Eq, 8.52 mmol) in DCM (15 mL) was added in a slow stream keeping the reaction around -60 °C. The reaction was allowed to warm to RT and stirred over the course of 1 hour and monitored by TLC. The TLC (20 % EtOAc in heptane) indicated the complete conversion of the starting material (R_{f} = 0.5) to one major product (R_{f} = 0.45). The mixture was quenched with 10 ml of 0.5M HCl, separated and the aq. layer was extracted twice more with 10 mL of DCM. The combined organic layers were dried over Na₂SO₄ and concentrated to afford the desired cyclized product tert-butyl (4R)-4-(difluoromethyl)-1,2,3-oxathiazolidine-3-carboxylate 2-oxide (2.00 g, 7.77 mmol, 91.2%) as colorless oil.

The oil thus obtained was then dissolved in MeCN (15 mL) and cooled to 0 °C. A portion of sodium periodate (2.10 g, 1.15 Eq, 9.80 mmol) was added, followed by ruthenium(III) chloride (177 mg, 0.1 Eq, 852 µmol) and water (15 mL). The mixture was stirred at 0 °C for 75 minutes. After this time, the black/intense red mixture was diluted with 15 mL of TBME and 15 mL of water and filtered on Celite. The filter cake was washed with 3x 20 ml of TBME and the organic and aqueous layers were separated. The organic layer was washed with brine, dried over Na₂SO₄, filtered and concentrated to afford tert-butyl (R)-4-(difluoromethyl)-1,2,3-oxathiazolidine-3-carboxylate 2,2-dioxide (2.00 g, 7.32 mmol, 85.9 %) as white crystalline material. ¹H NMR (299 MHz, CDCl₃) δ 6.14 (ddt, *J =* 56.3, 54.1, 2.1 Hz, 1H), 4.79 (m, 1H), 4.69 (m, 1H), 4.61 - 4.47 (m, 1H), 1.57 (s, 9H).

### Reference for tert-butyl (S)-4-(2-((tert-butyldimethylsilyl)oxy)ethyl)-1,2,3-oxathiazolidine-3-carboxylate 2,2-dioxide (AA-6): Zhang, N.; Arnold, M.A.; Dakka, A.; Karp, G.M.; Luong, T.T.; Narasimhan, J.; Naryshkin, N.A.; Wang, J.; Zhang, X. WO 2020167628.

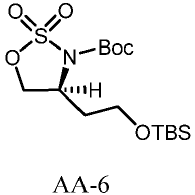

### Reference for tert-butyl (S)-4-(methoxymethyl)-1,2,3-oxathiazolidine-3-carboxylate 2,2-dioxide (AA-7) WO2017/080979A1.

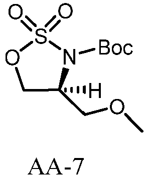

### Reference for tert-butyl (R)-4-(fluoromethyl)-1,2,3-oxathiazolidine-3-carboxylate 2,2-dioxide (AA-8) WO2020/167628A1.

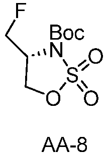

### Tert-butyl (S)-4-(2,2,2-trifluoroethyl)-1,2,3-oxathiazolidine-3-carboxylate 2,2-dioxide (AA-9) was synthesized from tert-butyl (S)-(4,4,4-trifluoro-1-hydroxybutan-2-yl)carbamate (WO2012/116279) according to the general method

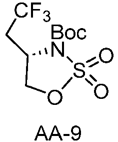

### Reference for tert-butyl 1,2,3-oxathiazinane-3-carboxylate 2,2-dioxide (AA-10) Moss, T.A.; Alonso, B.; Denwick, D.R.; Dixon, D.J. Angew. Chem. Int. Ed. 2010 , 49, 568 -571.

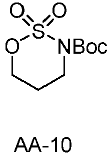

### Synthesis of tert-butyl 4-(oxetan-3-yl)-2,2-dioxo-1,2lambda6,3-oxathiazolidine-3-carboxylate(AA-11)

### Step 1.

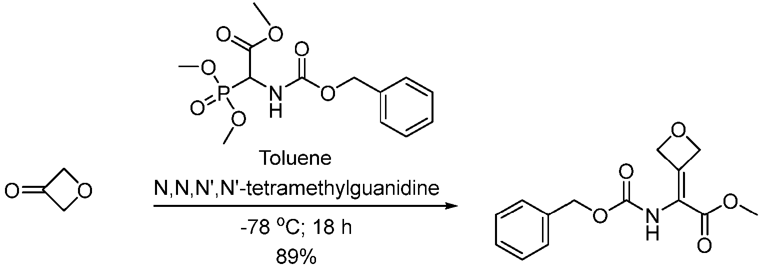

To a stirred solution of methyl 2-{[(benzyloxy)carbonyl]amino}-2-(dimethoxyphosphoryl)acetate (10 g, 30.188 mmol, 1 equiv) in toluene (100 mL) under an nitrogen atmosphere was added 1,1,3,3-tetramethylguanidine (3.48 g, 30.188 mmol, 1 equiv) at -78 °C and stirred for 30 min. Then a solution of 3-oxetanone (2.18 g, 30.188 mmol, 1 equiv) in toluene (10 mL) was added to the reaction mixture at -78 °C. The reaction mixture was warmed to room temperature and stirred for 18 h. After consumption of the starting material (monitored by TLC), the reaction mixture was diluted with water (250 mL) and extracted with EtOAc (2 x 250 mL) and 20% MeOH: CH₂Cl₂ (2 x 250 mL). The combined organic extracts were dried over sodium sulfate, filtered and concentrated in vacuo. The crude material was purified by column chromatography using 5-10% MeOH: CH₂Cl₂ to afford methyl 2-{[(benzyloxy)carbonyl]amino}-2-(oxetan-3-ylidene)acetate (7.45 g, 89.00%) as a white solid.

### Step 2.

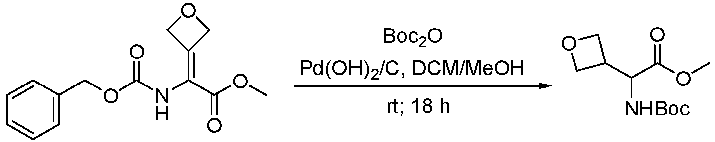

A solution of methyl 2-{[(benzyloxy)carbonyl]amino}-2-(oxetan-3-ylidene)acetate (4 g, 14.426 mmol, 1 equiv) in DCM/MeOH(1/1; 50 mL) was treated with Pd(OH)₂/C (800 mg, 5.697 mmol, 0.39 equiv) and Boc₂O (3.15 g, 14.426 mmol, 1 equiv). The resulting mixture was stirred for 18 h at room temperature. The reaction was monitored by LCMS. The residue was purified by reverse flash chromatography with the following conditions: column, C18 silica gel; mobile phase, MeCN in Water (10mmol/L NH₄HCO₃), 10% to 50% gradient in 10 min; detector, UV 254 nm. This resulted in methyl 2-[(tert-butoxycarbonyl)amino]-2-(oxetan-3-yl)acetate (1.3 g, 36.74%) as a yellow oil..

### Step 3.

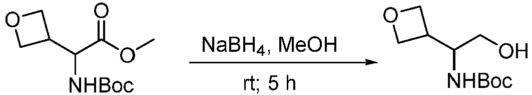

A solution of methyl 2-[(tert-butoxycarbonyl)amino]-2-(oxetan-3-yl)acetate (1.3 g, 5.300 mmol, 1 equiv) in MeOH (13.00 mL) was treated with NaBH₄ (0.60 g, 15.900 mmol, 3 equiv) at 0 °C. The resulting mixture was stirred for 5 h at room temperature. The reaction was monitored by LCMS. The residue was purified by silica gel column chromatography, eluted with PE / EA (1/1) to afford tert-butyl N-[2-hydroxy-1-(oxetan-3-yl)ethyl]carbamate (700 mg, 60.79%) as a yellow oil.

### Step 4.

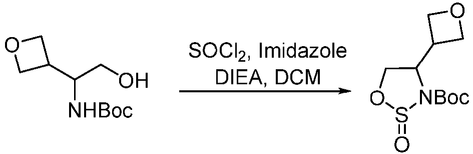

To a solution of Imidazole (1.00 g, 14.728 mmol, 4 equiv) in DCM (24 mL) were added SOCl2 (0.40 mL, 5.523 mmol, 1.5 equiv) followed by DIEA (1.28 mL, 7.364 mmol, 2 equiv) dropwise at 0°C. To the above mixture was added tert-butyl N-[2-hydroxy-1-(oxetan-3-yl)ethyl]carbamate (800 mg, 3.682 mmol, 1 equiv) dissolved in DCM (4 mL) dropwise at 0°C. The resulting mixture was stirred for additional 1h at room temperature. The resulting mixture was washed with 3 x 20 mL of conc. HCl(1M). The resulting mixture was concentrated under vacuum. The residue was purified by silica gel column chromatography, eluted with PE / EA (1: 1) to afford tert-butyl 4-(oxetan-3-yl)-2-oxo-1,2lambda4,3-oxathiazolidine-3-carboxylate (780 mg, 80.45%) as a yellow oil.

### Step 5.

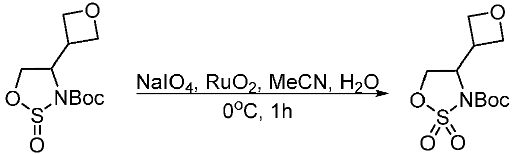

To a solution of tert-butyl 4-(oxetan-3-yl)-2-oxo-1,2lambda4,3-oxathiazolidine-3-carboxylate (780 mg, 2.962 mmol, 1 equiv) in MeCN (10 mL) and H₂O (6 mL) was added NaIO4 (760.32 mg, 3.554 mmol, 1.20 equiv) and ruthenium(iv) oxide hydrate (8.95 mg, 0.059 mmol, 0.02 equiv) at 0°C. The resulting mixture was stirred for 1h at 0°C. The resulting mixture was filtered through a Celite pad. The filtrate was extracted with EtOAc (3 x 20 mL). The combined organic layers were washed with brine (20 mL), dried over anhydrous Na2SO4. After filtration, the filtrate was concentrated under reduced pressure. The residue was purified by silica gel column chromatography, eluted with PE / EA (1:1) to afford tert-butyl 4-(oxetan-3-yl)-2,2-dioxo-1,2lambda6,3-oxathiazolidine-3-carboxylate (470 mg, 56.80%) as a white solid.

### Synthesis of tert-butyl (S)-4-(3,3-difluorocyclobutyl)-1,2,3-oxathiazolidine-3-carboxylate 2,2-dioxide (AA-12): Compound AA-12 was made from tert-butyl N-[(1S)-1-(3,3-difluorocyclobutyl)-2-hydroxyethyl]carbamate following the general procedure.

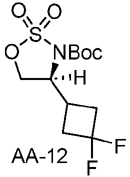

### Synthesis of tert-butyl N-[(1S)-1-(3,3-difluorocyclobutyl)-2-hydroxyethyl]carbamate

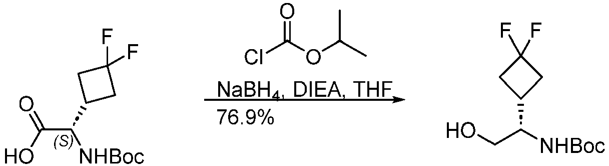

A solution of (S)-[(tert-butoxycarbonyl)amino](3,3-difluorocyclobutyl)acetic acid (4.8 g, 18.096 mmol, 1 equiv) in THF (50 mL) was treated with DIEA (4.68 g, 36.192 mmol, 2 equiv) and isopropyl chloroformate (2.88 g, 23.525 mmol, 1.3 equiv) at 0 °C for 30 min under nitrogen atmosphere followed by the addition of NaBH₄ (2.74 g, 72.384 mmol, 4 equiv) in portions at 0 °C. The resulting mixture was stirred for 2h at room temperature. The reaction was quenched with water at 0°C. The resulting mixture was extracted with EtOAc (3 x 50 mL). The combined organic layers were washed with brine (50 mL), dried over anhydrous Na₂SO₄. After filtration, the filtrate was concentrated under reduced pressure. The residue was purified by silica gel column chromatography, eluted with PE / EA (5: 1) to afford tert-butyl N-[(1S)-1-(3,3-difluorocyclobutyl)-2-hydroxyethyl]carbamate (3.5 g, 76.97%) as a colorless oil.

### Synthesis of tert-butyl (S)-4-(2,2-difluoroethyl)-1,2,3-oxathiazolidine-3-carboxylate (AA-13)

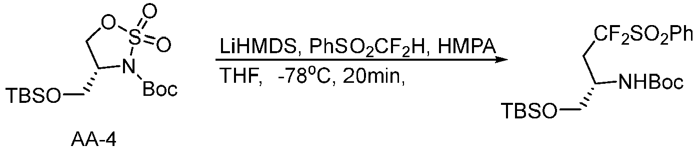

### Step 1: tert-butyl (S)-(1-((tert-butyldimethylsilyl)oxy)-4,4-difluoro-4-(phenylsulfonyl)butan-2-yl)carbamate

LiHMDS (1M solution in THF, 16.33 mL, 16.3 mmol, 1.2 equiv) was added slowly to a solution of tert-butyl (R)-4-(((tert-butyldimethylsilyl)oxy)methyl)-1,2,3-oxathiazolidine-3-carboxylate 2,2-dioxide (5 g, 13.6 mmol, 1 equiv) and (difluoromethane)sulfonylbenzene (2.13 mL, 15.0 mmol, 1.1 equiv) in THF (150 mL) and HMPA (6.90 mL, 39.5 mmol, 2.9 equiv) at -78 oC under N2. The reaction mixture was stirred at this temperature for 20 min, and then quenched with 20% aqueous sulfuric acid (60 mL). The mixture was extracted with EtOAc and the combined organic layers were washed with saturated NaHCO3 solution and then dried over Na2SO4. The sodium salts were filtered off and the filtrate was concentrated under reduced pressure. The residue was purified by silica gel column chromatography (PE / EA = 3:1) affording the product as colorless oil (6.3 g, 97%).

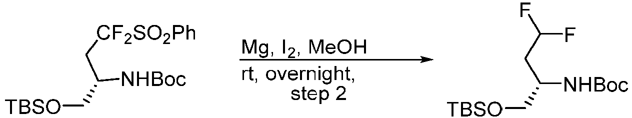

### Step 2: tert-butyl (S)-(1-((tert-butyldimethylsilyl)oxy)-4,4-difluorobutan-2-yl)carbamate

10 mL of methanol and I₂ (0.17 g, 0.69 mmol, 0.1 equiv) were added to Mg (5.02 g, 206.4 mmol, 30 equiv) stored under a nitrogen atmosphere. The mixture was cooled to 0 °C and a solution of tert-butyl (S)-(1-((tert-butyldimethylsilyl)oxy)-4,4-difluoro-4-(phenylsulfonyl)butan-2-yl)carbamate (3.3 g, 6.9 mmol, 1 equiv) in 30 mL methanol was added. The reaction mixture was allowed to warm to 20°C and stirred at this temperature overnight. The reaction was quenched with saturated ammonium chloride solution and extracted with EtOAc. The combined organic layers were washed with brine, dried over anhydrous sodium sulfate, and filtered. The solvents were evaporated, and the residue was purified by silica gel column chromatography (PE / EA = 7:1) affording the product as colorless oil (1.47 g, 63%).

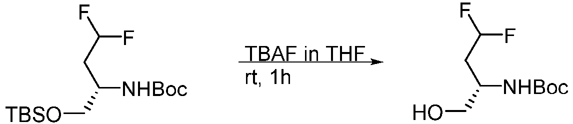

### Step 3: tert-butyl (S)-(4,4-difluoro-1-hydroxybutan-2-yl)carbamate

To a solution of tert-butyl (S)-(1-((tert-butyldimethylsilyl)oxy)-4,4-difluorobutan-2-yl)carbamate (700 mg, 2.06 mmol, 1 equiv) in THF (7 mL) was added TBAF (1.65 mL, 1.65 mmol, 0.8 equiv) dropwise at 0°C. The resulting mixture was stirred for 1h at room temperature and then concentrated under reduced pressure. The residue was purified by silica gel column chromatography (PE / EA = 1: 1) yielding the product as white solid (430 mg, 93%).

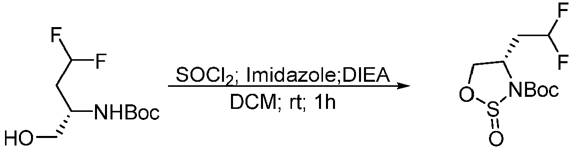

### Step 4. tert-butyl (4S)-4-(2,2-difluoroethyl)-1,2,3-oxathiazolidine-3-carboxylate 2-oxide

A portion of SOCl₂ (0.21 mL, 2.86 mmol, 1.5 equiv) followed by DIEA (0.67 mL, 3.82 mmol, 2 equiv) were added dropwise at 0°C to a solution of imidazole (520 mg, 7.64 mmol, 4 equiv) in DCM (13 mL). (S)-(4,4-difluoro-1-hydroxybutan-2-yl)carbamate (430 mg, 1.91 mmol, 1 equiv) dissolved in DCM (2 mL) was added dropwise at 0°C and the resulting mixture was stirred for 1h at room temperature. The mixture was washed with saturated NaHCO₃ solution and evaporated affording the product as yellow oil (500 mg, 97%).

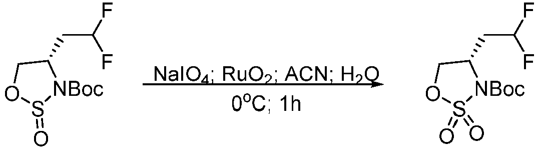

### Step 5: tert-butyl (S)-4-(2,2-difluoroethyl)-1,2,3-oxathiazolidine-3-carboxylate (AA-13)

To a solution of tert-butyl (4S)-4-(2,2-difluoroethyl)-1,2,3-oxathiazolidine-3-carboxylate 2-oxide (500 mg, 1.84 mmol, 1 equiv) in ACN (6.5 mL) and water (3.5 mL) were added NaIO₄ (591 mg, 2.76 mmol, 1.5 equiv) and ruthenium(iv) oxide hydrate (13.9 mg, 0.092 mmol, 0.05 equiv) at 0°C. The resulting mixture was stirred for 1h at 0°C, and then filtered. The filtrate was extracted with EtOAc and the combined organic layers were washed with brine (20 mL) and dried over anhydrous Na₂SO₄. The solvents were removed under reduced pressure and the residue was purified by silica gel column chromatography (PE / EA = 2:1). White solid (310 mg, 59%).

### Reference for tert-butyl (S)-4-phenyl-1,2,3-oxathiazolidine-3-carboxylate 2,2-dioxide (AA-14): James, T.; Simpson, I.; Grant, J. A.; Sridharan, V.; Nelson, A. Organic Letters, 2013, 15, 6094-6097.

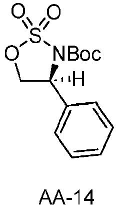

### Synthesis of tert-butyl (R)-4-((R)-1-fluoroethyl)-1,2,3-oxathiazolidine-3-carboxylate 2,2-dioxide (AA-15)

### Step 1. Synthesis of tert-butyl ((2R,3S)-1,3-dihydroxybutan-2-yl)carbamate

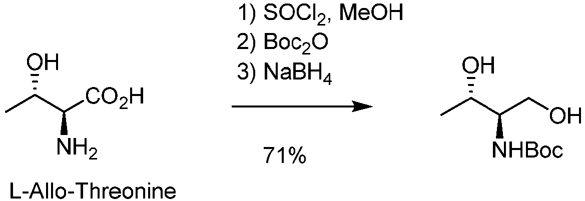

To a suspension of L-allothreonine (22.00 g, 97.00% Wt, 1 Eq, 179.1 mmol) in MeOH (500.00 mL) at 0°C, thionyl chloride (85.25 g, 52.30 mL, 4 Eq, 716.6 mmol) was added in a slow stream. The reaction mixture was allowed to warm to rt and refluxed for 3h and concentrated in vacuo. The crude material was diluted with DCM (500.00 mL) and Boc₂O (39.10 g, 41.2 mL, 1 Eq, 179.1 mmol) was added followed by triethylamine (54.38 g, 74.9 mL, 3 Eq, 537.4 mmol). The reaction mixture was stirred at rt for 90 minutes, washed with water twice and also brine, dried over sodium sulfate, filtered and concentrated in vacuo to afford methyl (tert-butoxycarbonyl)-L-allothreoninate (39.140 g, 167.80 mmol, 93.66 %). To a solution of methyl (tert-butoxycarbonyl)-L-allothreoninate (39.140 g, 1 Eq, 167.80 mmol) in EtOH (650.00 mL) at 0°C was added NaBH4 (12.70 g, 2 Eq, 335.59 mmol). The reaction mixture was stirred at 0°C overnight.

The reaction was quenched with water (400 mL) and diluted with DCM (500 mL). The mixture was stirred vigorously for 5 minutes. The phases were separated and the water layer was thoroughly extracted with DCM/EtOH 10% three times (3x 750 mL). The combined organic layers were washed with brine (500 mL) dried over sodium sulfate, filtered and concentrated in vacuo to afford tert-butyl ((2R,3S)-1,3-dihydroxybutan-2-yl)carbamate (26.11 g, 127.2 mmol, 75.81 %).

¹H NMR (299 MHz, CDCl₃) δ 5.57 - 5.13 (m, 1H), 4.10 - 3.93 (m, 2H), 3.87 - 3.66 (m, 1H), 3.51 (s, 1H), 2.62 (s, 2H), 1.47 (s, 9H), 1.31 (d, J = 6.4 Hz, 3H).

### Step 2, Synthesis of Tert-butyl ((2R,3S)-1-(benzyloxy)-3-hydroxybutan-2-yl)carbamate

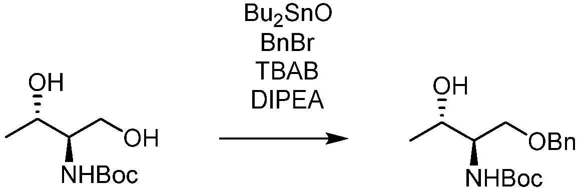

A mixture of dibutyltin oxide (2.814 g, 0.1 Eq, 11.30 mmol), tetrabutylammonium bromide (10.93 g, 0.30 Eq, 33.91 mmol), DIPEA (58.44 g, 78.8 mL, 4 Eq, 452.1 mmol) and benzyl bromide (77.33 g, 53.78 mL, 4 Eq, 452.1 mmol) and tert-butyl ((2R,3S)-1,3-dihydroxybutan-2-yl)carbamate (23.2g, 113 mmol) was stirred neat at 76°C for 18h under vigorous stirring. The reaction was cooled to room temperature and diluted with EtOAc and water. The water layer was extracted twice with EtOAc. The combined organic layers were washed with brine, dried over sodium sulfate, filtered and concentrated in vacuo. Purification by column chromatography (H/EA, 70/30) afforded tert-butyl ((2R,3S)-1-(benzyloxy)-3-hydroxybutan-2-yl)carbamate.

¹H NMR (299 MHz, CDCl₃) δ 7.51 - 7.31 (m, 5H), 5.28 (s, 1H), 4.64 - 4.48 (m, 2H), 3.96 - 3.77 (m, 2H), 3.73 = 3.56 (m, 2H), 2.92 (d, J = 8.2 Hz, 1H), 1.47 (s, 9H), 1.25 (d, 3H).

### Step 3. Synthesis of tert-butyl (4R,5S)-4-((benzyloxy)methyl)-5-methyl-1,2,3-oxathiazolidine-3-carboxylate 2,2-dioxide

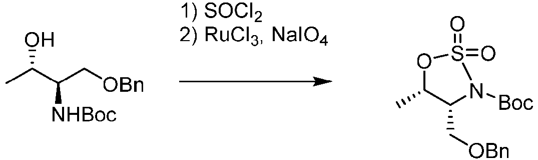

To a solution of imidazole (18.81 g, 4 Eq, 276.3 mmol) and triethylamine (17.47 g, 24.1 mL, 2.5 Eq, 172.7 mmol) in DCM (550.00 mL) at -60°C was added thionyl chloride (9.448 g, 5.796 mL, 1.15 Eq, 79.42 mmol) dropwise keeping the reaction mixture below -55°C. Next, a solution of tert-butyl ((2R,3S)-1-(benzyloxy)-3-hydroxybutan-2-yl)carbamate (20.40 g, 1 Eq, 69.06 mmol) in DCM (200.00 mL) was added dropwise keeping the reaction mixture below -55°C and the reaction mixture was allowed to warm to rt slowly. The reaction was quenched with 0.5N HCl (1L). The phases were separated and the acidic layer was extracted with DCM (2 x 500 mL). The combined organic layers were washed with brine (300 mL), dried over sodium sulfate, filtered and concentrated in vacuo. To a solution of the crude material in acetonitrile (200.00 mL) at 0°C was added sodium periodate (16.99 g, 1.15 Eq, 79.42 mmol) followed by ruthenium trichloride (1.433 g, 0.1 Eq, 6.906 mmol) and water (200.00 mL). The mixture was stirred at 0°C for 45 minutes and monitored by NMR for full conversion. The reaction was diluted with water (200 mL) and TBME (300 mL), and filtered through a pad of celite. The water layer was extracted with TBME (3 x 400 mL). The combined organic layers were washed with brine (300 mL), dried over sodium sulfate, filtered and concentrated in vacuo. The crude material was purified over a short plug of silica, eluting with TBME to afford tert-butyl (4R,5S)-4-((benzyloxy)methyl)-5-methyl-1,2,3-oxathiazolidine-3-carboxylate 2,2-dioxide (24.38 g, 68.21 mmol, 98.77 %).

¹H NMR (299 MHz, CDCl₃) δ 7.48 - 7.31 (m, 5H), 5.24 - 4.97 (m, 1H), 4.58 (s, 2H), 4.39 - 4.26 (m, 1H), 3.85 (dd, J = 10.2, 7.4 Hz, 1H), 3.67 (dd, J = 10.3, 2.9 Hz, 1H), 1.81 - 1.40 (m, 12H).

### Step 4. Synthesis of tert-butyl ((2R,3R)-1-(benzyloxy)-3-fluorobutan-2-yl)carbamate

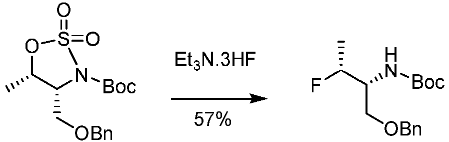

To a solution of tert-butyl (4R,5S)-4-((benzyloxy)methyl)-5-methyl-1,2,3-oxathiazolidine-3-carboxylate 2,2-dioxide (16.30 g, 1 Eq, 45.60 mmol) in toluene (250.00 mL) was added triethylamine trihydrofluoride (47.79 g, 48.3 mL, 6.5 Eq, 296.4 mmol) and the reaction was refluxed and stopped after 2.5 hours. The reaction was cooled to rt and the phases were separated. The bottom layer extracted twice with toluene (2 x 250 mL). The combined organic layers were washed with saturated sodium bicarbonate and brine, dried over sodium sulfate, filtered and concentrated in vacuo to afford 9 g of crude material. The crude was purified by column chromatography (Heptane/EA, 90/10) to afford tert-butyl ((2R,3R)-1-(benzyloxy)-3-fluorobutan-2-yl)carbamate (6.360 g, 21.39 mmol, 46.90 %). Additional material could be obtained as follows: further elution of the chromatography column afforded des-boc sulfamidate (Heptane/EA, 65/35). The initial aqueous layer was diluted with EtOAc (250 mL) and basified with a saturated sodium bicarbonate solution. The water layer was extracted twice with EtOAc (2 x 500 mL) and the combined organic layers washed with saturated sodium bicarbonate and brine, dried over sodium sulfate, filtered and concentrated in vacuo to afford 3.8 g of a mixture of compounds. The mixture was reprotected with Boc₂O and 1.57 g of additional desired product was isolated after column chromatography together with 1.7 g of de-boc sulfamidate which gave 2.36 g of boc-protected sulfamidate that was reconverted again to the desired product as described above. In total, 11.5 g of material were obtained starting from 24.4 g of starting material (57% yield).

¹H NMR (299 MHz, CDCl₃) δ 7.44 - 7.29 (m, 5H), 5.07 - 4.83 (m, 1H), 4.76 (d, J = 9.3 Hz, 1H), 4.55 (d, J = 2.0 Hz, 2H), 3.97 - 3.76 (m, 1H), 3.61 - 3.38 (m, 2H), 1.47 (s, 9H), 1.37 (dd, J = 24.4, 6.4 Hz, 3H).

### Step 5. Tert-butyl ((2R,3R)-3-fluoro-1-hydroxybutan-2-yl)carbamate

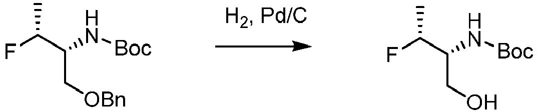

To a solution of tert-butyl ((2R,3R)-1-(benzyloxy)-3-fluorobutan-2-yl)carbamate (11.50 g, 1 Eq, 38.67 mmol) in EtOAc (250.00 mL) was added PdOH₂ (1.20 g, 0.221 Eq, 8.55 mmol) and the reaction mixture was stirred under an Hydrogen gas overnight. The reaction was deemed complete by NMR. The mixture was filtered over celite and the cake was washed twice with EtOAc (2 x 200 mL) and concentrated in vacuo. The crude material was purified over column chromatography (Heptanes/EA, 60/40) tp afford a total 5.80 g of tert-butyl ((2R,3R)-3-fluoro-1-hydroxybutan-2-yl)carbamate.

¹H NMR (299 MHz, CDCl₃) δ 5.24 - 4.56 (m, 2H), 3.90 - 3.48 (m, 3H), 2.09 (d, J = 14.2 Hz, 1H), 1.48 (s, 9H), 1.40 (dd, J = 24.7, 6.4 Hz, 3H).

### Step 6. Synthesis of tert-butyl (R)-4-((R)-1-fluoroethyl)-1,2,3-oxathiazolidine-3-carboxylate 2,2-dioxide

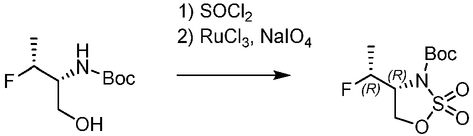

To a solution of imidazole (7.62 g, 4 Eq, 112 mmol), triethylamine (7.08 g, 9.75 mL, 2.5 Eq, 70.0 mmol) in DCM (160 mL) at -60°C was added dropwise thionyl chloride (3.99 g, 2.45 mL, 1.2 Eq, 33.6 mmol) followed by tert-butyl ((2R,3R)-3-fluoro-1-hydroxybutan-2-yl)carbamate (5.80 g, 1 Eq, 28.0 mmol) in DCM (60 mL) keeping the reaction mixture below -55°C. The turbid mixture was allowed to warm to rt slowly and stirred for 30 minutes. The reaction was quenched with 0.5N HCl (500 mL). The phases were separated and the water layer was extracted with DCM (2 x 200 mL). The combined organic layers were washed with brine (250 mL), dried over sodium sulfate, filtered, and concentrated in vacuo.

To a solution of the crude material in acetonitrile (60 mL) at 0°C was added sodium periodate (6.88 g, 1.15 Eq, 32.2 mmol) followed by ruthenium trichloride (580 mg, 187 µL, 0.1 Eq, 2.80 mmol) and water (60 mL). The mixture was stirred at 0°C for 45 minutes. Full conversion was noted by NMR. The reaction was diluted with water (200 mL) and TBME (300 mL), filtered over celite. The water layer was extracted with TBME (3 x 400 mL). The combined organic layers were washed with brine (300 mL), dried over sodium sulfate, filtered and concentrated in vacuo. Crude material purified by column chromatography (500 mL silica, Hept/EtOAc, 80/20 to 75/25) to afford tert-butyl (R)-4-((R)-1-fluoroethyl)-1,2,3-oxathiazolidine-3-carboxylate 2,2-dioxide (5.46 g, 20.3 mmol, 72.5 %) as a white solid.

¹H NMR (299 MHz, CDCl₃ δ 5.23 - 4.88 (m, 1H), 4.69 - 4.64 (m, 2H), 4.61 - 4.51 (m, 1H), 1.58 (s, 9H), 1.48 (dd, J = 24.4, 6.5 Hz, 3H).

### Synthesis of tert-butyl (4S)-4-(cyclopropoxymethyl)-2,2-dioxo-1,2lambda6,3-oxathiazolidine-3-carboxylate (AA-16)

### Step 1.

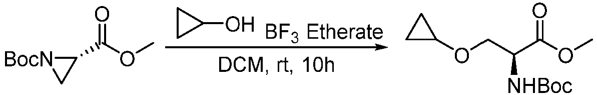

To a stirred mixture of 1-tert-butyl 2-methyl (2S)-aziridine-1,2-dicarboxylate vanadium (500 mg, 1.983 mmol, 1 equiv) and cyclopropanol (426.10 mg, 7.337 mmol, 3.7 equiv) in DCM (5 mL) was added BF₃.Et₂O (28.14 mg, 0.198 mmol, 0.1 equiv) dropwise at 0°C under nitrogen atmosphere. The resulting mixture was stirred for overnight at room temperature under nitrogen atmosphere. The resulting mixture was diluted with water (5mL). The resulting mixture was extracted with CH₂Cl₂ (3 x 50mL). The combined organic layers were washed with brine (1x30 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in Water (10mmol/L NH₄HCO₃), 30% to 100% gradient in 15 min; detector, UV 190 nm. This resulted in methyl (2S)-2-[(tert-butoxycarbonyl)amino]-3-cyclopropoxypropanoate (200 mg, 38.90%) as a yellow oil.

### Step 2.

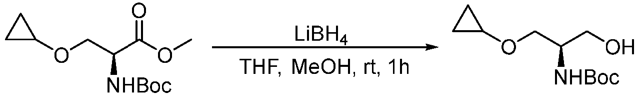

To a stirred solution of methyl (2S)-2-[(tert-butoxycarbonyl)amino]-3-cyclopropoxypropanoate (1.8 g, 6.942 mmol, 1 equiv) in tetrahydrofuran (20 mL) was added LiBH₄ (453.57 mg, 20.82 mmol, 3 equiv) dropwise at 0 °C under nitrogen atmosphere. The resulting mixture was stirred for 1 h at room temperature under nitrogen atmosphere. The reaction was quenched by the addition of water/Ice (10mL) at room temperature. The resulting mixture was extracted with EtOAc (3 x 50mL). The combined organic layers were washed with brine (1x50 mL), dried over anhydrous Na₂SO₄. After filtration, the filtrate was concentrated under reduced pressure. The residue was purified by silica gel column chromatography, eluted with PE / EA (4: 1) to afford tert-butyl N-[(2R)-1-cyclopropoxy-3-hydroxypropan-2-yl]carbamate (1.3 g, 80.97%) as a colorless oil.

### Step 3.

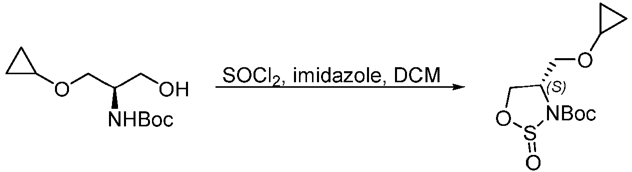

To a solution of Imidazole (1.05 g, 15.392 mmol, 4 equiv) in DCM (30 mL) were added SOCl₂ (0.42 mL, 5.772 mmol, 1.5 equiv) followed by DIEA (1.34 mL, 7.696 mmol, 2 equiv) dropwise at 0°C. To the above mixture was added tert-butyl N-[(2R)-1-cyclopropoxy-3-hydroxypropan-2-yl]carbamate (890 mg, 3.848 mmol, 1 equiv) dissolved in DCM (5 mL) dropwise at 0°C. The resulting mixture was stirred for additional 1h at room temperature. The resulting mixture was washed with 3 x 30 mL of conc. HCl (0.5 M). The resulting mixture was concentrated under vacuum to afford tert-butyl (4S)-4-(cyclopropoxymethyl)-2-oxo-1,2lambda4,3-oxathiazolidine-3-carboxylate (1.06 g, 99.33%) as a yellow solid.

### Step 4.

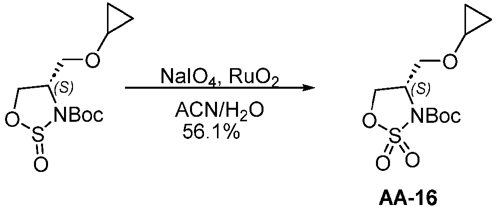

To a solution of tert-butyl (4S)-4-(cyclopropoxymethyl)-2-oxo-1,2lambda4,3-oxathiazolidine-3-carboxylate (1.06 g, 3.822 mmol, 1 equiv) in MeCN (13 mL) and H₂O (7 mL) was added NaIO₄ (0.98 g, 4.586 mmol, 1.2 equiv) and ruthenium(iv) oxide hydrate (11.55 mg, 0.076 mmol, 0.02 equiv) at 0°C. The resulting mixture was stirred for 1h at 0°C. The resulting mixture was filtered through a Celite pad. The filtrate was extracted with EtOAc (3 x 20 mL). The combined organic layers were washed with brine (20 mL), dried over anhydrous Na₂SO₄. After filtration, the filtrate was concentrated under reduced pressure. The residue was purified by silica gel column chromatography, eluted with PE / EA (3:1) to afford tert-butyl (4S)-4-(cyclopropoxymethyl)-2,2-dioxo-1,2lambda6,3-oxathiazolidine-3-carboxylate (AA-16, 0.63 g, 56.19%) as a colorless oil. Reference for 3-(tert-butyl) 4-methyl (S)-1,2,3-oxathiazolidine-3,4-dicarboxylate 2,2-dioxide (AA-17): Baig, N.; Chandrakala, R.N.; Sudhir, V.S.; Chandresekaran, S.; J. Org. Chem. 2010, 75, 2910-2921.

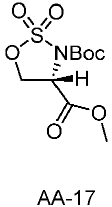

### Aldehydes Used: AL-1: (S)-N-Boc-2-aminopropanal

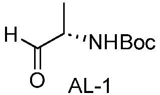

### AL-2: Tert-butyl (S)-4-formyl-2,2-dimethyloxazolidine-3-carboxylate

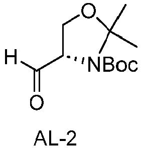

### Building blocks used:

### Reference for 3-amino-4-methylthiophene-2-carboxylic acid (BB-1): WO2008/118718 A2

### 3-amino-4-chlorothiophene-2-carboxylic acid (BB-2) was made by the following method:

### Step 1. Synthesis of methyl 3-acetamidothiophene-2-carboxylate

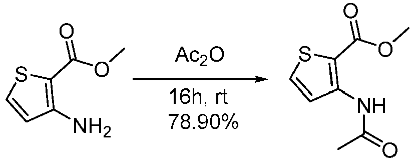

Into a 1L round-bottom flask were added methyl 3-aminothiophene-2-carboxylate (70 g, 445.321 mmol, 1 equiv) and acetic anhydride (100 g, 979.537 mmol, 2.20 equiv) at room temperature. The resulting mixture was stirred for overnight at room temperature. The resulting mixture was diluted with PE (600 mL). The precipitated solids were collected by filtration and washed with PE (2x100 mL). This resulted in methyl 3-acetamidothiophene-2-carboxylate (70 g, 78.90%) as a white solid.

### Step 2. Synthesis of methyl 4,5-dichloro-3-acetamidothiophene-2- carboxylate

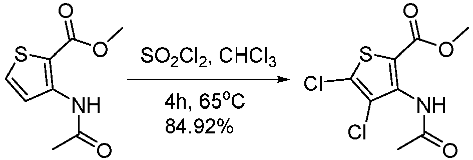

Into a 1 L 3-necked round-bottom flask were added methyl 3-acetamidothiophene-2-carboxylate (70 g, 351.370 mmol, 1 equiv), CHCl₃ (200 mL, 2479.685 mmol, 7.06 equiv) and SO₂Cl₂ (70 mL, 866.183 mmol, 2.47 equiv) at room temperature. The resulting mixture was stirred for 4 h at 65°C. The resulting mixture was concentrated under reduced pressure. This resulted in methyl 4,5-dichloro-3-acetamidothiophene-2- carboxylate (80 g, 84.92%) as a yellow solid.

### Step 3. Synthesis of methyl 4-chloro-3-acetamidothiophene-2-carboxylate

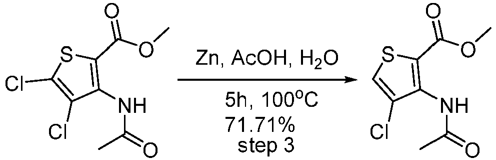

Into a 1 L 3-necked round-bottom flask were added methyl 4,5-dichloro-3-acetamidothiophene-2- carboxylate (80 g, 298.385 mmol, 1 equiv), H₂O (150 mL, 8326.395 mmol, 27.90 equiv), AcOH (50 mL, 872.577 mmol, 2.92 equiv) and Zn (78.03 g, 1193.540 mmol, 4 equiv) at room temperature. The resulting mixture was stirred for 5 h at 100°C. The resulting mixture was filtered, the filter cake was washed with DCM (2x100 mL). The aqueous layer was extracted with CH₂Cl₂ (3x200 mL). The resulting mixture was concentrated under reduced pressure. This resulted in methyl 4-chloro-3-acetamidothiophene-2-carboxylate (50 g, 71.71%) as a yellow solid.

### Step 4. Synthesis of methyl 3-amino-4-chlorothiophene-2-carboxylate

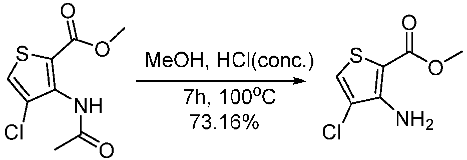

Into a 1 L round-bottom flask were added methyl 4-chloro-3-acetamidothiophene-2-carboxylate (50 g, 213.977 mmol, 1 equiv) and HCl (150 mL, 4936.917 mmol, 23.07 equiv) at room temperature. The resulting mixture was stirred for 7h at 100°C. The resulting mixture was concentrated under reduced pressure. The aqueous layer was extracted with EtOAc (5x100 mL).The resulting mixture was concentrated under vacuum. This resulted in methyl 3-amino-4-chlorothiophene-2-carboxylate (30 g, 73.16%) as a red solid.

### Step 5. Synthesis of 3-amino-4-chlorothiophene-2-carboxylic acid

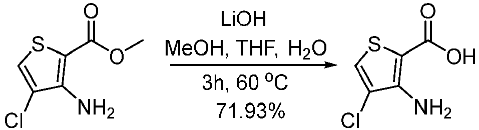

Into a 500 mL round-bottom flask were added methyl 3-amino-4-chlorothiophene-2-carboxylate (30 g, 156.552 mmol, 1 equiv), MeOH (100 mL), THF (100 mL), H₂O (20 mL) and lithiumol (15.00 g, 626.208 mmol, 4 equiv) at room temperature. The resulting mixture was stirred for 3h at 60°C. The resulting mixture was concentrated under reduced pressure and diluted with water (100 mL). The aqueous layer was extracted with EtOAc (3x100 mL). The resulting mixture was concentrated under reduced pressure. This resulted in 3-amino-4-chlorothiophene-2-carboxylic acid (20 g, 71.93%) as a yellow solid.

### Reference for 3-amino-4-bromothiophene-2-carboxylic acid (BB-3): Jeon, M-K; Kim, J-G,; Lee, D-H. Bull. Korean Chem. Soc. 2016, Vol. 37, 1406-1414.

### Synthesis of 3-amino-5-bromo-4-methylthiophene-2-carboxylic acid (BB-4):

### Step 1. Synthesis of methyl 3-amino-5-bromo-4-methylthiophene-2-carboxylate

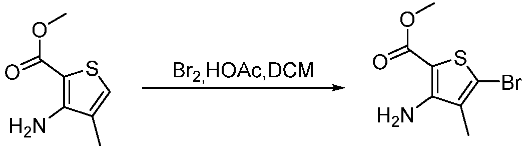

A solution of methyl 3-amino-4-methylthiophene-2-carboxylate (200.00 g, 1168.15 mmol, 1.00 equiv) in DCM (1500.00 mL) was treated with AcOH (500.00 mL) for 10 min at room temperature under nitrogen atmosphere followed by the addition of Br₂ (373.36 g, 2336.31 mmol, 2.00 equiv) dropwise at 0°C. The resulting mixture was stirred for additional overnight at 40°C. The resulting mixture was concentrated under reduced pressure. The residue was purified by trituration with diethyl ether (1.00 L) to afford methyl 3-amino-5-bromo-4-methylthiophene-2-carboxylate (200.00 g, 65.03%) as a yellow solid.

### Step 2. Synthesis of 3-amino-5-bromo-4-methylthiophene-2-carboxylic acid

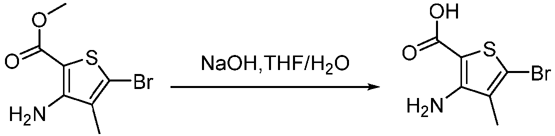

A mixture of methyl 3-amino-5-bromo-4-methylthiophene-2-carboxylate (200 g, 799.648 mmol, 1 equiv) and NaOH (120.00 g, 2409.63 mmol, 3.00 equiv) in H₂O (0.50 mL) and THF (1500 mL) was stirred for 4 h at room temperature under nitrogen atmosphere. The resulting mixture was diluted with water (1.5 L). The mixture was acidified to pH 5 with HCl (2 M). The resulting mixture was extracted with EtOAc (3 x 500 mL). The combined organic layers were washed with saturated NaCl (aq.) (1x1000 mL), dried over anhydrous Na₂SO₄. After filtration, the filtrate was concentrated under reduced pressure to afford 3-amino-5-bromo-4-methylthiophene-2-carboxylic acid (180 g, 90.58%) as a yellow solid.

### Synthesis of Compounds made by the General Schemes

Compounds were synthesized as illustrated in the general schemes or a combination of the general schemes in the order tabulated, with specific building blocks and reagents provided in Table 3 below.

**Table 3 Synthesis of specific compounds.**

| Compound Number | General Synthesis Scheme | Building Block | R²³ Reagent | R²¹ Reagent | R²⁴ Reagent |
|---|---|---|---|---|---|
| 1 | A, B | BB-1 | | 4-(chloromethyl)p yridin-1-ium chloride | |
| 3 | A, B | BB-1 | | Benzyl Bromide | |
| 4 | A, C | BB-1 | | furan-2-carbaldehyde | |
| 5 | A,B,D | BB-1 | AA-1 | Benzyl Bromide | |
| 6 | A,C,D | BB-1 | AA-1 | furan-2-carbaldehyde | |
| 7 | A,C,D | BB-1 | AA-1 | thiophene-2-carbaldehyde | |
| 8 | A,C,D | BB-1 | AA-3 | thiophene-2-carbaldehyde | |
| 9 | A,C,D | BB-1 | AA-3 | furan-2-carbaldehyde | |
| 10 | A,C,D | BB-1 | AA-8 | thiophene-2-carbaldehyde | |
| 12 | A,C,G | BB-4 | tert-butyl 3,6-diazabicyclo[3 .1.1]heptane-3-carboxylate | 4-(bromomethyl)p yridine | |
| 13 | A,C,G | BB-4 | tert-butyl hexahydropyrr olo[3,4-c]pyrrole-2(1H)-carboxylate | 1-(bromomethyl)-3-methylbenzene | |
| 14 | A,C,G | BB-4 | tert-butyl hexahydropyrr olo[3,4-c]pyrrole-2(1H)-carboxylate | 1-(bromomethyl)-3-fluorobenzene | |
| 15 | A,C,G | BB-4 | tert-butyl hexahydropyrr olo[3,4-c]pyrrole-2(1H)-carboxylate | 1-(bromomethyl)-2-fluorobenzene | |
| 16 | A,C,G | BB-4 | tert-butyl hexahydropyrr olo[3,4-c]pyrrole-2(1H)-carboxylate | 8-(bromomethyl)q uinoline | |
| 17 | E,C,D | BB-2 | AA-1 | thiophene-2-carbaldehyde | |
| 18 | E, C, D | BB-3 | AA-1 | thiophene-2-carbaldehyde | |
| 19 | E,C,D,F | BB-3 | AA-1 | thiophene-2-carbaldehyde | Zn(CN)₂ |
| 20 | E,C,D,F | BB-3 | AA-1 | thiophene-2-carbaldehyde | cyclopro pyltrifluo ro-lambda4-borane potassiu m |
| 21 | E,C,D,F | BB-3 | AA-1 | thiophene-2-carbaldehyde | 2-ethenyl-4,4,5,5-tetrameth yl-1,3,2-dioxabor olane |
| 22 | A,C,G | BB-4 | tert-butyl hexahydropyrr olo[3,4-c]pyrrole-2(1H)-carboxylate | 4-(bromomethyl)p yridine | |
| 23 | E,C,D | BB-2 | AA-1 | furan-2-carbaldehyde | |
| 24 | E,C,D | BB-3 | AA-1 | furan-2-carbaldehyde | |
| 25 | E,C,D,F | BB-3 | AA-1 | furan-2-carbaldehyde | KOH |
| 26 | E,C,D,I | BB-3 | AA-1 | furan-2-carbaldehyde | MeI |
| 27 | E,C,D,F | BB-3 | AA-1 | thiophene-2-carbaldehyde | KOH |
| 28 | E,C,D,I | BB-3 | AA-1 | thiophene-2-carbaldehyde | MeI |
| 30 | D | FC-1, Example X | AA-1 | thiophene-2-carbaldehyde | |
| 31 | E,C,D,F | BB-3 | AA-1 | furan-2-carbaldehyde | |
| 35 | E,C,J | BB-3 | AA-1 | thiophene-2-carbaldehyde | |
| 36 | A,B,D | BB-1 | AA-9 | thiophene-2-carbaldehyde | |
| 37 | A,B,D | BB-1 | AA-4 | thiophene-2-carbaldehyde | |
| 38 | E,C,D | BB-2 | AA-9 | thiophene-2-carbaldehyde | |
| 39 | E,C,D | BB-3 | AA-9 | thiophene-2-carbaldehyde | |
| 40 | E,C,D | BB-2 | AA-9 | furan-2-carbaldehyde | |
| 41 | E,C,D | BB-3 | AA-9 | furan-2-carbaldehyde | |
| 42 | E,C,D | BB-2 | AA-3 | thiophene-2-carbaldehyde | |
| 43 | E,C,D | BB-2 | AA-4 | thiophene-2-carbaldehyde | |
| 44 | E,C,D | BB-2 | AA-7 | thiophene-2-carbaldehyde | |
| 45 | A,B,D | BB-1 | AA-10 | thiophene-2-carbaldehyde | |
| 46 | E,C,D | BB-2 | AA-4 | furan-2-carbaldehyde | |
| 47 | E,C,D | BB-2 | AA-8 | thiophene-2-carbaldehyde | |
| 48 | E,C,D | BB-2 | AA-8 | furan-2-carbaldehyde | |
| 49 | E,C,D | BB-3 | AA-3 | thiophene-2-carbaldehyde | |
| 50 | E,C,D | BB-2 | AA-11 | thiophene-2-carbaldehyde | |
| 53 | E,C,D | BB-2 | AA-12 | thiophene-2-carbaldehyde | |
| 54 | A,C,D, K | BB-1 | | thiophene-2-carbaldehyde | |
| 55 | A,C,D, K, L | BB-1 | AA-4 | thiophene-2-carbaldehyde | |
| 57 | E,C,D | BB-2 | AA-3 | furan-2-carbaldehyde | |
| 58 | E,C,D | BB-2 | AA-3 | thiophene-2-carbaldehyde | |
| 59 | E,C,D | BB-3 | AA-4 | thiophene-2-carbaldehyde | |
| 60 | E,C,D | BB-3 | AA-4 | furan-2-carbaldehyde | |
| 61 | E,C,D | BB-3 | AA-3 | furan-2-carbaldehyde | |
| 62 | E,C,D | BB-3 | AA-7 | thiophene-2-carbaldehyde | |
| 63 | E,C,D | BB-3 | AA-7 | furan-2-carbaldehyde | |
| 64 | A,B,D | BB-1 | AA-7 | thiophene-2-carbaldehyde | |
| 65 | E,C,D | BB-2 | AA-7 | furan-2-carbaldehyde | |
| 66 | E,C,D,M | BB-3 | AA-1 | thiophene-2-carbaldehyde | |
| 67 | E,C,J | BB-3 | None | thiophene-2-carbaldehyde | |
| 68 | A,B.D | BB-1 | AA-13 | thiophene-2-carbaldehyde | |
| 69 | E,C,D | BB-2 | AA-13 | thiophene-2-carbaldehyde | |
| 70 | E,C,D | BB-3 | AA-13 | thiophene-2-carbaldehyde | |
| 71 | A,B,D | BB-1 | AA-14 | thiophene-2-carbaldehyde | |
| 72 | A,B,D | BB-1 | AA-2 | thiophene-2-carbaldehyde | |
| 73 | A,B,D | BB-1 | AA-15 | thiophene-2-carbaldehyde | |
| 74 | E,C,D | BB-2 | AA-2 | thiophene-2-carbaldehyde | |
| 75 | E,C,D | BB-2 | AA-15 | thiophene-2-carbaldehyde | |
| 76 | E,C,D | BB-2 | AA-1 | thiazole-2-carbaldehyde | |
| 79 | E,C,D,N | BB-1 | AA-1 | thiophene-2-carbaldehyde | |
| 80 | E,C,D | BB-3 | AA-12 | thiophene-2-carbaldehyde | |
| 81 | E,C,J, D | BB-3 | AA-1 | furan-2-carbaldehyde | |
| 82 | E,C,J | BB-3 | none | furan-2-carbaldehyde | |
| 84 | A,B,D | BB-1 | AA-12 | thiophene-2-carbaldehyde | |
| 85 | E,C,D,F | BB-2 | AA-1 | furan-2-carbaldehyde | NaSMe |
| 87 | E,C,D | BB-3 | AA-1 | thiophene-2-carbaldehyde | (tributyls tannyl)m ethanol |
| 88 | E,C,F | BB-3 | | thiophene-2-carbaldehyde | Acetami de |
| 90 | E,C,F | BB-2 | | furan-2-carbaldehyde | NaSMe |
| 93 | E,C,D | BB-3 | AA-2 | 3-fluorothiophene-2-carbaldehyde | |
| 94 | E,C,D | BB-2 | AA-12 | 3-fluorothiophene-2-carbaldehyde | |
| 95 | E,C,D | BB-2 | AA-2 | 3-fluorothiophene-2-carbaldehyde | |
| 96 | E,C,D | BB-2 | AA-2 | 5-fluorothiophene-2-carbaldehyde | |
| 97 | E,C,D,F | BB-3 | AA-2 | thiophene-2-carbaldehyde | 2-ethenyl-4,4,5,5-tetrameth yl-1,3,2-dioxabor olane |
| 98 | E,C,R,O | BB-2 | AL-1 | thiophene-2-carbaldehyde | |
| 99 | E,C,R,O | BB-3 | AL-1 | thiophene-2-carbaldehyde | |
| 100 | E,C,D | BB-3 | AA-16 | thiophene-2-carbaldehyde | |
| 101 | E,C,D | BB-2 | AA-5 | furan-2-carbaldehyde | |
| 102 | E,C,R,P | BB-2 | AL-1 | thiophene-2-carbaldehyde | |
| 103 | E,C,R,P | BB-2 | | thiophene-2-carbaldehyde | |
| 104 | E,C,D | BB-3 | AA-8 | thiazole-2-carbaldehyde | |
| 105 | E,C,D | BB-3 | AA-2 | thiazole-2-carbaldehyde | |
| 106 | E,C,D | BB-3 | AA-15 | thiazole-2-carbaldehyde | |
| 107 | E,C,D | BB-3 | AA-8 | furan-2-carbaldehyde | |
| 108 | E,C,D | BB-3 | AA-8 | thiophene-2-carbaldehyde | |
| 110 | E,C,R,O | BB-2 | AL-1 | furan-2-carbaldehyde | |
| 111 | E,C,D | BB-3 | AA-5 | furan-2-carbaldehyde | |
| 112 | E,C,R,O | BB-3 | AL-2 | thiazole-2-carbaldehyde | |
| 113 | E,C | BB-3 | | thiophene-2-carbaldehyde | |
| 114 | Example 18 | BB-3 | | thiophene-2-carbaldehyde | |
| 115 | E,C,R,Q | BB-3 | AA-17 | thiophene-2-carbaldehyde | |
| 116 | E,C,R,Q | BB-3 | AA-17 | thiophene-2-carbaldehyde | |
| 117 | E,C,R,Q | BB-3 | AA-17 | thiophene-2-carbaldehyde | |
| 118 | E,C,R,Q | BB-3 | AA-17 | thiophene-2-carbaldehyde | |
| 119 | E,C,R,Q | BB-3 | AA-17 | thiophene-2-carbaldehyde | |
| 120 | E,C,R,Q | BB-3 | AA-17 | thiophene-2-carbaldehyde | |
| 121 | E,C,R,Q | BB-3 | AA-17 | thiophene-2-carbaldehyde | |
| 122 | E,C,R,Q | BB-3 | AA-17 | thiophene-2-carbaldehyde | |
| 123 | E,C,R,Q | BB-3 | AA-17 | thiophene-2-carbaldehyde | |
| 124 | E,C,R,Q | BB-3 | AA-17 | thiophene-2-carbaldehyde | |
| 125 | E,C,R,Q | BB-3 | AA-17 | thiophene-2-carbaldehyde | |
| 126 | E,C,R,Q | BB-3 | AA-17 | thiophene-2-carbaldehyde | |
| 127 | E,C,R,Q | BB-3 | AA-17 | thiophene-2-carbaldehyde | |
| 128 | E,C,D,F | BB-3 | | thiophene-2-carbaldehyde | Phenylbo ronic acid |
| 129 | E,C,D,F | BB-3 | AA-1 | furan-2-carbaldehyde | phenylbo ronic acid |

**Table 4: Spectral Data for Specific Compounds**

| Compound Number | LC/MS Ion (M+H) | ¹H NMR |
|---|---|---|
| 1 | 262.0 | (300 MHz, DMSO-d6) δ 8.64 (m, 2H), 8.39 (t, J = 9.1 Hz, 1H), 7.60 (q, J = 1.2 Hz, 1H), 7.48-7.41 (m, 2H), 4.53 (d, J = 6.1 Hz, 2H), 2.19 (d, J = 1.3 Hz, 3H). |
| 3 | 261.0 | (300 MHz, DMSO-d6) δ 8.25 (t, J = 5.9 Hz, 1H), 7.52 (d, J = 1.6 Hz, 1H), 7.44-7.21 (m, 5H), 4.39 (d, J = 5.9 Hz, 2H), 2.13 (d, J = 1.3 Hz, 3H). |
| 4 | 251.0 | (300 MHz, DMSO-d6) δ 8.15 (t, J = 5.7 Hz, 1H), 7.64 (d, J = 1.4 Hz, 1H), 7.53 (q, J = 1.3 Hz, 1H), 6.43 (d, J = 1.8 Hz, 2H), 2.14 (d, J = 1.3 Hz, 3H). |
| 5 | 317.0 | (300 MHz, DMSO-d6) δ 8.21 (t, J = 5.9 Hz, 1H), 7.43-7.19 (m, 5H), 6.73 (s, 5H), 4.39 (d, J = 5.8 Hz, 2H), 3.29 (q, J = 6.6 Hz, 1H), 2.93(dd, J = 14.4, 6.1 Hz, 1H), 2.80(dd, J = 14.5,7.9 Hz, 1H),2.09(s, 3H), 1.14 (d, J = 6.4 Hz, 3H). |
| 6 | 308.0 | (300 MHz, DMSO-d6) δ 8.10 (t, J = 5.8 Hz, 1H), 7.64 (dd, J = 1.8, 0.9 Hz, 1H), 6.42 (qd, J = 3.2, 1.3 Hz, 2H), 5.99 (d, J = 87.3 Hz, 1H), 4.36 (d, J = 5.7 Hz, 2H), 3.31-3.17 (m, 1H), 2.90(dd, J = 14.4, 6.2 Hz, 1H),2.81-2.70 (m, 1H), 2.10(s, 3H), 1.12 (d, J = 6.4 Hz, 3H). |
| 7 | 324.0 | (300 MHz, DMSO-d6) δ 8.19 (t, J = 5.8 Hz, 1H), 7.46 (dd, J = 5.1, 1.3 Hz, 1H), 7.12 (dd, J = 3.5, 1.2 Hz, 1H), 7.00 (dd, J = 5.1, 3.4 Hz, 1H), 4.56 (d, J = 5.7 Hz, 2H), 3.14 (q, J = 5.7 Hz, 1H), 2.75 (qd, J = 14.2, 6.6 Hz, 2H),2.08 (s,3H), 1.06 (d, J = 6.3 Hz, 3H). |
| 8 | 364.0 | (300 MHz, DMSO-d6) δ 8.16 (t, J = 5.8 Hz, 1H), 7.46 (dd, J = 5.0, 1.3 Hz, 1H), 7.12 (dd, J = 3.4,1.2 Hz, 1H), 7.00 (dd, J = 5.1, 3.4 Hz, 1H), 4.57 (d, J = 5.7 Hz, 2H), 3.85 (s, 3H), 3.09 (p, J = 6.5 Hz, 1H),1.42-1.19 (m,2H), 0.88-0.73 (m, 1H), 0.53-0.32 (m, 2H), 0.13-0.03 (m, 1H), 0.03-0 (m, 1H). |
| 9 | 348.0 | (300 MHz, DMSO-d6) δ 8.03(t, J = 5.7 Hz, 1H), 7.64 (dd, J = 1.8, 0.9 Hz, 1H), 6.60-6.27 (m, 2H), 4.36 (d, J = 5.7Hz, 2H), 3.01 (tt, J = 7.5, 5.4 Hz, 1H), 2.81 (dd, J = 14.4, 5.7 Hz, 1H), 2.66 (dd, J = 14.4, 7.6 Hz, 1H),2.41 (s,2H), 2.07 (s, 3H), 1.37-1.13 (m, 2H), 0.91-0.73 (m, 1H), 0.52-0.32 (m, 2H), 0.06 (tdd, J = 6.1, 4.2, 2.7 Hz, 1H), 0.02-0 (m, 1H). |
| 10 | 342.0 | (400 MHz, Methanol-d4) δ 7.33 (t, J = 5.1, 1.2 Hz, 1H), 7.10 (dd, J = 3.5, 1.1 Hz, 1H), 6.97 (dd, J = 5.1, 3.5 Hz, 1H), 4.62 (d, J = 0.9 Hz, 2H), 4.35 (ddd, J = 47.5, 21.3, 4.7 Hz, 2H), 3.24 (tdd, J = 7.1, 4.4, 2.8 Hz, 1H), 2.90 (dd, J = 32.4, 7.1 Hz, 2H),2.16 (s,3H). |
| 12 | 358.0 | (300 MHz, DMSO-d6): δ 9.56 - 9.40 (m, 1H), 8.97 - 8.88 (m, 2H), 8.07 - 7.99 (m, 2H), 4.75 (s, 2H), 4.46 - 4.38 (m, 2H), 3.68 - 3.50 (m, 2H), 3.42 - 3.28 (m, 2H), 2.31 - 2.14 (m, 2H), 1.96 (s, 3H). |
| 13 | 385.0 | (300 MHz, DMSO-d6): 9.09 (m, 1H), 7.36 - 7.06 (m, 4H), 4.42 (s, 2H), 3.61 - 3.48 (m, 4H), 3.46 - 3.30 (m, 2H), 3.19 - 3.00 (m, 4H), 2.30 (s, 3H), 2.17 (s, 3H). |
| 14 | 389.0 | (300 MHz, DMSO-d6): δ 9.00 - 8.77 (m, 1H), 7.51 - 7.33 (m, 2H), 7.30 - 7.15 (m, 2H), 4.50 (s, 2H), 3.57 - 3.45 (m, 4H), 3.40 (s, 2H), 3.18 - 2.99 (m, 4H), 2.16 (s, 3H). |
| 15 | 389.0 | (300 MHz, DMSO-d6): δ 9.00 - 8.77 (m, 1H), 7.51 - 7.33 (m, 2H), 7.30 - 7.15 (m, 2H), 4.50 (s, 2H), 3.57 - 3.45 (m, 4H), 3.40 (s, 2H), 3.18 - 2.99 (m, 4H), 2.16 (s, 3H). |
| 16 | 422.0 | (300 MHz, DMSO-d6): δ 9.01 (dd, J = 4.2, 1.8 Hz, 1H), 8.47 (dd, J = 8.4, 1.8 Hz, 1H), 7.99 (d, J = 8.3 Hz, 1H), 7.80 (d, J = 7.0 Hz, 1H), 7.69 - 7.58 (m, 2H), 5.11 (s, 2H), 3.53 - 3.43 (m, 4H), 3.42 - 3.30 (m, 2H), 3.17 - 2.98 (m, 4H), 2.16 (s, 3H). |
| 17 | 344.0 | (300 MHz, DMSO-d6) δ 8.50 (s, 1H), 7.48 (m, 1H), 7.14 (m, 1H), 7.01 (m, 1H), 4.58 (d, J = 4.9 Hz, 2H), 3.13 (m, 2H), 2.90 (s, 1H), 2.78 (d, J = 6.6 Hz, 2H), 1.03 (d, J = 6.3 Hz, 3H). |
| 18 | 389.9 | (300 MHz, Methanol-d4) δ 7.35 (dd, J = 5.1, 1.3 Hz, 1H), 7.16 - 7.08 (m, 1H), 6.98 (dd, J = 5.1, 3.5 Hz, 1H), 4.64 (d, J = 0.9 Hz, 2H), 3.41 (q, J = 6.6 Hz, 2H), 2.98 (dd, J = 7.0, 2.9 Hz, 2H), 1.22 (d, J = 6.4 Hz, 3H). |
| 19 | 335.0 | (400 MHz, Methanol-d4) δ 7.36 (dt, J = 5.1, 1.4 Hz, 1H), 7.13 (ddt, J = 3.5, 2.2, 1.2 Hz, 1H), 6.99 (ddd, J = 5.2, 3.5, 1.9 Hz, 1H), 4.65 (dd, J = 7.5, 0.9 Hz, 2H), 4.11 - 4.02 (m, 1H), 3.28 (d, J = 6.5 Hz, 1H), 3.12 - 2.97 (m, 2H), 1.43 (dd, J = 6.5, 1.3 Hz, 1H), 1.17 (d, J = 6.4 Hz, 2H). |
| 20 | 350.1 | (400 MHz, Methanol-d4) δ 8.53 (s, 1H), 7.32 (dd, J = 5.1, 1.2 Hz, 1H), 7.08 (d, J = 3.7 Hz, 1H), 6.96 (dd, J = 5.1, 3.5 Hz, 1H), 4.60 (d, J = 1.0 Hz, 2H), 2.93 (dd, J = 6.9, 2.5 Hz, 2H), 1.84 - 1.76 (m, 1H), 1.29 - 1.14 (m, 6H), 0.86 (dd, J = 8.5, 2.5 Hz, 3H). |
| 21 | 336.0 | (400 MHz, DMSO-d6) δ 8.28 (t, J = 5.8 Hz, 1H), 7.46 (dd, J = 5.1, 1.3 Hz, 1H), 7.13 (d, J = 3.5 Hz, 1H), 7.00 (dd, J = 5.1, 3.4 Hz, 1H), 6.78 (dd, J = 17.3, 11.3 Hz, 2H), 6.58 (dd, J = 17.3, 2.7 Hz, 1H), 5.33 (dd, J = 11.3, 2.7 Hz, 1H), 4.57 (d, J = 5.7 Hz, 2H), 3.06 (t, J = 6.4 Hz, 1H), 2.78 (td, J = 15.0, 14.3, 6.6 Hz, 2H), 1.02 (d, J = 6.2 Hz, 3H). |
| 22 | 372.0 | 300 MHz, DMSO-d6): δ 8.97 - 8.92 (m, 1H), 8.92-8.88 (m, 2H), 8.02 (d, J = 6.3 Hz, 2H), 4.82 (s, 2H), 3.52 - 3.45 (m, 4H), 3.41 - 3.35 (m, 2H), 3.14 - 3.03 (m, 4H), 2.05 (s, 3H). |
| 23 | 328.0 | (400 MHz, Methanol-d4) δ 7.48 (dd, J = 1.9, 0.9 Hz, 1H), 6.44 - 6.37 (m, 2H), 4.43 (s, 2H), 3.00 - 2.88 (m, 2H), 1.19 (d, J = 6.4 Hz, 3H). |
| 24 | 372.0 | (400 MHz, Methanol-d4) δ 7.47 (dd, J = 1.9, 0.9 Hz, 1H), 6.43 - 6.35 (m, 2H), 4.43 (s, 2H), 3.35 (d, J = 6.6 Hz, 1H), 3.00 - 2.88 (m, 2H), 1.18 (d, J = 6.4 Hz, 3H). |
| 25 | 310.1 | (400 MHz, Methanol-d4) δ 7.44 (d, J = 14.5 Hz, 1H), 6.42 - 6.31 (m, 2H), 4.40 (d, J = 10.3 Hz, 2H), 3.59 (dd, J = 14.0, 7.1 Hz, 1H), 2.96 (t, J = 8.9 Hz, 2H), 1.34 (p, J = 7.1, 5.2 Hz, 3H). |
| 26 | 324.0 | (400 MHz, Methanol-d4) δ 7.46 (t, J = 1.3 Hz, 1H), 6.41 - 6.34 (m, 2H), 4.41 (s, 2H), 4.10 (s, 3H), 3.55 (q, J = 6.7 Hz, 1H), 2.98 (qd, J = 14.7, 6.8 Hz, 2H), 1.34 (d, J = 6.5 Hz, 3H). |
| 27 | 326.1 | (400 MHz, Methanol-d4) δ 7.34 (td, J = 4.8, 1.2 Hz, 1H), 7.11 (ddt, J = 4.5, 2.2, 1.1 Hz, 1H), 6.97 (dt, J = 5.1, 3.3 Hz, 1H), 4.63 (dd, J = 9.0, 0.9 Hz, 2H), 3.59 (h, J = 7.0 Hz, 1H), 3.18 - 2.90 (m, 2H), 1.36 (dd, J = 6.6, 4.2 Hz, 3H). |
| 28 | 340.1 | (400 MHz, Methanol-d4) δ 7.34 (dd, J = 5.1, 1.3 Hz, 1H), 7.11 (dq, J = 3.4, 1.1 Hz, 1H), 6.97 (dd, J = 5.1, 3.5 Hz, 1H), 4.65 - 4.60 (m, 2H), 4.07 (s, 3H), 3.40 (h, J = 6.5 Hz, 1H), 2.98 - 2.82 (m, 2H), 1.26 (d, J = 6.5 Hz, 3H). |
| 30 | 328.0 | (400 MHz, Methanol-d4) δ 8.53 (s, 1H), 6.80 - 6.70 (m, 2H), 6.41 (dd, J = 4.0, 1.9 Hz, 1H), 4.58 - 4.50 (m, 2H), 3.58 (dt, J = 13.6, 6.8 Hz, 1H), 3.17 - 3.03 (m, 2H), 1.35 (d, J = 6.5 Hz, 3H). |
| 31 | 334.1 | (400 MHz, Methanol-d4) δ 7.34 (dd, J = 5.1, 1.2 Hz, 1H), 7.11 (dq, J = 3.3, 1.0 Hz, 1H), 6.97 (dd, J = 5.1, 3.5 Hz, 1H), 4.62 (d, J = 1.0 Hz, 2H), 3.7-3.32 (m, 2H), 3.02-2.92 (m, 2H), 2.10 (s, 3H),1.18 (d, J = 6.4 Hz, 3H). |
| 35 | 378.1 | (400 MHz, Methanol-d4) δ 7.36 (dd, J = 5.1, 1.2 Hz, 1H), 7.13 (dt, J = 3.3, 1.1 Hz, 1H), 6.99 (dd, J = 5.1, 3.5 Hz, 1H), 4.66 (d, J = 0.9 Hz, 2H), 3.67 - 3.56 (m, 1H), 3.35 (s, 1H), 3.25 - 3.16 (m, 1H), 1.34 (d, J = 6.5 Hz, 3H). |
| 36 | 392.0 | (400 MHz, Methanol-d4) δ 7.33 (dd, J = 5.1 Hz, 1H), 7.13-7.05 (m, 1H), 6.97 (dd, J = 5.1, 3.5 Hz, 1H), 4.63 (d, J = 1.0 Hz, 2H), 3.50-3.36 (m, 1H), 2.95 (dd, J = 14.6, 6.0 Hz, 1H), 2.86 (dd, J = 14.7, 8.0 Hz, 1H), 2.45-2.33 (m, 1H), 2.27 (ddd, J = 15.1, 11.3, 8.1 Hz, 1H), 2.17(s, 3H). |
| 37 | 340.0 | (400 MHz, Methanol-d4) δ 7.33 (dd, J = 5.1, 1.2 Hz, 1H), 7.16-7.03 (m, 1H), 6.97 (dd, J = 5.1, 3.5 Hz, 1H), 4.62 (d, J = 0.9 Hz, 2H), 3.59 (dd, J = 10.8, 4.4 Hz, 1H), 3.45 (dd, J = 10.9,6.3 Hz, 1H), 3.16 (dt, J = 11.5, 6.4 Hz, 1H),2.97 (dd, J = 14.6, 6.5 Hz, 1H), 2.80 (dd, J = 14.6, 7.5 Hz, 1H) , 2.17 (s, 3H). |
| 38 | 412.0 | (400 MHz, Methanol-d4) δ 7.36 (dd, J = 5.1, 1.2 Hz, 1H), 7.13 (dt, J = 3.3, 1.1 Hz, 1H), 6.98 (dd, J = 5.1, 3.5 Hz, 1H), 4.64 (d, J = 0.9 Hz, 2H), 3.55 - 3.44 (m, 1H), 3.07 (dd, J = 14.6, 6.0 Hz, 1H), 2.94 (dd, J = 14.6, 7.8 Hz, 1H), 2.40 (ddd, J = 15.7, 11.5, 4.3 Hz, 1H), 2.30 (ddd, J = 15.1, 11.2, 8.0 Hz, 1H). |
| 39 | 458.0 | (400 MHz, Methanol-d4) δ 7.35 (dd, J = 5.1, 1.3 Hz, 1H), 7.12 (dt, J = 3.5, 1.1 Hz, 1H), 6.98 (dd, J = 5.1, 3.5 Hz, 1H), 4.64 (d, J = 0.9 Hz, 2H), 3.52 (tdd, J = 0.9 Hz, 2H), 3.52 (tdd, J = 8.0, 6.1, 4.4 Hz, 1H), 3.07 (dd, J = 14.6, 7.8 Hz, 1H), 2.48-2.17 (m,2H). |
| 40 | 396.0 | 400 MHz, Methanol-d4) δ 7.50 - 7.45 (m, 1H), 6.44 - 6.32 (m, 2H), 4.43 (s, 2H), 3.50 (d, J = 5.5 Hz, 1H), 3.07 (dd, J = 14.6, 6.0 Hz, 1H), 2.94 (dd, J = 14.7, 7.7 Hz, 1H), 2.34 (dddd, J = 34.0, 15.1, 11.3, 7.6 Hz, 2H), 1.28 (s, 2H), 0.92 - 0.85 (m, 1H). |
| 41 | 440.0 | (400 MHz, Methanol-d4) δ 7.47 (dd, J = 1.9, 0.8 Hz, 1H), 6.44 - 6.35 (m, 2H), 4.44 (s, 2H), 3.91 (dd, J = 7.4, 5.0 Hz, 1H), 3.28 - 3.20 (m, 2H), 2.75 - 2.56 (m, 2H). |
| 42 | 380.0 | (400 MHz, Methanol-d4) δ 7.35 (dd, J = 5.1, 1.2 Hz, 1H), 7.12 (dt, J = 3.5, 1.1 Hz, 1H), 6.98 (dd, J = 5.1, 3.5 Hz, 1H), 5.80 (td, J = 56.2, 3.3 Hz, 1H), 4.64 (d, J = 0.9 Hz, 2H), 3.42 - 3.33 (m, 1H), 3.15 (dd, J = 14.9, 4.8 Hz, 1H), 2.92 (dd, J = 14.9, 9.2 Hz, 1H). |
| 43 | 360.0 | (400 MHz, Methanol-d4) δ 7.35 (dd, J = 5.1, 1.2 Hz, 1H), 7.12 (dt, J = 3.4, 1.1 Hz, 1H), 6.98 (dd, J = 5.1, 3.5 Hz, 1H), 4.64 (d, J = 0.9 Hz, 2H), 3.59 (dd, J = 10.9, 4.6 Hz, 1H), 3.46 (dd, J = 10.9, 6.4 Hz, 1H), 3.20 (dtd, J = 7.6, 6.3, 4.5 Hz, 1H), 3.04 (dd, J = 14.6, 6.2 Hz, 1H), 2.87 (dd, J = 14.6, 7.6 Hz, 1H). |
| 44 | 374.0 | (400 MHz, Methanol-d4) δ 7.37 (dd, J = 5.1, 1.2 Hz, 1H), 7.15 - 7.06 (m, 1H), 6.99 (dd, J = 5.1, 3.5 Hz, 1H), 4.82 (s, 2H), 3.65 (dd, J = 11.1, 4.3 Hz, 1H), 3.52 (dd, J = 11.1, 6.2 Hz, 1H), 3.34 (s, 1H), 3.15 (s, 3H), 3.14 - 3.06 (m, 1H), 2.97 (dd, J = 14.6, 7.4 Hz, 1H). |
| 45 | 324.0 | (400 MHz, Methanol-d4) δ 7.33 (dd, J = 5.1, 1.2 Hz, 1H), 7.09 (dt, J = 3.4, 1.1 Hz, 1H), 6.97 (dd, J = 5.1, 3.5 Hz, 1H), 4.63 (d, J =1.0 Hz, 2H), 2.94-2.79 (m, 4H), 2.15 (s, 3H), 1.94 (p, J = 7.7 Hz, 2H). |
| 46 | 344.0 | (400 MHz, Methanol-d4) δ 7.48 (dd, J = 1.9, 0.9 Hz, 1H), 6.46 - 6.33 (m, 2H), 4.43 (s, 2H), 3.60 (dd, J = 10.6, 4.7 Hz, 1H), 3.47 (d, J = 17.5 Hz, 1H), 3.22 (s, 1H), 3.07 - 2.99 (m, 1H), 2.89 (dd, J = 14.3, 7.5 Hz, 1H). |
| 47 | 361.0 | (400 MHz, Methanol-d4) δ 7.35 (dd, J = 5.1, 1.2 Hz, 1H), 7.12 (dd, J = 3.5, 1.2 Hz, 1H), 6.98 (dd, J = 5.1, 3.5 Hz, 1H), 4.64 (d, J = 1.0 Hz, 2H), 4.47 - 4.33 (m, 1H), 4.33 - 4.15 (m, 1H), 3.42 - 3.31 (m, 2H), 3.05 (dd, J = 14.6, 6.6 Hz, 1H), 2.93 (ddd, J = 14.6, 7.6, 1.1 Hz, 1H). |
| 48 | 346.0 | (400 MHz, Methanol-d4) δ 7.48 (dd, J = 1.9, 0.8 Hz, 1H), 6.43 - 6.35 (m, 2H), 4.50 - 4.38 (m, 3H), 4.38 - 4.24 (m, 1H), 3.37 (s, 1H), 3.05 (dd, J = 14.7, 6.6 Hz, 1H), 2.98 - 2.88 (m, 1H). |
| 49 | 427.9 | (300 MHz, Methanol-d4) δ 8.35 (s, 1H), 7.20 (dd, J = 5.1, 1.2 Hz, 1H), 6.96 (dt, J = 3.3, 1.1 Hz, 1H), 6.82 (dd, J = 5.1, 3.5 Hz, 1H), 4.49 (d, J = 0.9 Hz, 2H), 3.48 (p, J = 6.9 Hz, 1H), 3.11 (d, J = 7.2 Hz, 1H), 2.98 (dd, J = 14.9, 7.2 Hz, 1H), 1.43 (t, J = 6.8 Hz, 2H), 0.66 (ddd, J = 12.5, 7.9, 4.8 Hz, 1H), 0.51 - 0.33 (m, 2H). |
| 50 | 386.0 | (400 MHz, Methanol-d4) δ 8.46 (s, 1H), 7.36 (dd, J = 5.1, 1.2 Hz, 1H), 7.13 (dt, J = 3.4, 1.1 Hz, 1H), 6.99 (dd, J = 5.1, 3.5 Hz, 1H), 4.76 (dd, J = 7.9, 6.5 Hz, 1H), 4.71 - 4.59 (m, 4H), 4.39 (t, J = 6.5 Hz, 1H), 3.85 (dt, J = 9.6, 6.8 Hz, 1H), 3.31 - 3.19 (m, 1H), 3.13 - 2.99 (m, 2H). |
| 53 | 419.0 | (300 MHz, Methanol-d4) δ 7.38 (dd, J = 5.1, 1.2 Hz, 1H), 7.15 (dd, J = 3.6, 1.2 Hz, 1H), 7.01 (dd, J = 5.1, 3.5 Hz, 1H), 4.67 (d, J = 1.0 Hz, 2H), 3.13 - 2.96 (m, 2H), 2.85 (dd, J = 14.5, 7.7 Hz, 1H), 2.70 - 2.26 (m, 2H), 2.17 (s, 1H). |
| 55 | 348.0 | (400 MHz, Methanol-d4) δ 7.35 (dd, J = 5.1, 1.2 Hz, 1H), 7.13 - 7.05 (m, 1H), 6.98 (dd, J = 5.1, 3.5 Hz, 1H), 4.84 - 4.77 (m, 2H), 4.39 (ddd, J = 9.0, 5.9, 2.3 Hz, 1H), 3.39 - 3.33 (m, 1H), 3.29 (d, J = 2.2 Hz, 1H), 3.12 (s, 3H), 2.25 (s, 3H). |
| 57 | 380.0 | (400 MHz, Methanol-d4) δ 8.51 (s, 1H), 7.48 (dd, J = 1.9, 0.8 Hz, 1H), 6.44 - 6.35 (m, 2H), 4.44 (s, 2H), 3.60 (p, J = 6.9 Hz, 1H), 3.30 - 3.20 (m, 1H), 3.14 (dd, J = 15.0, 7.3 Hz, 1H), 1.59 (td, J = 7.2, 6.7, 1.7 Hz, 2H), 0.82 (ddt, J = 10.2, 7.4, 3.6 Hz, 1H), 0.65 - 0.51 (m, 2H), 0.23 - 0.08 (m, 2H). |
| 58 | 383.0 | (400 MHz, Methanol-d4) δ 8.51 (s, 1H), 7.36 (dd, J = 5.1, 1.2 Hz, 1H), 7.13 (dt, J = 3.5, 1.1 Hz, 1H), 6.99 (dd, J = 5.1, 3.5 Hz, 1H), 4.65 (d, J = 0.9 Hz, 2H), 3.61 (p, J = 7.0 Hz, 1H), 3.30 - 3.21 (m, 1H), 3.14 (dd, J = 15.0, 7.3 Hz, 1H), 1.59 (td, J = 6.7, 1.7 Hz, 2H), 0.89 - 0.75 (m, 1H), 0.65 - 0.52 (m, 2H), 0.23 - 0.08 (m, 2H). |
| 59 | 404.0 | (300 MHz, DMSO-d6) δ 8.50 (t, J = 5.7 Hz, 1H), 7.48 (d, J = 5.1 Hz, 1H), 7.14 (d, J = 3.4 Hz, 1H), 7.01 (dd, J = 5.1, 3.4 Hz, 1H), 4.89 (s, 1H), 4.58 (d, J = 5.6 Hz, 2H), 3.00 (dd, J = 23.1, 8.8 Hz, 2H), 2.72 (dd, J = 14.4, 7.1 Hz, 1H). |
| 60 | 388.0 | (300 MHz, DMSO-d6) δ 8.44 (t, J = 5.6 Hz, 1H), 7.65 (t, J = 1.4 Hz, 1H), 6.44 (s, 1H), 5.32 (s, 2H), 5.05 (s, 1H), 4.38 (d, J = 5.6 Hz, 2H), 3.15 (s, 1H), 3.02 (dd, J = 14.4, 6.0 Hz, 1H), 2.81 (dd, J = 14.5, 7.0 Hz, 1H). |
| 61 | 412.0 | (300 MHz, Methanol-d4) δ 7.48 (dd, J = 1.9, 0.9 Hz, 1H), 6.45 - 6.34 (m, 2H), 4.44 (s, 2H), 3.63 (q, J = 6.8 Hz, 1H), 3.36 - 3.23 (m, 1H), 3.14 (dd, J = 14.9, 7.3 Hz, 1H), 1.60 (t, J = 6.7 Hz, 2H), 0.83 (dd, J = 8.9, 5.7 Hz, 1H), 0.67 - 0.49 (m, 2H), 0.25 - 0.08 (m, 2H). |
| 62 | 418 | (300 MHz, Methanol-d4) δ 7.35 (dd, J = 5.1, 1.2 Hz, 1H), 7.12 (dq, J = 3.3, 1.1 Hz, 1H), 6.98 (dd, J = 5.1, 3.5 Hz, 1H), 4.65 (d, J = 1.0 Hz, 2H), 3.74 - 3.62 (m, 1H), 3.57 (dd, J = 10.5, 3.2 Hz, 1H), 3.46 (dd, J = 10.4, 5.2 Hz, 1H), 3.41 (s, 3H), 3.31 - 3.10 (m, 2H). |
| 63 | 402.0 | (300 MHz, Methanol-d4) δ 7.47 (dd, J = 1.9, 0.9 Hz, 1H), 6.45 - 6.34 (m, 2H), 4.44 (s, 2H), 3.71 (dddd, J = 8.4, 6.5, 5.1, 3.1 Hz, 1H), 3.58 (dd, J = 10.5, 3.2 Hz, 1H), 3.48 (dd, J = 10.5, 5.2 Hz, 1H), 3.42 (s, 3H), 3.21 (h, J = 6.3 Hz, 2H). |
| 64 | 304.0 | (400 MHz, Methanol-d4) δ 7.33 (dd, J = 5.0, 1.2 Hz, 1H), 7.10 (dq, J = 3.3, 1.0 Hz, 1H), 6.97 (dd, J = 5.1, 3.5 Hz, 1H), 4.62 (d, J = 0.9 Hz, 2H), 3.41 - 3.34 (m, 4H), 3.28 - 3.17 (m, 1H), 2.95 (dd, J = 14.5, 6.9 Hz, 1H), 2.82 (dd, J = 14.5, 6.9 Hz, 1H), 2.16 (s, 3H). |
| 65 | 357.9 | (400 MHz, Methanol-d4) δ 7.47 (dd, J = 1.9, 0.9 Hz, 1H), 6.51 - 6.12 (m, 2H), 4.43 (d, J = 0.7 Hz, 2H), 3.42 - 3.38 (m, 1H), 3.36 (s, 3H), 3.07 - 2.98 (m, 1H), 2.92 - 2.78 (m, 1H), 1.37 - 1.20 (m, 1H). |
| 66 | 310.0 | (400 MHz, Methanol-d4) δ 7.33 (dd, J = 5.1, 1.3 Hz, 1H), 7.10 (dt, J = 3.4, 1.1 Hz, 1H), 6.97 (dd, J = 5.1, 3.5 Hz, 1H), 6.63 (d, J = 1.1 Hz, 1H), 4.63 (d, J = 0.9 Hz, 2H), 3.20 (p, J = 6.5 Hz, 1H), 2.86 - 2.80 (m, 2H), 1.16 (d, J = 6.4 Hz, 3H). |
| 67 | 321.0 | (400 MHz, Methanol-d4) δ 8.40 (q, J = 1.3 Hz, 1H), 7.36 (dd, J = 5.1, 1.2 Hz, 1H), 7.14 (dq, J = 3.4, 1.0 Hz, 1H), 6.99 (dd, J = 5.1, 3.5 Hz, 1H), 4.66 (d, J = 0.9 Hz, 2H). |
| 68 | 438.0 | 300 MHz, Methanol-d4) δ 7.43 - 7.33 (m, 1H), 7.19 - 7.11 (m, 1H), 7.06 - 6.96 (m, 1H), 6.32 - 5.86 (m, 1H), 4.67 (s, 2H), 3.47 - 3.37 (m, 1H), 3.11 - 2.92 (m, 2H), 2.17 - 1.86 (m, 2H). |
| 69 | 393.0 | (300 MHz, Methanol-d4) δ 7.38 (dd, J = 5.1, 1.2 Hz, 1H), 7.15 (dt, J = 3.4, 1.1 Hz, 1H), 7.01 (dd, J = 5.1, 3.5 Hz, 1H), 6.31 - 5.89 (m, 1H), 4.66 (d, J = 1.0 Hz, 2H), 3.41 (d, J = 13.4 Hz, 1H), 3.00 (qd, J = 14.6, 6.9 Hz, 2H), 2.14 - 1.87 (m, 2H). |
| 70 | 438.0 | 300 MHz, Methanol-d4) δ 7.43 - 7.33 (m, 1H), 7.19 - 7.11 (m, 1H), 7.06 - 6.96 (m, 1H), 6.32 - 5.86 (m, 1H), 4.67 (s, 2H), 3.47 - 3.37 (m, 1H), 3.11 - 2.92 (m, 2H), 2.17 - 1.86 (m, 2H). |
| 71 | 386.0 | 300 MHz, Methanol-d4) δ 7.40 - 7.20 (m, 6H), 7.16 - 7.06 (m, 1H), 7.04 - 6.94 (m, 1H), 4.63 (d, J = 1.0 Hz, 2H), 4.24 - 4.14 (m, 1H), 3.20 - 3.10 (m, 2H), 1.87 (s, 3H). |
| 72 | 356.0 | (300 MHz, Methanol-d4) δ 7.33 (dd, J = 5.1, 1.3 Hz, 1H), 7.10 (dq, J = 3.3, 1.0 Hz, 1H), 6.97 (dd, J = 5.1, 3.5 Hz, 1H), 4.68 - 4.60 (m, 2H), 4.55 - 4.45 (m, 1H), 3.16 - 2.93 (m, 2H), 2.90 - 2.77 (m, 1H), 2.17 (s, 3H), 1.37 (dd, J = 24.4, 6.3 Hz, 3H). |
| 73 | 356.0 | (300 MHz, Methanol-d4) δ 7.33 (dd, J = 5.1, 1.3 Hz, 1H), 7.10 (dq, J = 3.3, 1.0 Hz, 1H), 6.97 (dd, J = 5.1, 3.5 Hz, 1H), 4.69 - 4.61 (m, 2H), 4.55 - 4.43 (m, 1H), 3.16 - 2.93 (m, 2H), 2.90 - 2.77 (m, 1H), 2.17 (s, 3H), 1.37 (dd, J = 24.4, 6.3 Hz, 3H). |
| 74 | 376 | (400 MHz, Methanol-d4) δ 7.36 (dd, J = 5.1, 1.2 Hz, 1H), 7.13 (dt, J = 3.5, 1.1 Hz, 1H), 6.99 (dd, J = 5.1, 3.5 Hz, 1H), 4.94 (dd, J = 6.5, 3.2 Hz, 1H), 4.65 (d, J = 1.0 Hz, 2H), 3.76 - 3.59 (m, 1H), 3.04 (dd, J = 15.2, 9.1 Hz, 1H), 1.46 (dd, J = 24.3, 6.5 Hz, 3H). |
| 75 | 376.0 | (400 MHz, Methanol-d4) δ 7.35 (dd, J = 5.1, 1.2 Hz, 1H), 7.12 (dq, J = 3.3, 1.0 Hz, 1H), 6.98 (dd, J = 5.1, 3.5 Hz, 1H), 4.66 - 4.61 (m, 2H), 4.56 - 4.47 (m, 1H), 3.18 - 3.00 (m, 2H), 2.98 - 2.83 (m, 1H), 1.37 (dd, J = 24.3, 6.3 Hz, 3H). |
| 76 | 346.0 | (400 MHz, Methanol-d4) δ 8.52 (s, 1H), 7.78 (d, J = 3.3 Hz, 1H), 7.58 (d, J = 3.3 Hz, 1H), 4.85 (d, J = 16.0 Hz, 28H), 3.62 (dt, J = 7.8, 6.4 Hz, 1H), 3.21 - 3.07 (m, 2H), 1.34 (d, J = 6.6 Hz, 4H), 1.31 - 1.25 (m, 1H), 0.89 (d, J = 8.9 Hz, 0H). |
| 79 | 366.0 | (400 MHz, Methanol-d4) δ 7.46 - 7.32 (m, 1H), 6.98 (d, J = 3.7 Hz, 2H), 5.65 (s, 2H), 3.48 (dt, J = 7.8, 6.3 Hz, 1H), 3.15 - 2.87 (m, 2H), 2.47 (s, 3H), 2.27 (s, 3H), 1.28 (d, J = 6.5 Hz, 3H). |
| 80 | 400.0 | 300 MHz, Methanol-d4) δ 7.4 - 7.3 (m, 1H), 7.17 - 7.07 (m, 1H), 7.04 - 6.94 (m, 1H), 4.65 (d, J = 0.9 Hz, 2H), 3.10 - 2.99 (m, 1H), 2.96 - 2.86 (m, 1H), 2.83 - 2.73 (m, 1H), 2.68 - 2.40 (m, 3H), 2.40 - 2.24 (m, 1H), 2.19 (s, 3H), 2.16 (s, 1H). |
| 81 | 362.0 | (400 MHz, Methanol-d4) δ 7.48 (dd, J = 2.0, 0.9 Hz, 1H), 6.44 - 6.30 (m, 2H), 4.60 (s, 1H), 4.43 (s, 2H), 3.06 (d, J = 7.1 Hz, 2H), 1.18 (d, J = 6.4 Hz). |
| 82 | 305.0 | 400 MHz, Methanol-d4) δ 8.41 (q, J = 1.3 Hz, 1H), 7.48 (dd, J = 1.9, 0.9 Hz, 1H), 6.40 (ddd, J = 14.9, 3.2, 1.3 Hz, 2H), 4.45 (s, 2H). |
| 84 | 464.0 | 300 MHz, Methanol-d4) δ 7.43 - 7.33 (m, 1H), 7.15 (d, J = 3.2 Hz, 1H), 7.06 - 6.96 (m, 1H), 4.67 (d, J = 1.0 Hz, 2H), 3.18 - 3.09 (m, 1H), 3.07 - 2.97 (m, 1H), 2.9 - 2.8 (m, 1H), 2.53 - 2.43 (m, 4H), 2.24 - 2.16 (m, 1H). |
| 85 | 367.0 | (400 MHz, Methanol-d4) δ 7.47 (dd, J = 1.8, 0.9 Hz, 1H), 6.42 - 6.32 (m, 2H), 4.42 (s, 2H), 3.34 (d, J = 6.7 Hz, 2H), 3.09 (d, J = 7.0 Hz, 2H), 2.40 (s, 3H), 1.39 - 1.26 (m, 1H), 1.18 (d, J = 6.4 Hz, 3H). |
| 87 | 340.0 | (400 MHz, Methanol-d4) δ 8.52 (s, 1H), 7.33 (dd, J = 5.1, 1.3 Hz, 1H), 7.10 (dd, J = 3.3, 1.2 Hz, 1H), 6.97 (dd, J = 5.1, 3.5 Hz, 1H), 4.71 (d, J = 12.5 Hz, 1H), 4.67 - 4.61 (m, 3H), 3.61 (h, J = 6.8 Hz, 1H), 3.22 (dd, J = 14.8, 6.9 Hz, 1H), 3.13 (dd, J = 14.8, 7.2 Hz, 1H), 1.37 (d, J = 6.6 Hz, 3H). |
| 88 | 310.0 | (400 MHz, Methanol-d4) δ 8.05 (s, 1H), 7.34 (dd, J = 5.1, 1.2 Hz, 1H), 7.12 (dq, J = 3.3, 1.0 Hz, 1H), 6.98 (dd, J = 5.1, 3.5 Hz, 1H), 4.64 (d, J = 0.9 Hz, 2H), 2.16 (s, 3H). |
| 90 | 283.0 | (400 MHz, Methanol-d4) δ 7.47 (dd, J = 1.7, 1.0 Hz, 1H), 6.68 (s, 1H), 6.38 (t, J = 1.4 Hz, 2H), 4.40 (s, 2H), 2.59 (s, 3H). |
| 93 | 438.0 | (400 MHz, Methanol-d4) δ 7.34 (dd, J = 5.6, 4.0 Hz, 1H), 6.85 (d, J = 5.6 Hz, 1H), 4.68 - 4.49 (m, 3H), 3.27 - 3.08 (m, 2H), 2.89 - 2.74 (m, 1H), 1.39 (dd, J = 24.4, 6.3 Hz, 3H). |
| 94 | 376.0 | (400 MHz, Methanol-d4) δ 7.35 (dd, J = 5.1, 1.2 Hz, 1H), 7.12 (dq, J = 3.3, 1.0 Hz, 1H), 6.98 (dd, J = 5.1, 3.5 Hz, 1H), 4.66 - 4.61 (m, 2H), 4.56 - 4.47 (m, 1H), 3.18 - 3.00 (m, 2H), 2.98 - 2.83 (m, 1H), 1.37 (dd, J = 24.3, 6.3 Hz, 3H). |
| 95 | 371.0 | (400 MHz, Methanol-d4) δ 8.65 (dt, J = 5.0, 1.3 Hz, 1H), 8.09 (d, J = 7.9 Hz, 1H), 7.93 (td, J = 7.7, 1.9 Hz, 1H), 7.44 - 7.18 (m, 2H), 7.13 (dd, J = 3.4, 1.2 Hz, 1H), 6.98 (dd, J = 5.1, 3.5 Hz, 1H), 4.67 (d, J = 0.9 Hz, 2H), 3.49 (h, J = 6.5 Hz, 1H), 3.27 - 3.09 (m, 2H), 1.27 (d, J = 6.5 Hz, 3H). |
| 96 | 389.0 | (400 MHz, Methanol-d4) δ 6.80 (t, J = 3.8 Hz, 1H), 6.42 (dt, J = 4.1, 2.0 Hz, 1H), 4.66 - 4.38 (m, 3H), 3.23 - 3.07 (m, 2H), 2.84 (dd, J = 14.6, 8.8 Hz, 1H), 1.47 - 1.13 (m, 3H). |
| 97 | 367.0 | (400 MHz, Methanol-d4) δ 7.33 (dd, J = 5.1, 1.2 Hz, 1H), 7.11 (dd, J = 3.5, 1.1 Hz, 1H), 6.97 (dd, J = 5.1, 3.5 Hz, 1H), 6.79 (dd, J = 17.4, 11.4 Hz, 1H), 6.50 (dd, J = 17.4, 2.2 Hz, 1H), 5.41 (dd, J = 11.4, 2.2 Hz, 1H), 4.70 - 4.46 (m, 3H), 3.23 - 3.09 (m, 2H), 2.93 - 2.77 (m, 1H), 1.37 (dd, J = 24.5, 6.3 Hz, 3H). |
| 98 | 380.0 | (400 MHz, Methanol-d4) 7.36 (dd, J = 5.1, 1.2 Hz, 1H), 7.13 (dd, J = 3.5, 1.1 Hz, 1H), 6.99 (dd, J = 5.1, 3.5 Hz, 1H), 4.66 (s, 2H), 3.44 (dd, J = 8.0, 6.6 Hz, 1H), 1.24 (d, J = 6.7 Hz, 3H). |
| 99 | 424.0 | 400 MHz, Methanol-d4) δ 7.41 - 7.35 (m, 1H), 7.17 - 7.12 (m, 1H), 7.00 (m, 1H), 4.66 (s, 2H), 3.91 - 3.77 (m, 1H), 1.19 (d, J = 6.8 Hz, 3H). |
| 100 | 444.0 | (400 MHz, Methanol-d4) δ 7.35 (dd, J = 5.1, 1.2 Hz, 1H), 7.12 (dt, J = 3.5, 1.0 Hz, 1H), 6.98 (dd, J = 5.1, 3.5 Hz, 1H), 4.64 (d, J = 0.9 Hz, 2H), 3.52 (dd, J = 9.5, 4.3 Hz, 1H), 3.42 (dd, J = 9.5, 6.0 Hz, 1H), 3.34 (s, 1H), 3.29 - 3.24 (m, 1H), 3.01 (dd, J = 14.5, 7.0 Hz, 1H), 2.88 (dd, J = 14.5, 7.1 Hz, 1H), 0.62 - 0.32 (m, 4H). |
| 101 | 364.0 | (400 MHz, Methanol-d4) δ 7.48 (dd, J = 1.9, 0.8 Hz, 1H), 6.46 - 6.35 (m, 2H), 5.81 (td, J = 56.2, 3.3 Hz, 1H), 4.43 (s, 2H), 3.36 (dt, J = 12.7, 4.4 Hz, 1H), 3.15 (dd, J = 14.9, 4.9 Hz, 1H), 2.92 (dd, J = 14.9, 9.1 Hz, 1H). |
| 102 | 360.0 | (400 MHz, Methanol-d4) δ 8.54 (s, 1H), 7.36 (dd, J = 5.1, 1.2 Hz, 1H), 7.13 (dd, J = 3.5, 1.1 Hz, 1H), 6.99 (dd, J = 5.1, 3.5 Hz, 1H), 5.01 (d, J = 8.2 Hz, 1H), 4.66 (s, 2H), 3.44 (dd, J = 8.0, 6.6 Hz, 1H), 1.24 (d, J = 6.7 Hz, 3H). |
| 104 | 408.8 | (400 MHz, Methanol-d4) δ 7.78 (d, J = 3.3 Hz, 1H), 7.57 (d, J = 3.3 Hz, 1H), 4.81 (s, 2H), 4.51 - 4.25 (m, 2H), 3.47 - 3.32 (m, 1H), 3.07 (dd, J = 14.6, 6.7 Hz, 1H), 2.95 (ddd, J = 14.6, 7.6, 1.1 Hz, 1H). |
| 105 | 422.9 | (400 MHz, Methanol-d4) δ 7.78 (d, J = 3.3 Hz, 1H), 7.57 (d, J = 3.3 Hz, 1H), 4.81 (s, 2H), 4.72 - 4.51 (m, 1H), 3.24 (dt, J = 9.2, 4.9 Hz, 1H), 3.17 (dd, J = 14.8, 4.7 Hz, 1H), 2.89 - 2.75 (m, 1H), 1.39 (dd, J = 24.3, 6.3 Hz, 3H). |
| 106 | 422.8 | (400 MHz, Methanol-d4) δ 7.78 (d, J = 3.3 Hz, 1H), 7.57 (d, J = 3.3 Hz, 1H), 4.81 (s, 2H), 4.71 - 4.48 (m, 1H), 3.16 - 3.04 (m, 2H),2.98 - 2.86 (m, 1H), 1. 37 (dd, J = 24.3, 6.4 Hz, 3H). |
| 107 | 390.0 | (400 MHz, Methanol-d4) δ 7.47 (dd, J = 1.8, 0.9 Hz, 1H), 6.45 - 6.31 (m, 2H), 4.52 - 4.38 (m, 3H), 4.40 - 4.24 (m, 1H), 3.41 (dtdd, J = 19.8, 7.1, 5.3, 4.1 Hz, 1H), 3.08 (dd, J = 14.7, 6.8 Hz, 1H), 2.96 (ddd, J = 14.6, 7.5, 1.1 Hz, 1H). |
| 108 | 408.0 | (400 MHz, Methanol-d4) δ 7.35 (dd, J = 5.1, 1.2 Hz, 1H), 7.12 (dq, J = 3.3, 1.0 Hz, 1H), 6.98 (dd, J = 5.1, 3.5 Hz, 1H), 4.64 (d, J = 0.9 Hz, 2H), 4.53 - 4.25 (m, 2H), 3.49 - 3.34 (m, 1H), 3.08 (dd, J = 14.6, 6.8 Hz, 1H), 2.95 (ddd, J = 14.6, 7.5, 1.2 Hz, 1H). |
| 110 | 364.0 | (400 MHz, Methanol-d4) δ 7.49 (d, J = 1.8 Hz, 1H), 6.44 (d, J = 3.3 Hz, 1H), 6.39 (dd, J = 3.3, 1.9 Hz, 1H), 4.45 (s, 2H), 3.81 - 3.70 (m, 1H), 3.64 - 3.57 (m, 0H), 3.18 - 3.09 (m, 0H), 1.39 - 1.26 (m, 3H), 1.20 (d, J = 6.8 Hz, 3H). |
| 111 | 409.8 | (400 MHz, Methanol-d4) δ 7.47 (dd, J = 1.9, 0.9 Hz, 1H), 6.48 - 6.33 (m, 2H), 5.81 (td, J = 56.2, 3.3 Hz, 1H), 4.43 (s, 2H), 3.38 (dt, J = 8.6, 4.5 Hz, 1H), 3.16 (dd, J = 14.9, 4.8 Hz, 1H), 2.92 (dd, J = 14.8, 9.2 Hz, 1H). |
| 112 | 442.7 | (400 MHz, Methanol-d4) δ 8.26 (s, 1H), 7.79 (d, J = 3.3 Hz, 1H), 7.59 (d, J = 3.4 Hz, 1H), 4.84 (s, 2H), 4.02 (dddd, J = 15.9, 11.4, 7.4, 4.0 Hz, 1H), 3.79 (dd, J = 11.4, 4.0 Hz, 1H), 3.61 (dd, J = 11.5, 7.4 Hz, 1H). |
| 115 | 487.0 | (400 MHz, Chloroform-d) δ 7.61 (s, 1H), 6.95-6.94 (d, J = 2.7 Hz, 1H), 6.75-6.74 (d, J = 2.9 Hz, 1H), 5.40-5.38 (m, 1H), 4.60-4.59 (d, J = 5.4 Hz, 2H), 3.68-3.62 (m, 5H), 3.56 - 3.41 (m, 3H), 3.38-3.27 (m, 1H), 3.15-3.12 (m, 1H), 2.98-2.94 (m, 1H). |
| 116 | 511.0 | (400 MHz, DMSO-d6) δ 8.57-8.54 (m, 1H), 8.10 (s, 1H), 8.06-8.02 (m, 1H), 7.29 - 7.22 (m, 1H), 7.16-7.11 (m, 2H), 6.97-6.96 (d, J = 3.0 Hz, 1H), 6.82-6.81 (d, J = 3.0 Hz, 1H), 4.52-4.51 (d, J = 5.2 Hz, 2H), 3.64-3.61 (m, 1H), 3.22-3.17 (m, 1H), 3.02-2.96(m, 1H). |
| 117 | 431.0 | (400 MHz, Chloroform-d) δ 7.61 (s, 1H), 7.36 - 7.31 (m, 1H), 6.97-6.95 (m, 1H), 6.77-6.74 (m, 1H), 5.49-5.42(m, 1H), 4.60-4.59 (d, J = 5.2 Hz, 2H), 3.60-3.57 (m, 1H), 3.38-3.34 (m, 1H), 3.07-3.01 (m, 1H), 2.83-2.81 (d, J = 4.8 Hz, 3H). |
| 118 | 511.0 | 400 MHz, DMSO-d6) δ 8.59- 8.50 (m, 1H), 8.10 (s, 1H), 7.62-7.44 (m, 1H), 7.32 (s, 2H), 6.95 (d, J = 3.2 Hz, 1H), 6.79 (d, J = 3.1 Hz, 1H), 4.50 (d, J = 5.4 Hz, 2H), 3.62-3.33 (m, 1H), 3.18-3.14 (m, 1H), 3.02 - 2.95 (m, 1H). |
| 119 | 507.0 | (400 MHz, DMSO-d6) δ 9.53 (br, 1H), 8.58-8.55 (m, 1H), 8.11 (s, 1H), 7.66-7.64 (m, 1H), 7.17-7.12 (m, 2H), 7.05-7.01 (m, 1H), 6.97-6.96 (m, 1H), 6.82-6.81 (m, 1H), 4.52-4.51 (d, J=5.5Hz, 2H), 3.61-3.58 (m, 1H), 3.21-3.16 (m, 1H), 3.04-2.99 (m, 1H), 2.11 (s, 3H). |
| 120 | 507.0 | (400 MHz, DMSO-d6) δ 9.78 (br, 1H), 8.54-8.53 (m, 1H), 8.09 (s, 1H), 7.42-7.38 (m, 1H), 7.38-7.36 (m, 1H), 7.18-7.14 (m, 1H), 6.94-6.93 (m, 1H), 6.87-6.85 (m, 1H), 6.78-6.77 (m, 1H), 4.51-4.50 (d, J=5.5Hz, 2H), 3.53-3.50 (m, 1H), 3.16-3.11 (m, 1H), 2.95-2.89 (m, 1H), 2.27 (s, 3H). |
| 121 | 493.0 | (400 MHz, DMSO-d6) δ 9.77(br, 1H), 8.53-8.51 (m, 1H), 8.07 (s, 1H), 7.57-7.55 (m, 2H), 7.28-7.24 (m, 2H), 7.03-6.99 (m, 1H), 6.92-6.91 (m, 1H), 6.76-6.75 (m, 1H), 4.49-4.47 (d, J=5.49Hz, 2H), 3.51-3.48 (m, 1H), 3.14-3.09 (m, 1H), 2.93-2.88 (m, 1H). |
| 122 | 485.0 | (400 MHz, DMSO-d6) δ 8.58-8.56 (m, 1H), 8.11 (s, 1H), 6.94-6.93 (m, 1H), 6.72-6.71 (m, 1H), 4.51-4.49 (d, J = 5.6 Hz, 2H), 3.81-3.78 (m, 1H), 3.46-3.37 (m, 1H), 3.22-3.14 (m, 1H), 2.96-2.91 (m, 1H), 2.79-2.74 (m, 1H), 1.83 (s, 2H), 1.54-1.29 (m, 6H). |
| 123 | 445.0 | (400 MHz, DMSO-d6) δ 8.60-8.58 (m, 1H), 8.32-8.29 (m, 1H), 8.12 (s, 1H),7.00-6.99 (m, 1H), 6.80-6.79 (m, 1H), 4.54-4.52 (d, J = 5.7 Hz, 2H), 3.83-3.80 (m, 1H), 3.17-3.15 (m, 2H), 3.08-3.02 (m, 2H), 0.95-0.92 (t, J = 7.2 Hz, 3H). |
| 124 | 523.0 | (400 MHz, DMSO-d6) δ 9.78(br, 1H), 8.56-8.54 (m, 1H), 8.10 (s, 1H), 7.48-7.46 (d, J = 8.5 Hz, 2H), 6.95-6.94 (d, J = 3.0 Hz, 1H), 6.86-6.84 (d, J = 8.5 Hz, 2H), 6.78-6.77 (d, J = 3.0 Hz, 1H), 4.52-4.50 (d, J = 5.4 Hz, 2H), 3.72 (s, 3H), 3.52-3.47 (m, 1H), 3.15-3.10 (m, 1H), 2.95-2.90 (m, 1H). |
| 125 | 494.0 | (400 MHz, DMSO-d6) δ 8.74 (s, 1H), 8.55 (m, 1H), 8.28-8.27(m, 1H), 8.10 (s, 1H), 8.00-7.98 (m, 1H), 7.35-7.31 (m, 1H), 6.97-6.96 (d, J = 3.3 Hz, 1H), 6.81-6.80 (d, J = 3.2 Hz, 1H), 4.52-4.50 (m, 2H), 3.71-3.68 (m, 1H), 3.25-3.17 (m, 1H), 3.07-3.02 (m, 1H). |
| 126 | 523.0 | H-(400 MHz, DMSO-d6) δ10.03(s, 1H), 8.56-8.53 (m, 1H), 8.27-8.25 (m, 1H), 8.10 (s, 1H), 7.04-7.03 (m, 2H), 6.97-6.96 (d, J = 3.2 Hz, 1H), 6.93-6.89 (m, 1H), 6.84-6.83 (d, J = 3.2 Hz, 1H), 4.51-4.50 (d, J = 5.5 Hz, 2H), 3.83 (s, 3H), 3.57 -3.55(m, 1H), 3.26-3.21 (m, 1H), 3.04-2.98 (m, 1H). |
| 127 | 494.0 | (400 MHz, DMSO-d6) δ 8.55-8.52 (m, 1H), 8.31-8.28 (m, 1H), 8.10 (s, 1H), 7.80-7.76 (m, 1H), 7.12-7.09 (m, 1H), 6.95-6.94 (m, 1H), 6.80-6.79 (m, 1H), 4.51-4.49 (d, J = 5.3 Hz, 2H), 3.65-3.60 (m, 1H), 3.21-3.16 (m, 1H), 2.98-2.92 (m, 1H). |
| 128 | 386.1 | 1H NMR (400 MHz, Methanol-d4) δ 7.49 - 7.43 (m, 4H), 7.42 - 7.31 (m, 2H), 7.15 - 7.09 (m, 1H), 6.98 (dd, J = 5.1, 3.4 Hz, 1H), 4.66 (s, 2H), 3.21 - 2.86 (m, 3H), 1.11 (d, J = 6.4 Hz, 3H). |
| 129 | 370.0 | 1H NMR (400 MHz, Methanol-d4) δ 7.47-7.43 (t, J = 5.8 Hz, 5H), 7.38-7.35 (dd, J = 5.0, 1.3 Hz, 1H), 6.39-6.36 (dd, J = 3.4, 1.2 Hz, 2H), 4.44 (dd, J = 5.1, 3.4 Hz, 2H), 3.31-3.29 (d, J = 5.7 Hz, 1H), 2.96-2.94 (s, 2H), 1.07-1.05 (m,3H). |

### Example 2: Specific Example of General Scheme A: Synthesis of 6-methylthieno[3,2-c] [1,2]thiazol-3-amine

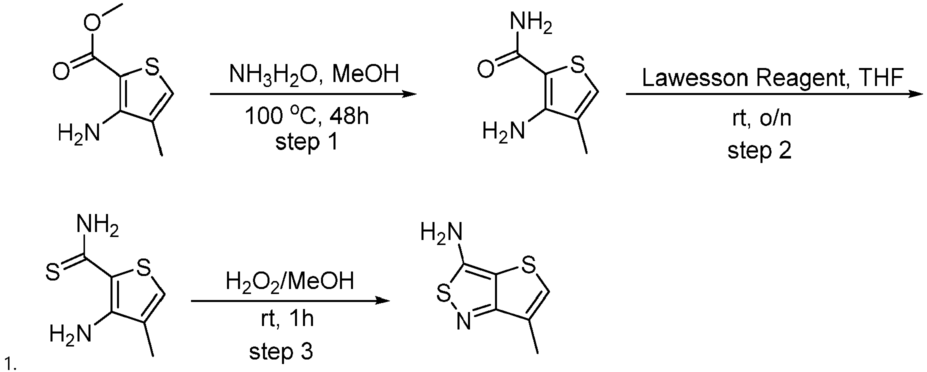

Step 1 .A solution of methyl 3-amino-4-methylthiophene-2-carboxylate (10 g, 58.408 mmol, 1.0 equiv) in THF (50 mL) and H₂O (50 mL) was added LiOH (4.20 g, 175.224 mmol, 3.0 equiv). The reaction was stirred for overnight at 70 °C under N₂ atmosphere. The resulting mixture was concentrated under reduced pressure. The mixture was acidified to pH 4 with citric acid. The precipitated solids were collected by filtration and washed with H₂O (3 x 20 mL) to afford the 3-amino-4-methylthiophene-2-carboxylic acid (4 g, 43.57%) as a white solid.

Step 2. A solution of 3-amino-4-methylthiophene-2-carboxylic acid (3 g, 19.085 mmol, 1.0 equiv) and NH₄Cl (5.10 g, 95.425 mmol, 5.0 equiv), EDCI (4.39 g, 22.902 mmol, 1.2 equiv), HOBT (3.35 g, 24.811 mmol, 1.3 equiv) and DIEA (4.93 g, 38.170 mmol, 2.0 equiv) in DMF (30 mL) was stirred for 10 h at room temperature under nitrogen atmosphere. The reaction was monitored by LCMS. The mixture was allowed to cool down to room temperature. The reaction was quenched with H₂O (30 mL). The resulting mixture was extracted with EA (50x3 mL). The combined organic layers were dried over anhydrous Na₂SO₄. After filtration, the filtrate was concentrated under reduced pressure. The residue was purified by silica gel column chromatography, eluted with EA/PE (1/1) to afford 3-amino-4-methylthiophene-2-carboxamide (1.3 g, 43.61%) as a brown solid.

Step 3. A solution of 3-amino-4-methylthiophene-2-carboxamide (1.5 g, 9.603 mmol, 1.0 equiv) and Lawesson Reagent (3.88 g, 9.603 mmol, 1.0 equiv) in THF (15 mL) was stirred for overnight at room temperature under air atmosphere. The resulting mixture was concentrated under reduced pressure. The resulting mixture was diluted with saturated aqueous NaHCO₃ (30 mL). The resulting mixture was extracted with EA (3 x 20ml). The combined organic layers were washed with saturated aqueous NaCl, dried over anhydrous Na₂SO₄. After filtration, the filtrate was concentrated under reduced pressure. The residue was purified by silica gel column chromatography, eluted with PE/EA (4/1) to afford 3-amino-4-methylthiophene-2-carbothioamide (230 mg, 13.90%) as a yellow solid.

Step 4. A solution of 3-amino-4-methylthiophene-2-carbothioamide (210 mg, 1.219 mmol, 1.0 equiv) and H₂O₂ (30%) (0.7 mL, 30.046 mmol, 24.65 equiv) in MeOH (2 mL) was stirred for 1 h at room temperature under air atmosphere. The resulting mixture was concentrated under reduced pressure. The resulting mixture was diluted with saturated aqueous Na₂O₃S₂ (5 mL). The resulting mixture was extracted with EA (3 x 10 ml). The combined organic layers were washed with saturated aqueous NaCl, dried over anhydrous Na₂SO₄. After filtration, the filtrate was concentrated under reduced pressure. The residue was purified by silica gel column chromatography, eluted with PE/EA (2/1) to 6-methylthieno[3,2-c][1,2]thiazol-3-amine (100 mg, 48.18%). LC/MS (M+H) 171.0.

### Example 3: Specific Example of General Synthesis Scheme B: Synthesis of N-benzyl-6-methylthieno[3,2-c] [1,2]thiazol-3-amine (Compound 3)

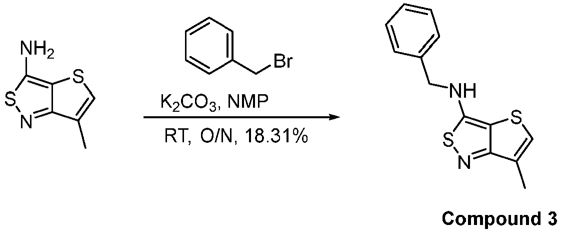

A solution of 6-methylthieno[3,2-c][1,2]thiazol-3-amine (100 mg, 0.587 mmol, 1.0 equiv) in NMP (5 mL) was treated with benzyl bromide (100.46 mg, 0.587 mmol, 1.0 equiv) and K₂CO₃ (162.36 mg, 1.174 mmol, 2.0 equiv) for overnight at room temperature under nitrogen atmosphere. The reaction was monitored by LCMS. The crude product (100 mg) was purified by Prep-HPLC with the following conditions (Column: XBridge Prep C18 OBD Column, 19* 150 mm, 5µm; Mobile Phase A: Water (10 mmol/L NH₄HCO₃), Mobile Phase B: ACN; Flow rate: 25 mL/min; Gradient: 10% B to 35% B in 7 min, 35% B; Wave Length: 254/220 nm; RT1(min): 6.35; Number Of Runs: 0) to afford N-benzyl-6-methylthieno[3,2-c][1,2]thiazol-3-amine (Compound 3) (28 mg, 18.31%).
LCMS-[M+H]⁺=261
¹H NMR (300 MHz, DMSO-*d*₆) δ 8.25 (t, *J =* 5.9 Hz, 1H), 7.52 (d, *J =* 1.6 Hz, 1H), 7.44-7.21 (m, 5H), 4.39 (d, *J =* 5.9 Hz, 2H), 2.13 (d, *J =* 1.3 Hz, 3H).

### Example 4: Specific Example of General Scheme C N-[(furan-2-yl)methyl]-6-methylthieno[3,2-c] [1,2]thiazol-3-amine (Compound 4)

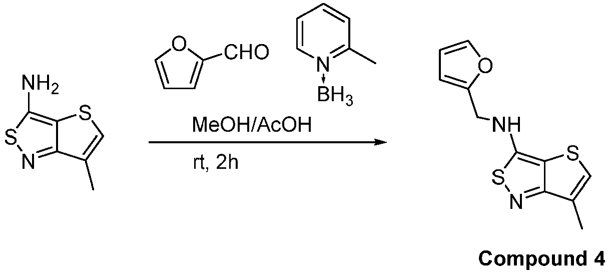

A mixture of 6-methylthieno[3,2-c][1,2]thiazol-3-amine (100 mg, 0.587 mmol, 1.0 equiv), furfural (56.44 mg, 0.587 mmol, 1.0 equiv) and 2-Methylpyridine Borane (109.18 mg, 1.174 mmol, 2 equiv) in MeOH/AcOH (2.2 mL, V/V=10/1) was stirred for 2 h at room temperature. The reaction was monitored by LCMS. The crude product (100 mg) was purified by Prep-HPLC with the following conditions (Column: XBridge Prep C18 OBD Column, 19*150 mm, 5µm; Mobile Phase A: Water (10 mmol/L NH₄HCO₃), Mobile Phase B: ACN; Flow rate: 25 mL/min; Gradient: 10% B to 35% B in 7 min, 35% B; Wave Length: 254/220 nm; RT1(min): 6.35) to afford N-(furan-2-ylmethyl)-6-methylthieno[3,2-c][1,2]thiazol-3-amine (22.4 mg, 15.23%).
LCMS [M+H]⁺=251
¹H NMR (300 MHz, DMSO-*d*₆) δ 8.15 (t, *J =* 5.7 Hz, 1H), 7.64 (d, *J =* 1.4 Hz, 1H), 7.53 (q, *J =* 1.3 Hz, 1H), 6.43 (d, *J =* 1.8 Hz, 2H), 2.14 (d, *J =* 1.3 Hz, 3H).

### Example 5, Specific example General Scheme D, synthesis of 5-[(2S)-2-aminopropyl]-N-benzyl-6-methylthieno[3,2-c][1,2]thiazol-3-amine (compound 5).

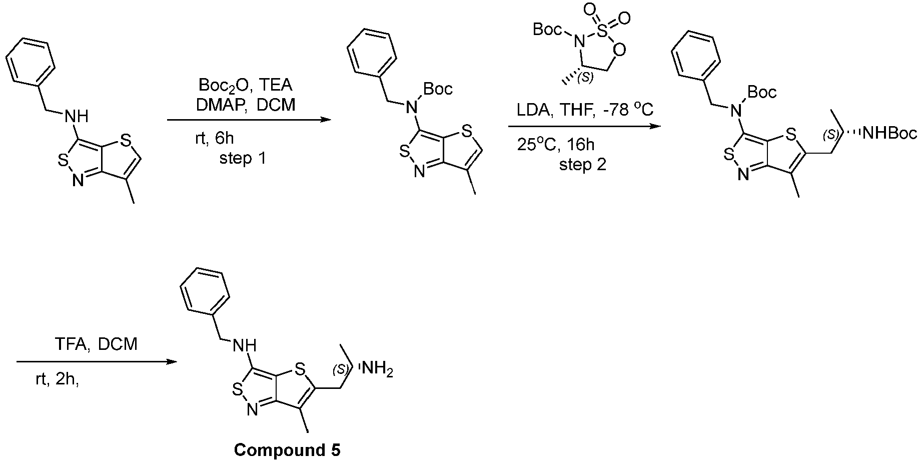

Step 1: A solution of N-benzyl-6-methylthieno[3,2-c][1,2]thiazol-3-amine (220 mg, 0.845 mmol, 1.0 equiv) in DCM (4 mL) was treated with TEA (128.25 mg, 1.268 mmol, 1.5 equiv), Boc₂O (202.85 mg, 0.929 mmol, 1.1 equiv) and DMAP (10.32 mg, 0.085 mmol, 0.1 equiv) for 6 h at room temperature. The reaction was monitored by LCMS. The reaction was quenched by the addition of water (5 mL). The resulting mixture was extracted with EA (3 x 30 mL). The combined organic layers were dried over anhydrous Na₂SO₄. After filtration, the filtrate was concentrated under reduced pressure. The residue was purified by silica gel column chromatography, eluted with EA/PE (1/2) to afford tert-butyl N-benzyl-N-{6-methylthieno[3,2-c][1,2]thiazol-3-yl}carbamate (170 mg, 55.81%) as a yellow solid. LC/MS (M+H) = 361.2

Step 2: Into a 8-mL vial purged and maintained with an inert atmosphere of nitrogen, was placed tert-butyl N-benzyl-N-{6-methylthieno[3,2-c][1,2]thiazol-3-yl}carbamate (120 mg, 0.333 mmol, 1.0 equiv), THF (4 mL). The LDA (71.32 mg, 0.666 mmol, 2.0 equiv) was added drop-wise to the above solution at -78 °C. The resulting solution was stirred for 0.5 h at -78 °C. Then tert-butyl (4S)-4-methyl-2,2-dioxo-1,21ambda6,3-oxathiazolidine-3-carboxylate (118.47 mg, 0.500 mmol, 1.5 equiv) was added drop-wise into the above mixture at -78 °C. Subsequently, the resulting solution was stirred for 16 h at 25 °C. The reaction was then quenched by the addition of 2 mL of water at 0 °C. The resulting mixture was extracted with 3x20 mL of ethyl acetate and the organic layers combined and dried over anhydrous sodium sulfate. The residue was applied onto a silica gel column with dichloromethane/methanol (10/1) to afford tert-butyl N-benzyl-N-{5-[(2S)-2-[(tert-butoxycarbonyl)amino]propyl]-6-methylthieno[3,2-c][1,2]thiazol-3-yl}carbamate (100 mg, 58.03%) as a yellow solid. (LC/MS) (M+H) = 518.2.

Step 3: A solution of tert-butyl N-benzyl-N-{5-[(2S)-2-[(tert-butoxycarbonyl)amino]propyl]-6-methylthieno [3,2-c][1,2]thiazol-3-yl}carbamate (90 mg, 0.174 mmol, 1.0 equiv) in DCM (5 mL) was treated with TFA (2.5 mL) for 1 min at room temperature under air atmosphere. The resulting mixture was stirred for 2 h at room temperature under air atmosphere. The resulting mixture was concentrated under reduced pressure. The crude product (90 mg) was purified by Prep-HPLC with the following conditions (Column: XBridge Prep C18 OBD Column, 19*150 mm, 5µm; Mobile Phase A: Water (10 mmol/L NH₄HCO₃), Mobile Phase B: ACN; Flow rate: 25 mL/min; Gradient: 10% B to 35% B in 7 min, 35% B; Wave Length: 254/220 nm; RT1(min): 6.35) to afford 5-[(2S)-2-aminopropyl]-N-benzyl-6-methylthieno[3,2-c][1,2]thiazol-3-amine (40 mg, 72.48%).

LCMS-[M+H]⁺=318 ¹H NMR (300 MHz, DMSO-*d*₆) δ 8.21 (t, *J =* 5.9 Hz, 1H), 7.43-7.19 (m, 5H), 6.73 (s, 5H), 4.39 (d, *J =* 5.8 Hz, 2H), 3.29 (q, *J =* 6.6 Hz, 1H), 2.93(dd, *J* = 14.4, 6.1 Hz, 1H), 2.80(dd, *J =* 14.5,7.9 Hz, 1H),2.09(s, 3H), 1.14 (d, *J =* 6.4 Hz, 3H).

### Example 6: Specific Example of General Scheme E: Synthesis of 6-bromo-N-(thiophen-2-ylmethyl) thieno[3,2-c][1,2]thiazol-3-amine

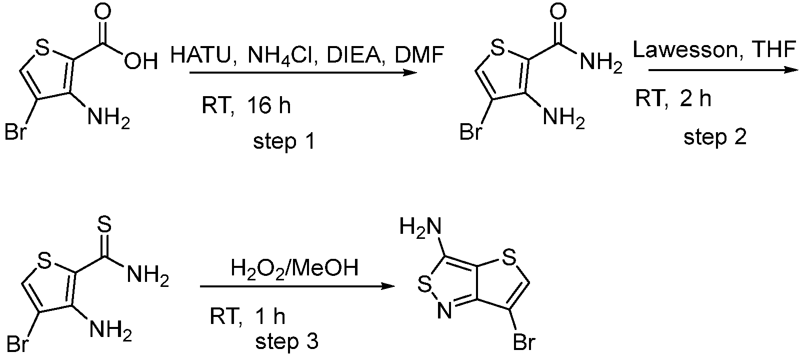

Into a 100-mL vial purged and maintained with an inert atmosphere of nitrogen, was placed 3-amino-4-bromothiophene-2-carboxylic acid (16 g, 72.053 mmol, 1 equiv), HATU (54.79 g, 144.106 mmol, 2 equiv), NH₄Cl (19.27 g, 360.265 mmol, 5 equiv), DIEA (18.63 g, 144.106 mmol, 2 equiv), DMF (50 mL). The resulting solution was stirred for 16 h at 25 degrees C. The reaction was then quenched by the addition of 2 mL of water. The resulting solution was extracted with 3x10 mL of ethyl acetate and the organic layers combined and dried over anhydrous sodium sulfate. The residue was applied onto a silica gel column with dichloromethane/methanol (10:1). This resulted in 12 g (75.34%) of 3-amino-4-bromothiophene-2-carboxamide as a yellow solid.

Into a 25-mL vial purged and maintained with an inert atmosphere of nitrogen, was placed 3-amino-4-bromothiophene-2-carboxamide (16 g, 72.375 mmol, 1 equiv), Lawesson Reagent (29.27 g, 72.375 mmol, 1 equiv), THF (20 mL). The resulting solution was stirred for 2 h at 25 degrees C. The reaction was then quenched by the addition of 2 mL of water. The resulting solution was extracted with 3x10 mL of ethyl acetate and the organic layers combined and dried over anhydrous sodium sulfate. The residue was applied onto a silica gel column with dichloromethane/methanol (10:1). This resulted in 7 g (40.79%) of 3-amino-4-bromothiophene-2-carbothioamide as a yellow solid.

Into a 50-mL vial purged and maintained with an inert atmosphere of nitrogen, was placed 3-amino-4-bromothiophene-2-carbothioamide (11.3 g, 47.653 mmol, 1 equiv), H₂O₂ (15 mL), MeOH (20 mL). The resulting solution was stirred for 1 h at 25 degrees C. The reaction was then quenched by the addition of 2 mL of water. The resulting solution was extracted with 3x10 mL of ethyl acetate and the organic layers combined and dried over anhydrous sodium sulfate. The residue was applied onto a silica gel column with dichloromethane/methanol (10:1). This resulted in 7 g (62.48%) of 6-bromothieno[3,2-c][1,2]thiazol-3-amine.

### Example 7: Specific Example of General Synthesis Scheme F: Synthesis of 5-[(2S)-2-aminopropyl]-6-ethenyl-N-(thiophen-2-ylmethyl) thieno[3,2-c] [1,2]thiazol-3-amine (Compound 21)

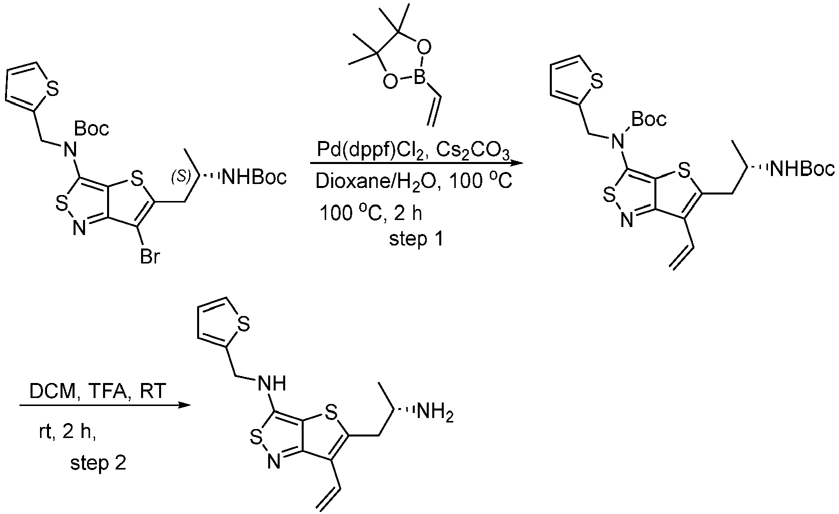

Into a 25-mL vial purged and maintained with an inert atmosphere of nitrogen, was placed tert-butyl N-{6-bromo-5-[(2S)-2-[(tert-butoxycarbonyl)amino]propyl]thieno[3,2-c][1,2]thiazol-3-yl}-N-(thiophen-2-ylmethyl)carbamate (90 mg, 0.153 mmol, 1 equiv), 2-ethenyl-4,4,5,5-tetramethyl-1,3,2-dioxaborolane (30.62 mg, 0.199 mmol, 1.3 equiv), Pd(dppf)Cl₂ (22.38 mg, 0.031 mmol, 0.2 equiv), Cs₂CO₃ (99.64 mg, 0.306 mmol, 2 equiv), dioxane (2 mL), H₂O (0.5 mL). The resulting solution was stirred for 2 h at 100 degrees C. The reaction was then quenched by the addition of 2 mL of water. The resulting solution was extracted with 3x10 mL of ethyl acetate and the organic layers combined and dried over anhydrous sodium sulfate. The residue was applied onto a silica gel column with dichloromethane/methanol (10:1). This resulted in 35 mg (42.73%) of tert-butyl N-{5-[(2S)-2-[(tert-butoxycarbonyl)amino]propyl]-6-ethenylthieno[3,2-c][1,2]thiazol-3-yl}-N-(thiophen-2-ylmethyl)carbamate as a yellow solid.

Into a 8-mL vial purged and maintained with an inert atmosphere of nitrogen, was placed tert-butyl N-{5-[(2S)-2-[(tert-butoxycarbonyl)amino]propyl]-6-ethenylthieno[3,2-c][1,2]thiazol-3-yl}-N-(thiophen-2-ylmethyl)carbamate (20 mg, 0.037 mmol, 1 equiv), TFA (1 mL), DCM (2 mL). The resulting solution was stirred for 2 hr at room temperature. Then concentrate the reaction solution under vacuum. The residue was applied on the HP-Flash with acetonitrile/NH₄HCO₃ (4:1). The desired fractions were collected and concentrated under reduced pressure to remove most of the acetonitrile. Then the mixture was lyophilizied to dryness. This resulted in 3.2 mg (25.55%) of 5-[(2S)-2-aminopropyl]-6-ethenyl-N-(thiophen-2-ylmethyl) thieno[3,2-c][1,2]thiazol-3-amine.

LC/MS C₁₃H₁₇N₃S₃calc. [M+H] + 335.06 found 336.05.

¹H NMR (400 MHz, DMSO-*d*₆) δ 8.28 (t, *J* = 5.8 Hz, 1H), 7.46 (dd, *J =* 5.1, 1.3 Hz, 1H), 7.13 (d*, J* = 3.5 Hz, 1H), 7.00 (dd, *J =* 5.1, 3.4 Hz, 1H), 6.78 (dd, *J =* 17.3, 11.3 Hz, 2H), 6.58 (dd, *J* = 17.3, 2.7 Hz, 1H), 5.33 (dd, *J =* 11.3, 2.7 Hz, 1H), 4.57 (d, *J =* 5.7 Hz, 2H), 3.06 (t, *J =* 6.4 Hz, 1H), 2.78 (td, *J =* 15.0, 14.3, 6.6 Hz, 2H), 1.02 (d, *J =* 6.2 Hz, 3H).

### Example 8: Specific Example of General Scheme G: Synthesis of Compound 12

### Step 1. : Synthesis of 5-bromo-6-methyl-N-(oxan-2-yl)thieno[3,2-c][1,2]thiazol-3-amine

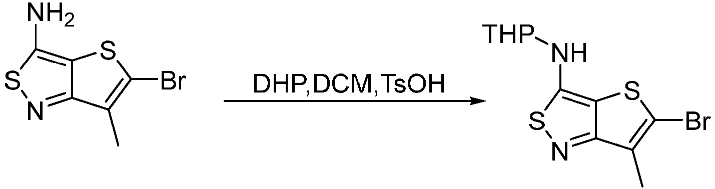

A mixture of 5-bromo-6-methylthieno[3,2-c][1,2]thiazol-3-amine (50.00 g, 200.69 mmol, 1.00 equiv) and DHP (50.65 g, 602.07 mmol, 3.00 equiv) and TsOH (3.46 g, 20.06 mmol, 0.10 equiv) in DCM (500.00 mL) was stirred for overnight at room temperature under nitrogen atmosphere. The resulting mixture was concentrated under reduced pressure. The resulting mixture was diluted with diethyl ether (100.00 mL). The precipitated solids were collected by filtration and washed with diethyl ether (1x100 mL) to afford 5-bromo-6-methyl-N-(oxan-2-yl)thieno[3,2-c][1,2]thiazol-3-amine (58.00 g, 82.38%) as a yellow solid.

### Step 2. Synthesis of 5-bromo-6-methyl-N-(oxan-2-yl)-N-(pyridin-4-ylmethyl)thieno[3,2-c][1,2]thiazol-3-amine.

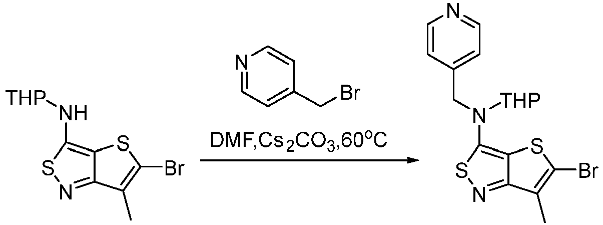

A mixture of 5-bromo-6-methyl-N-(oxan-2-yl)thieno[3,2-c][1,2]thiazol-3-amine (2.5 g, 7.502 mmol, 1 equiv) and 4-(bromomethyl)pyridine (1.55 g, 9.00 mmol, 1.20 equiv) and Cs₂CO₃ (7.33 g, 22.50 mmol, 3.00 equiv) in DMF (30.00 mL) was stirred for 6 h at 60°C under nitrogen atmosphere. The mixture was allowed to cool down to room temperature. The resulting mixture was diluted with water (100.00 mL). The resulting mixture was extracted with EtOAc (3 x 50.00 mL). The combined organic layers were washed with water (3x50.00 mL), dried over anhydrous Na₂SO₄. After filtration, the filtrate was concentrated under reduced pressure. The residue was purified by silica gel column chromatography, eluted with PE / EA (1:1) to afford 5-bromo-6-methyl-N-(oxan-2-yl)-N-(pyridin-4-ylmethyl)thieno[3,2-c][1,2]thiazol-3-amine (2.50 g, 74.60%) as a yellow solid.

### Step 3. Synthesis of tert-butyl 6-(6-methyl1-3-((pyridin-4-ylmethyl)(tetrahydro-2H-pyran-2-yl)amino)thieno[3,2-c]isothiazol-5-yl)-3,6-diazabicyclo[3.1.1]heptane-3-carboxylate

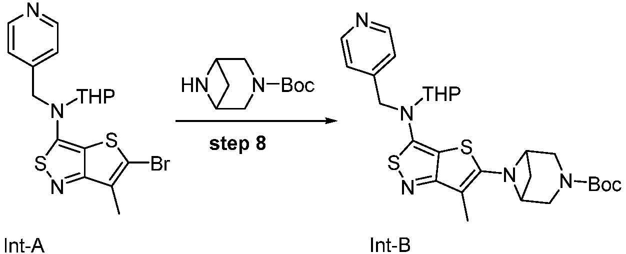

Into a 40-mL vial tube, was placed 5-bromo-6-methyl-N-(pyridin-4-ylmethyl)-N-(tetrahydro-2H-pyran-2-yl)thieno[3,2-c]isothiazol-3-amine (120 mg, 0.28 mmol, 1.0 equiv), tert-butyl 3,6-diazabicyclo[3.1.1]heptane-3-carboxylate (67 mg, 0.34 mmol, 1.2 equiv), Cs₂CO₃ (274 mg, 0.84 mmol, 3.0 equiv), Pd₂(dba)₃ (26 mg, 0.028 mmol, 0.10 equiv), BINAP (18 mg, 0.028 mmol, 0.10 equiv), 1,4-Dioxane(3.0 mL) under N₂ atmosphere. The resulting solution was stirred for 12 h at 100°C. The resulting mixture was purified by C18 column (Flash Column: Welch Flash AQ C18, Flow rate: 80 ml/min; Mobile phase A: 0.05% HCl; Mobile phase B: CAN) to give tert-butyl 6-(6-methyl-3-((pyridin-4-ylmethyl)(tetrahydro-2H-pyran-2-yl)amino)thieno[3,2-c]isothiazol-5-yl)-3,6-diazabicyclo[3.1.1]heptane-3-carboxylate (50 mg, 32.7%) as a solid.

### Step 4.

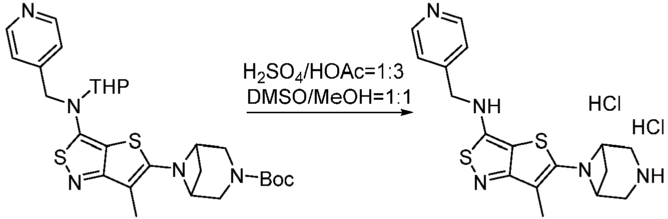

Into a 40-mL vial tube, was placed tert-butyl 6-(6-methyl-3-((pyridin-4-ylmethyl) (tetrahydro-2H-pyran-2-yl) amino) thieno[3,2-c] isothiazol-5-yl)-3,6-diazabicyclo [3.1.1] heptane-3-carboxylate (50 mg, 0.09 mmol, 1.0 equiv) and DMSO/MeOH =1:1 (3.0 mL). This was followed by addition of H₂SO₄/HOAc=1:3 (0.5 mL). The mixture was stirred at 60°C for 2 h. The resulting mixture was purified by Prep-HPLC (Column: Welch Xtimate C18 30* 150 mm, 10 um, Flow rate: 35 ml/min, Mobile phase A: 0.05% HCl; Mobile phase B: CAN). The eluent with final compound was concentrated and lyophilized to give 5-(3,6-diazabicyclo [3.1.1] heptan-6-yl)-6-methyl-N-(pyridin-4-ylmethyl) thieno[3,2-c] isothiazol-3-amine hydrogen chloride (32.8 mg, 82.7%).

LCMS (ES, m/z): [M+H]⁺= 358. ¹H-NMR (300 MHz, DMSO-*d₆*): δ 9.56 - 9.40 (m, 1H), 8.97 - 8.88 (m, 2H), 8.07 - 7.99 (m, 2H), 4.75 (s, 2H), 4.46 - 4.38 (m, 2H), 3.68 - 3.50 (m, 2H), 3.42 - 3.28 (m, 2H), 2.31 - 2.14 (m, 2H), 1.96 (s, 3H).

### Example 9, Specific example of General Scheme I, Synthesis of 5-[(2S)-2-aminopropyl]-N-(furan-2-ylmethyl)-6-methoxythieno[3,2-c] [1,2]thiazol-3-amine Compound 26

### Step 1. Synthesis of tert-butyl N-{5-[(2S)-2-[(tert-butoxycarbonyl)amino]propyl]-6-hydroxythieno[3,2-c][1,2]thiazol-3-yl}-N-(furan-2-ylmethyl)

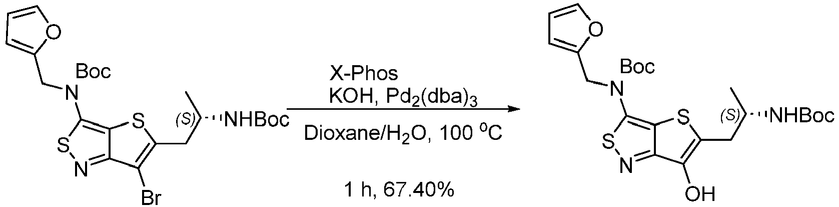

Into a 25-mL vial purged and maintained with an inert atmosphere of nitrogen, was placed tert-butyl N-{6-bromo-5-[(2S)-2-[(tert-butoxycarbonyl)amino]propyl]thieno[3,2-c][1,2]thiazol-3-yl}-N-(furan-2-ylmethyl)carbamate (500 mg, 0.873 mmol, 1 equiv), X-Phos (83.27 mg, 0.175 mmol, 0.2 equiv), Pd₂(dba)₃ (79.97 mg, 0.087 mmol, 0.1 equiv), KOH (97.99 mg, 1.746 mmol, 2 equiv), dioxane (8 mL), H₂O (1 mL). The resulting solution was stirred for 1 h at 100 degrees C. The reaction was then quenched by the addition of 2 mL of water. The resulting solution was extracted with 3x10 mL of ethyl acetate and the organic layers combined and dried over anhydrous sodium sulfate. The residue was applied onto a silica gel column with dichloromethane/methanol (10:1). This resulted in 300 mg (67.40%) of tert-butyl N-{5-[(2S)-2-[(tert-butoxycarbonyl)amino]propyl]-6-hydroxythieno[3,2-c][1,2]thiazol-3-yl}-N-(furan-2-ylmethyl)carbamate as a yellow solid.

### Step 2. Synthesis of tert-butyl N-{5-[(2S)-2-[(tert-butoxycarbonyl)amino]propyl]-6-methoxythieno[3,2-c] [1,2]thiazol-3-yl}-N-(furan-2-ylmethyl)carbamate

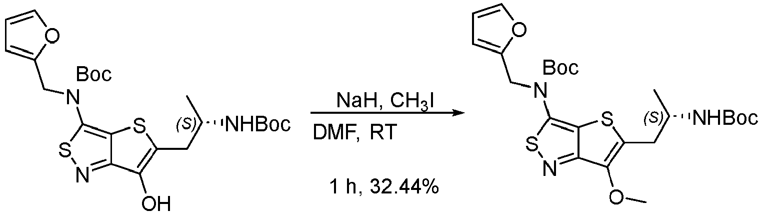

Into a 25-mL vial purged and maintained with an inert atmosphere of nitrogen, was placed tert-butyl N-{5-[(2S)-2-[(tert-butoxycarbonyl)amino]propyl]-6-hydroxythieno[3,2-c][1,2]thiazol-3-yl}-N-(furan-2-ylmethyl)carbamate (300 mg, 0.589 mmol, 1 equiv), DMF (5 mL), NaH (28.25 mg, 1.178 mmol, 2 equiv). The resulting solution was stirred for 0.5 h at 25 degrees C. Then CH₃I (125.33 mg, 0.883 mmol, 1.5 equiv) was added into the above mixture for one time at room temperature. Subsequently, the resulting solution was stirred for 1 h at 25 degrees C. The reaction was then quenched by the addition of 2 mL of water. The resulting solution was extracted with 3x10 mL of ethyl acetate and the organic layers combined and dried over anhydrous sodium sulfate. The residue was applied onto a silica gel column with dichloromethane/methanol (10:1). This resulted in 100 mg (32.44%) of tert-butyl N-{ 5-[(2S)-2-[(tert-butoxycarbonyl)amino]propyl]-6-methoxythieno[3,2-c][1,2]thiazol-3-yl}-N-(furan-2-ylmethyl)carbamate as a yellow solid.

### Step 3. Synthesis of 5-[(2S)-2-aminopropyl]-N-(furan-2-ylmethyl)-6-methoxythieno[3,2-c][1,2]thiazol-3-amine

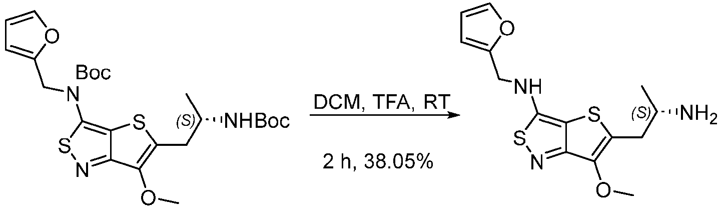

Into a 8-mL vial purged and maintained with an inert atmosphere of nitrogen, was placed tert-butyl N-{5-[(2S)-2-[(tert-butoxycarbonyl)amino]propyl]-6-methoxythieno[3,2-c][1,2]thiazol-3-yl}-N-(furan-2-ylmethyl)carbamate (100 mg, 0.191 mmol, 1 equiv), DCM (2 mL), TFA (1 mL). The resulting solution was stirred for 2 hr at room temperature. Then concentrate the reaction solution under vacuum. The residue was applied on the HP-Flash with acetonitrile/NH₄HCO₃ (4:1). The desired fractions were collected and concentrated under reduced pressure to remove most of the acetonitrile. Then the mixture was lyophilizied to dryness. This resulted in 23.5 mg (38.05%) of 5-[(2S)-2-aminopropyl]-N-(furan-2-ylmethyl)-6-methoxythieno[3,2-c][1,2]thiazol-3-amine.

LCMS: C₁₄H₁₇N₃O₂S₂calc. [M+H]⁺ 324.08 found324.00.

HNMR: ¹H NMR (400 MHz, Methanol-*d*₄) δ 7.46 (t, *J =* 1.3 Hz, 1H), 6.41 - 6.34 (m, 2H), 4.41 (s, 2H), 4.10 (s, 3H), 3.55 (q, *J =* 6.7 Hz, 1H), 2.98 (qd, *J =* 14.7, 6.8 Hz, 2H), 1.34 (d, *J =* 6.5 Hz, 3H).

### Example 10. Specific Example of General Scheme J, Synthesis of 7-[(S)-2-aminopropyl]-4-thenylamino-8-(trifluoromethyl)-3,6-dithia-2-azabicyclo[3.3.0] octa-1,4,7-triene Compound 35

### Step1. Synthesis of tert-butyl N-[6-(4,4,5,5-tetramethyl-1,3,2-dioxaborolan-2-yl)thieno[3,2-c][1,2]thiazol-3-yl]-N-(thiophen-2-ylmethyl)carbamate

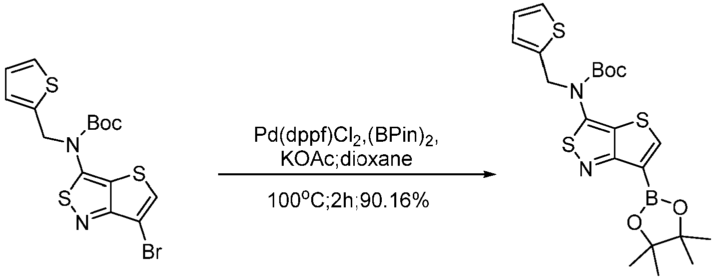

To a solution of tert-butyl N-{6-bromothieno[3,2-c][1,2]thiazol-3-yl}-N-(thiophen-2-ylmethyl)carbamate (1 g, 2.318 mmol, 1 equiv) and 4,4,5,5-tetramethyl-2-(4,4,5,5-tetramethyl-1,3,2-dioxaborolan-2-yl)-1,3,2-dioxaborolane(1.17 mg, 4.636 mmol, 2 equiv) in dioxane (10 mL) were added Pd(dppf)Cl₂ (169.62 mg, 0.232 mmol, 0.1 equiv) and KOAc (455.01 mg, 4.636 mmol, 2 equiv). After stirring for 2h at 100°C under a nitrogen atmosphere, the resulting mixture was concentrated under reduced pressure. The residue was purified by Prep-TLC, eluted with PE / EA (5:1) to afford tert-butyl N-[6-(4,4,5,5-tetramethyl-1,3,2-dioxaborolan-2-yl)thieno[3,2-c][1,2]thiazol-3-yl]-N-(thiophen-2-ylmethyl)carbamate (1 g, 90.16%) as a yellow solid.

### Step2. Synthesis of tert-butyl N-(thiophen-2-ylmethyl)-N-[6-(trifluoromethyl)thieno[3,2-c] [1,2]thiazol-3-yl]carbamate

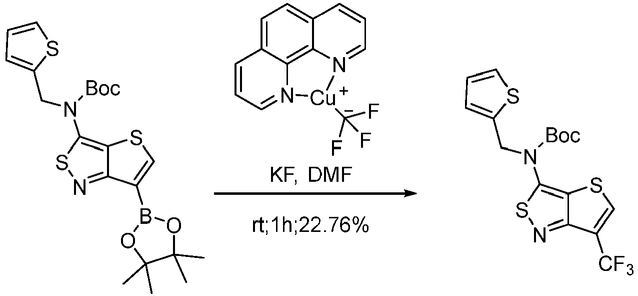

Into a 40 mL vial were added tert-butyl N-[6-(4,4,5,5-tetramethyl-1,3,2-dioxaborolan-2-yl)thieno[3,2-c][1,2]thiazol-3-yl]-N-(thiophen-2-ylmethyl)carbamate (1 g, 2.090 mmol, 1 equiv), trifluoro(1lambda4,12lambda4-diaza-15-cupratetracyclo[10.2.1.0^{5,14 }.0^{8,13}]pentadeca-1,3,5,7,9,11,13-heptaen-15-ylium-15-yl)methanuide (1.31 g, 4.180 mmol, 2 equiv), KF (121.43 mg, 2.090 mmol, 1 equiv) and DMF (20 mL) at room temperature. The resulting mixture was stirred for 1h at room temperature under nitrogen atmosphere. The residue was purified by reversed-phase flash chromatography with the following conditions: column, C₁₈; mobile phase, MeCN in Water (10mmol/L NH₄HCO₃), 40% to 100% gradient in 10 min; detector, UV 254 nm. This resulted in tert-butyl N-(thiophen-2-ylmethyl)-N-[6-(trifluoromethyl)thieno[3,2-c][1,2]thiazol-3-yl]carbamate (200 mg, 22.76%) as a yellow solid.

### Step 3. Synthesis of tert-butyl N-{5-[(2S)-2-[(tert-butoxycarbonyl)amino]propyl]-6-(trifluoromethyl)thieno[3,2-c] [1,2]thiazol-3-yl}-N-(thiophen-2-ylmethyl)carbamate

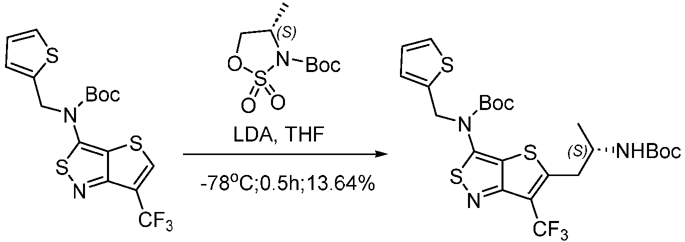

In a 50-mL round bottom flask, to a solution of tert-butyl N-(thiophen-2-ylmethyl)-N-[6-(trifluoromethyl)thieno[3,2-c][1,2]thiazol-3-yl]carbamate (200 mg, 0.476 mmol, 1 equiv) in THF (10 mL) was added dropwise LDA(in 2M THF) (0.476 mL, 0.952 mmol, 2 equiv) at -78 °C under N₂ atmosphere. The reaction mixture was stirred at -78 °C for 30 mins. Then a solution of tert-butyl (4S)-4-methyl-2,2-dioxo-1,21ambda6,3-oxathiazolidine-3-carboxylate (169.29 mg, 0.714 mmol, 1.5 equiv) in 3 mL THF was added dropwise and the mixture was stirred for another 30 mins. The residue was purified by reversed-phase flash chromatography with the following conditions: column, C₁₈; mobile phase, MeCN in Water (10mmol/L NH₄HCO₃), 10% to 50% gradient in 10 min; detector, UV 254 nm. This resulted in tert-butyl N-{5-[(2S)-2-[(tert-butoxycarbonyl)amino]propyl]-6-(trifluoromethyl)thieno[3,2-c] [1,2]thiazol-3-yl }-N-(thiophen-2-ylmethyl)carbamate (30 mg, 13.64%) as a yellow solid.

### Step 4. Synthesis of 7-[(S)-2-aminopropyl]-4-thenylamino-8-(trifluoromethyl)-3,6-dithia-2-azabicyclo[3.3.0]octa-1,4,7-triene

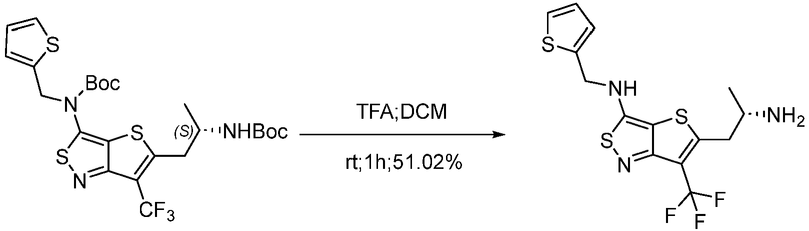

Into a 8 mL vial were added tert-butyl N-{5-[(2S)-2-[(tert-butoxycarbonyl)amino]propyl]-6-(trifluoromethyl)thieno[3,2-c][1,2]thiazol-3-yl}-N-(thiophen-2-ylmethyl)carbamate (30 mg, 0.052 mmol, 1 equiv), DCM (2 mL) and TFA (1 mL) at 0°C. The resulting mixture was stirred for 1h at room temperature. The resulting mixture was concentrated under vacuum. The residue was purified by reversed-phase flash chromatography with the following conditions: column, C₁₈; mobile phase, MeCN in Water (10mmol/L NH₄HCO₃), 10% to 50% gradient in 10 min; detector, UV 254 nm. This resulted in 5-[(2S)-2-aminopropyl]-N-(thiophen-2-ylmethyl)-6-(trifluoromethyl)thieno[3,2-c][1,2]thiazol-3-amine (10 mg, 51.02%).

LC-MS (ES, *m*/*z):* [M+H]⁺= 378.10. ¹H NMR (400 MHz, Methanol-*d*₄) δ 7.36 (dd, *J =* 5.1, 1.2 Hz, 1H), 7.13 (dt*, J* = 3.3, 1.1 Hz, 1H), 6.99 (dd, *J =* 5.1, 3.5 Hz, 1H), 4.66 (d, *J =* 0.9 Hz, 2H), 3.67 - 3.56 (m, 1H), 3.35 (s, 1H), 3.25 - 3.16 (m, 1H), 1.34 (d, *J =* 6.5 Hz, 3H).

### Example 11: Specific Example of General Scheme K, Synthesis of 7-[(R)-2-amino-3-butynyl]-8-methyl-4-thenylamino-3,6-dithia-2-azabicyclo[3.3.0]octa-1,4,7-triene Compound 54

### Step 1. Synthesis of tert-butyl N-{5-[(2R)-2-[(tert-butoxycarbonyl)amino]-3-oxopropyl]-6-methylthieno[3,2-c][1,2]thiazol-3-yl}-N-(thiophen-2-ylmethyl)carbamate

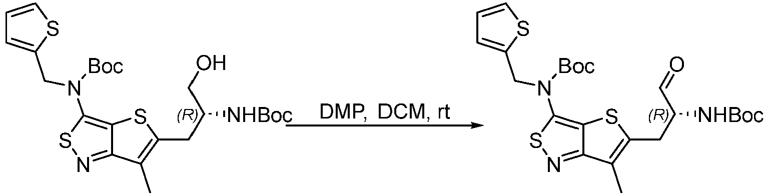

A solution of tert-butyl N-{5-[(2R)-2-[(tert-butoxycarbonyl)amino]-3-hydroxypropyl]-6-methylthieno[3,2-c][1,2]thiazol-3-yl}-N-(thiophen-2-ylmethyl)carbamate (550 mg, 1.019 mmol, 1 equiv) in DCM (10 mL) was treated with DMP (561.89 mg, 1.325 mmol, 1.3 equiv) at room temperature. The resulting mixture was stirred for additional 2 h. The crude product was used in the next step directly without further purification.

### Step 2. Synthesis of tert-butyl N-{5-[(2R)-2-[(tert-butoxycarbonyl)amino]but-3-yn-1-yl]-6-methylthieno[3,2-c][1,2]thiazol-3-yl}-N-(thiophen-2-ylmethyl)carbamate

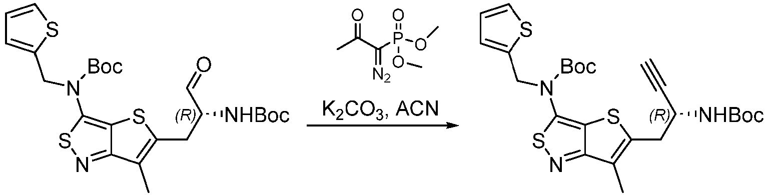

A solution of tert-butyl N-{5-[(2R)-2-[(tert-butoxycarbonyl)amino]-3-oxopropyl]-6-methylthieno[3,2-c][1,2]thiazol-3-yl}-N-(thiophen-2-ylmethyl)carbamate (300 mg, 0.558 mmol, 1 equiv) and K₂CO₃ (154.22 mg, 1.116 mmol, 2 equiv) in MeOH (6.00 mL) was treated with dimethyl (1-diazo-2-oxopropyl)phosphonate (128.62 mg, 0.670 mmol, 1.2 equiv) for 1 min at 0 °C under nitrogen atmosphere followed. The residue was purified by silica gel column chromatography, eluted with PE / EA (9:1) to afford tert-butyl N-{5-[(2R)-2-[(tert-butoxycarbonyl)amino]but-3-yn-1-yl]-6-methylthieno[3,2-c][1,2]thiazol-3-yl}-N-(thiophen-2-ylmethyl)carbamate (200 mg, 67.17%) as a yellow oil.

### Step 3. Synthesis of 7-[(R)-2-amino-3-butynyl]-8-methyl-4-thenylamino-3,6-dithia-2-azabicyclo[3.3.0]octa-1,4,7-triene

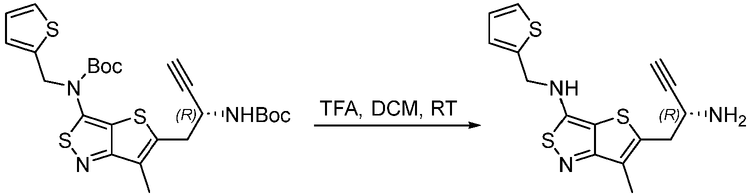

A solution of tert-butyl N-{5-[(2R)-2-[(tert-butoxycarbonyl)amino]but-3-yn-1-yl]-6-methylthieno[3,2-c][1,2]thiazol-3-yl}-N-(thiophen-2-ylmethyl)carbamate (200 mg, 0.375 mmol, 1 equiv) in DCM (4 mL) was treated with TFA (2 mL) for 1 min at room temperature. The resulting mixture was stirred for additional 2 h at room temperature. The reaction was monitored by LCMS. The crude product (90 mg) was purified by Prep-HPLC with the following conditions (Column: XBridge Prep C18 OBD Column, 19*150 mm, 5µm; Mobile Phase A: Water (10 mmol/L NH₄HCO₃), Mobile Phase B: ACN; Flow rate: 25 mL/min; Gradient: 10% B to 35% B in 7 min, 35% B; Wave Length: 254/220 nm; RT1(min): 6.35; Number Of Runs: 0) to afford 5-[(2R)-2-aminobut-3-yn-1-yl]-6-methyl-N-(thiophen-2-ylmethyl)thieno[3,2-c][1,2]thiazol-3-amine (56 mg, 44.81%).

LCMS [M+H]⁺=334 ¹H NMR (400 MHz, DMSO-*d*₆) δ 8.16 (t, *J =* 5.8 Hz, 1H), 7.45 (dd, *J =* 5.1, 1.3 Hz, 1H), 7.11 (dd, *J* = 3.4*,* 1.2 Hz, 1H), 6.99 (dd, *J =* 5.1, 3.4 Hz, 1H), 4.55 (d, *J =* 5.7 Hz, 2H), 3.71 (td, *J =* 6.9, 2.2 Hz, 1H), 3.12 (d, *J =* 2.2 Hz, 1H), 2.99 - 2.84 (m, 2H), 2.30 (d, *J =* 31.0 Hz, 2H), 2.10 (s, 3H).

### Example 12: Specific Example of General Scheme L, Synthesis of 7-[(R)-2-amino-3-pentynyl]-8-methyl-4-thenylamino-3,6-dithia-2-azabicyclo[3.3.0]octa-1,4,7-triene Compound 55

### Step 1. Synthesis of tert-butyl N-{6-methyl-5-[(2R)-2-(2,2,10,10-tetramethyl-8-oxo-5,9-dioxa-7-aza-2-silaundecan-7-yl)but-3-yn-1-yl]thieno[3,2-c][1,2]thiazol-3-yl}-N-(thiophen-2-ylmethyl)carbamate

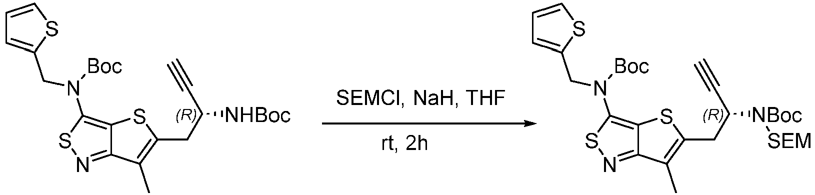

A solution of tert-butyl N-{5-[(2R)-2-[(tert-butoxycarbonyl)amino]but-3-yn-1-yl]-6-methylthieno[3,2-c][1,2]thiazol-3-yl}-N-(thiophen-2-ylmethyl)carbamate (300 mg, 0.562 mmol, 1 equiv) in THF (4 mL) was treated with NaH (60%) (26.98 mg, 0.674 mmol, 1.2 equiv) for 30 min at 0 °C under nitrogen atmosphere. To the above mixture was added SEMCl (187.42 mg, 1.124 mmol, 2 equiv) dropwise over 1 min at 0 °C. The resulting mixture was stirred for additional 2 h at room temperature. The residue was purified by silica gel column chromatography, eluted with PE / EA (5:1) to afford tert-butyl N-{6-methyl-5-[(2R)-2-(2,2,10,10-tetramethyl-8-oxo-5,9-dioxa-7-aza-2-silaundecan-7-yl)but-3-yn-1-yl]thieno[3,2-c][1,2]thiazol-3-yl}-N-(thiophen-2-ylmethyl)carbamate (200 mg, 53.59%) as a yellow solid.

### Step 2. Synthesis of tert-butyl N-{6-methyl-5-[(2R)-2-(2,2,10,10-tetramethyl-8-oxo-5,9-dioxa-7-aza-2-silaundecan-7-yl)pent-3-yn-1-yl]thieno[3,2-c][1,2]thiazol-3-yl}-N-(thiophen-2-ylmethyl)carbamate

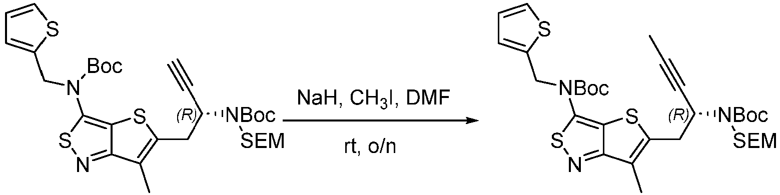

A solution of tert-butyl N-{6-methyl-5-[(2R)-2-(2,2,10,10-tetramethyl-8-oxo-5,9-dioxa-7-aza-2-silaundecan-7-yl)but-3-yn-1-yl]thieno[3,2-c][1,2]thiazol-3-yl}-N-(thiophen-2-ylmethyl)carbamate (300 mg, 0.452 mmol, 1 equiv) in DMF (6 mL) was treated with NaH (60%) (23.5 mg, 0.588 mmol, 1.3 equiv) for 30 min at 0 °C under a nitrogen atmosphere. To the above mixture was added CH₃I (70.54 mg, 0.497 mmol, 1.1 equiv) dropwise over 1 min at 0 °C. The resulting mixture was stirred for additional overnight at room temperature. The residue was purified by silica gel column chromatography, eluted with PE / EA (6: 1) to afford tert-butyl N-{6-methyl-5-[(2R)-2-(2,2,10,10-tetramethyl-8-oxo-5,9-dioxa-7-aza-2-silaundecan-7-yl)pent-3-yn-1-yl]thieno[3,2-c][1,2]thiazol-3-yl}-N-(thiophen-2-ylmethyl)carbamate (150 mg, 48.97%) as a yellow solid.

### Step 3. Synthesis of 7-[(R)-2-amino-3-pentynyl]-8-methyl-4-thenylamino-3,6-dithia-2-azabicyclo[3.3.0]octa-1,4,7-triene

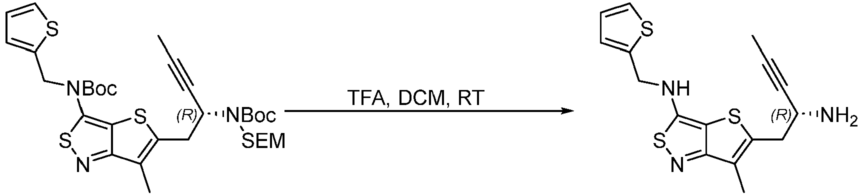

A solution of tert-butyl N-{6-methyl-5-[(2R)-2-(2,2,10,10-tetramethyl-8-oxo-5,9-dioxa-7-aza-2-silaundecan-7-yl)pent-3-yn-1-yl]thieno[3,2-c][1,2]thiazol-3-yl}-N-(thiophen-2-ylmethyl)carbamate (200 mg, 0.295 mmol, 1 equiv) in DCM (4 mL) was treated with TFA (2 mL) for 1 min at room temperature. The resulting mixture was stirred for additional 2 h at room temperature. The reaction was monitored by LCMS. The crude product (90 mg) was purified by Prep-HPLC with the following conditions (Column: XBridge Prep C18 OBD Column, 19*150 mm, 5µm; Mobile Phase A: Water (10 mmol/L NH₄HCO₃), Mobile Phase B: ACN; Flow rate: 25 mL/min; Gradient: 10% B to 35% B in 7 min, 35% B; Wave Length: 254/220 nm; RT1(min): 6.35) to afford 7-[(R)-2-amino-3-pentynyl]-8-methyl-4-thenylamino-3,6-dithia-2-azabicyclo[3.3.0]octa-1,4,7-triene (30 mg, 29.27%).

LCMS: [M+H]⁺=348. ¹H NMR (400 MHz, Methanol-*d*₄) δ 7.35 (dd, *J =* 5.1, 1.2 Hz, 1H), 7.13 -7.05 (m, 1H), 6.98 (dd, *J* = 5.1*,* 3.5 Hz, 1H), 4.84 - 4.77 (m, 2H), 4.39 (ddd, *J =* 9.0, 5.9, 2.3 Hz, 1H), 3.39 - 3.33 (m, 1H), 3.29 (d, *J =* 2.2 Hz, 1H), 3.12 (s, 3H), 2.25 (s, 3H).

### Example 13: Specific Example of General Method M, Synthesis of 5-[(2S)-2-aminopropyl]-N-(thiophen-2-ylmethyl)thieno[3,2-c][1,2]thiazol-3-amine Compound 66

### Step 1. Synthesis of tert-butyl N-{5-[(2S)-2-[(tert-butoxycarbonyl)amino]propyl]thieno[3,2-c][1,2]thiazol-3-yl}-N-(thiophen-2-ylmethyl)carbamate

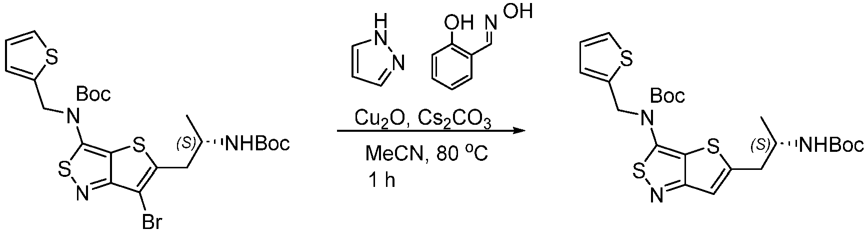

Into a 25-mL vial purged and maintained with an inert atmosphere of nitrogen, was placed tert-butyl N-{6-bromo-5-[(2S)-2-[(tert-butoxycarbonyl)amino]propyl]thieno[3,2-c][1,2]thiazol-3-yl}-N-(thiophen-2-ylmethyl)carbamate (100 mg, 0.170 mmol, 1 equiv), pyrazole (13.88 mg, 0.204 mmol, 1.2 equiv), 2-[(1E)-(hydroxyimino)methyl]phenol (23.30 mg, 0.170 mmol, 1 equiv), Cu₂O (2.43 mg, 0.017 mmol, 0.1 equiv), Cs₂CO₃ (55.36 mg, 0.170 mmol, 1 equiv), MeCN (5 mL). The resulting solution was stirred for 1 h at 80 degrees C. The reaction was then quenched by the addition of 2 mL of water. The resulting solution was extracted with 3x10 mL of ethyl acetate and the organic layers combined and dried over anhydrous sodium sulfate. The residue was applied onto a silica gel column with dichloromethane/methanol (10:1). This resulted in 50 mg (57.74%) of tert-butyl N-{5-[(2S)-2-[(tert-butoxycarbonyl)amino]propyl]thieno[3,2-c][1,2]thiazol-3-yl}-N-(thiophen-2-ylmethyl)carbamate as a yellow solid.

### Step 2. Synthesis of 5-[(2S)-2-aminopropyl]-N-(thiophen-2-ylmethyl)thieno[3,2-c][1,2]thiazol-3-amine

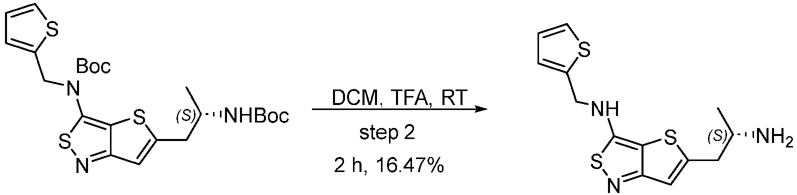

Into a 8-mL vial purged and maintained with an inert atmosphere of nitrogen, was placed tert-butyl N-{5-[(2S)-2-[(tert-butoxycarbonyl)amino]propyl]thieno[3,2-c][1,2]thiazol-3-yl}-N-(thiophen-2-ylmethyl) carbamate (50 mg, 0.098 mmol, 1 equiv), DCM (2 mL), TFA (1 mL). The resulting solution was stirred for 2 hr at room temperature. Then concentrate the reaction solution under vacuum. The residue was applied on the HP-Flash with acetonitrile/NH₄HCO₃ (4:1). The desired fractions were collected and concentrated under reduced pressure to remove most of the acetonitrile. Then the mixture was lyophilized to dryness. This resulted in 5 mg (16.47%) of 5-[(2S)-2-aminopropyl]-N-(thiophen-2-ylmethyl)thieno[3,2-c][1,2]thiazol-3-amine.

LCMS: [M+H]⁺ = 310.10. ¹HNMR (400 MHz, Methanol-*d*₄) δ 7.33 (dd, *J =* 5.1, 1.3 Hz, 1H), 7.10 (dt, *J* = 3.4, 1.1 Hz, 1H), 6.97 (dd, *J* = 5.1, 3.5 Hz, 1H), 6.63 (d, *J =* 1.1 Hz, 1H), 4.63 (d,*J* = 0.9 Hz, 2H), 3.20 (p, *J =* 6.5 Hz, 1H), 2.86 - 2.80 (m, 2H), 1.16 (d, *J =* 6.4 Hz, 3H).

### Example 14: Specific Example of General Scheme N, Synthesis of N-[(2S)-1-{6-methyl-3-[(thiophen-2-ylmethyl)amino]thieno[3,2-c][1,2]thiazol-5-yl}propan-2-yl]acetamide Compound 79

### Step 1. Synthesis of tert-butyl N-{5-[(2S)-2-[N-(tert-butoxycarbonyl)acetamido]propyl]-6-methylthieno[3,2-c][1,2]thiazol-3-yl}-N-(thiophen-2-ylmethyl)carbamate

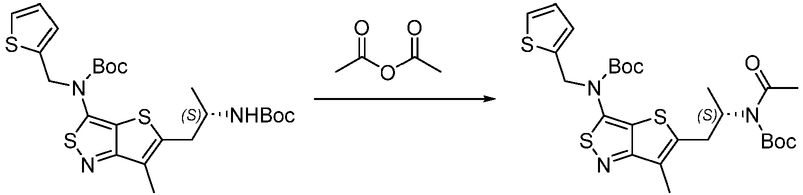

A mixture of tert-butyl N-{5-[(2S)-2-[(tert-butoxycarbonyl)amino]propyl]-6-methylthieno[3,2-c][1,2]thiazol-3-yl}-N-(thiophen-2-ylmethyl)carbamate (200 mg, 0.382 mmol, 1 equiv) in acetic anhydride (3 mL) was stirred for 3 days at 90 °C. The residue was purified by reversed-phase flash chromatography with the following conditions: column, C₁₈; mobile phase, MeCN in Water (10mmol/L NH₄HCO₃), 10% to 50% gradient in 10 min; detector, UV 254 nm. This resulted in tert-butyl N-{5-[(2S)-2-[N-(tert-butoxycarbonyl)acetamido]propyl]-6-methylthieno[3,2-c][1,2]thiazol-3-yl}-N-(thiophen-2-ylmethyl)carbamate (100 mg, 46.29%) as a yellow solid.

### Step 2. Synthesis of N-[(2S)-1- { 6-methyl-3-[(thiophen-2-ylmethyl)amino]thieno[3,2-c][1,2]thiazol-5-yl}propan-2-yl] acetamide

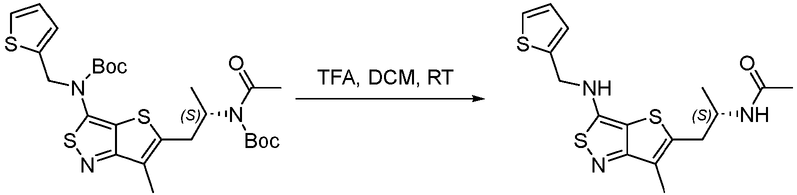

A solution of tert-butyl N-{5-[(2S)-2-[N-(tert-butoxycarbonyl)acetamido]propyl]-6-methylthieno[3,2-c][1,2]thiazol-3-yl}-N-(thiophen-2-ylmethyl)carbamate (100 mg, 0.177 mmol, 1 equiv) in DCM (4 mL) was treated with TFA (2 mL) for 1 min at room temperature. The reaction was monitored by LCMS. The crude product (90 mg) was purified by Prep-HPLC with the following conditions (Column: XBridge Prep C18 OBD Column, 19*150 mm, 5µm; Mobile Phase A: Water (10 mmol/L NH₄HCO₃), Mobile Phase B: ACN; Flow rate: 25 mL/min; Gradient: 10% B to 35% B in 7 min, 35% B; Wave Length: 254/220 nm; RT1(min): 6.35) to afford N-[(2S)-1-{6-methyl-3-[(thiophen-2-ylmethyl)amino]thieno[3,2-c][1,2]thiazol-5-yl}propan-2-yl]acetamide (24 mg, 37.15%).

LCMS [M+H]⁺=366. ¹H NMR (400 MHz, Methanol-*d*₄) δ 7.46 - 7.32 (m, 1H), 6.98 (d, *J =* 3.7 Hz, 2H), 5.65 (s, 2H), 3.48 (dt, *J* = 7.8*,* 6.3 Hz, 1H), 3.15 - 2.87 (m, 2H), 2.47 (s, 3H), 2.27 (s, 3H), 1.28 (d, *J =* 6.5 Hz, 3H).

### Example 15: Specific Example of General Scheme O, 5-[(2S)-2-amino-1,1-difluoropropyl]-6-bromo-N-(thiophen-2-ylmethyl)thieno[3,2-c][1,2]thiazol-3-amine Compound 99

### Step 1. Synthesis of tert-butyl N-{6-bromo-5-[(2S)-2-[(tert-butoxycarbonyl)amino]-1-hydroxypropyl]thieno[3,2-c][1,2]thiazol-3-yl}-N-(thiophen-2-ylmethyl)carbamate

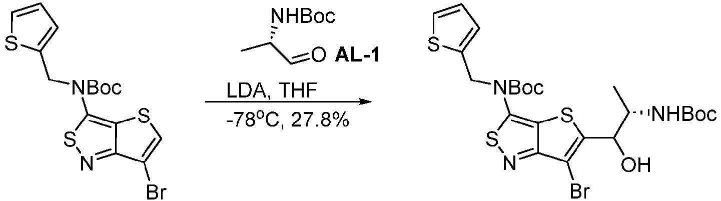

Into a 40-mL vial, to a solution of tert-butyl N-{6-bromothieno[3,2-c][1,2]thiazol-3-yl}-N-(thiophen-2-ylmethyl)carbamate (1 g, 2.318 mmol, 1 equiv) in THF (10 mL) was added LDA (2.32 mL, 4.636 mmol, 2 equiv) dropwise at -78 degrees C under N₂ atmosphere. The reaction mixture was stirred at -78 degrees C for 30 mins. Then a solution of tert-butyl N-[(2S)-1-oxopropan-2-yl]carbamate (0.60 g, 3.477 mmol, 1.5 equiv) in 3 mL THF was added dropwise and the mixture was stirred for another 1h -78 degrees C. The reaction was quenched with sat. NH₄Cl (aq.) at 0°C. The residue was purified by reversed-phase flash chromatography with the following conditions: column, C18 silica gel; mobile phase, MeCN in water (10mmol/L NH₄HCO₃), 0% to 100% gradient in 100 min; detector, UV 254 nm. This resulted in tert-butyl N-{6-bromo-5-[(2S)-2-[(tert-butoxycarbonyl)amino]-1-hydroxypropyl]thieno[3,2-c][1,2]thiazol-3-yl}-N-(thiophen-2-ylmethyl)carbamate (390 mg, 27.83%) as a yellow solid.

### Step 2. Synthesis of tert-butyl N-{6-bromo-5-[(2S)-2-[(tert-butoxycarbonyl)amino]propanoyl]thieno[3,2-c] [1,2]thiazol-3-yl}-N-(thiophen-2-ylmethyl)carbamate (200 mg, 60.81%)

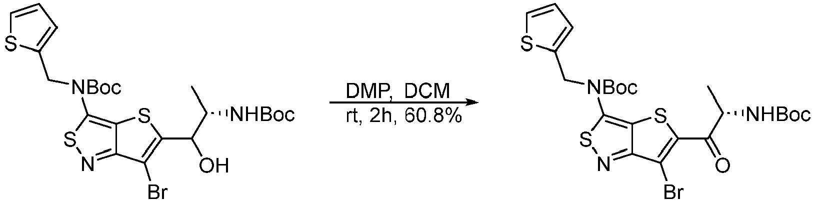

Into a 20-mL vial were added tert-butyl N-{6-bromo-5-[(2S)-2-[(tert-butoxycarbonyl)amino]-1-hydroxypropyl]thieno[3,2-c][1,2]thiazol-3-yl}-N-(thiophen-2-ylmethyl)carbamate (330 mg, 0.546 mmol, 1 equiv) and DMP (347.26 mg, 0.819 mmol, 1.5 equiv) in DCM (4 mL). The resulting mixture was stirred for 2h at room temperature. The reaction was quenched with sat. sodium hyposulfite (aq.) (10 mL) at 0°C. The resulting mixture was extracted with DCM (3 x 10 mL). The combined organic layers were washed with sat.NaHCO₃ (3 x 10 mL), dried over anhydrous Na₂SO₄. After filtration, the filtrate was concentrated under reduced pressure. The residue was purified by reversed-phase flash chromatography with the following conditions: column, C₁₈ silica gel; mobile phase, MeCN in Water (10mmol/L NH₄HCO₃), 20% to 100% gradient in 20 min; detector, UV 254 nm. This resulted in tert-butyl N-{6-bromo-5-[(2S)-2-[(tert-butoxycarbonyl)amino]propanoyl]thieno[3,2-c][1,2]thiazol-3-yl}-N-(thiophen-2-ylmethyl)carbamate (200 mg, 60.81%) as a yellow solid.

### Step 3. Synthesis of tert-butyl N-[(2S)-1-{6-bromo-3-[(tert-butoxycarbonyl)(thiophen-2-ylmethyl)amino]thieno[3,2-c][1,2]thiazol-5-yl}-1,1-difluoropropan-2-yl]carbamate

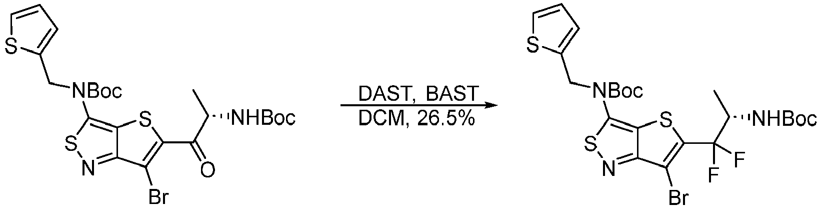

To a stirred solution of tert-butyl N-{6-bromo-5-[(2S)-2-[(tert-butoxycarbonyl)amino]propanoyl]thieno[3,2-c][1,2]thiazol-3-yl}-N-(thiophen-2-ylmethyl)carbamate (200 mg, 0.332 mmol, 1 equiv) in DCM (2 mL) was added DAST (1605.00 mg, 9.960 mmol, 30 equiv) dropwise at room temperature under nitrogen atmosphere. The resulting mixture was stirred for overnight at room temperature. To the above mixture was added BAST (367.15 mg, 1.660 mmol, 5 equiv) dropwise at 0°C. The resulting mixture was stirred for additional 6h at room temperature. The reaction was quenched by the addition of sat. NH₄Cl (aq.) (20mL) and sat. NaHCO₃ (aq.)(20 mL) at 0°C. The resulting mixture was extracted with EtOAc (3 x 20 mL). The combined organic layers were washed with brine (2 x 20 mL), dried over anhydrous Na₂SO₄. After filtration, the filtrate was concentrated under reduced pressure. The residue was purified by reversed-phase flash chromatography with the following conditions: column, C₁₈ silica gel; mobile phase, MeCN in Water (10mmol/L NH₄HCO₃), 20% to 100% gradient in 20 min; detector, UV 254 nm. This resulted in tert-butyl N-[(2S)-1-{6-bromo-3-[(tert-butoxycarbonyl)(thiophen-2-ylmethyl)amino]thieno[3,2-c][1,2]thiazol-5-yl}-1,1-difluoropropan-2-yl]carbamate (55 mg, 26.53%) as a yellow solid.

### Step 4. Synthesis of 5-[(2S)-2-amino-1,1-difluoropropyl]-6-bromo-N-(thiophen-2-ylmethyl)thieno[3,2-c][1,2]thiazol-3-amine

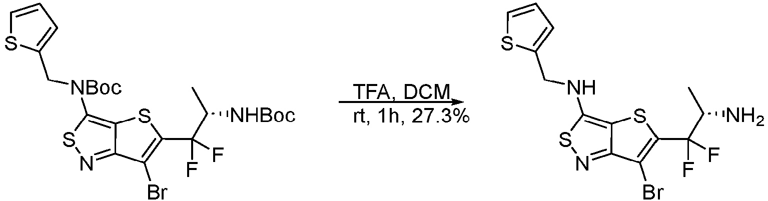

To a stirred solution of tert-butyl N-[(2S)-1-{6-bromo-3-[(tert-butoxycarbonyl)(thiophen-2-ylmethyl)amino]thieno[3,2-c][1,2]thiazol-5-yl}-1,1-difluoropropan-2-yl]carbamate (55 mg, 0.088 mmol, 1 equiv) in DCM (0.3 mL) was added TFA (0.1 mL) dropwise at 0°C. The resulting mixture was stirred for 1h at room temperature. The mixture was basified to pH 8 with DIEA at 0°C. The resulting mixture was concentrated under vacuum. The residue was purified by reversed-phase flash chromatography with the following conditions: column, C₁₈ silica gel; mobile phase, MeCN in Water (10mmol/L NH₄HCO₃), 0% to 100% gradient in 20 min; detector, UV 254 nm. This resulted in 5-[(2S)-2-amino-1,1-difluoropropyl]-6-bromo-N-(thiophen-2-ylmethyl)thieno[3,2-c] [1,2]thiazol-3-amine (10.3 mg, 27.34%).

LC-MS: (ES, m/z): [M+H]⁺=424 ¹H-NMR(400 MHz, Methanol-d4) δ 7.41 - 7.35 (m, 1H), 7.17 - 7.12 (m, 1H), 7.00 (m, 1H), 4.66 (s, 2H), 3.91 - 3.77 (m, 1H), 1.19 (d, J = 6.8 Hz, 3H).

### Example 16: Specific Example of General Method P, synthesis of (2S)-2-amino-1-{6-bromo-3-[(thiophen-2-ylmethyl)amino]thieno[3,2-c] [1,2]thiazol-5-yl}propan-1-ol Compound 103.

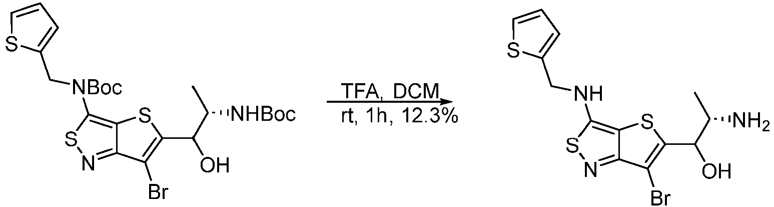

To a stirred solution of tert-butyl N-{6-bromo-5-[(2S)-2-[(tert-butoxycarbonyl)amino]-1-hydroxypropyl]thieno[3,2-c][1,2]thiazol-3-yl}-N-(thiophen-2-ylmethyl)carbamate (Step 1 of Example General Procedure O) (40 mg, 0.066 mmol, 1 equiv) in DCM (0.3 mL) was added TFA (0.1 mL) dropwise at 0°C. The resulting mixture was stirred for 1h at room temperature. The mixture was basified to pH 8 with DIEA at 0°C. The resulting mixture was concentrated under vacuum. The residue was purified by reverse-phase flash chromatography with the following conditions: column, C₁₈ silica gel; mobile phase, MeCN in Water (10mmol/L NH₄HCO₃), 0% to 100% gradient in 20 min; detector, UV 254 nm. This resulted in (2S)-2-amino-1-{6-bromo-3-[(thiophen-2-ylmethyl)amino]thieno[3,2-c][1,2]thiazol-5-yl}propan-1-ol (3.3 mg, 12.30%) as a white solid.

LC-MS [M+H]⁺=404 H-NMR400 MHz, Methanol-d4) δ 7.4 - 7.3 (m, 1H), 7.12 (d, J = 3.5 Hz, 1H), 7.03 - 6.93 (m, 1H), 4.83 (d, J = 7.4 Hz, 1H), 4.65 (s, 2H), 3.27 - 3.18 (m, 1H), 1.89 (s, 1H), 1.13 (d, J = 6.6 Hz, 3H).

### Example 17: Specific Example of General Scheme Q, Synthesis of (S)-2-amino-3-(6-bromo-3-((thiophen-2-ylmethyl)amino)thieno[3,2-c]isothiazol-5-yl)-1-morpholinopropan-1-one Compound 115.

### Step 1. Synthesis of methyl (S)-3-(6-bromo-3-((tert-butoxycarbonyl)(thiophen-2-ylmethyl)amino)thieno [3,2-c]isothiazol-5-yl)-2-((tert-butoxycarbonyl)amino)propanoate

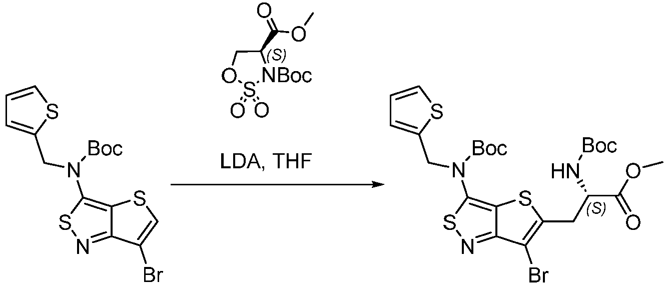

To a solution of tert-butyl (6-bromothieno[3,2-c]isothiazol-3-yl)(thiophen-2-ylmethyl)carbamate (1.0 g, 1 equiv, 2.3 mmol) in tetrahydrofuran (20 mL) was added lithium diisopropylamide (2.3 mL, 2 equiv, 4.6 mmol) dropwise at -78°C. After stirring for 30 mins at -78°C, 3-(tert-butyl) 4-methyl (S)-1,2,3-oxathiazolidine-3,4-dicarboxylate 2,2-dioxide (0.97 g, 1.5 equiv, 3.5 mmol) dissolved in tetrahydrofuran (5 mL) was added dropwise at -78°C. After the addition was completed, the reaction mixture was allowed to warm to room temperature. The reaction was quenched by addition of saturated ammonium chloride aqueous (5 mL) and the resulting mixture was extracted with ethyl acetate (20 mL x 2). The combined organic phases were dried over anhydrous sodium sulfate and concentrated under reduced pressure. The residue was purified by silica gel column chromatography, eluted with petroleum ether/ethyl acetate (3:1) to afford methyl (S)-3-(6-bromo-3-((tert-butoxycarbonyl)(thiophen-2-ylmethyl)amino)thieno [3,2-c]isothiazol-5-yl)-2-((tert-butoxycarbonyl)amino)propanoate (0.53 g, 40 %) as a yellow solid.

### Step 2. Synthesis of (S)-3-(6-bromo-3-((tert-butoxycarbonyl)(thiophen-2-ylmethyl)amino)thieno[3,2-c]isothiazol-5-yl) -2-((tert-butoxycarbonyl)amino)propanoic acid

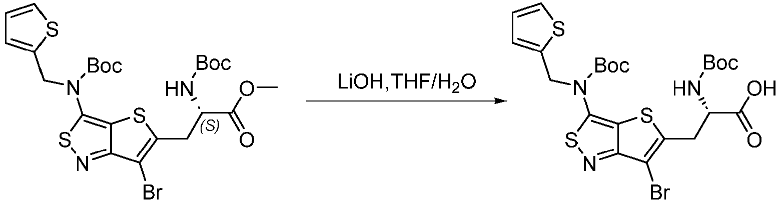

A solution of methyl (S)-3-(6-bromo-3-((tert-butoxycarbonyl)(thiophen-2-ylmethyl)amino)thieno [3,2-c]isothiazol-5-yl)-2-((tert-butoxycarbonyl)amino)propanoate (500 mg, 1 equiv, 0.79 mmol) in tetrahydrofuran (4 mL) and water (1 mL) was added lithium hydroxide (66 mg, 2 equiv, 1.58 mmol) at room temperature. The resulting mixture was stirred for 2 h at 40°C. After the reaction was cooled to room temperature, the resulting mixture was concentrated under reduced pressure. The resulting mixture was diluted with water (4 mL) and adjusted to pH 3-4 with 2M hydrochloric acid (4 mL). The precipitated solids were collected by filtration and washed with water (10 mL). This resulted in (S)-3-(6-bromo-3-((tert-butoxycarbonyl)(thiophen-2-ylmethyl)amino)thieno[3,2-c]isothiazol-5-yl) -2-((tert-butoxycarbonyl)amino)propanoic acid (483 mg, 99 %) as a yellow solid.

### Step 3. Synthesis of tert-butyl (S)-(6-bromo-5-(2-((tert-butoxycarbonyl)amino)-3-morpholino-3-oxopropyl)thieno[3,2-c]isothiazol-3-yl)(thiophen-2-ylmethyl)carbamate

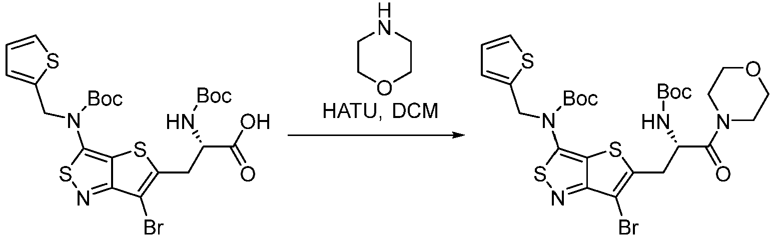

A solution of (S)-3-(6-bromo-3-((tert-butoxycarbonyl)(thiophen-2-ylmethyl)amino)thieno[3,2-c]isothiazol -5-yl)-2-((tert-butoxycarbonyl)amino)propanoic acid (100 mg, 1 equiv, 0.16 mmol) in dichloromethane (1 mL) was added morpholine (21 mg, 1.5 equiv, 0.24 mmol), 2-(7-Azabenzotriazol-1-yl)-*N,N,N',N'* -tetramethyluronium hexafluorophosphate (92 mg, 1.5 equiv, 0.24 mmol) and N,N-diisoproylethylamine (63 mg, 3 equiv, 0.48 mmol). The resulting reaction mixture was stirred at 40°C for 2 h. After the reaction was cooled to room temperature, the mixture was concentrated under reduced pressure and the residue was purified by pre-TLC (petroleum ether/ethyl acetate=5: 1) to afford tert-butyl (S)-(6-bromo-5-(2-((tert-butoxycarbonyl)amino)-3-morpholino-3-oxopropyl)thieno[3,2-c]isothiazol-3-yl)(thiophen-2-ylmethyl)carbamate (85 mg, 77%) as a white solid.

### Step 4. Synthesis of (S)-2-amino-3-(6-bromo-3-((thiophen-2-ylmethyl)amino)thieno[3,2-c]isothiazol-5-yl)-1-morpholinopropan-1-one

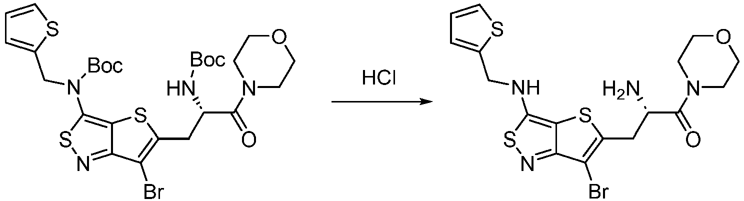

A solution of tert-butyl (S)-(6-bromo-5-(2-((tert-butoxycarbonyl)amino)-3-morpholino-3-oxopropyl)thieno[3,2-c]isothiazol-3-yl)(thiophen-2-ylmethyl)carbamate (80 mg, 1 equiv, 0.12 mmol) in 4N HCl/dioxane (1 mL) was stirred for 2 h at room temperature. The reaction was concentrated under reduced pressure and purified by Prep-HPLC with the following conditions: column, C₁₈; mobile phase, NH₃•H₂O/ACN, 10% to 60% gradient in 15 mins; detector, UV 254 nm. The fraction was concentrated under reduced pressure and dried by lyophilization to afford (S)-2-amino-3-(6-bromo-3-((thiophen-2-ylmethyl)amino)thieno[3,2-c]isothiazol-5-yl)-1-morpholinopropan-1-one (26 mg, 45%) as a white solid.

LCMS [M+H]⁺ = 487 ¹H-NMR (400 MHz, Chloroform-d) δ 7.61 (s, 1H), 6.95-6.94 (d, *J =* 2.7 Hz, 1H), 6.75-6.74 (d, *J =* 2.9 Hz, 1H), 5.40-5.38 (m, 1H), 4.60-4.59 (d, *J =* 5.4 Hz, 2H), 3.68-3.62 (m, 5H), 3.56 - 3.41 (m, 3H), 3.38-3.27 (m, 1H), 3.15-3.12 (m, 1H), 2.98-2.94 (m, 1H).

### Example 18: Synthesis of 5-[(2S)-2-amino-1-methoxypropyl]-6-bromo-N-(thiophen-2-ylmethyl)thieno[3,2-c] [1,2]thiazol-3-amine (8.1 mg, 20.08%) as a yellow solid. Compound 114

### Step 1. Synthesis of tert-butyl N-{6-bromo-5-[(2S)-2-[(tert-butoxycarbonyl)amino]-1-methoxypropyl]thieno[3,2-c][1,2]thiazol-3-yl}-N-(thiophen-2-ylmethyl)carbamate

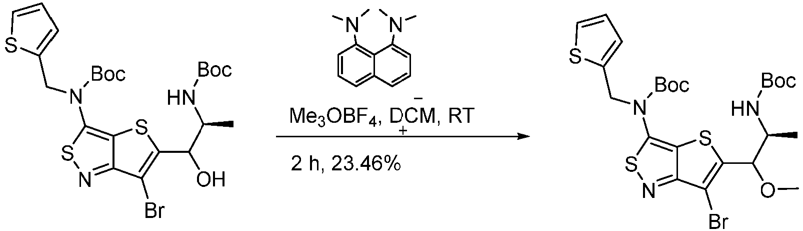

Into a 25-mL vial purged and maintained with an inert atmosphere of nitrogen, was placed tert-butyl N-{6-bromo-5-[(2R)-2-[(tert-butoxycarbonyl)amino]-1-hydroxypropyl]thieno[3,2-c][1,2]thiazol-3-yl}-N-(thiophen-2-ylmethyl)carbamate (500 mg, 0.827 mmol, 1 equiv), N1,N1,N8,N8-tetramethylnaphthalene-1,8-diamine (354.47 mg, 1.654 mmol, 2 equiv), DCM (20mL), Me₃OBF₄ (458.68 mg, 1.654 mmol, 2 equiv) . The resulting solution was stirred for 2 h at 25 degrees C. The reaction was then quenched by the addition of 2 mL of water. The resulting solution was extracted with 3x10 mL of ethyl acetate and the organic layers combined and dried over anhydrous sodium sulfate. The residue was applied onto a silica gel column with dichloromethane/methanol (10:1). This resulted in 120 mg (23.46%) of tert-butyl N-{6-bromo-5-[(2S)-2-[(tert-butoxycarbonyl)amino]-1-methoxypropyl]thieno[3,2-c][1,2]thiazol-3-yl}-N-(thiophen-2-ylmethyl)carbamate as a yellow solid.

### Step 2. Synthesis of 5-[(2S)-2-amino-1-methoxypropyl]-6-bromo-N-(thiophen-2-ylmethyl)thieno[3,2-c] [1,2]thiazol-3-amine

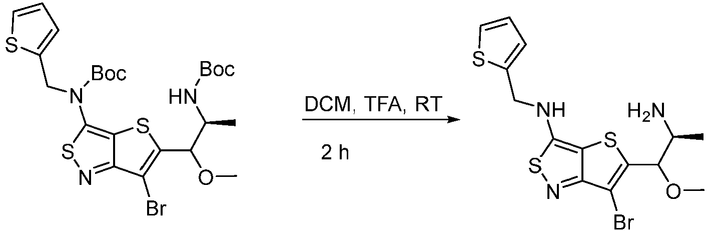

Into a 8-mL vial purged and maintained with an inert atmosphere of nitrogen, was placed tert-butyl N-{5-[(2S)-2-amino-1-methoxypropyl]-6-bromothieno[3,2-c] [1,2]thiazol-3-yl}-N-(thiophen-2-ylmethyl)carbamate (50 mg, 0.096 mmol, 1 equiv), DCM (2 mL), TFA (1 mL). The resulting solution was stirred for 2 hr at room temperature. Then concentrate the reaction solution under vacuum. The residue was applied on the HP-Flash with MeCN in Water (10mmol/L NH₄HCO₃), 10% to 100% gradient in 10 min; detector, UV 254 nm. The desired fractions were collected and concentrated under reduced pressure to remove most of the acetonitrile. Then the mixture was lyophilizied to dryness. This resulted in 5-[(2S)-2-amino-1-methoxypropyl]-6-bromo-N-(thiophen-2-ylmethyl)thieno[3,2-c][1,2]thiazol-3-amine (8.1 mg, 20.08%) .

LCMS [M+H]⁺ 417.80. ¹HNMR (300 MHz, Methanol-*d*₄) δ 7.35 (dd, *J =* 5.1, 1.2 Hz, 1H), 7.11 - 7.04 (m, 1H), 6.99 (dd, *J =* 5.1, 3.4 Hz, 1H), 4.79 (d, *J* = 6.7 Hz, 1H), 4.52 (s, 2H), 3.52 *(*d, *J* = 1.9 Hz, 3H), 3.19 (p, *J =* 6.5 Hz, 1H), 1.12 (dd, *J* = 11.7, 6.6 Hz, 3H).

### Example 19: Synthesis of tert-butyl N-{6-fluorothieno[3,2-c] [1,2]thiazol-3-yl}-N-(thiophen-2-ylmethyl)carbamate (BB-5)

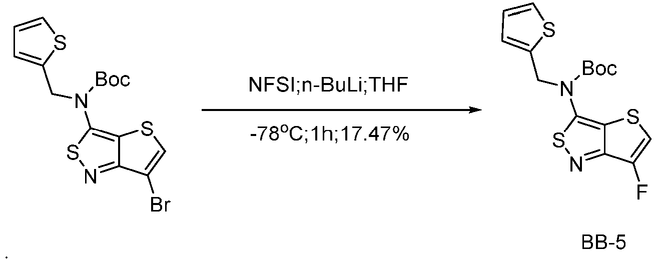

In a 50-mL round bottom flask, to a solution of tert-butyl N-{6-bromothieno[3,2-c][1,2]thiazol-3-yl}-N-(thiophen-2-ylmethyl)carbamate (1 g, 2.318 mmol, 1 equiv) in THF (20 mL) was added dropwise n-BuLi in hexanes (2.5 M in hexanes,0.66 mL, 6.954 mmol, 3 equiv) at -78 degrees C under N₂ atmosphere. The reaction mixture was stirred at -78 degrees C for 30 mins. Then a solution of NFSI (1461.96 mg, 4.636 mmol, 2 equiv) in 3 mL THF was added dropwise and the mixture was stirred for another 30 mins. The residue was purified by reversed-phase flash chromatography with the following conditions: column, C₁₈; mobile phase, MeCN in Water (10mmol/L NH₄HCO₃), 10% to 100% gradient in 20 min; detector, UV 254 nm. This resulted in tert-butyl N-{6-fluorothieno[3,2-c][1,2]thiazol-3-yl}-N-(thiophen-2-ylmethyl)carbamate (150 mg, 17.47%) as a yellow solid.

### Example 20: ATXN3 Quantitative Splicing Assay.

Human neuroblastoma SK-N-MC cells were plated in 384-well plates at 20,000 cells/well. Twenty-four hours after plating, cells were treated with compounds for 24 h at appropriate concentrations ranging from 30 µM to 0.6 nM (0.3% DMSO). Treated cells were lysed in 15 µL of lysis buffer, and cDNA was synthesized using the Fast Advanced Cells-to-Ct kit. Two µL of each cDNA was used in qPCR reactions to confirm the exon 4 skipped transcripts of ATXN3. A second set of primers/probe E4E5 was used to detect the transcripts containing exon 4. The third set of primers/probe E8E9 was used to detect total gene level of ATXN3. The qPCR reactions were prepared in 384-well plates in 10 µL volume, using TaqMan^{™} Fast Advanced Master Mix with primers and probes shown in the table below. Reactions were run in a Quant Studio 6 qPCR instrument with default settings.

The primers and probes are listed below in **Table 5.**

**Table 5.**

| **Target Sequence** | **Forward Primer** | **Probe** | **Reverse Primer** |
|---|---|---|---|
| ATXN3 E4skpping -FAM | SEQ ID NO: 2 5' GCAGCCTTCTGGAAA TATGG 3' | SEQ ID NO: 3 5' TTCTCTATTCAGAAAT GAAAGATCATT 3' | SEQ ID NO: 4 5' CTGGACCCGTCAAGA GAGAA 3' |
| ATXN3 E4E5-Cy5 | SEQ ID NO: 5 5' TGTTCAACAGTCCAG AGTATCAG 3' | SEQ ID NO: 6 5' AGGCTCAGGATCGAT CCTATAAATGAAAGA 3' | SEQ ID NO: 7 5' ACCCGTCAAGAGAGA ATTCAAG 3' |
| ATXN3 E8E9-total-FAM | SEQ ID NO: 8 5’ GATGAGGAGGATTTG CAGAGG 3’ | SEQ ID NO: 9 5' ATGTTTCTGGAACTAC CTTGCATACTTAGCTG 3' | SEQ ID NO: 10 5' CCTGATGTCTGTGTCA TATCTTGA 3' |
| TBP-YAK (endogeno us control) | SEQ ID NO: 11 5' TCGGAGAGTTCTGGG ATT 3' | SEQ ID NO: 12 5’ CCGCAGCTGCAAAAT ATTGTATCCACA 3’ | SEQ ID NO: 13 5' AAGTGCAATGGTCTTT AGGT 3' |

### Example 21: ATXN3 total protein assay.

Human neuroblastoma SK-N-MC cells were seeded at 10,000 cells/well in 384 well plates one day prior to compound treatment. The concentrations of compounds were tested at appropriate doses ranging from 30 µM to 0.6 nM. After incubation for 48 hours, the cells were lysed with 25 µL of lysis buffer containing protease inhibitors, and total ATXN3 protein levels were assessed by Mesoscale Discovery (MSD) assay developed with one pair of anti-ATXN3 antibodies. The capture and detect antibodies were raised in mouse and rabbit respectively. Anti-rabbit MSD-ST antibody was used for secondary antibody.

ATXN3 recombinant protein was used for standards. The readouts were captured with 35 µL of MSD read buffer and multi-array 384-well high binding plates.

One plate replica was carried out for parallel viability testing by CellTiter Glo^{®} 2.0 with a seeding density of 4,000 cells/well. Compounds were incubated for 48 hours. The viability readouts were carried out by Envision according to the manufacturer's instructions.

Compounds were tested as outlined in Examples 6 and 7 above and the results are shown below in **Table 6.**

**Table 6**

| * IC₅₀ range (nM): 0.01 ≤ A ≤ 100; 101 ≤ B ≤ 500; 501 ≤ C ≤ 5000; 5001 ≤ D ≤ 10000; 10001 ≤ E ≤ 40,000. * EC₅₀ range (nM): 0.01 ≤ A ≤ 100; 101 ≤ B ≤ 500: 501 ≤ C ≤ 5000; 5001 ≤ D ≤ 10000; 10001 ≤ E ≤ 40,000. | | |
|---|---|---|
| Compound Number | ATXN3 Splicing E4E5 IC50 | ATXN3 Protein IC50 |
| 1 | E | E |
| 3 | E | E |
| 4 | E | E |
| 5 | D | C |
| 6 | C | C |
| 7 | C | C |
| 8 | D | D |
| 9 | C | C |
| 10 | C | C |
| 17 | C | B |
| 18 | B | B |
| 19 | C | C |
| 20 | E | E |
| 21 | E | E |
| 23 | B | B |
| 24 | B | B |
| 25 | E | E |
| 26 | D | D |
| 27 | E | E |
| 28 | D | E |
| 30 | D | E |
| 31 | D | D |
| 35 | C | C |
| 36 | C | C |
| 37 | C | C |
| 38 | C | C |
| 39 | C | C |
| 40 | C | C |
| 41 | C | C |
| 42 | C | C |
| 43 | C | B |
| 44 | D | C |
| 45 | E | E |
| 46 | B | B |
| 47 | B | B |
| 48 | B | B |
| 49 | C | C |
| 53 | C | C |
| 54 | C | C |
| 55 | E | E |
| 57 | B | C |
| 58 | C | C |
| 59 | B | B |
| 60 | A | A |
| 61 | B | B |
| 62 | B | C |
| 63 | B | B |
| 64 | C | C |
| 65 | C | B |
| 66 | E | E |
| 67 | E | E |
| 68 | E | E |
| 69 | E | E |
| 70 | E | E |
| 71 | E | D |
| 72 | C | B |
| 73 | E | D |
| 74 | B | B |
| 75 | B | C |
| 76 | B | B |
| 79 | E | E |
| 80 | C | D |
| 81 | C | C |
| 82 | E | E |
| 84 | C | D |
| 85 | D | E |
| 87 | E | E |
| 88 | E | E |
| 89 | D | C |
| 90 | E | E |
| 93 | C | C |
| 94 | D | D |
| 95 | C | C |
| 96 | C | D |
| 97 | C | C |
| 98 | C | C |
| 99 | C | C |
| 100 | C | C |
| 101 | C | C |
| 102 | E | E |
| 103 | E | E |
| 104 | C | C |
| 105 | B | B |
| 106 | C | C |
| 110 | C | C |
| 111 | C | C |
| 113 | E | E |
| 114 | E | E |
| 128 | E | E |

## Claims

1. A compound of Formula (I): 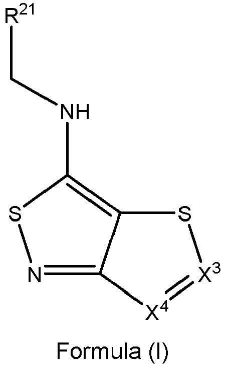
or a pharmaceutically acceptable salt thereof;
wherein,
X³ is selected from the group consisting of N and CR²³;
X⁴ is selected from the group consisting of N and CR²⁴;
R²¹ is selected from the group consisting of phenyl, 5-6 membered heteroaryl, and 5-6 membered heterocycloalkyl, each of which is unsubstituted or substituted with 1, 2, 3 or 4, independently selected R^{1A} groups; each R^{1A} is independently selected from halo, CN, NO₂, C₁₋₆ alkyl, C₂₋₆ alkenyl, C₂₋₆ alkynyl, C₁₋₆ haloalkyl, C₁₋₆ alkoxy, -C(=O)OH, -C(=O)C₁₋₆ alkyl, -C(=O)C₁₋₆ haloalkyl, and -C(=O)C₁₋₆ alkoxy;
R²³ is selected from the group consisting of hydrogen, azido, halogen, -CN, -NO₂, C₁₋₆ haloalkyl, C₁₋₆ alkyl, C₂₋₆ alkenyl, C₂₋₆ alkynyl, C₁₋₆ heteroalkyl, -(C₁₋₆ alkylene)-C₃₋₁₀ cycloalkyl, -(C₁₋₆ alkylene)-4-10 membered heterocycloalkyl, -(C₁₋₆ heteroalkylene)-C₃₋₁₀ cycloalkyl, -(C₁₋₆ heteroalkylene)-4-10 membered heterocycloalkyl, -(C₁₋₆ alkylene)-C₆₋₁₀ aryl, -(C₁₋₆ alkylene)-5-10 membered heteroaryl, -(C₁₋₆ heteroalkylene)-C₆₋₁₀ aryl, -(C₁₋₆ heteroalkylene)-5-10 membered heteroaryl, C₃₋₁₀ cycloalkyl, C₆₋₁₀ aryl, 5-10 membered heteroaryl, 4-10 membered heterocycloalkyl, -OR^{a3}, -SR^{a3}, -C(=O)R^{b3}, -C(=O)OR^{b3}, -NR^{c3}R^{d3}, C(=O)NR^{c3}R^{d3} -OC(=O)NR^{c3}R^{d3}, -NR^{C3}C(=O)R^{b3,} -NR^{C3}C(=O)OR^{b3}, -NR^{C3}C(=O)NR^{C3}R^{d3}, -NR^{c3}S(=O)₂R^{b3}, -_{NR}^{c3}S(=O)₂NR^{c3}R^{d3}, -S(O)_{NR}^{c3}R^{d3}, and -S(O)₂NR^{c3}R^{d3}, wherein the C₁₋₆ alkyl, C₁₋₆ alkylene, C₁₋₆ heteroalkylene, C₁₋₆ haloalkyl, C₂₋₆ alkenyl, C₂₋₆ alkynyl, C₁₋₆ heteroalkyl, C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
R²⁴ is selected from the group consisting of hydrogen, azido, halogen, -CN, -NO₂, C₁₋₆ haloalkyl, C₁₋₆ alkyl, C₂₋₆ alkenyl, C₁₋₆ heteroalkyl, C₃₋₁₀ cycloalkyl, C₆₋₁₀ aryl, -OR^{a4}, -SR^{a4}, -S(=O)₂R^{a4}, -C(=O)R^{b4}, -C(=O)OR^{b4}, -NR^{c4}R^{d4}, -C(=O)NR^{c4}R^{d4}, -OC(=O)NR^{c4} R^{d4}, -NR^{c4}C(=O)R^{b4}, -NR^{c4}C(=O)OR^{b4}, -NR^{c4}C(=O)NR^{c4}R^{d4}, -NR^{c4}S(=O)₂R^{b4}, -NR^{c4}S(=O)2NR^{c4}R^{d4}, -S(O)NR^{c4}R^{d4}, and -S(O)₂NR^{c4}R^{d4}, wherein the C₁₋₆ alkyl, C₁₋₆ haloalkyl, C₂₋₆ alkenyl, C₁₋₆ heteroalkyl,C₃₋₁₀ cycloalkyl, and C₆₋₁₀ aryl are unsubstituted or substituted independently with 1, 2, 3, or 4 R^{20d} groups;
each R^{a3}, R^{b3}, R^{c3}, R^{d3}, R^{a4}, R^{b4}, R^{c4} and R^{d4} is independently selected from the group consisting of hydrogen, C₁₋₆ alkyl, C₂₋₆ alkenyl, C₂₋₆ alkynyl, C₁₋₆ hydroxyalkyl, C₁₋₆ haloalkyl, C₁₋₆ alkoxy, - (C₁₋₆ alkylene)-C₁₋₆ alkoxy, C₃₋₁₀ cycloalkyl, -(C₁₋₆ alkylene)-C₃₋₁₀ cycloalkyl, C₆₋₁₀ aryl, 5-10 membered heteroaryl, 4-10 membered heterocycloalkyl, wherein the C₁₋₆ alkyl, C₂₋₆ alkenyl, C₂₋₆ alkynyl, C₃₋₁₀ cycloalkyl, -(C₁₋₆ alkylene)-C₃₋₁₀ cycloalkyl, C₆₋₁₀ aryl, 5-10 membered heteroaryl, and 4-10 membered heterocycloalkyl are unsubstituted or substituted independently with 1, 2, 3, or 4 R²⁰ groups;
or R^{c3} and R^{d3} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups;
or R^{c4} and R^{d4} together with the N atom to which they are connected, come together to form a 5-10 membered heteroaryl or 4-10 membered heterocycloalkyl, wherein the 5-10 membered heteroaryl and 4-10 membered heterocycloalkyl are unsubstituted or substituted independently with 1, 2, 3, or 4 R²⁰ groups;
each R²⁰ is independently selected from the group consisting of -OH, -SH, -CN, -NO₂, halogen, oxo, C₁₋₄ alkyl, C₂₋₄ alkenyl, C₂₋₄ alkynyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, 5-6 membered heteroaryl, 4-6 membered heterocycloalkyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino; and
each R^{20d} is independently selected from the group consisting of -OH, -SH, -CN, -NO₂, halogen, oxo, C₁₋₄ alkyl, C₂₋₄ alkenyl, C₁₋₄ haloalkyl, C₁₋₄ cyanoalkyl, C₁₋₄ hydroxyalkyl, C₁₋₄ alkoxy, -(C₁₋₄ alkyl)-(C₁₋₄ alkoxy), -(C₁₋₄ alkoxy)-(C₁₋₄ alkoxy), C₁₋₄ haloalkoxy, C₃₋₆ cycloalkyl, phenyl, amino, C₁₋₄ alkylamino, di(C₁₋₄ alkyl)amino, carbamyl, C₁₋₄ alkylcarbamyl, di(C₁₋₄ alkyl)carbamyl, carbamoyl, C₁₋₄ alkylcarbamoyl, di(C₁₋₄ alkyl)carbamoyl, C₁₋₄ alkylcarbonyl, C₁₋₄ alkoxycarbonyl, C₁₋₄ alkylcarbonylamino, C₁₋₄ alkylsulfonylamino, aminosulfonyl, C₁₋₄ alkylaminosulfonyl, di(C₁₋₄ alkyl)aminosulfonyl, aminosulfonylamino, C₁₋₄ alkylaminosulfonylamino, di(C₁₋₄ alkyl)aminosulfonylamino, aminocarbonylamino, C₁₋₄ alkylaminocarbonylamino, and di(C₁₋₄ alkyl)aminocarbonylamino.

2. The compound of claim 1, or a pharmaceutically acceptable salt thereof, wherein:
R²¹ is C₆₋₁₀ aryl or 5-6 membered heteroaryl, wherein the C₆₋₁₀ aryl and 5-6 membered heteroaryl are unsubstituted or substituted independently with 1, 2, 3, or 4 R^{1A} groups, wherein each R^{1A} is independently selected from the group consisting of halogen, -CN, -NO₂, -CF₃, -CHF₂, C₁₋₆ alkyl, C₂₋₆ alkenyl, C₂₋₆ alkynyl, C₁₋₆ haloalkyl, C₁₋₆ alkoxy, -C(=O)OH, -C(=O)C₁₋₆ alkyl, -C(=O)C₁₋₆ haloalkyl, and -C(=O)C₁₋₆ alkoxy.

3. The compound of claim 2, or a pharmaceutically acceptable salt thereof, wherein:
R²¹ is selected from the group consisting of

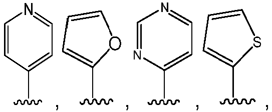

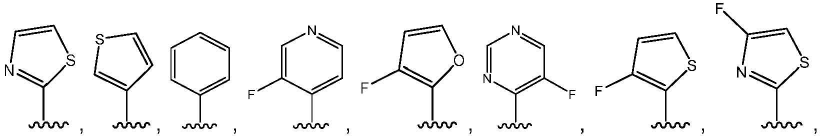

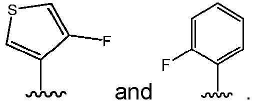

4. The compound of any one of claims 1-3, or a pharmaceutically acceptable salt thereof, wherein:
X³ is CH.

5. The compound of any one of claims 1-3, or a pharmaceutically acceptable salt thereof, wherein:
X³ is CR²³.

6. The compound of claim 5, or a pharmaceutically acceptable salt thereof, wherein:
R²³ is C₁₋₆ alkyl or C₁₋₆ heteroalkyl, wherein the C₁₋₆ alkyl and C₁₋₆ heteroalkyl are unsubstituted or substituted independently with 1, 2, 3, or 4 R²⁰ groups.

7. The compound of claim 6, or a pharmaceutically acceptable salt thereof, wherein:
X³ is CCH₂CH(NH₂)CH₃;
X³ is CCH₂CHNH₂CH₂OH;
X³ is CCH₂CH(NH₂)CH₂CH₃;
X³ is CCH₂CH(NH₂)CH₂CH₂OH;
X³ is CCH₂CH(NH₂)CH₂CH₂F;
X³ is CCH₂CH(NH₂)CH₂CHF₂; or
X³ is CCH₂CH(NH₂)CH₂CH(CH₃)₂.

8. The compound of any one of claims 1-7, or a pharmaceutically acceptable salt thereof, wherein:
X⁴ is N; or
X⁴ is CH.

9. The compound of any one of claims 1-7, or a pharmaceutically acceptable salt thereof, wherein:
X⁴ is CR²⁴.

10. The compound of claim 9, wherein:
R²⁴ is selected from the group consisting of halogen, -CN, and C₁₋₆ alkyl, wherein C₁₋₆ alkyl is unsubstituted or substituted independently with 1, 2, 3, or 4 R^{20d} groups.

11. The compound of any one of claims 1-7, or a pharmaceutically acceptable salt thereof, wherein:
X⁴ is CCI;
X⁴ is CBr;
X⁴ is CF;
X⁴ is CCN;
X⁴ is CCH₃; or
X⁴ is C(cyclopropyl).

12. The compound of claim 1, or a pharmaceutically acceptable salt thereof, which is a compound selected from the following compounds, or a pharmaceutically acceptable salt thereof:
| | |
|---|---|
| 1 | 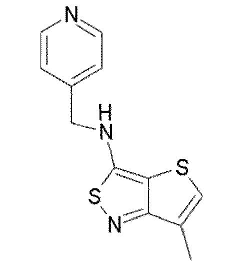 |
| 2 | 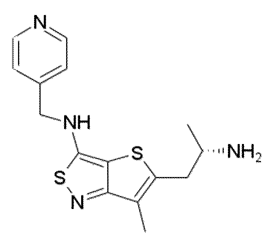 |
| 3 | 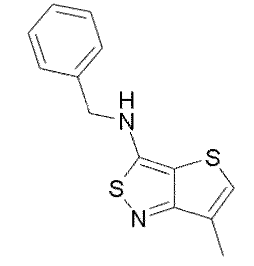 |
| 4 | 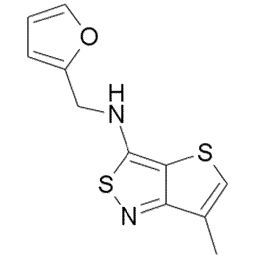 |
| 5 | 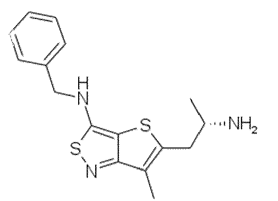 |
| 6 | 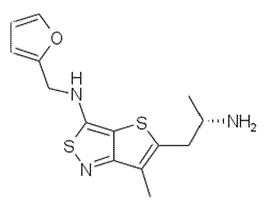 |
| 7 | 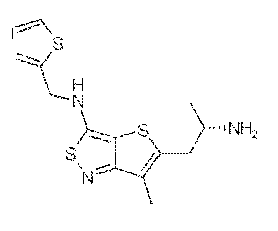 |
| 8 | 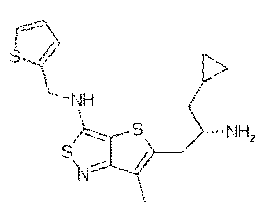 |
| 9 | 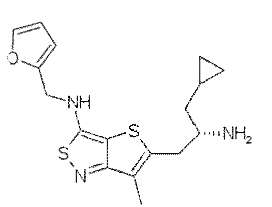 |
| 10 | 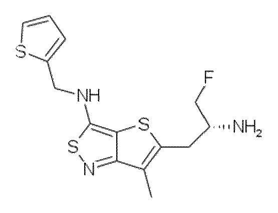 |
| 11 | 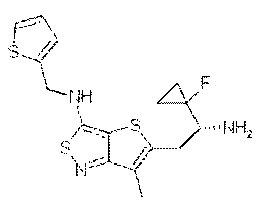 |
| 12 | 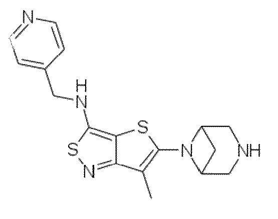 |
| 13 | 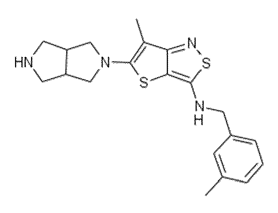 |
| 14 | 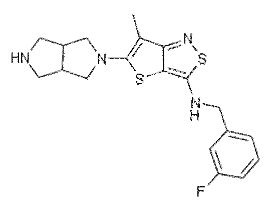 |
| 15 | 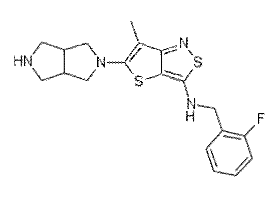 |
| 16 | 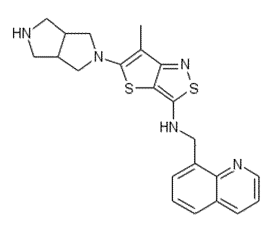 |
| 17 | 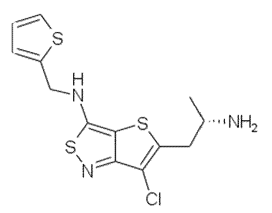 |
| 18 | 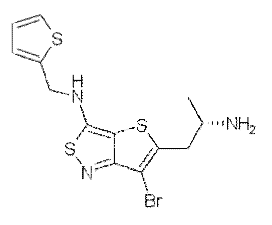 |
| 19 | 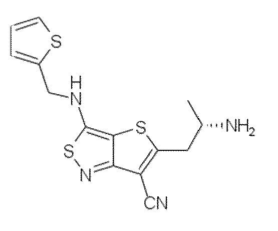 |
| 20 | 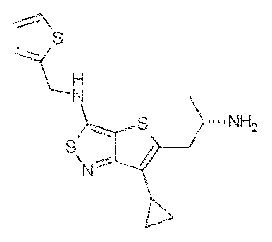 |
| 21 | 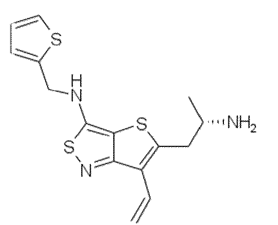 |
| 22 | 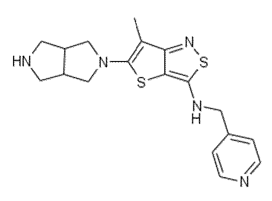 |
| 23 | 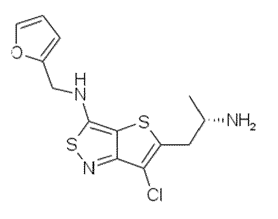 |
| 24 | 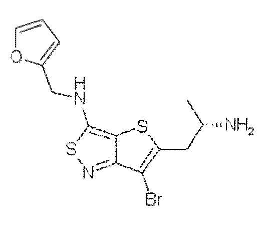 |
| 25 | 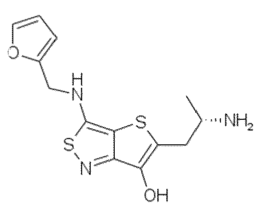 |
| 26 | 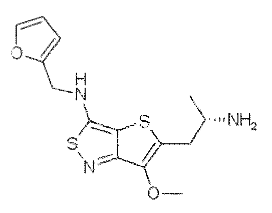 |
| 27 | 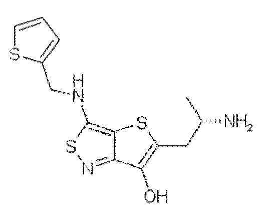 |
| 28 | 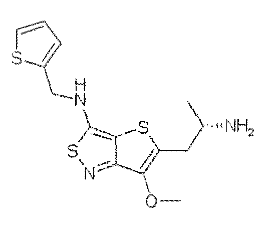 |
| 29 | 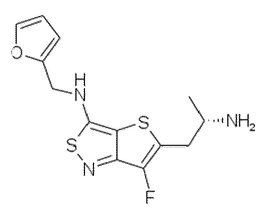 |
| 30 | 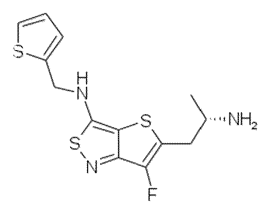 |
| 31 | 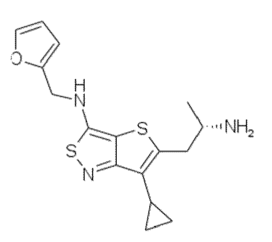 |
| 32 | 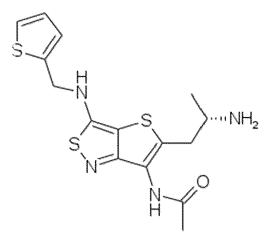 |
| 33 | 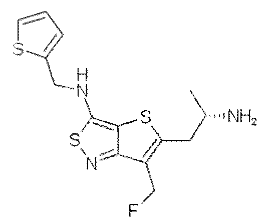 |
| 34 | 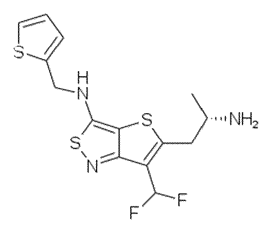 |
| 35 | 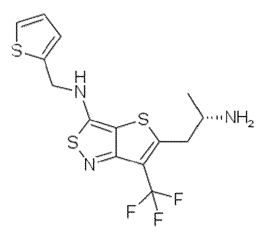 |
| 36 | 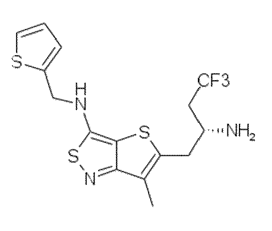 |
| 37 | 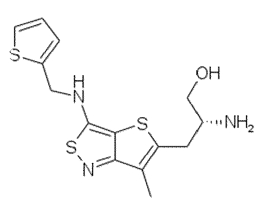 |
| 38 | 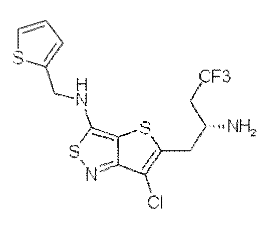 |
| 39 | 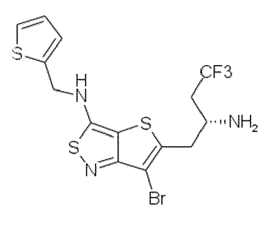 |
| 40 | 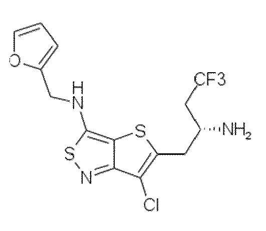 |
| 41 | 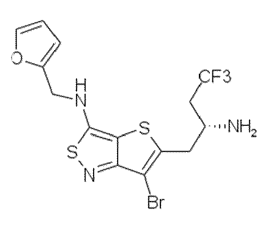 |
| 42 | 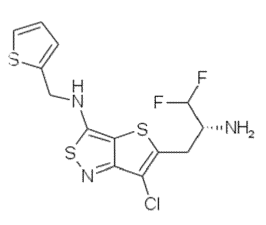 |
| 43 | 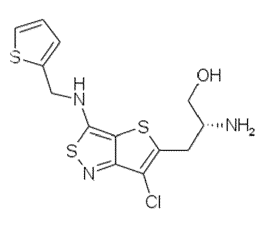 |
| 44 | 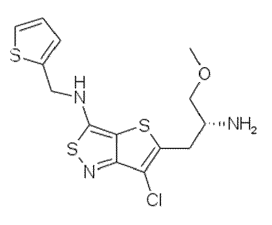 |
| 45 | 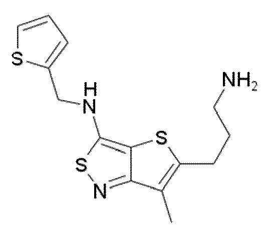 |
| 46 | 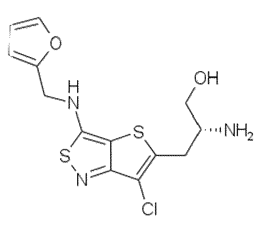 |
| 47 | 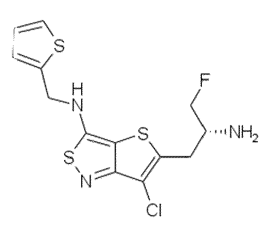 |
| 48 | 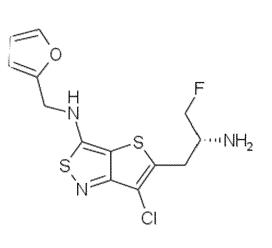 |
| 49 | 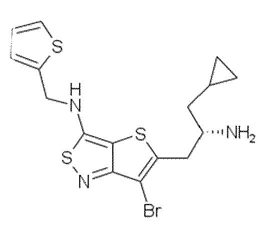 |
| 50 | 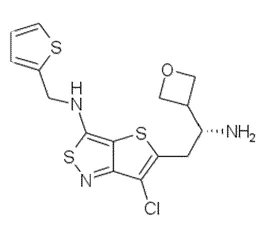 |
| 51 | 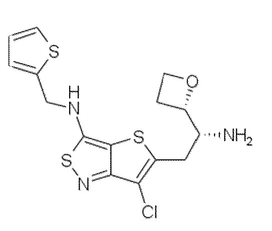 |
| 52 | 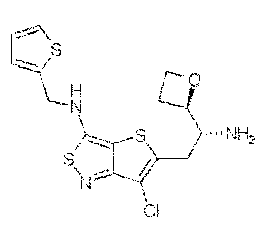 |
| 53 | 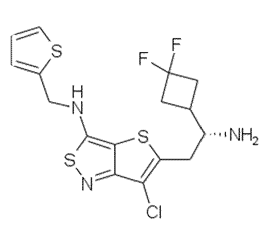 |
| 54 | 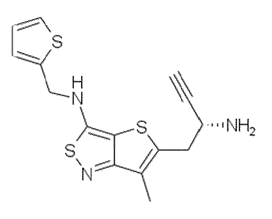 |
| 55 | 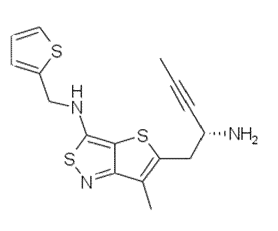 |
| 56 | 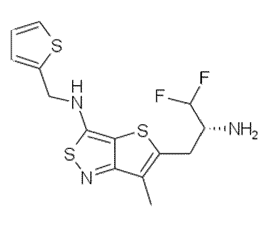 |
| 57 | 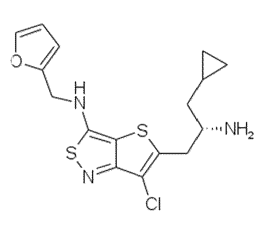 |
| 58 | 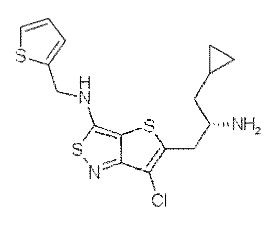 |
| 59 | 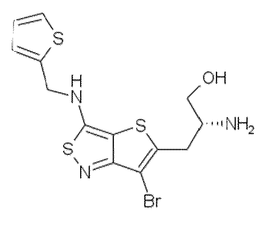 |
| 60 | 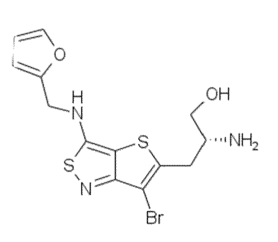 |
| 61 | 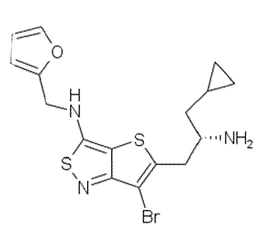 |
| 62 | 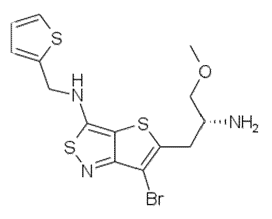 |
| 63 | 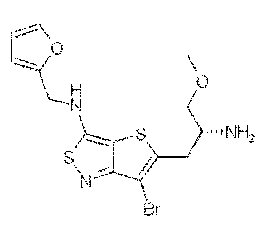 |
| 64 | 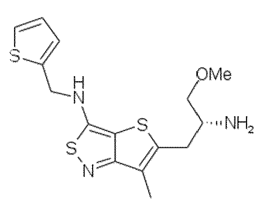 |
| 65 | 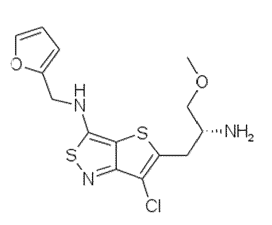 |
| 66 | 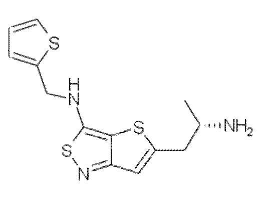 |
| 67 | 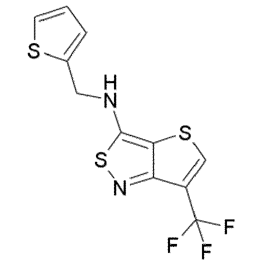 |
| 68 | 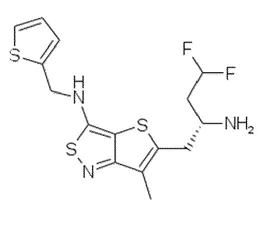 |
| 69 | 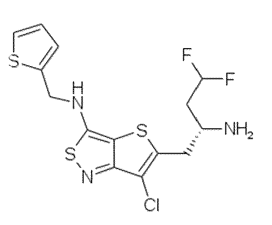 |
| 70 | 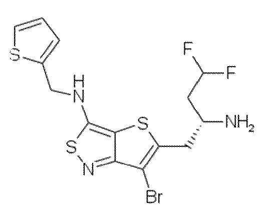 |
| 71 | 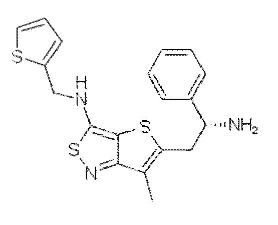 |
| 72 | 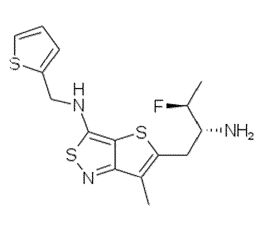 |
| 73 | 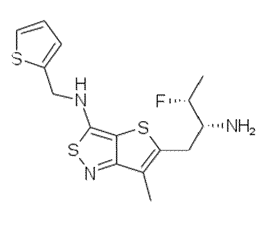 |
| 74 | 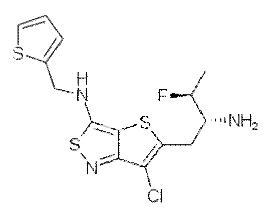 |
| 75 | 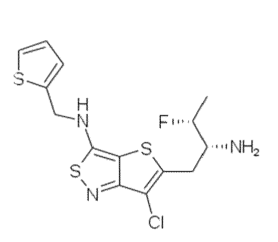 |
| 76 | 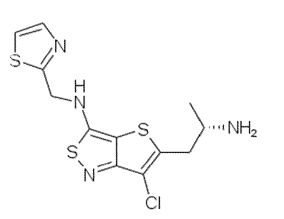 |
| 79 | 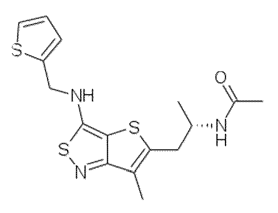 |
| 80 | 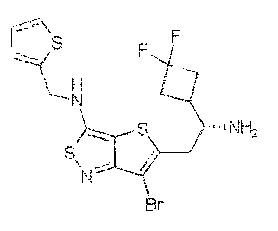 |
| 81 | 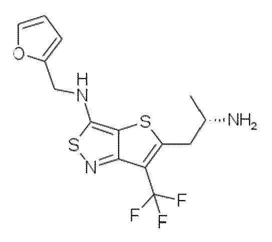 |
| 82 | 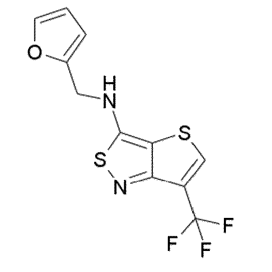 |
| 83 | 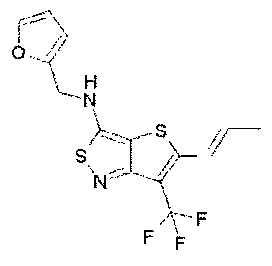 |
| 84 | 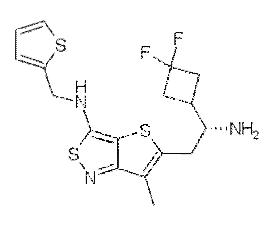 |
| 85 | 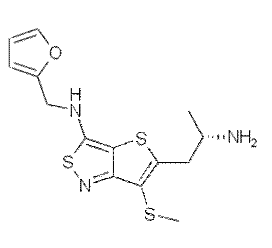 |
| 86 | 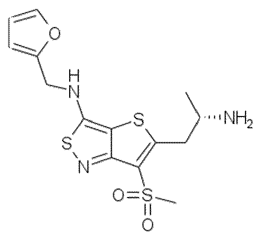 |
| 87 | 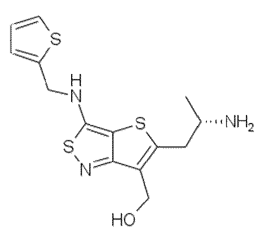 |
| 88 | 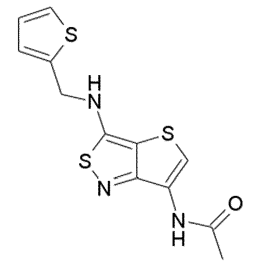 |
| 89 | 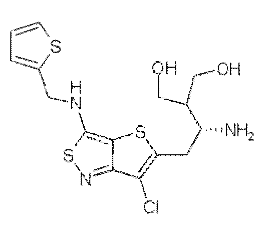 |
| 90 | 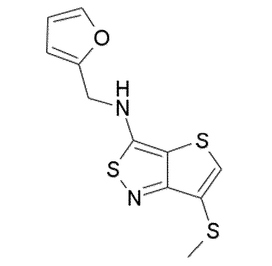 |
| 91 | 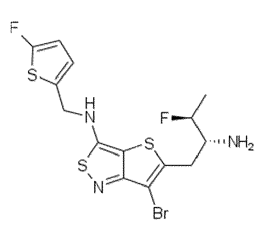 |
| 92 | 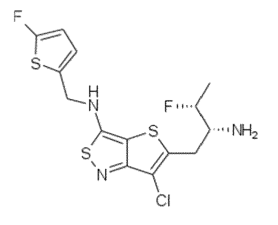 |
| 93 | 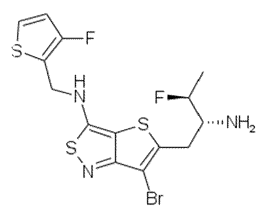 |
| 94 | 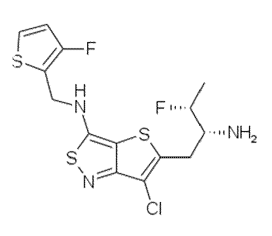 |
| 95 | 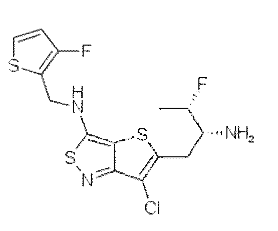 |
| 96 | 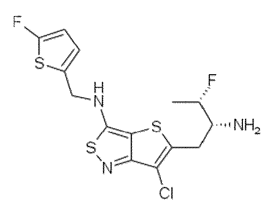 |
| 97 | 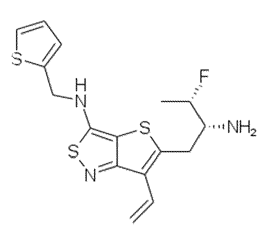 |
| 98 | 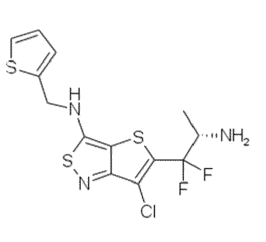 |
| 99 | 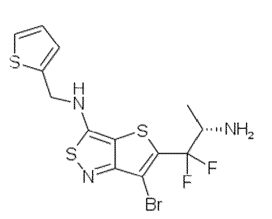 |
| 100 | 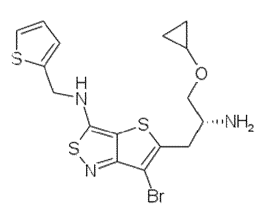 |
| 101 | 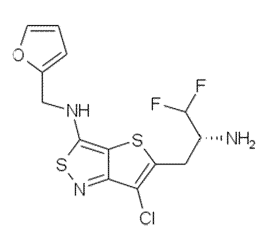 |
| 102 | 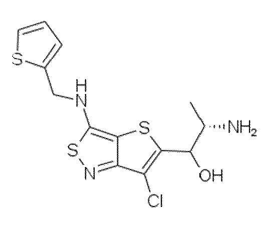 |
| 103 | 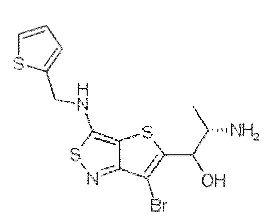 |
| 104 | 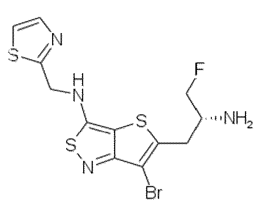 |
| 105 | 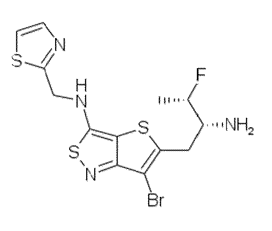 |
| 106 | 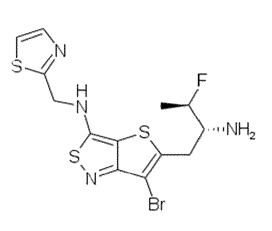 |
| 107 | 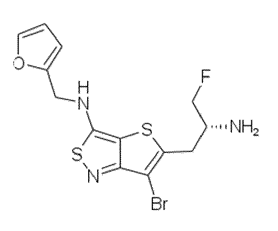 |
| 108 | 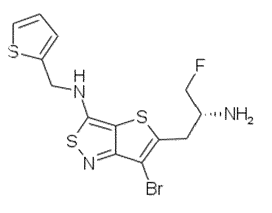 |
| 109 | 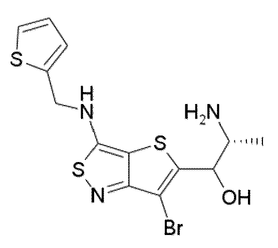 |
| 110 | 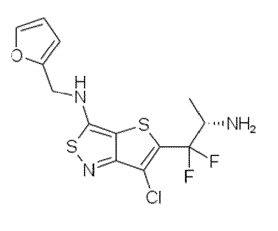 |
| 111 | 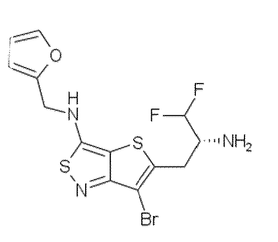 |
| 112 | 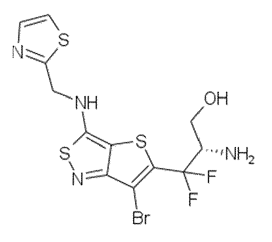 |
| 113 | 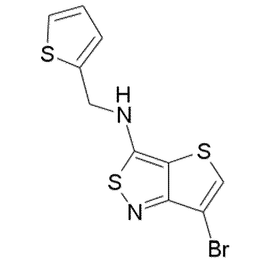 |
| 114 | 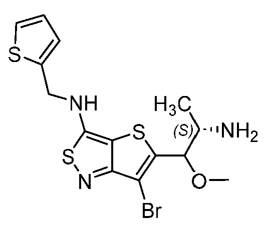 |
| 115 | 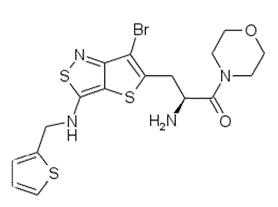 |
| 116 | 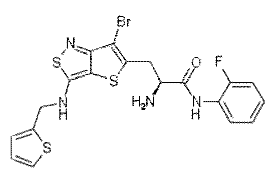 |
| 117 | 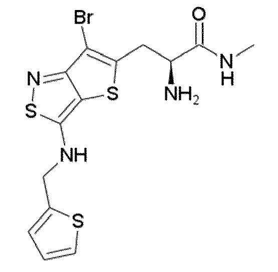 |
| 118 | 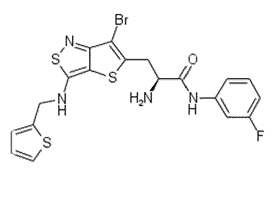 |
| 119 | 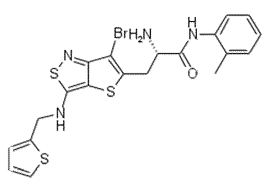 |
| 120 | 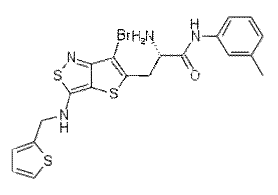 |
| 121 | 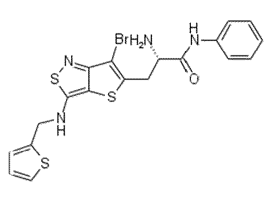 |
| 122 | 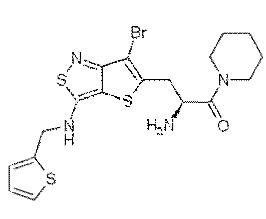 |
| 123 | 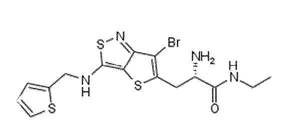 |
| 124 | 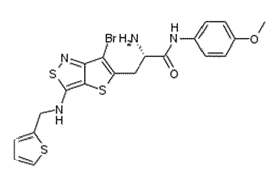 |
| 125 | 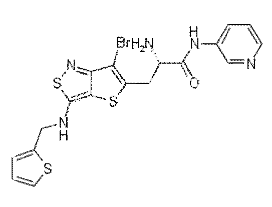 |
| 126 | 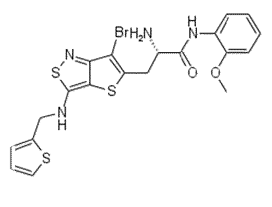 |
| 127 | 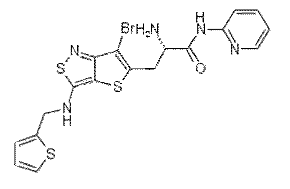 |
| 128 | 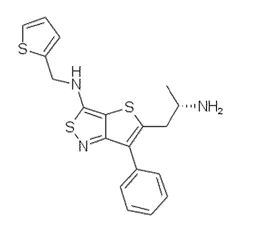 |
| 129 | 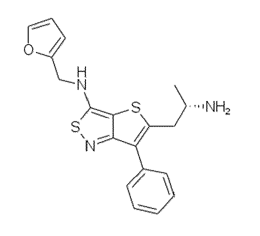 |

13. A pharmaceutical composition comprising:
a compound of any one of the preceding claims, or a pharmaceutically acceptable salt thereof, and
at least one pharmaceutically acceptable excipient or carrier.

## Patentansprüche

1. Verbindung der Formel (I): 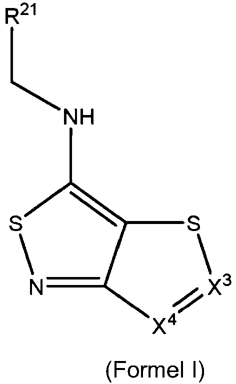
oder ein pharmazeutisch annehmbares Salz davon;
worin
X³ aus der aus N und CR²³ bestehenden Gruppe ausgewählt ist;
X⁴ aus der aus N und CR²⁴ bestehenden Gruppe ausgewählt ist;
R²¹ aus der aus Phenyl, 5- bis 6-gliedrigem Heteroaryl und 5- bis 6-gliedrigem Heterocycloalkyl bestehenden Gruppe ausgewählt ist, die jeweils unsubstituiert oder mit 1, 2, 3 oder 4 jeweils unabhängig ausgewählten Gruppen R^{1A} substituiert sind, wobei die R^{1A} jeweils unabhängig aus Halogen, CN, NO₂, C₁₋₆-Alkyl, C₂₋₆-Alkenyl, C₂₋₆-Alkinyl, C₁₋₆-Halogenalkyl, C₁₋₆-Alkoxy, -C(=O)OH, -C(=O)C₁₋₆-Alkyl, -C(=O)C₁₋₆-Halogenalkyl und -C(=O)C₁₋₆-Alkoxy ausgewählt sind;
R²³ aus der aus Wasserstoff, Azido, Halogen, -CN, -NO₂, C₁₋₆-Halogenalkyl, C₁₋₆-Alkyl, C₂₋₆-Alkenyl, C₂₋₆-Alkinyl, C₁₋₆-Heteroalkyl, -(C₁₋₆-Alkylen)-C₃₋₁₀-cycloalkyl, -(C₁₋₆-Alkylen)-4- bis 10-gliedriges Heterocycloalkyl, -(C₁₋₆-Heteroalkylen)-C₃₋₁₀-cycloalkyl, -(C₁₋₆-Heteroalkylen)-4- bis 10-gliedriges Heterocycloalkyl, -(C₁₋₆-Alkylen)-C₆₋₁₀-aryl, -(C₁₋₆-Alkylen)-5- bis 10-gliedriges Heteroaryl, -(C₁₋₆-Heteroalkylen)-C₆₋₁₀-aryl, -(C₁₋₆-Heteroalkylen)-5- bis 10-gliedriges Heteroaryl, C₃₋₁₀-Cycloalkyl, C₆₋₁₀-Aryl, 5- bis 10-gliedrigem Heteroaryl, 4- bis 10-gliedrigem Heterocycloalkyl, -OR^{a3}, -SR^{a3}, -C(=O)R^{b3}, -C(=O)OR^{b3}, -NR^{c3}R^{d3,} -C(=O)-NR^{c3}R^{d3}, -OC(=O)NR^{c3} R^{d3}, -NR^{c3}C(=O)R^{b3}, -NR^{c3}C(=O)OR^{b3}, -NR^{c3}C(=O)NR^{c3}R^{d3}, -NR^{c3}-S(=O)₂R^{b3}, -NR^{c3}S(=O)₂NR^{c3}R^{d3}, -S(O)NR^{c3}R^{d3} und -S(O)₂NR^{c3}R^{d3} bestehenden Gruppe ausgewählt ist, wobei das C₁₋₆-Alkyl, C₁₋₆-Alkylen, C₁₋₆-Heteroalkylen, C₁₋₆-Halogenalkyl, C₂₋₆-Alkenyl, C₂₋₆-Alkinyl, C₁₋₆-Heteroalkyl, C₃₋₁₀-Cycloalkyl, C₆₋₁₀-Aryl, 5- bis 10-gliedrige Heteroaryl und 4- bis 10-gliedrige Heterocycloalkyl unsubstituiert oder jeweils unabhängig mit 1, 2, 3 oder 4 Gruppen R²⁰ substituiert sind;
R²¹ aus der aus Wasserstoff, Azido, Halogen, -CN, -NO₂, C₁₋₆-Halogenalkyl, C₁₋₆-Alkyl, C₂₋₆-Alkenyl, C₁₋₆-Heteroalkyl, C₃₋₁₀-Cycloalkyl, C₆₋₁₀-Aryl, -OR^{a4}, -SR^{a4}, -S(=O)₂R^{a4}, -C(=O)R^{b4}, -C(=O)OR^{b4}, -NR^{c4}R^{d4}, -C(=O)NR^{c4}R^{d4}, -OC(=O)NR^{c4}R^{d4}, -NR^{C4}C(=O)R^{b4}, -NR^{c4}C(=O)OR^{b4}, -NR^{c4}C(=O)NR^{c4}R^{d4}, -NR^{c4}S(=O)₂R^{b4}, -NR^{c4}S(=O)₂NR^{c4}R^{d4}, -S(O)NR^{c4}R^{d4} und -S(O)₂NR^{c4}R^{d4} bestehenden Gruppe ausgewählt ist, wobei das C₁₋₆-Alkyl, C₁₋₆-Halogenalkyl, C₂₋₆-Alkenyl, C₁₋₆-Heteroalkyl, C₃₋₁₀-Cycloalkyl und C₆₋₁₀-Aryl unsubstituiert oder jeweils unabhängig mit 1, 2, 3 oder 4 Gruppen R^{20d} substituiert sind;
die R^{a3}, R^{b3}, R^{c3}, R^{d3}, R^{a4}, R^{b4}, R^{c4} und R^{d4} jeweils unabhängig aus der aus Wasserstoff, C₁₋₆-Alkyl, C₂₋₆-Alkenyl, C₂₋₆-Alkinyl, C₁₋₆-Hydroxyalkyl, C₁₋₆-Halogenalkyl, C₁₋₆-Alkoxy, -(C₁₋₆-Alkylen)-C₁₋₆-alkoxy, C₃₋₁₀-Cycloalkyl, -(C₁₋₆-Alkylen)-C₃₋₁₀-cycloalkyl, C₆₋₁₀-Aryl, 5- bis 10-gliedrigem Heteroaryl und 4- bis 10-gliedrigem Heterocycloalkyl bestehenden Gruppe ausgewählt sind, wobei das C₁₋₆-Alkyl, C₂₋₆-Alkenyl, C₂₋₆-Alkinyl, C₃₋₁₀-Cycloalkyl, -(C₁₋₆-Alkylen)-C₃₋₁₀-cycloalkyl, C₆₋₁₀-Aryl, 5- bis 10-gliedrige Heteroaryl und 4- bis 10-gliedrige Heterocycloalkyl unsubstituiert oder jeweils unabhängig mit 1, 2, 3 oder 4 Gruppen R²⁰ substituiert sind;
oder R^{c3} und R^{d3} zusammen mit dem N-Atom, an das sie gebunden sind, ein 5- bis 10-gliedriges Heteroaryl oder 4- bis 10-gliedriges Heterocycloalkyl bilden, wobei das 5- bis 10-gliedrige Heteroaryl und 4- bis 10-gliedrige Heterocycloalkyl unsubstituiert oder jeweils unabhängig mit 1, 2, 3 oder 4 R²⁰-Gruppen substituiert sind;
oder R^{c4} und R^{d4} zusammen mit dem N-Atom, an das sie gebunden sind, ein 5- bis 10-gliedriges Heteroaryl oder 4- bis 10-gliedriges Heterocycloalkyl bilden, wobei das 5- bis 10-gliedrige Heteroaryl und 4- bis 10-gliedrige Heterocycloalkyl unsubstituiert oder jeweils unabhängig mit 1, 2, 3 oder 4 Gruppen R²⁰ substituiert sind;
die R²⁰ jeweils unabhängig aus der aus -OH, -SH, -CN, -NO₂, Halogen, Oxo, C₁₋₄-Alkyl, C₂₋₄-Alkenyl, C₂₋₄-Alkinyl, C₁₋₄-Halogenalkyl, C₁₋₄-Cyanoalkyl, C₁₋₄-Hydroxyalkyl, C₁₋₄-Alkoxy, -(C₁₋₄-Alkyl)-(C₁₋₄-alkoxy), -(C₁₋₄-Alkoxy)-(C₁₋₄-alkoxy), C₁₋₄-Halogenalkoxy, C₃₋₆-Cycloalkyl, Phenyl, 5- bis 6-gliedrigem Heteroaryl, 4- bis 6-gliedrigem Heterocycloalkyl, Amino, C₁₋₄-Alkylamino, Di(C₁₋₄-alkyl)amino, Carbamyl, C₁₋₄-Alkylcarbamyl, Di(C₁₋₄-alkyl)-carbamyl, Carbamoyl, C₁₋₄-Aalkylcarbamoyl, Di(C₁₋₄-alkyl)carbamoyl, C₁₋₄-Alkylcarbonyl, C₁₋₄-Alkoxycarbonyl, C₁₋₄-Alkylcarbonylamino, C₁₋₄-Alkylsulfonylamino, Aminosulfonyl, C₁₋₄-Alkylaminosulfonyl, Di(C₁₋₄-alkyl)aminosulfonyl, Aminosulfonylamino, C₁₋₄-Alkylaminosulfonyl-amino, Di(C₁₋₄-alkyl)aminosulfonylamino, Aminocarbonylamino, C₁₋₄-Alkylaminocarbonyl-amino und Di(C₁₋₄-alkyl)aminocarbonylamino bestehenden Gruppe ausgewählt sind; und
die R^{20d} jeweils unabhängig aus der aus -OH, -SH, -CN, -NO₂, Halogen, Oxo, C₁₋₄-Alkyl, C₂₋₄-Alkenyl, C₁₋₄-Halogenalkyl, C₁₋₄-Cyanoalkyl, C₁.₄-Hydroxyalkyl, C₁₋₄-Alkoxy, -(C₁₋₄-Alkyl)-(C₁₋₄-alkoxy), -(C₁₋₄-Alkoxy)-(C₁₋₄-alkoxy), C₁₋₄-Halogenalkoxy, C₃₋₆-Cycloalkyl, Phenyl, Amino, C₁₋₄-Alkylamino, Di(C₁₋₄-alkyl)amino, Carbamyl, C₁₋₄-Alkylcarbamyl, Di(C₁₋₄-alkyl)-carbamyl, Carbamoyl, C₁₋₄-Aalkylcarbamoyl, Di(C₁₋₄-alkyl)carbamoyl, C₁₋₄-Alkylcarbonyl, C₁₋₄-Alkoxycarbonyl, C₁₋₄-Alkylcarbonylamino, C₁₋₄-Alkylsulfonylamino, Aminosulfonyl, C₁₋₄-Alkylaminosulfonyl, Di(C₁₋₄-alkyl)aminosulfonyl, Aminosulfonylamino, C₁₋₄-Alkylaminosulfonyl-amino, Oi(C₁₋₄-alkyl)aminosulfonylamino, Aminocarbonylamino, C₁₋₄-Alkylaminocarbonyl-amino und Di(C₁₋₄-alkyl)aminocarbonylamino bestehenden Gruppe ausgewählt sind.

2. Verbindung nach Anspruch 1 oder pharmazeutisch annehmbares Salz davon, worin:
R²¹ C₆₋₁₀-Aryl oder 5- bis 6-gliedriges Heteroaryl ist, wobei das C₆₋₁₀-Aryl und 5- bis 6-gliedrige Heteroaryl unsubstituiert oder jeweils unabhängig mit 1, 2, 3 oder 4 Gruppen R^{1A} substituiert sind, wobei die R^{1A} jeweils unabhängig aus der aus Halogen, -CN, -NO₂, -CF₃, -CHF₂, C₁₋₆-Alkyl, C₂₋₆-Alkenyl, C₂₋₆-Alkinyl, C₁₋₆-Halogenalkyl, C₁₋₆-Alkoxy, -C(=O)OH, -C(=O)C₁₋₆-Alkyl, -C(=O)C₁₋₆-Halogenalkyl und -C(=O)C₁₋₆-Alkoxy bestehenden Gruppe ausgewählt sind.

3. Verbindung nach Anspruch 2 oder pharmazeutisch annehmbares Salz davon, worin
R²¹ aus der aus 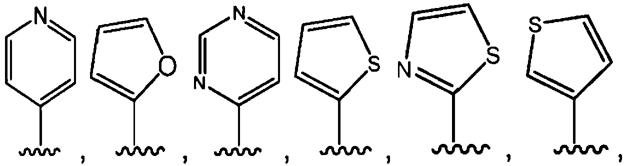 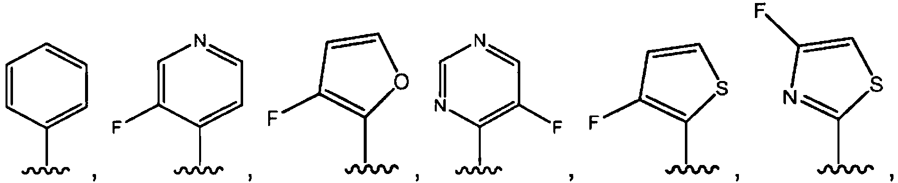 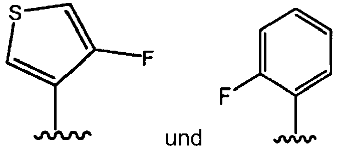 bestehenden Gruppe ausgewählt ist.

4. Verbindung nach einem der Ansprüche 1 bis 3 oder ein pharmazeutisch annehmbares Salz davon, worin:
X³ CH ist.

5. Verbindung nach einem der Ansprüche 1 bis 3 oder ein pharmazeutisch annehmbares Salz davon, worin:
X³ CR²³ ist.

6. Verbindung nach Anspruch 5 oder ein pharmazeutisch annehmbares Salz davon, worin:
R²³ C₁₋₆-Alkyl oder C₁₋₆-Heteroalkyl ist, wobei das C₁₋₆-Alkyl und C₁₋₆-Heteroalkyl unsubstituiert oder jeweils unabhängig mit 1, 2, 3 oder 4 Gruppen R²⁰ substituiert sind.

7. Verbindung nach Anspruch 6 oder pharmazeutisch annehmbares Salz davon, worin:
X³ CCH₂CH(NH₂)CH₃ ist;
X³ CCH₂CHNH₂CH₂OH ist;
X³ CCH₂CH(NH₂)CH₂CH₃ ist;
X³ CCH₂CH(NH₂)CH₂CH₂OH ist;
X³ CCH₂CH(NH₂)CH₂CH₂F ist;
X³ CCH₂CH(NH₂)CH₂CHF₂ ist; oder
X³ CCH₂CH(NH₂)CH₂CH(CH₃)₂ ist.

8. Verbindung nach einem der Ansprüche 1 bis 7 oder ein pharmazeutisch annehmbares Salz davon, worin:
X⁴ N ist; oder
X⁴ CH ist.

9. Verbindung nach einem der Ansprüche 1 bis 7 oder ein pharmazeutisch annehmbares Salz davon, worin:
X⁴ CR²⁴ ist.

10. Verbindung nach Anspruch 9, worin:
R²⁴ aus der aus Halogen, -CN und C₁₋₆-Alkyl bestehenden Gruppe ausgewählt ist, wobei das C₁₋₆-Alkyl unsubstituiert oder jeweils unabhängig mit 1, 2, 3, oder 4 Gruppen R^{20d} substituiert ist.

11. Verbindung nach einem der Ansprüche 1 bis 7 oder ein pharmazeutisch annehmbares Salz davon, worin:
X⁴ CCl ist;
X ⁴ CBr ist;
X⁴ CF ist;
X⁴ CCN ist;
X⁴ CCH₃ ist; oder
X⁴ C(Cyclopropyl) ist.

12. Verbindung nach Anspruch 1 oder pharmazeutisch annehmbares Salz davon, bei der es sich um eine Verbindung, die aus folgenden Verbindungen ausgewählt ist, oder ein pharmazeutisch annehmbares Salz davon handelt:
| | |
|---|---|
| 1 | 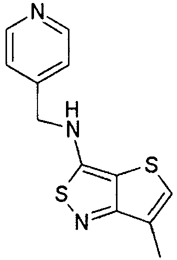 |
| 2 | 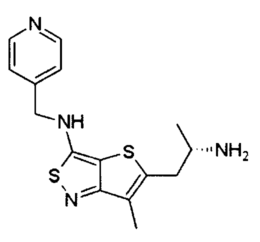 |
| 3 | 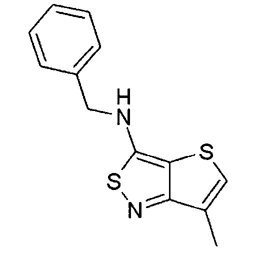 |
| 4 | 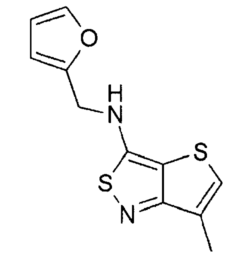 |
| 5 | 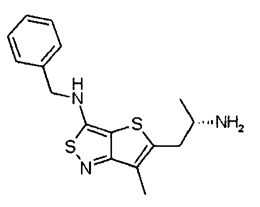 |
| 6 | 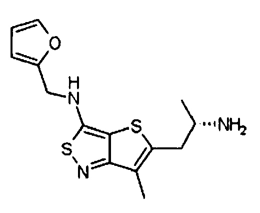 |
| 7 | 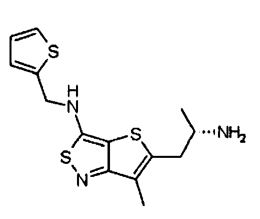 |
| 8 | 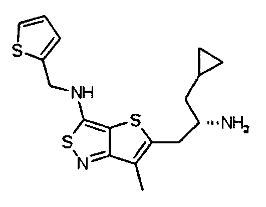 |
| 9 | 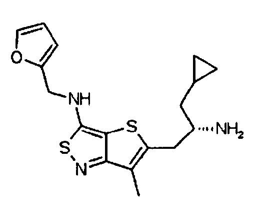 |
| 10 | 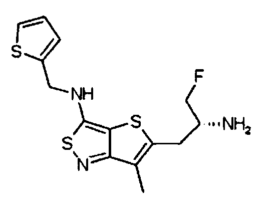 |
| 11 | 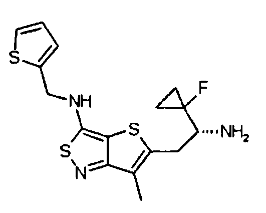 |
| 12 | 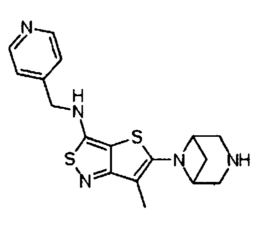 |
| 13 | 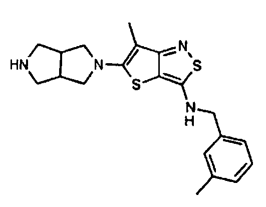 |
| 14 | 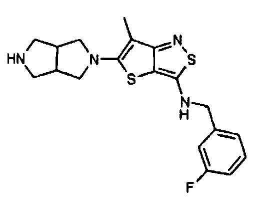 |
| 15 | 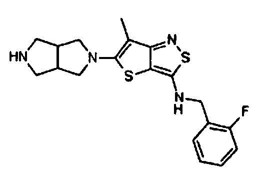 |
| 16 | 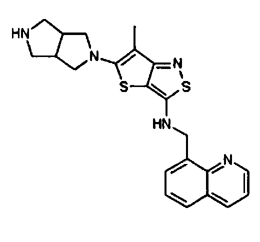 |
| 17 | 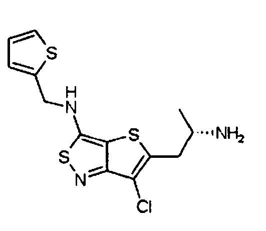 |
| 18 | 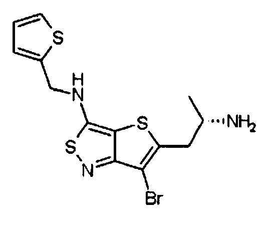 |
| 19 | 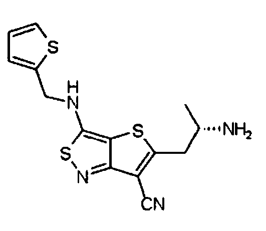 |
| 20 | 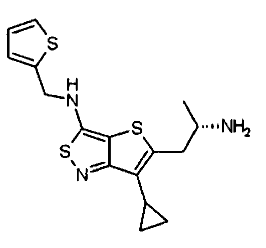 |
| 21 | 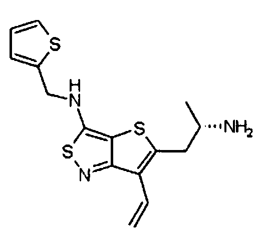 |
| 22 | 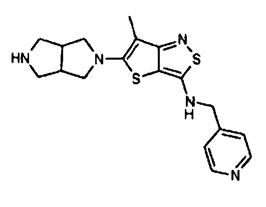 |
| 23 | 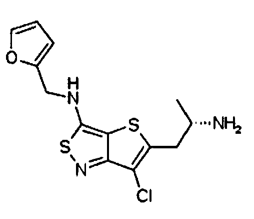 |
| 24 | 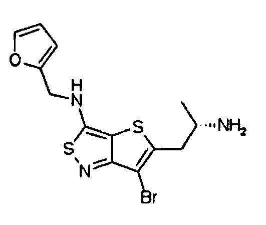 |
| 25 | 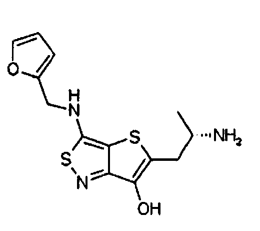 |
| 26 | 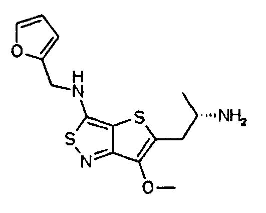 |
| 27 | 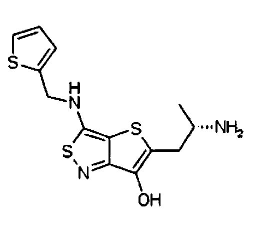 |
| 28 | 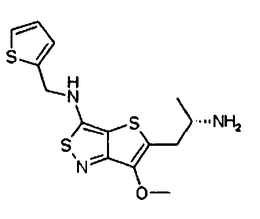 |
| 29 | 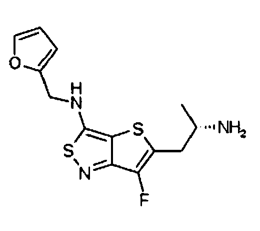 |
| 30 | 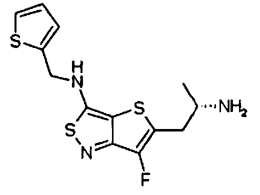 |
| 31 | 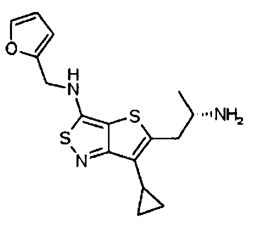 |
| 32 | 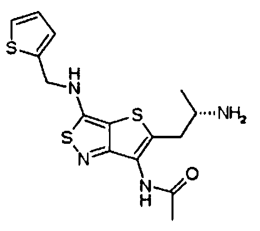 |
| 33 | 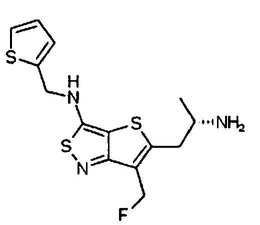 |
| 34 | 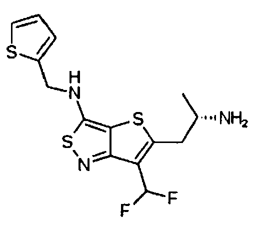 |
| 35 | 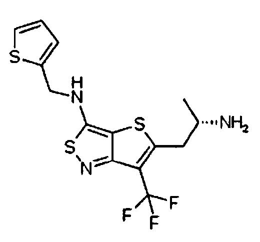 |
| 36 | 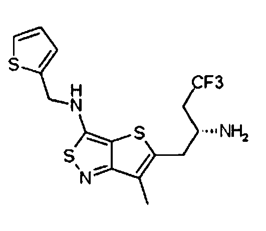 |
| 37 | 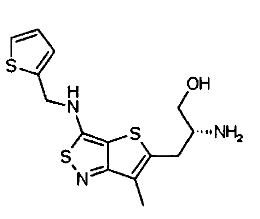 |
| 38 | 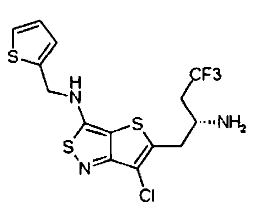 |
| 39 | 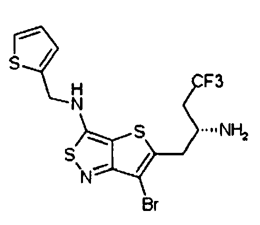 |
| 40 | 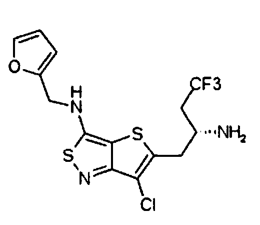 |
| 41 | 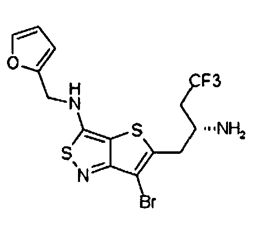 |
| 42 | 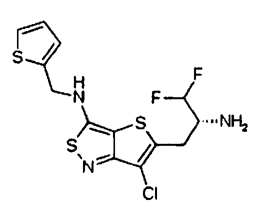 |
| 43 | 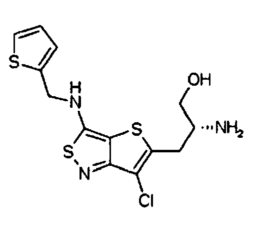 |
| 44 | 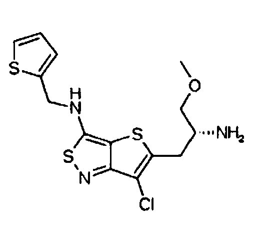 |
| 45 | 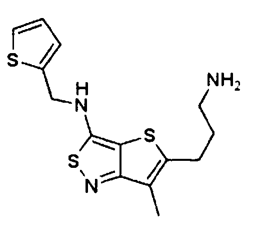 |
| 46 | 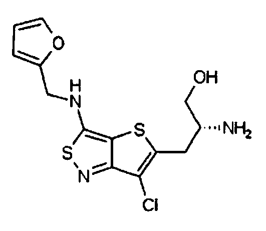 |
| 47 | 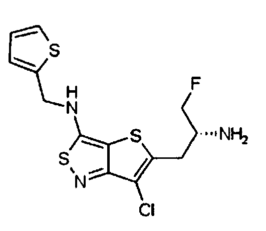 |
| 48 | 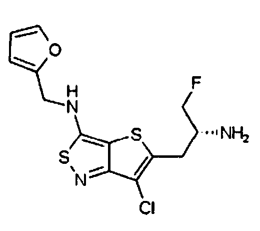 |
| 49 | 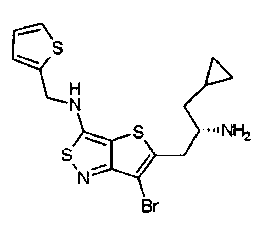 |
| 50 | 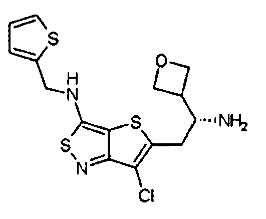 |
| 51 | 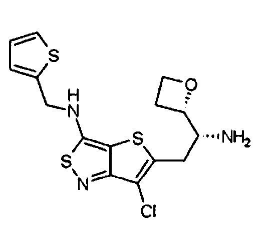 |
| 52 | 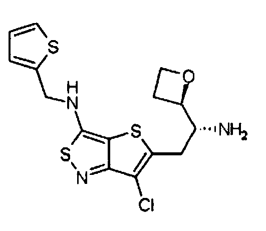 |
| 53 | 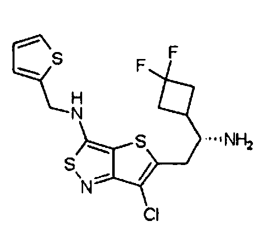 |
| 54 | 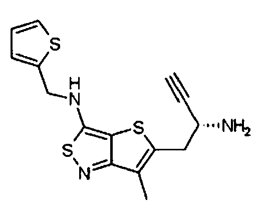 |
| 55 | 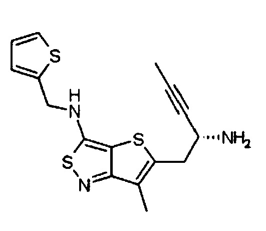 |
| 56 | 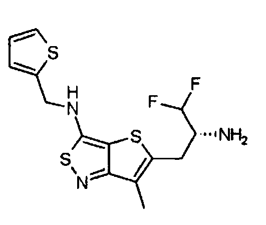 |
| 57 | 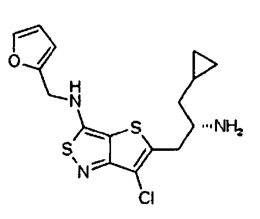 |
| 58 | 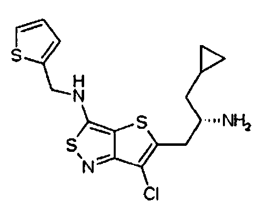 |
| 59 | 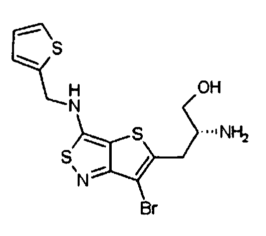 |
| 60 | 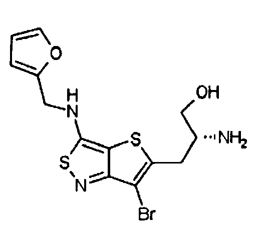 |
| 61 | 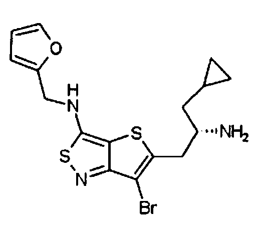 |
| 62 | 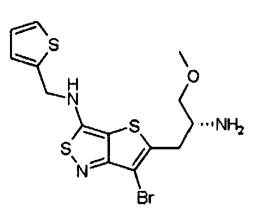 |
| 63 | 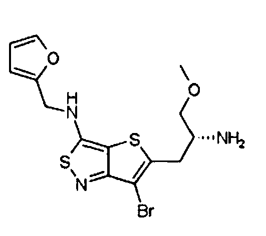 |
| 64 | 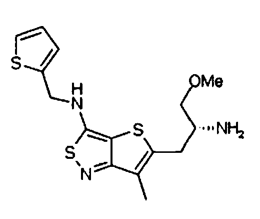 |
| 65 | 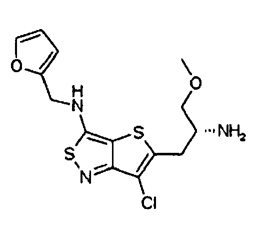 |
| 66 | 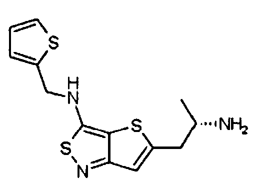 |
| 67 | 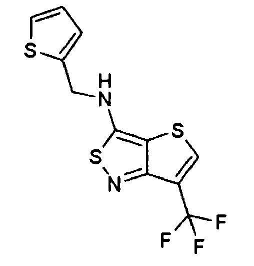 |
| 68 | 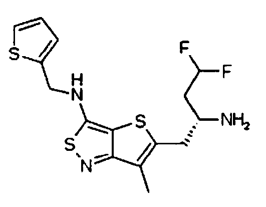 |
| 69 | 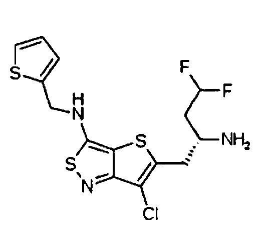 |
| 70 | 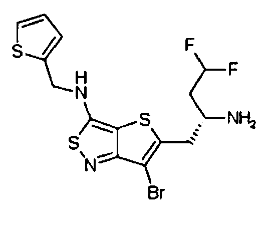 |
| 71 | 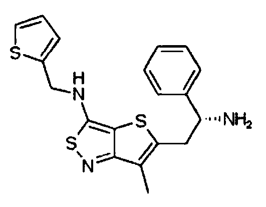 |
| 72 | 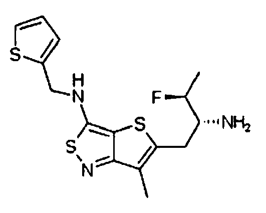 |
| 73 | 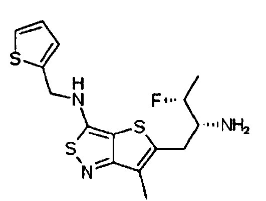 |
| 74 | 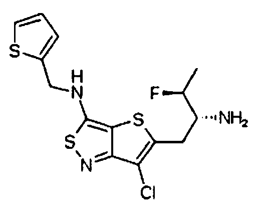 |
| 75 | 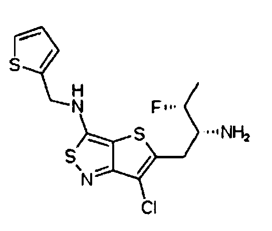 |
| 76 | 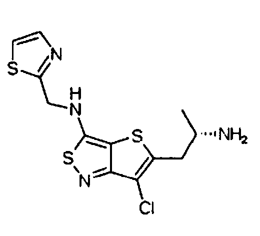 |
| 79 | 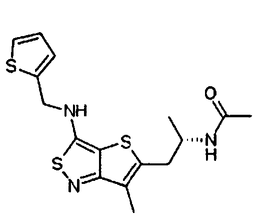 |
| 80 | 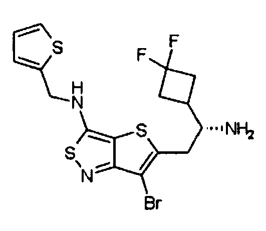 |
| 81 | 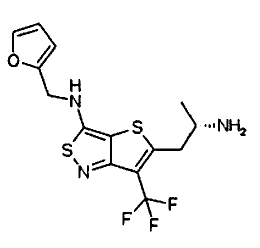 |
| 82 | 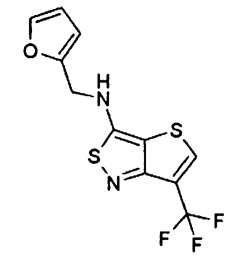 |
| 83 | 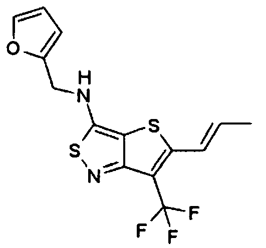 |
| 84 | 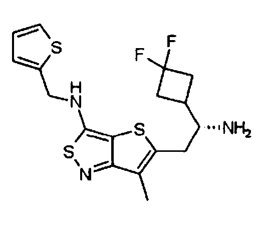 |
| 85 | 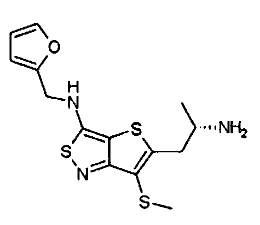 |
| 86 | 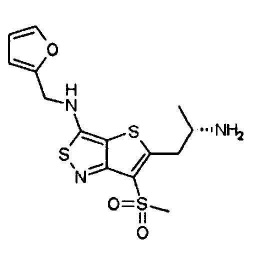 |
| 87 | 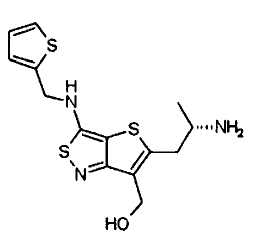 |
| 88 | 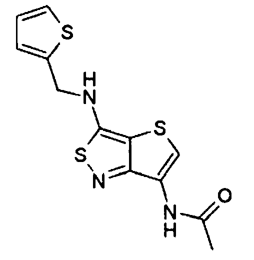 |
| 89 | 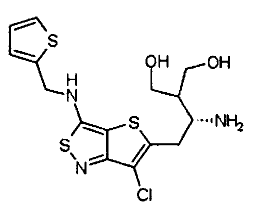 |
| 90 | 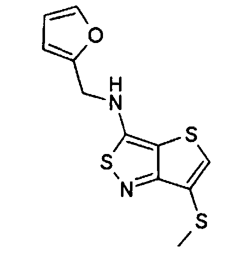 |
| 91 | 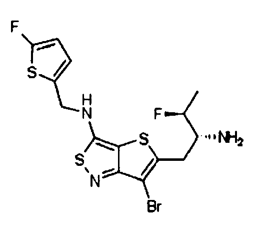 |
| 92 | 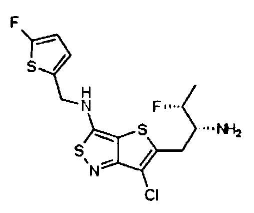 |
| 93 | 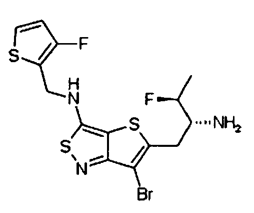 |
| 94 | 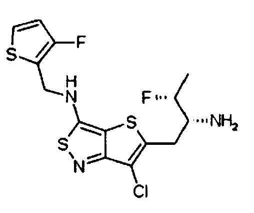 |
| 95 | 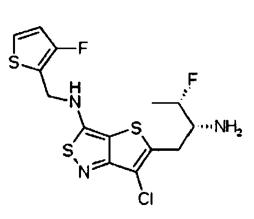 |
| 96 | 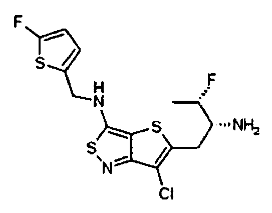 |
| 97 | 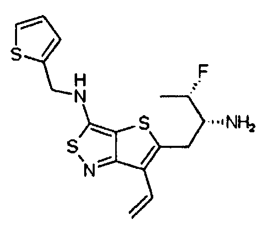 |
| 98 | 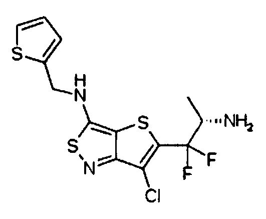 |
| 99 | 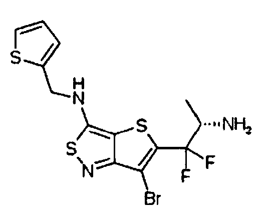 |
| 100 | 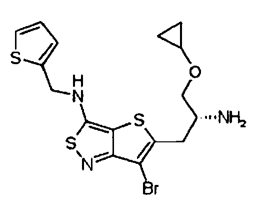 |
| 101 | 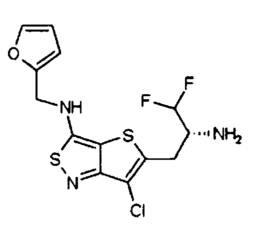 |
| 102 | 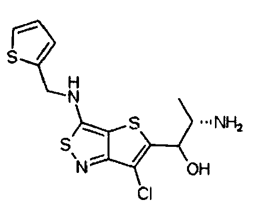 |
| 103 | 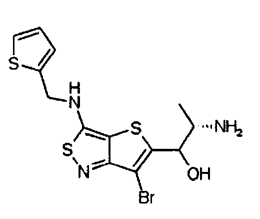 |
| 104 | 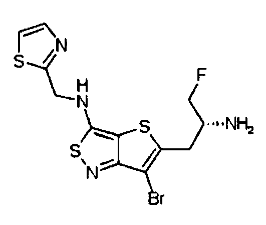 |
| 105 | 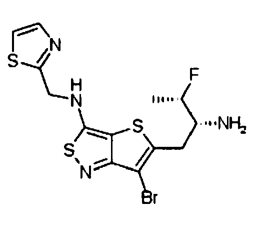 |
| 106 | 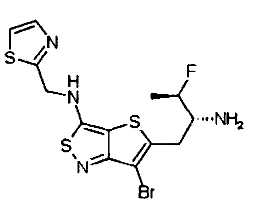 |
| 107 | 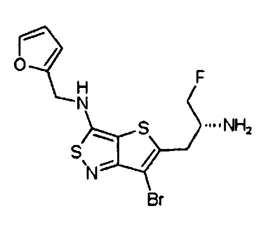 |
| 108 | 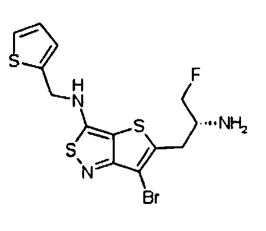 |
| 109 | 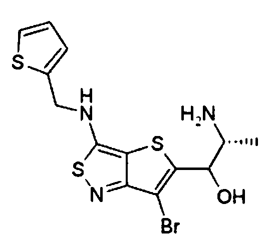 |
| 110 | 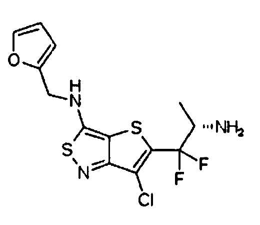 |
| 111 | 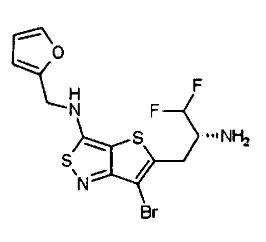 |
| 112 | 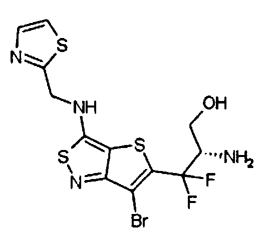 |
| 113 | 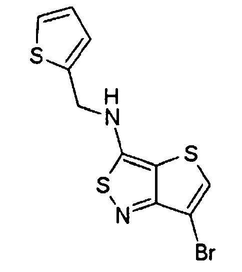 |
| 114 | 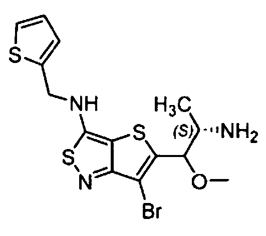 |
| 115 | 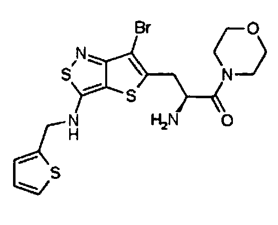 |
| 116 | 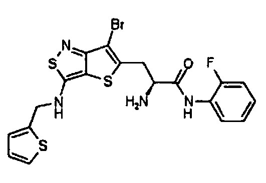 |
| 117 | 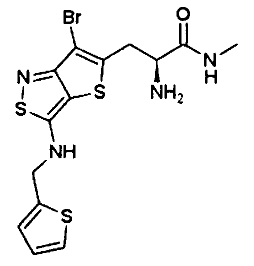 |
| 118 | 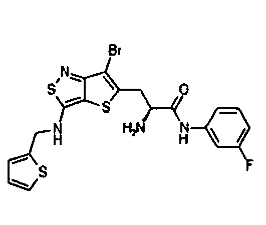 |
| 119 | 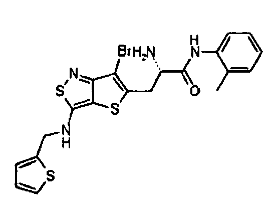 |
| 120 | 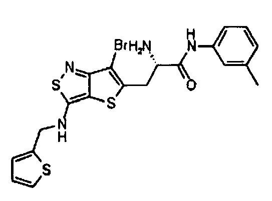 |
| 121 | 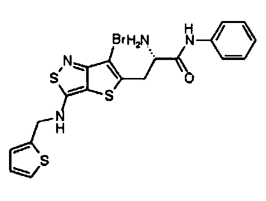 |
| 122 | 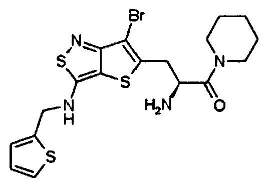 |
| 123 | 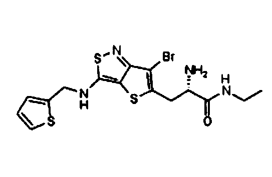 |
| 124 | 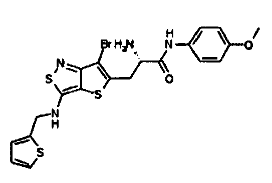 |
| 125 | 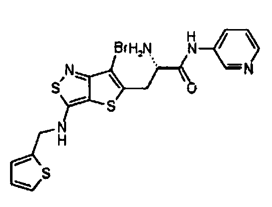 |
| 126 | 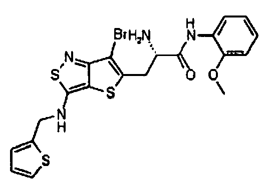 |
| 127 | 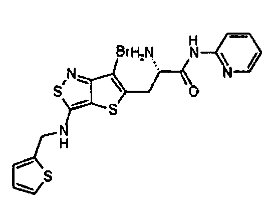 |
| 128 | 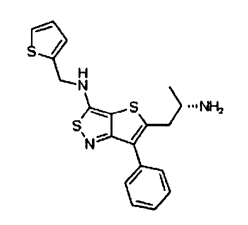 |
| 129 | 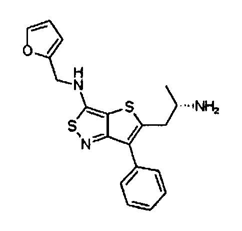 |

13. Pharmazeutische Zusammensetzung, die Folgendes umfasst:
eine Verbindung nach einem der vorangegangenen Ansprüche oder ein pharmazeutisch annehmbares Salz davon und
zumindest einen pharmazeutisch annehmbaren Hilfsstoff oder Träger.

## Revendications

1. Composé de formule (I) : 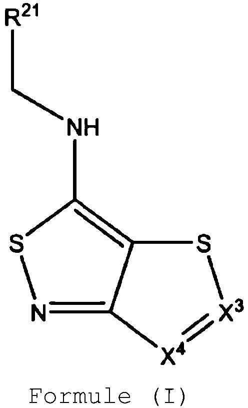
ou un sel pharmaceutiquement acceptable de celui-ci ;
dans lequel
X³ est choisi dans le groupe constitué par N et CR²³ ;
X⁴ est CR²⁴ ;
R²¹ est choisi dans le groupe constitué par un groupe phényle, un groupe hétéroaryle à 5 à 6 chaînons et un groupe hétérocycloalkyle à 5 à 6 chaînons, chacun est non substitué ou substitué par 1, 2, 3 ou 4 groupes R^{1A} indépendamment choisis ; chaque R^{1A} est choisi indépendamment parmi un atome d'halogène, CN, NO₂, un groupe alkyle en C₁₋₆, un groupe alcényle en C₂₋₆, un groupe alcynyle en C₂₋₆, un groupe halogénoalkyle en C₁₋₆, un groupe alcoxy en C₁₋₆, -C(=O)OH,-C(=O)alkyle en C₁₋₆, -C(=O)halogénoalkyle en C₁₋₆ et-C(=O)alcoxy en C₁₋₆ ;
R²³ est choisi dans le groupe constitué par un atome d'hydrogène, un groupe azido, un atome d'halogène, -CN, -NO₂, un groupe halogénoalkyle en C₁₋₆, un groupe alkyle en C₁₋₆, un groupe alcényle en C₂₋₆, un groupe alcynyle en C₂₋₆, un groupe hétéroalkyle en C₁₋₆, un groupe - (alkylène en C₁₋₆)-cycloalkyle en C₃₋₁₀, un groupe - (alkylène en C₁₋₆)-hétérocycloalkyle à 4 à 10 chaînons, un groupe - (hétéroalkylène en C₁₋₆)-cycloalkyle en C₃₋₁₀, un groupe - (hétéroalkylène en C₁₋₆)-hétérocycloalkyle à 4 à 10 chaînons, un groupe - (alkylène en C₁₋₆)-aryle en C₆₋₁₀, un groupe - (alkylène en C₁₋₆)-hétéroaryle à 5 à 10 chaînons, un groupe - (hétéroalkylène en C₁₋₆)-aryle en C₆₋₁₀, un groupe - (hétéroalkylène en C₁₋₆)-hétéroaryle à 5 à 10 chaînons, un groupe cycloalkyle en C₃₋₁₀, un groupe aryle en C₆₋₁₀, un groupe hétéroaryle à 5 à 10 chaînons, un groupe hétérocycloalkyle à 4 à 10 chaînons, -OR^{a3}, -SR^{a3}, -C(=O)R^{b3}, -C(=O)OR^{b3}, -NR^{c3}R^{d3}, -C(=O)NR^{c3}R^{d3}, -OC(=O)NR^{c3}R^{d3}, -NR^{c3}C(=O)R^{b3}, -NR^{c3}C(=O)OR^{b3},-NR^{c3}C(=O)NR^{c3}R^{d3}, -NR^{c3}S(=O)₂R^{b3}, -NR^{c3}S(=O)₂NR^{c3}R^{d3}, -S(O)NR^{c3}R^{d3} et -S(O)₂NR^{c3}R^{d3}, dans lequel les groupes alkyle en C₁₋₆, alkylène en C₁₋₆, hétéroalkylène en C₁₋₆, halogénoalkyle en C₁₋₆, alcényle en C₂₋₆, alcynyle en C₂₋₆, hétéroalkyle en C₁₋₆, cycloalkyle en C₃₋₁₀, aryle en C₆₋₁₀, hétéroaryle à 5 à 10 chaînons et hétérocycloalkyle à 4 à 10 chaînons sont non substitués ou substitués indépendamment par 1, 2, 3 ou 4 groupes R²⁰ ;
R²⁴ est choisi dans le groupe constitué par un atome d'hydrogène, un groupe azido, un atome d'halogène, -CN, -NO₂, un groupe halogénoalkyle en C₁₋₆, un groupe alkyle en C₁₋₆, un groupe alcényle en C₂₋₆, un groupe hétéroalkyle en C₁₋₆, un groupe cycloalkyle en C₃₋₁₀, un groupe aryle en C₆₋₁₀, -OR^{a4},-SR^{a4}, -S(=O)₂R^{a4}, -C(=O)R^{b4}, -C (=O) ORb4, -NR^{c4}R^{d4}, -C(=O)NR^{c4}R^{d4}, -OC(=O)NR^{c4}R^{d4}, -NR^{c4}C(=O)Rb⁴, -NR^{c4}C(=O)OR^{b4}, -NR^{c4}C(=O)NR^{c4}R^{d4}, -NR^{c4}S(=O)₂R^{b4}, -NR^{c4}S(=O)₂NR^{c4}R^{d4}, -S(O)NR^{c4}R^{d4} et -S(O)₂NR^{c4}R^{d4}, dans lequel les groupes alkyle en C₁₋₆, halogénoalkyle en C₁₋₆, alcényle en C₂₋₆, hétéroalkyle en C₁₋₆, cycloalkyle en C₃₋₁₀ et aryle en C₆₋₁₀ sont non substitués ou substitués indépendamment par 1, 2, 3 ou 4 groupes R^{20d} ;
chaque R^{a3}, R^{b3}, R^{c3}, R^{d3}, R^{a4}, R^{b4}, R^{c4} et R^{d4} est indépendamment choisi dans le groupe constitué par un atome d'hydrogène, un groupe alkyle en C₁₋₆, un groupe alcényle en C₂₋₆, un groupe alcynyle en C₂₋₆, un groupe hydroxyalkyle en C₁₋₆, un groupe halogénoalkyle en C₁₋₆, un groupe alcoxy en C₁₋₆, un groupe - (alkylène en C₁₋₆) -alcoxy en C₁₋₆, un groupe cycloalkyle en C₃₋₁₀, un groupe - (alkylène en C₁₋₆)-cycloalkyle en C₃₋₁₀, un groupe aryle en C₆₋₁₀, un groupe hétéroaryle à 5 à 10 chaînons, un groupe hétérocycloalkyle à 4 à 10 chaînons, dans lequel les groupes alkyle en C₁₋₆, alcényle en C₂₋₆, alcynyle en C₂₋₆, cycloalkyle en C₃₋₁₀, - (alkylène en C₁₋₆)-cycloalkyle en C₃₋₁₀, aryle en C₆₋₁₀, hétéroaryle à 5 à 10 chaînons et hétérocycloalkyle à 4 à 10 chaînons sont indépendamment non substitués ou substitués par 1, 2, 3 ou 4 groupes R²⁰ ;
ou R^{c3} et R^{d3} conjointement avec l'atome N auquel ils sont liés, se réunissent pour former un groupe hétéroaryle à 5 à 10 chaînons ou un groupe hétérocycloalkyle à 4 à 10 chaînons, dans lequel les groupes hétéroaryle à 5 à 10 chaînons et hétérocycloalkyle à 4 à 10 chaînons sont non substitués ou substitués indépendamment par 1, 2, 3 ou 4 groupes R²⁰ ;
ou R^{c4} et R^{d4} conjointement avec l'atome N auquel ils sont liés, se réunissent pour former un groupe hétéroaryle à 5 à 10 chaînons ou un groupe hétérocycloalkyle à 4 à 10 chaînons, dans lequel les groupes hétéroaryle à 5 à 10 chaînons et hétérocycloalkyle à 4 à 10 chaînons sont non substitués ou substitués indépendamment par 1, 2, 3 ou 4 groupes R²⁰ ;
chaque R²⁰ est indépendamment choisi dans le groupe constitué par -OH, -SH, -CN, -NO₂, un atome d'halogène, un groupe oxo, un groupe alkyle en C₁₋₄, un groupe alcényle en C₂₋₄, un groupe alcynyle en C₂₋₄, un groupe halogénoalkyle en C₁₋₄, un groupe cyanoalkyle en C₁₋₄, un groupe hydroxyalkyle en C₁₋₄, un groupe alcoxy en C₁₋₄, un groupe - (alkyle en C₁₋₄)-(alcoxy en C₁₋₄), un groupe -(alcoxy en C₁₋₄)-(alcoxy en C₁₋₄), un groupe halogénoalcoxy en C₁₋₄, un groupe cycloalkyle en C₃₋₆, un groupe phényle, un groupe hétéroaryle à 5 à 6 chaînons, un groupe hétérocycloalkyle à 4 à 6 chaînons, un groupe amino, un groupe alkyle en C₁₋₄-amino, un groupe di(alkyle en C₁₋₄)amino, un groupe carbamyle, un groupe alkyle en C₁₋₄-carbamyle, un groupe di (alkyle en C₁₋₄)carbamyle, un groupe carbamoyle, un groupe alkyle en C₁₋₄-carbamoyle, un groupe di (alkyle en C₁₋₄) carbamoyle, un groupe alkyle en C₁₋₄-carbonyle, un groupe alcoxy en C₁₋₄-carbonyle, un groupe alkyle en C₁₋₄-carbonylamino, un groupe alkyle en C₁₋₄-sulfonylamino, un groupe aminosulfonyle, un groupe alkyle en C₁₋₄-aminosulfonyle, un groupe di (alkyle en C₁₋₄)aminosulfonyle, un groupe aminosulfonylamino, un groupe alkyle en C₁₋₄-aminosulfonylamino, un groupe di(alkyle en C₁₋₄)aminosulfonylamino, un groupe aminocarbonylamino, un groupe alkyle en C₁₋₄-aminocarbonylamino et un groupe di(alkyle en C₁₋₄)aminocarbonylamino ; et
chaque R^{20d} est indépendamment choisi dans le groupe constitué par -OH, -SH, -CN, -NO₂, un atome d'halogène, un groupe oxo, un groupe alkyle en C₁₋₄, un groupe alcényle en C₂₋₄, un groupe halogénoalkyle en C₁₋₄, un groupe cyanoalkyle en C₁₋₄, un groupe hydroxyalkyle en C₁₋₄, un groupe alcoxy en C₁₋₄, un groupe hétéroalkyle en C₁₋₄, un groupe -(alkyle en C₁₋₄)-(alcoxy en C₁₋₄), un groupe -(alcoxy en C₁₋₄)-(alcoxy en C₁₋₄), un groupe halogénoalcoxy en C₁₋₄, un groupe cycloalkyle en C₃₋₆, un groupe phényle, un groupe amino, un groupe alkyle en C₁₋₄-amino, un groupe di(alkyle en C₁₋₄)amino, un groupe carbamyle, un groupe alkyle en C₁₋₄-carbamyle, un groupe di(alkyle en C₁₋₄)carbamyle, un groupe carbamoyle, un groupe alkyle en C₁₋₄-carbamoyle, un groupe di(alkyle en C₁₋₄)carbamoyle, un groupe alkyle en C₁₋₄-carbonyle, un groupe alcoxy en C₁₋₄-carbonyle, un groupe alkyle en C₁₋₄-carbonylamino, un groupe alkyle en C₁₋₄-sulfonylamino, un groupe aminosulfonyle, un groupe alkyle en C₁₋₄-aminosulfonyle, un groupe di(alkyle en C₁₋₄)aminosulfonyle, un groupe aminosulfonylamino, un groupe alkyle en C₁₋₄-aminosulfonylamino, un groupe di(alkyle en C₁₋₄)aminosulfonylamino, un groupe aminocarbonylamino, un groupe alkyle en C₁₋₄-aminocarbonylamino et un groupe di(alkyle en C₁₋₄)aminocarbonylamino.

2. Composé selon la revendication 1, ou un sel pharmaceutiquement acceptable de celui-ci, dans lequel :
R²¹ est un groupe aryle en C₆₋₁₀ ou un groupe hétéroaryle à 5 à 6 chaînons, dans lequel les groupes aryle en C₆₋₁₀ et hétéroaryle à 5 à 6 chaînons sont non substitués ou substitués indépendamment par 1, 2, 3 ou 4 groupes R^{1A}, dans lequel chaque R^{1A} est choisi indépendamment dans le groupe constitué par un atome d'halogène, -CN, -NO₂, -CF₃, -CHF₂, un groupe alkyle en C₁₋₆, un groupe alcényle en C₂₋₆, un groupe alcynyle en C₂₋₆, un groupe halogénoalkyle en C₁₋₆, un groupe alcoxy en C₁₋₆, -C(=O)OH, -C(=O)alkyle en C₁₋₆, -C(=O)halogénoalkyle en C₁₋₆ et -C(=O)alcoxy en C₁₋₆.

3. Composé selon la revendication 2, ou un sel pharmaceutiquement acceptable de celui-ci, dans lequel :
R²¹ est choisi dans le groupe constitué par

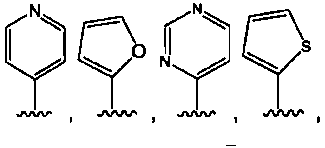

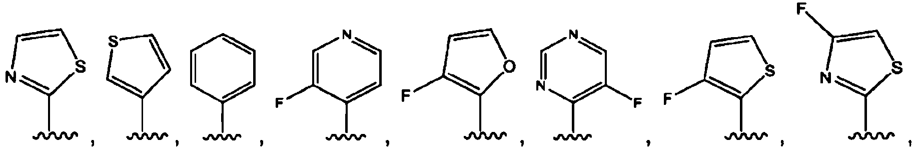

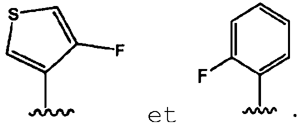

4. Composé selon l'une quelconque des revendications 1 à 3, ou un sel pharmaceutiquement acceptable de celui-ci, dans lequel :
X³ est CH.

5. Composé selon l'une quelconque des revendications 1 à 3, ou un sel pharmaceutiquement acceptable de celui-ci, dans lequel :
X³ est CR²³.

6. Composé selon la revendication 5, ou un sel pharmaceutiquement acceptable de celui-ci, dans lequel
R²³ est un groupe alkyle en C₁₋₆ ou un groupe hétéroalkyle en C₁₋₆, dans lequel les groupes alkyle en C₁₋₆ et hétéroalkyle en C₁₋₆ sont non substitués ou substitués indépendamment par 1, 2, 3 ou 4 groupes R²⁰.

7. Composé selon la revendication 6, ou un sel pharmaceutiquement acceptable de celui-ci, dans lequel :
X³ est CCH₂CH(NH₂)CH₃ ;
X³ est CCH₂CH(NH₂)CH₂OH ;
X³ est CCH₂CH(NH₂)CH₂CH₃ ;
X³ est CCH₂CH(NH₂)CH₂CH₂OH ;
X³ est CCH₂CH(NH₂)CH₂CH₂F ;
X³ est CCH₂CH(NH₂)CH₂CHF₂ ; ou
X³ est CCH₂CH(NH₂)CH₂CH(CH₃)₂.

8. Composé selon l'une quelconque des revendications 1 à 7, ou un sel pharmaceutiquement acceptable de celui-ci, dans lequel :
X⁴ est N ; ou
X⁴ est CH.

9. Composé selon l'une quelconque des revendications 1 à 7, ou un sel pharmaceutiquement acceptable de celui-ci, dans lequel :
X⁴ est CR²⁴.

10. Composé selon la revendication 9, dans lequel :
R²⁴ est choisi dans le groupe constitué par un atome d'halogène, -CN et un groupe alkyle en C₁₋₆, dans lequel le groupe alkyle en C₁₋₆ est non substitué ou substitué indépendamment par 1, 2, 3 ou 4 groupes R^{20d}.

11. Composé selon l'une quelconque des revendications 1 à 7, ou un sel pharmaceutiquement acceptable de celui-ci, dans lequel :
X⁴ est CCl ;
X⁴ est CBr ;
X⁴ est CF ;
X⁴ est CCN ;
X⁴ est CCH₃ ; ou
X⁴ est C(cyclopropyle).

12. Composé selon la revendication 1, ou un sel pharmaceutiquement acceptable de celui-ci, qui est un composé choisi parmi les composés, ou un sel pharmaceutiquement acceptable de ceux-ci :
| | |
|---|---|
| 1 | 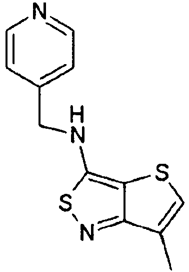 |
| 2 | 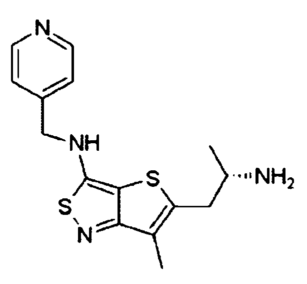 |
| 3 | 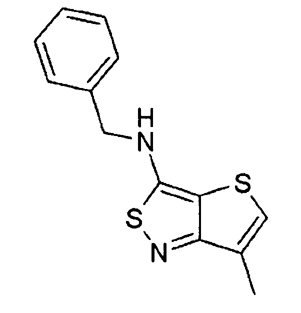 |
| 4 | 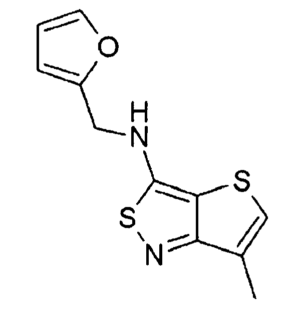 |
| 5 | 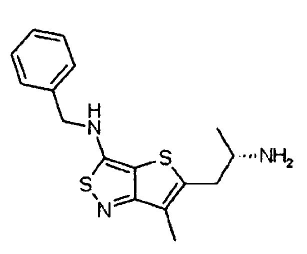 |
| 6 | 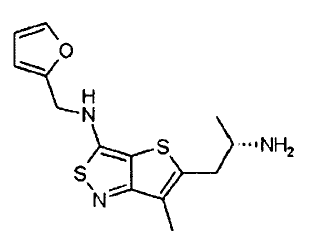 |
| 7 | 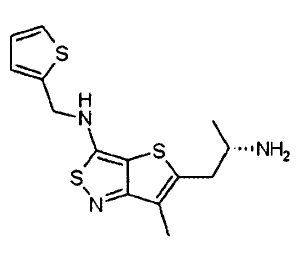 |
| 8 | 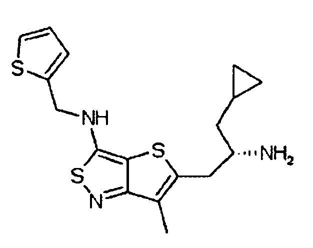 |
| 9 | 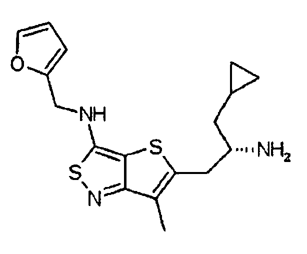 |
| 10 | 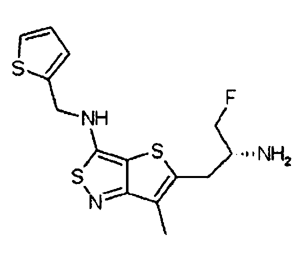 |
| 11 | 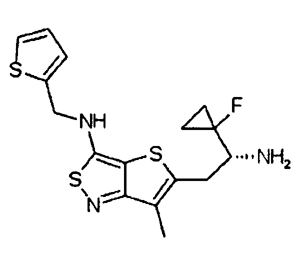 |
| 12 | 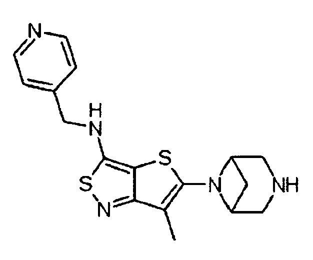 |
| 13 | 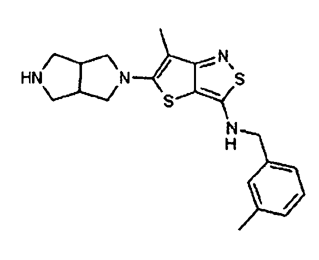 |
| 14 | 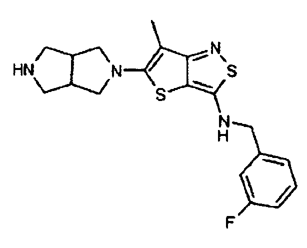 |
| 15 | 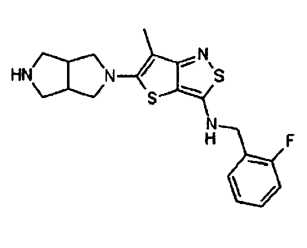 |
| 16 | 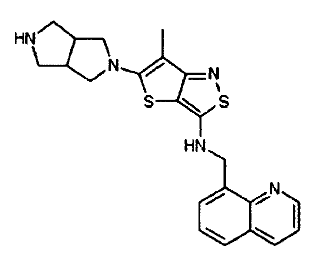 |
| 17 | 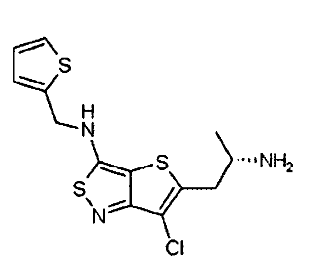 |
| 18 | 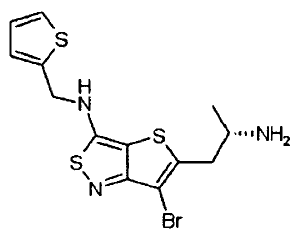 |
| 19 | 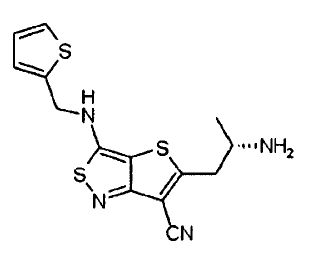 |
| 20 | 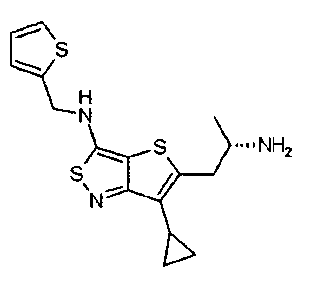 |
| 21 | 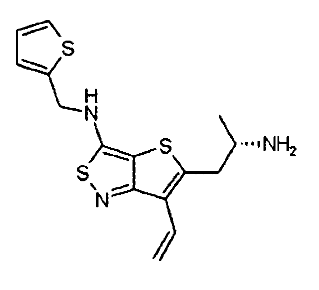 |
| 22 | 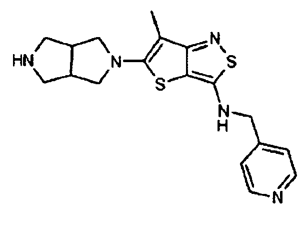 |
| 23 | 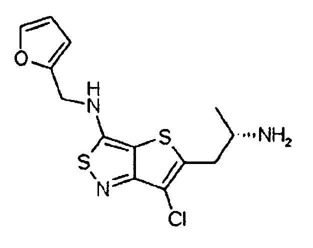 |
| 24 | 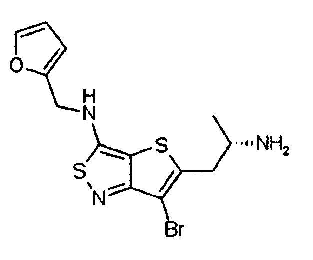 |
| 25 | 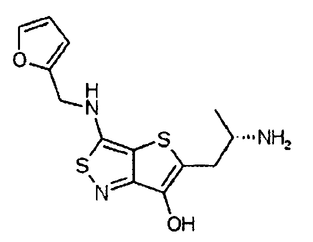 |
| 26 | 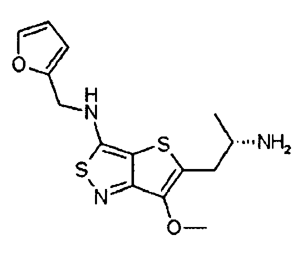 |
| 27 | 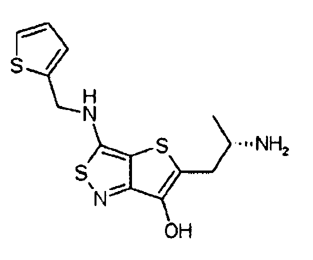 |
| 28 | 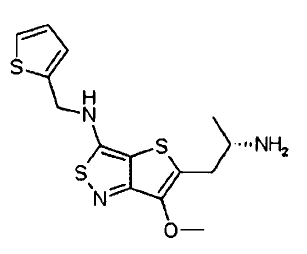 |
| 29 | 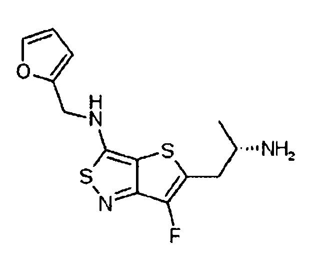 |
| 30 | 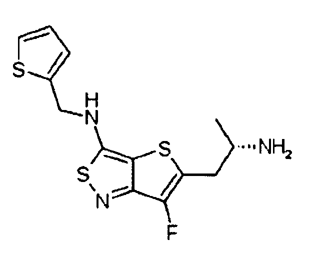 |
| 31 | 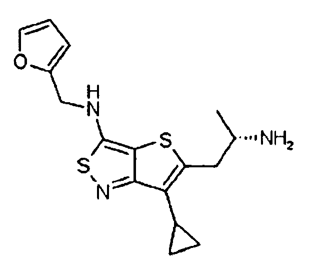 |
| 32 | 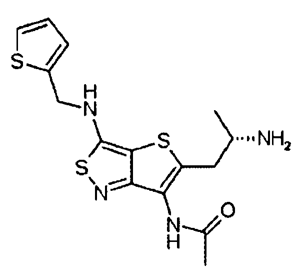 |
| 33 | 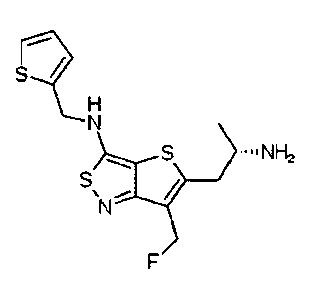 |
| 34 | 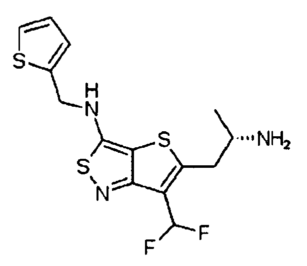 |
| 35 | 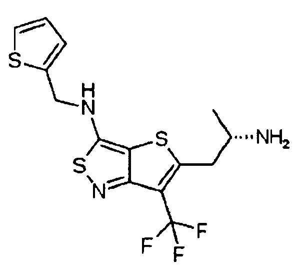 |
| 36 | 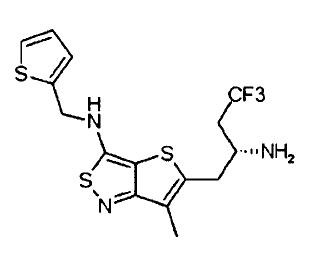 |
| 37 | 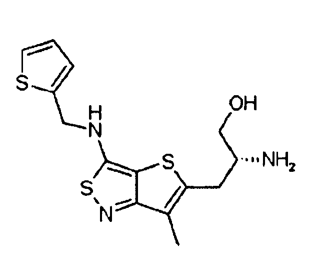 |
| 38 | 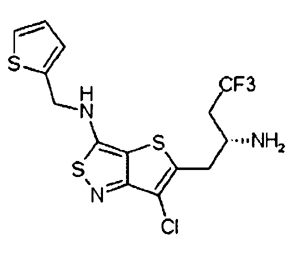 |
| 39 | 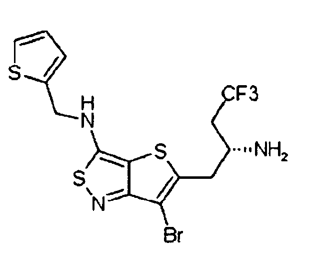 |
| 40 | 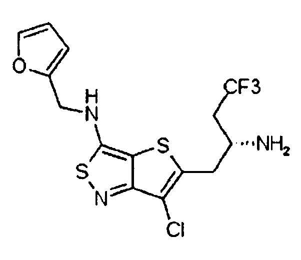 |
| 41 | 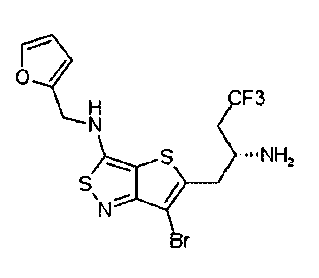 |
| 42 | 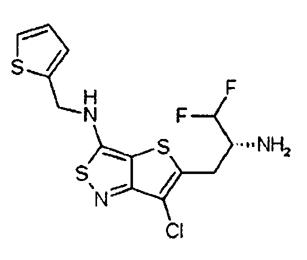 |
| 43 | 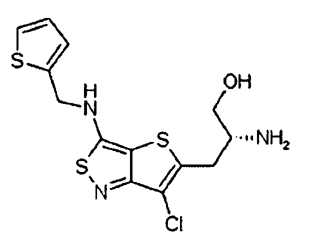 |
| 44 | 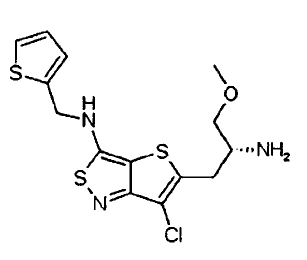 |
| 45 | 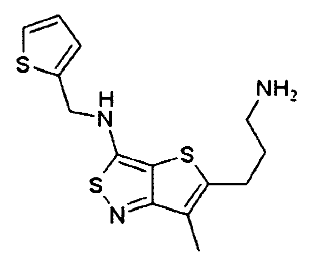 |
| 46 | 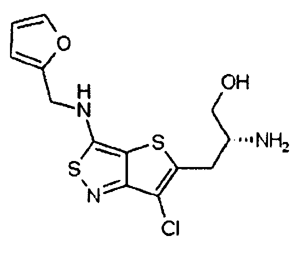 |
| 47 | 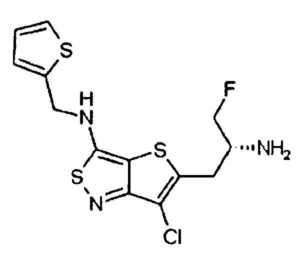 |
| 48 | 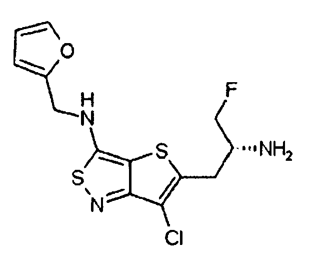 |
| 49 | 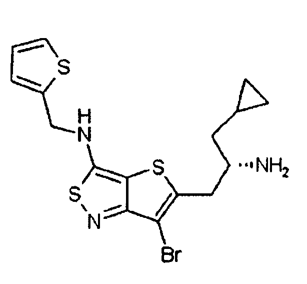 |
| 50 | 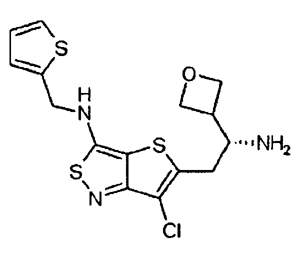 |
| 51 | 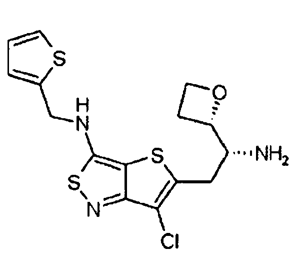 |
| 52 | 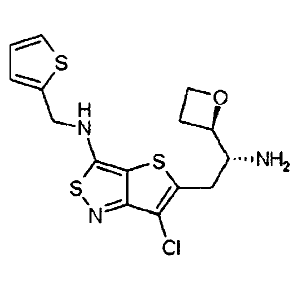 |
| 53 | 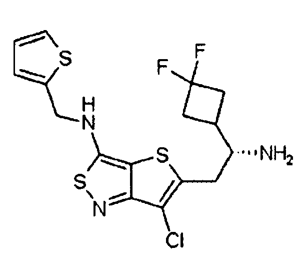 |
| 54 | 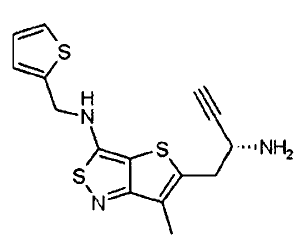 |
| 55 | 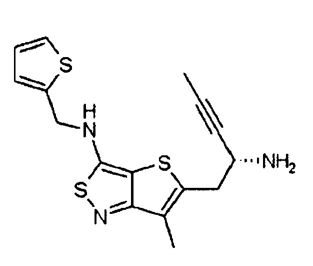 |
| 56 | 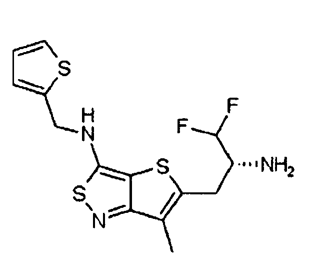 |
| 57 | 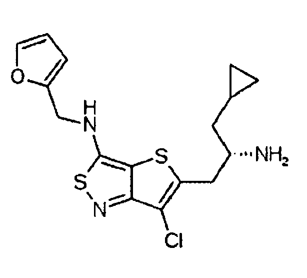 |
| 58 | 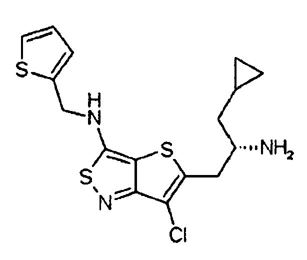 |
| 59 | 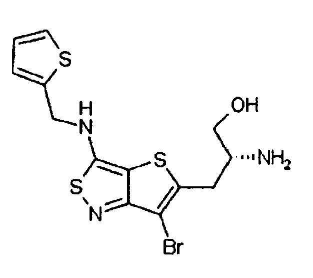 |
| 60 | 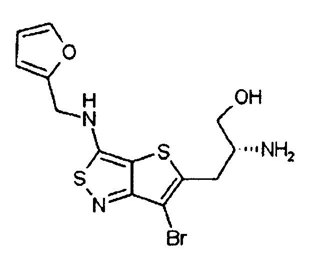 |
| 61 | 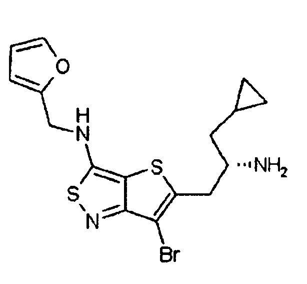 |
| 62 | 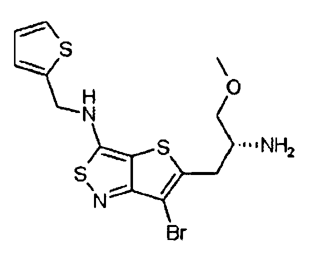 |
| 63 | 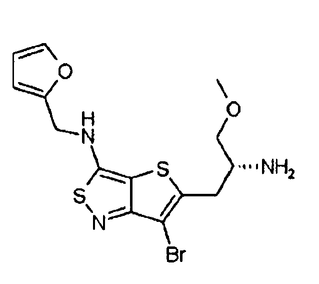 |
| 64 | 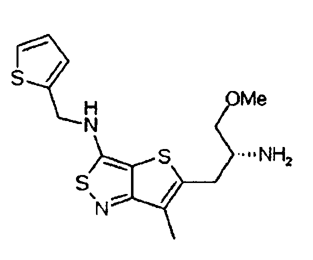 |
| 65 | 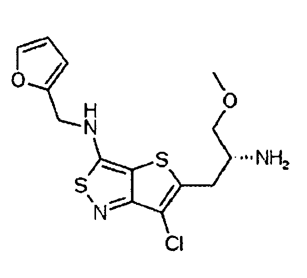 |
| 66 | 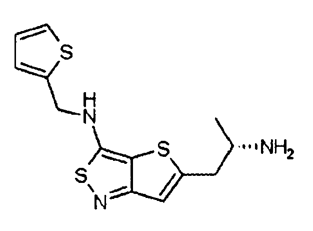 |
| 67 | 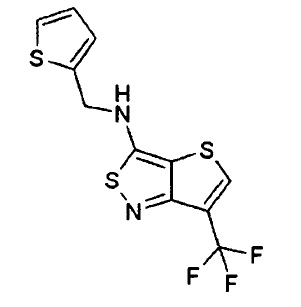 |
| 68 | 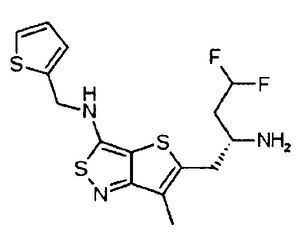 |
| 69 | 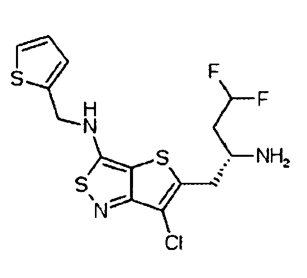 |
| 70 | 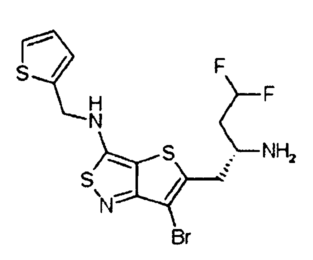 |
| 71 | 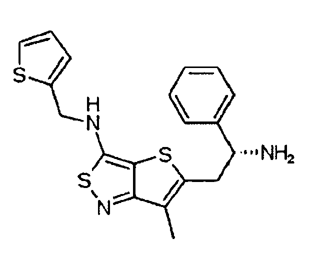 |
| 72 | 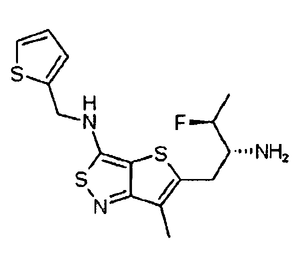 |
| 73 | 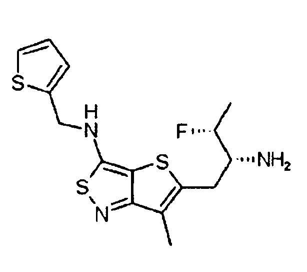 |
| 74 | 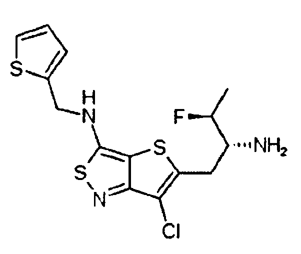 |
| 75 | 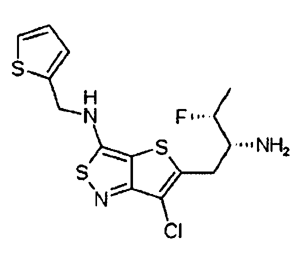 |
| 76 | 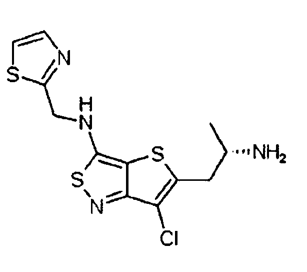 |
| 79 | 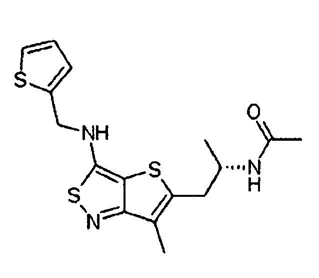 |
| 80 | 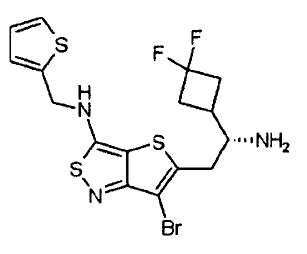 |
| 81 | 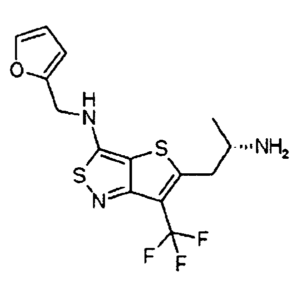 |
| 82 | 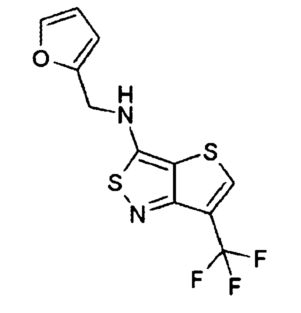 |
| 83 | 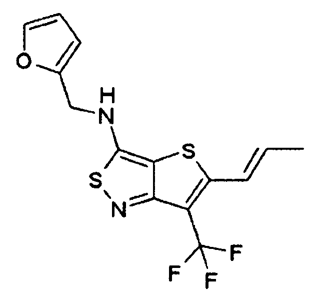 |
| 84 | 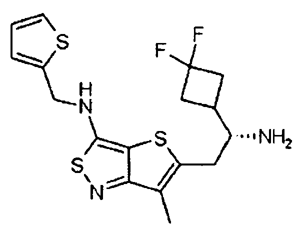 |
| 85 | 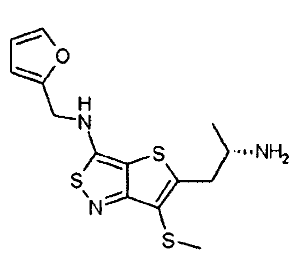 |
| 86 | 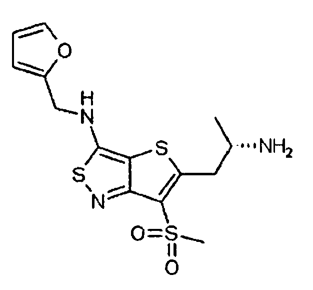 |
| 87 | 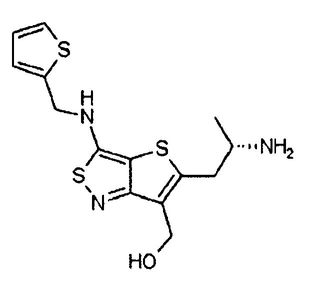 |
| 88 | 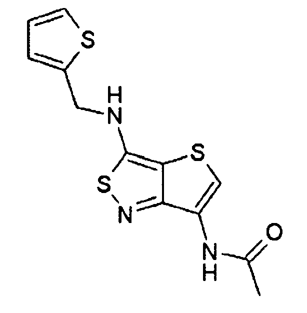 |
| 89 | 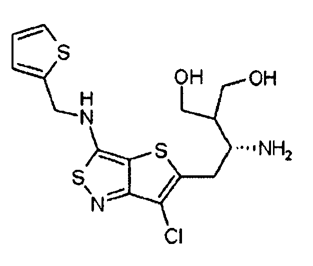 |
| 90 | 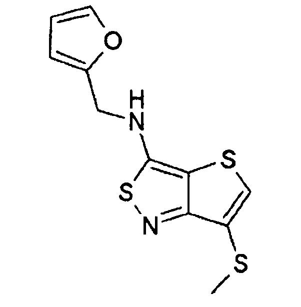 |
| 91 | 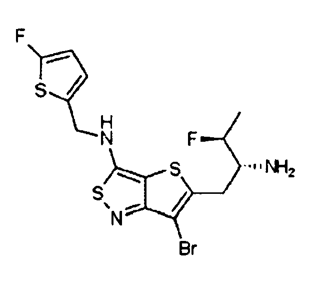 |
| 92 | 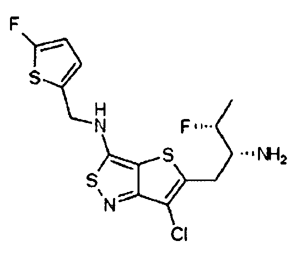 |
| 93 | 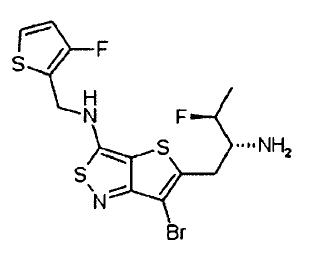 |
| 94 | 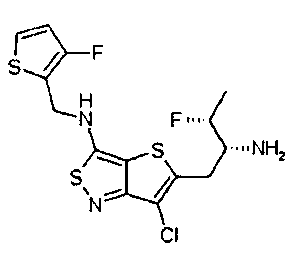 |
| 95 | 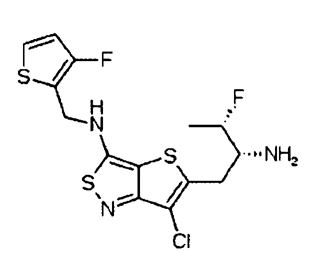 |
| 96 | 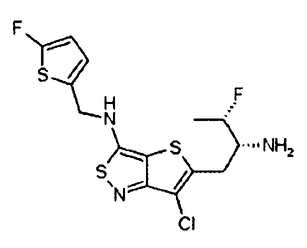 |
| 97 | 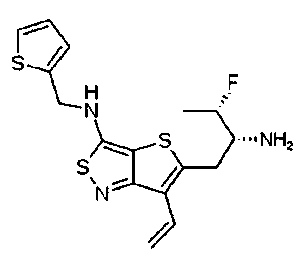 |
| 98 | 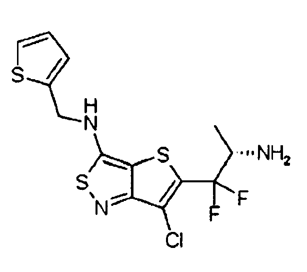 |
| 99 | 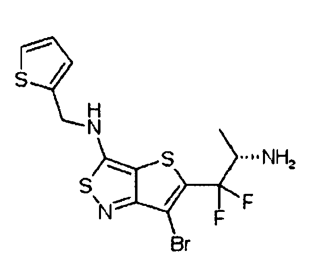 |
| 100 | 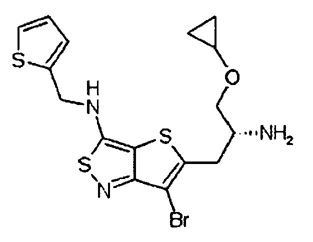 |
| 101 | 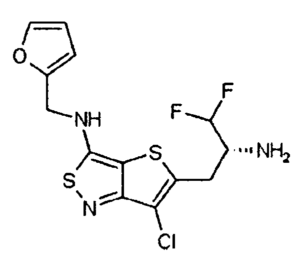 |
| 102 | 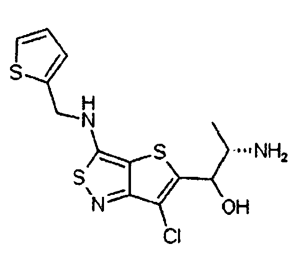 |
| 103 | 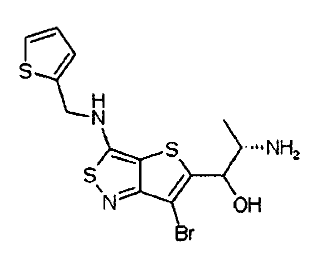 |
| 104 | 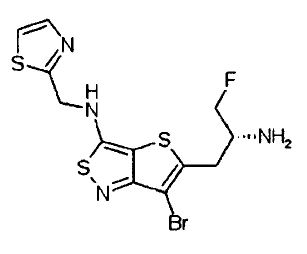 |
| 105 | 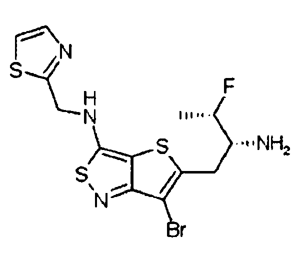 |
| 106 | 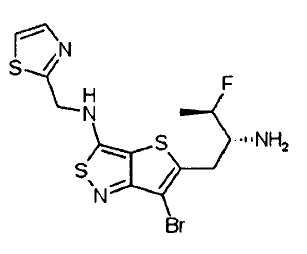 |
| 107 | 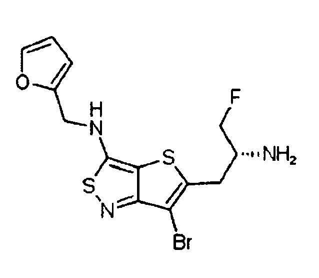 |
| 108 | 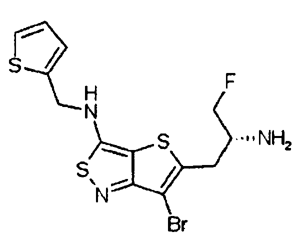 |
| 109 | 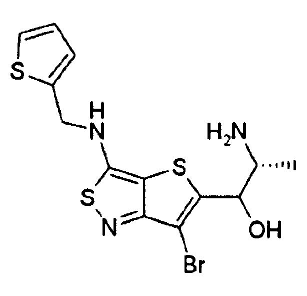 |
| 110 | 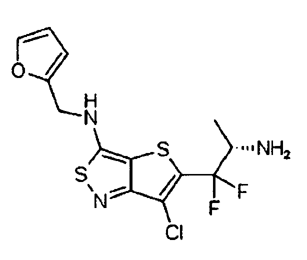 |
| 111 | 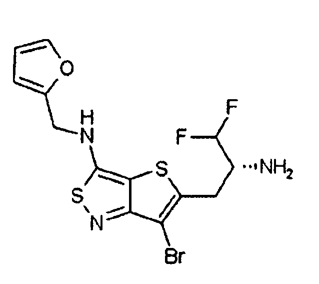 |
| 112 | 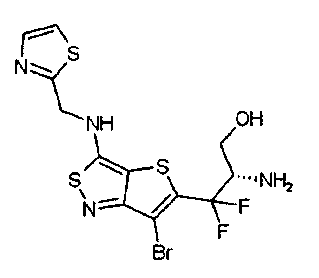 |
| 113 | 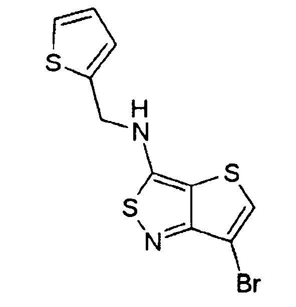 |
| 114 | 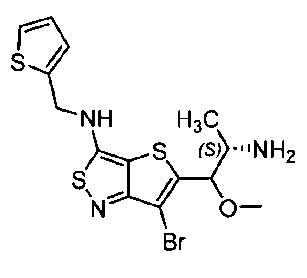 |
| 115 | 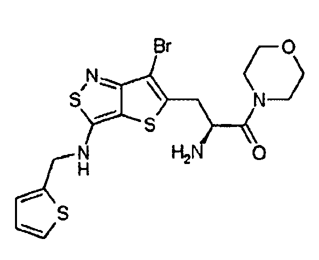 |
| 116 | 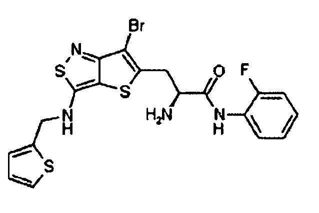 |
| 117 | 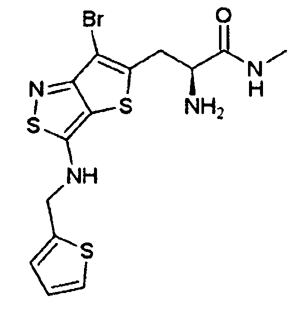 |
| 118 | 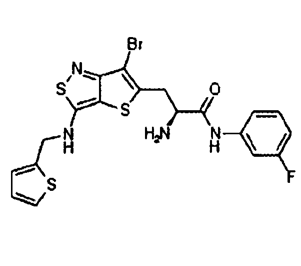 |
| 119 | 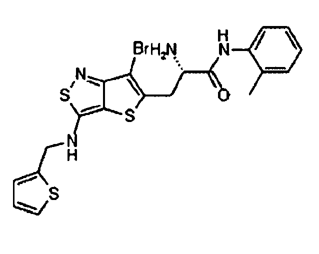 |
| 120 | 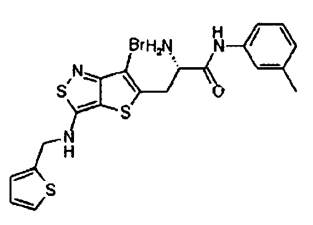 |
| 121 | 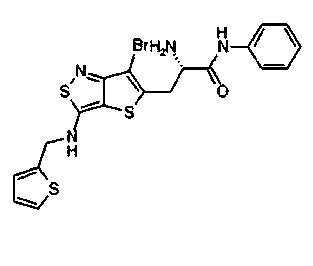 |
| 122 | 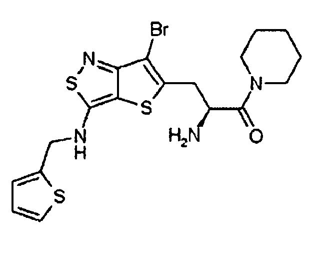 |
| 123 | 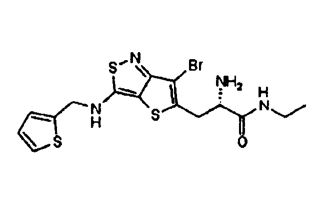 |
| 124 | 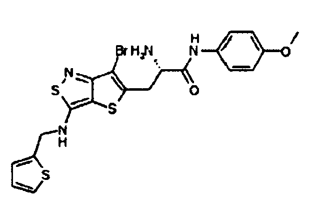 |
| 125 | 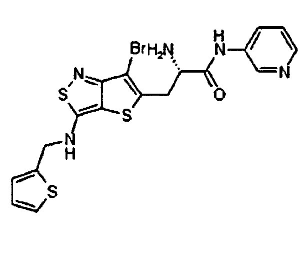 |
| 126 | 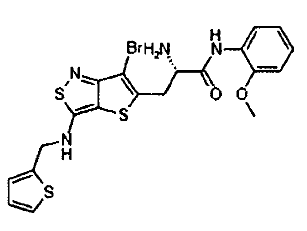 |
| 127 | 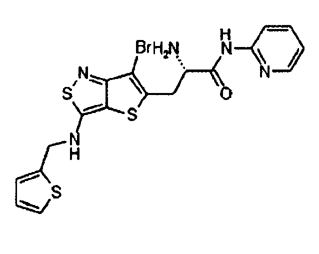 |
| 128 | 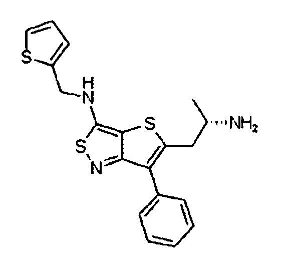 |
| 129 | 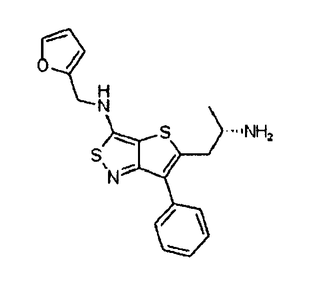 |

13. Composition pharmaceutique comprenant :
un composé selon l'une quelconque des revendications précédentes, ou un sel pharmaceutiquement acceptable de celui-ci, et
au moins un excipient ou véhicule pharmaceutiquement acceptable.
